# EUROPEAN PATENT APPLICATION

(11) **EP 2 019 116 A1**
(43) Date of publication of application: **28.01.2009**
(21) Application number: 07014680.8
(22) Date of filing: 26.07.2007
(51) Int. Cl.: C07K 14/195, C07K 14/71

(54) **Inhibitor of the met-receptor and its use**

(71) Applicant: Helmholtz-Zentrum für Infektionsforschung GmbH, 38124 Braunschweig (DE)
(72) Inventor: Heinz, Dirk, 38304, Wolfenbüttel (DE); Niemann, Hartmut, 38124, Braunschweig (DE)
(74) Representative: Kröncke, Rolf

(57) **Abstract**

The present invention relates to inhibitors of the Met-receptor, in particular, the present invention relates to inhibitors of the Met-receptor useful for preventing or treating cancer. In addition, the present invention relates to methods for screening and obtaining said inhibitors using a new binding site for said inhibitor with the Met-receptor. Moreover, the present invention is directed to the use of the new binding site identified with the Met-receptor for identifying new inhibitors useful in treating and preventing cancer, in particular metastasing cancer. Finally, the present invention relates to a crystalline form of said Met-receptor as well as its use in preventing or treating cancer.

SEQUENCE LISTING

<110>  Helmholtz Zentrum für Infektionsforschung
       Medical Research Council

<120>  Met inhibitor

<130>  3275014

<160>  5

<170>  PatentIn version 3.3

<210>  1
<211>  1390
<212>  PRT
<213>  human

<400>  1

<210>  2
<211>  630
<212>  PRT
<213>  Listeria monocytogenes

<400>  2

Met Lys Glu Lys His Asn Pro Arg Arg Lys Tyr Cys Leu Ile Ser Gly
1               5                   10                  15

Leu Ala Ile Ile Phe Ser Leu Trp Ile Ile Ile Gly Asn Gly Ala Lys
            20                  25                  30

Val Gln Ala Glu Thr Ile Thr Val Pro Thr Pro Ile Lys Gln Ile Phe
        35                  40                  45

Ser Asp Asp Ala Phe Ala Glu Thr Ile Lys Asp Asn Leu Lys Lys Lys
    50                  55                  60

Ser Val Thr Asp Ala Val Thr Gln Asn Glu Leu Asn Ser Ile Asp Gln
65                  70                  75                  80

Ile Ile Ala Asn Asn Ser Asp Ile Lys Ser Val Gln Gly Ile Gln Tyr
                85                  90                  95

Leu Pro Asn Val Thr Lys Leu Phe Leu Asn Gly Asn Lys Leu Thr Asp

            100                 105                 110

Ile Lys Pro Leu Ala Asn Leu Lys Asn Leu Gly Trp Leu Phe Leu Asp
        115                 120                 125

Glu Asn Lys Val Lys Asp Leu Ser Ser Leu Lys Asp Leu Lys Lys Leu
    130                 135                 140

Lys Ser Leu Ser Leu Glu His Asn Gly Ile Ser Asp Ile Asn Gly Leu
145                 150                 155                 160

Val His Leu Pro Gln Leu Glu Ser Leu Tyr Leu Gly Asn Asn Lys Ile
                165                 170                 175

Thr Asp Ile Thr Val Leu Ser Arg Leu Thr Lys Leu Asp Thr Leu Ser
            180                 185                 190

Leu Glu Asp Asn Gln Ile Ser Asp Ile Val Pro Leu Ala Gly Leu Thr
        195                 200                 205

Lys Leu Gln Asn Leu Tyr Leu Ser Lys Asn His Ile Ser Asp Leu Arg
    210                 215                 220

Ala Leu Ala Gly Leu Lys Asn Leu Asp Val Leu Glu Leu Phe Ser Gln
225                 230                 235                 240

Glu Cys Leu Asn Lys Pro Ile Asn His Gln Ser Asn Leu Val Val Pro
                245                 250                 255

Asn Thr Val Lys Asn Thr Asp Gly Ser Leu Val Thr Pro Glu Ile Ile
            260                 265                 270

Ser Asp Asp Gly Asp Tyr Glu Lys Pro Asn Val Lys Trp His Leu Pro
        275                 280                 285

Glu Phe Thr Asn Glu Val Ser Phe Ile Phe Tyr Gln Pro Val Thr Ile
    290                 295                 300

Gly Lys Ala Lys Ala Arg Phe His Gly Arg Val Thr Gln Pro Leu Lys
305                 310                 315                 320

Glu Val Tyr Thr Val Ser Tyr Asp Val Asp Gly Thr Val Ile Lys Thr
                325                 330                 335

Lys Val Glu Ala Gly Thr Arg Ile Thr Ala Pro Lys Pro Pro Thr Lys
            340                 345                 350

Gln Gly Tyr Val Phe Lys Gly Trp Tyr Thr Glu Lys Asn Gly Gly His
        355                 360                 365

Glu Trp Asn Phe Asn Thr Asp Tyr Met Ser Gly Asn Asp Phe Thr Leu

370                 375                 380

Tyr Ala Val Phe Lys Ala Glu Thr Thr Glu Lys Ala Val Asn Leu Thr
385                 390                 395                 400

Arg Tyr Val Lys Tyr Ile Arg Gly Asn Ala Gly Ile Tyr Lys Leu Pro
                405                 410                 415

Arg Glu Asp Asn Ser Leu Lys Gln Gly Thr Leu Ala Ser His Arg Cys
            420                 425                 430

Lys Ala Leu Thr Val Asp Arg Glu Ala Arg Asn Gly Gly Lys Leu Trp
        435                 440                 445

Tyr Arg Leu Lys Asn Ile Gly Trp Thr Lys Ala Glu Asn Leu Ser Leu
    450                 455                 460

Asp Arg Tyr Asp Lys Met Glu Tyr Asp Lys Gly Val Thr Ala Tyr Ala
465                 470                 475                 480

Arg Val Arg Asn Ala Ser Gly Asn Ser Val Trp Thr Lys Pro Tyr Asn
                485                 490                 495

Thr Ala Gly Ala Lys His Val Asn Lys Leu Ser Val Tyr Gln Gly Lys
            500                 505                 510

Asn Met Arg Ile Leu Arg Glu Ala Lys Thr Pro Ile Thr Thr Trp Tyr
        515                 520                 525

Gln Phe Ser Ile Gly Gly Lys Val Ile Gly Trp Val Asp Thr Arg Ala
    530                 535                 540

Leu Asn Thr Phe Tyr Lys Gln Ser Met Glu Lys Pro Thr Arg Leu Thr
545                 550                 555                 560

Arg Tyr Val Ser Ala Asn Lys Ala Gly Glu Ser Tyr Tyr Lys Val Pro
                565                 570                 575

Val Ala Asp Asn Pro Val Lys Arg Gly Thr Leu Ala Lys Tyr Lys Asn
            580                 585                 590

Gln Lys Leu Ile Val Asp Cys Gln Ala Thr Ile Glu Gly Gln Leu Trp
        595                 600                 605

Tyr Arg Ile Arg Thr Ser Ser Thr Phe Ile Gly Trp Thr Lys Ala Ala
    610                 615                 620

Asn Leu Arg Ala Gln Lys
625                 630

<210>  3

<211> 321
<212> PRT
<213> Listeria monocytogenes

<400> 3

Met Lys Glu Lys His Asn Pro Arg Arg Lys Tyr Cys Leu Ile Ser Gly
1               5                   10                  15

Leu Ala Ile Ile Phe Ser Leu Trp Ile Ile Ile Gly Asn Gly Ala Lys
            20                  25                  30

Val Gln Ala Glu Thr Ile Thr Val Pro Thr Pro Ile Lys Gln Ile Phe
        35                  40                  45

Ser Asp Asp Ala Phe Ala Glu Thr Ile Lys Asp Asn Leu Lys Lys Lys
    50                  55                  60

Ser Val Thr Asp Ala Val Thr Gln Asn Glu Leu Asn Ser Ile Asp Gln
65                  70                  75                  80

Ile Ile Ala Asn Asn Ser Asp Ile Lys Ser Val Gln Gly Ile Gln Tyr
                85                  90                  95

Leu Pro Asn Val Thr Lys Leu Phe Leu Asn Gly Asn Lys Leu Thr Asp
            100                 105                 110

Ile Lys Pro Leu Ala Asn Leu Lys Asn Leu Gly Trp Leu Phe Leu Asp
        115                 120                 125

Glu Asn Lys Val Lys Asp Leu Ser Ser Leu Lys Asp Leu Lys Lys Leu
    130                 135                 140

Lys Ser Leu Ser Leu Glu His Asn Gly Ile Ser Asp Ile Asn Gly Leu
145                 150                 155                 160

Val His Leu Pro Gln Leu Glu Ser Leu Tyr Leu Gly Asn Asn Lys Ile
                165                 170                 175

Thr Asp Ile Thr Val Leu Ser Arg Leu Thr Lys Leu Asp Thr Leu Ser
            180                 185                 190

Leu Glu Asp Asn Gln Ile Ser Asp Ile Val Pro Leu Ala Gly Leu Thr
        195                 200                 205

Lys Leu Gln Asn Leu Tyr Leu Ser Lys Asn His Ile Ser Asp Leu Arg
    210                 215                 220

Ala Leu Ala Gly Leu Lys Asn Leu Asp Val Leu Glu Leu Phe Ser Gln
225                 230                 235                 240

Glu Cys Leu Asn Lys Pro Ile Asn His Gln Ser Asn Leu Val Val Pro
                245                 250                 255

Asn Thr Val Lys Asn Thr Asp Gly Ser Leu Val Thr Pro Glu Ile Ile
            260                 265                 270

Ser Asp Asp Gly Asp Tyr Glu Lys Pro Asn Val Lys Trp His Leu Pro
        275                 280                 285

Glu Phe Thr Asn Glu Val Ser Phe Ile Phe Tyr Gln Pro Val Thr Ile
    290                 295                 300

Gly Lys Ala Lys Ala Arg Phe His Gly Arg Val Thr Gln Pro Leu Lys
305                 310                 315                 320

Glu

<210>  4
<211>  241
<212>  PRT
<213>  Listeria monocytogenes

<400>  4

Met Lys Glu Lys His Asn Pro Arg Arg Lys Tyr Cys Leu Ile Ser Gly
1               5                   10                  15

Leu Ala Ile Ile Phe Ser Leu Trp Ile Ile Ile Gly Asn Gly Ala Lys
            20                  25                  30

Val Gln Ala Glu Thr Ile Thr Val Pro Thr Pro Ile Lys Gln Ile Phe
        35                  40                  45

Ser Asp Asp Ala Phe Ala Glu Thr Ile Lys Asp Asn Leu Lys Lys Lys
    50                  55                  60

Ser Val Thr Asp Ala Val Thr Gln Asn Glu Leu Asn Ser Ile Asp Gln
65                  70                  75                  80

Ile Ile Ala Asn Asn Ser Asp Ile Lys Ser Val Gln Gly Ile Gln Tyr
                85                  90                  95

Leu Pro Asn Val Thr Lys Leu Phe Leu Asn Gly Asn Lys Leu Thr Asp
            100                 105                 110

Ile Lys Pro Leu Ala Asn Leu Lys Asn Leu Gly Trp Leu Phe Leu Asp
        115                 120                 125

Glu Asn Lys Val Lys Asp Leu Ser Ser Leu Lys Asp Leu Lys Lys Leu
    130                 135                 140

Lys Ser Leu Ser Leu Glu His Asn Gly Ile Ser Asp Ile Asn Gly Leu
145                 150                 155                 160

Val His Leu Pro Gln Leu Glu Ser Leu Tyr Leu Gly Asn Asn Lys Ile
                165                 170                 175

Thr Asp Ile Thr Val Leu Ser Arg Leu Thr Lys Leu Asp Thr Leu Ser
            180                 185                 190

Leu Glu Asp Asn Gln Ile Ser Asp Ile Val Pro Leu Ala Gly Leu Thr
        195                 200                 205

Lys Leu Gln Asn Leu Tyr Leu Ser Lys Asn His Ile Ser Asp Leu Arg
    210                 215                 220

Ala Leu Ala Gly Leu Lys Asn Leu Asp Val Leu Glu Leu Phe Ser Gln
225                 230                 235                 240

Glu

<210>  5
<211>  420
<212>  PRT
<213>  human

<400>  5

Thr Thr Ala Leu Gln Arg Val Asp Leu Phe Met Gly Gln Phe Ser Glu
1               5                   10                  15

Val Leu Leu Thr Ser Ile Ser Thr Phe Ile Lys Gly Asp Leu Thr Ile
            20                  25                  30

Ala Asn Leu Gly Thr Ser Glu Gly Arg Phe Met Gln Val Val Val Ser
        35                  40                  45

Arg Ser Gly Pro Ser Thr Pro His Val Asn Phe Leu Leu Asp Ser His
    50                  55                  60

Pro Val Ser Pro Glu Val Ile Val Glu His Thr Leu Asn Gln Asn Gly
65                  70                  75                  80

Tyr Thr Leu Val Ile Thr Gly Lys Lys Ile Thr Lys Ile Pro Leu Asn
                85                  90                  95

Gly Leu Gly Cys Arg His Phe Gln Ser Cys Ser Gln Cys Leu Ser Ala
            100                 105                 110

Pro Pro Phe Val Gln Cys Gly Trp Cys His Asp Lys Cys Val Arg Ser
        115                 120                 125

Glu Glu Cys Leu Ser Gly Thr Trp Thr Gln Gln Ile Cys Leu Pro Ala
    130                 135                 140

Ile Tyr Lys Val Phe Pro Asn Ser Ala Pro Leu Glu Gly Gly Thr Arg
145                 150                 155                 160

Leu Thr Ile Cys Gly Trp Asp Phe Gly Phe Arg Arg Asn Asn Lys Phe
                165                 170                 175

Asp Leu Lys Lys Thr Arg Val Leu Leu Gly Asn Glu Ser Cys Thr Leu
            180                 185                 190

Thr Leu Ser Glu Ser Thr Met Asn Thr Leu Lys Cys Thr Val Gly Pro
        195                 200                 205

Ala Met Asn Lys His Phe Asn Met Ser Ile Ile Ile Ser Asn Gly His
    210                 215                 220

Gly Thr Thr Gln Tyr Ser Thr Phe Ser Tyr Val Asp Pro Val Ile Thr
225                 230                 235                 240

Ser Ile Ser Pro Lys Tyr Gly Pro Met Ala Gly Gly Thr Leu Leu Thr
                245                 250                 255

Leu Thr Gly Asn Tyr Leu Asn Ser Gly Asn Ser Arg His Ile Ser Ile
            260                 265                 270

Gly Gly Lys Thr Cys Thr Leu Lys Ser Val Ser Asn Ser Ile Leu Glu
        275                 280                 285

Cys Tyr Thr Pro Ala Gln Thr Ile Ser Thr Glu Phe Ala Val Lys Leu
    290                 295                 300

Lys Ile Asp Leu Ala Asn Arg Glu Thr Ser Ile Phe Ser Tyr Arg Glu
305                 310                 315                 320

Asp Pro Ile Val Tyr Glu Ile His Pro Thr Lys Ser Phe Ile Ser Gly
                325                 330                 335

Gly Ser Thr Ile Thr Gly Val Gly Lys Asn Leu Asn Ser Val Ser Val
            340                 345                 350

Pro Arg Met Val Ile Asn Val His Glu Ala Gly Arg Asn Phe Thr Val
        355                 360                 365

Ala Cys Gln His Arg Ser Asn Ser Glu Ile Ile Cys Cys Thr Thr Pro
    370                 375                 380

Ser Leu Gln Gln Leu Asn Leu Gln Leu Pro Leu Lys Thr Lys Ala Phe
385                 390                 395                 400

Phe Met Leu Asp Gly Ile Leu Ser Lys Tyr Phe Asp Leu Ile Tyr Val
                405                 410                 415

His Asn Pro Val
            420

## Description

The present invention relates to inhibitors of the Met-receptor, in particular, the present invention leads to inhibitors of the Met-receptor useful for preventing or treating cancer. In addition, the present invention relates to methods for screening and obtaining said inhibitors using a new binding site for said inhibitor with the Met-receptor. Further, the present invention is directed to the use of the new binding site identified with the Met-receptor for identifying new inhibitors useful in treating and preventing cancer, in particular metastasing cancer. Finally, the present invention leads to a crystalline form of said Met-receptor as well as its use in preventing or treating cancer.

### Background art

Pathogenic bacteria have evolved elaborate ways to subvert host cell signalling pathways to their own benefit. The facultative intracellular Gram-positive pathogen *Listeria monocytogenes* has at its disposal two surface molecules of the internalin family that engage the extracellular region of human receptors to relay signals across the membrane into the host-cell cytoplasm. Receptor activation induces rearrangements of the actin cytoskeleton and ultimately causes bacterial invasion of normally non-phagocytic cells. The cytoplasm constitutes a protected niche where *L*. *monocytogenes* can replicate and spread to cause systemic disease.

The intestinal epithelium forms the first barrier that *L* .*monocytogenes* encounters following uptake with contaminated food. Binding of InIA, also called internalin, to E-cadherin exposed at the tip of intestinal villi initiates uptake of bacteria into epithelial cells, enabling them to breach this barrier. Colonization of deeper tissues requires the related protein InIB that mediates uptake into a number of cell types including hepatocytes and endothelial cells.

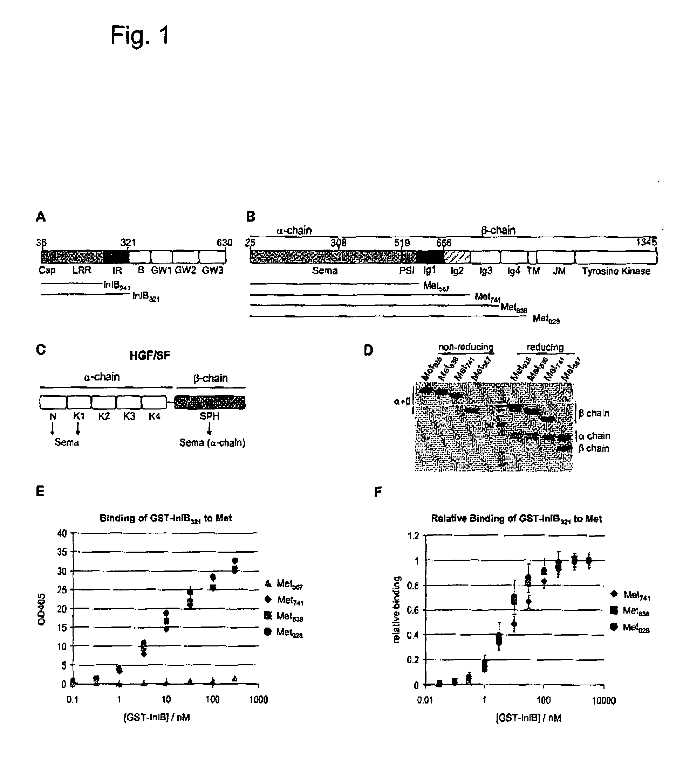

InIB is a bacterial surface protein of 630 amino acids. It shares with InIA the organization of the N-terminal intemalin domain comprising a Cap, a variable number of leucine rich repeats (LRRs) and a so called internepeat (IR) region (Schubert and Heinz, 2003) (Figure 1A). A poorly characterized B-repeat is followed by three C-terminal GW domains that non-covalently anchor InIB on the surface of *Listeria* through interaction with lipoteichoic acid (Braun et al., 1997). In addition to the bacteria-bound form, a substantial fraction of InIB is released into the medium as a soluble molecule and elicits a cellular response reminiscent of that caused by hepatocyte growth factor/scatter factor (HGF/SF) (Braun et al., supra). This observation contributed to the identification of the receptor tyrosine kinase Met as receptor for InIB (Shen et al., 2000). Normally, HGF/SF and Met mediate signals critical for cell survival and migration in embryogenesis and tissue regeneration but deregulation of Met also plays a major role in tumour invasion (Birchmeier et al., 2003). Two further receptors for InIB have been described, the complement receptor gC1qR (Braun et al., 2000) and heparan sulphate proteo glycans (HPSGs) (Jonquieres et al., 2001). While the exact function of gC1qR is still under debate, it is clear now that HSPGs significantly enhance InIB-induced Met signaling (Banerjee et al., 2004).

InIB is structurally well characterized and the interaction sites for all three receptors are known. A fragment comprising Cap and LRR (InIB₂₄₁; Figure 1A) is sufficient for Met binding (Shen et al., 2000, supra) and InIB₃₂₁, a fragment further comprising the IR region, is the minimal fragment capable of receptor activation (Banerjee et al., 2004, supra). The Met binding site maps to the concave face of the LRR region where several aromatic amino acids essential for interaction with Met are located (Machner et al., 2003). In addition to their interaction with lipoteichoic acid, the highly basic GW domains bind gC1qR and HSPGs, and binding of these receptors to the C-terminus of InIB is competitive (Jonquieres et al., supra).

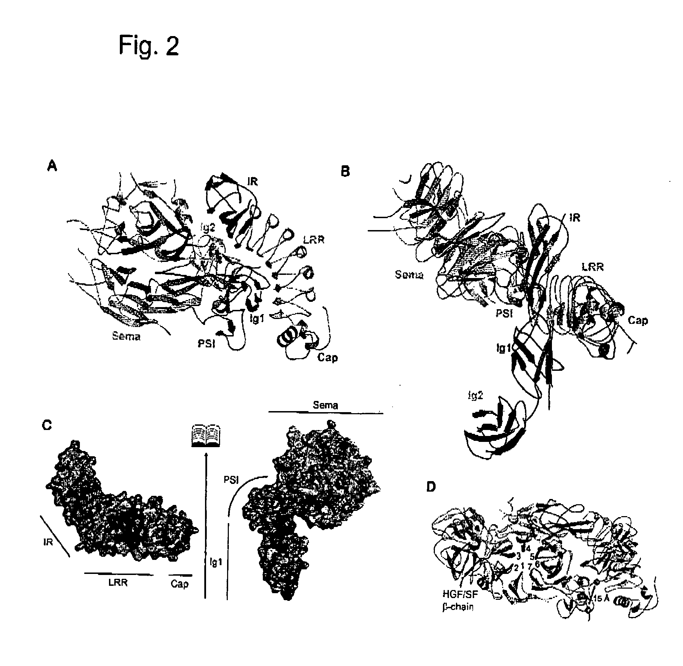

Less is known about the six domains of the Met ectodomain that comprises some 900 amino acids (Birchmeier et al., 2003, supra). Met is produced as a 1390 amino acid single-chain precursor that is cleaved by the cellular protease furin between residues 307 and 308 to yield a disulfide-linked two chain heterodimer (Figure 2B). The completely extracellular α-chain, together with amino acids 308-514 of the β-chain, forms the N-terminal semaphorin (Sema) domain. The β-chain further contains a small cystine-rich (PSI) and four immunoglobulin-like domains (Ig1 - Ig4), a transmembrane helix and the cytoplasmic juxtamembrane and tyrosine kinase domains. The Sema domain, a seven-bladed β-propeller, includes the binding site for the HGF/SF β-chain (Figure 1C) (Stamos et al., 2004). Two of the five domains within the HGF/SF α-chain bind to Sema as well (Holmes et al., 2007). The Met Ig domains do not bind HGF/SF and have been proposed to act as a stalk presenting the ligand binding Sema domain (Gherardi et al., 2003). The crystal structure of the Sema-PSI fragment in complex with the HGF/SF β-chain has been determined (Stamos et al., 2004, supra), and homology models are available for the Met Ig domains (Gherardi et al., 2003, supra). The InIB binding site on Met is so far unknown but it appears to be distinct from that for HGF/SF as no competition for binding to Met was observed (Shen et al., supra).

For example, W2005/108424 describes the crystal structure of the complex of HGF/SF β-chain with Met receptor and claims its use for the design, identification and selection of ligands that modulate the Met receptor and the interaction of HGF/SF with Met receptor. Therein, the crystal structure of the Met receptor is described whereby the Met receptor is composed of the Sema domain and the PSI domain. The involvement of the Met receptor in various complex pathways in a cell including cell division, breakdown of cell-cell contact and promotion of cell motility (jointly known as scattering), inhibition of apoptosis and tubule formation is known, see above. Further, the Met receptor is essential during vertebrate development and contributes to organ development by promoting epithelial to mesenchymal transition (EMT). In EMT, cells leave their tissue and migrate through the body, a process known as invasive growth.

Cancer metastasis resembles EMT and actually both HGF/SF and Met are involved in cancer progression of many tumors. Abnormal Met activation can be due to activating mutations in the cytoplasmic tyrosine kinase (TK) or juxtamembrane domains, but mutations are also found in the HGF/SF binding extracellular Sema domain. Up-regulation of both the ligand HGF/SF and the Met receptor are also found in many cancers. Up-regulation of HGF/SF contributes to metastasis of cell types that express both HGF/SF and Met (autocrine activation), whereas up-regulation of Met is often found in cell types that do not express HGF/SF themselves but are activated in a paracrine manner.

Thus, inhibition of abnormal Met signalling may block EMT like processes of cancer metastases and contribute to effective cancer therapy. Hence, Met receptor is an attractive target for cancer treatment and inhibitors which inhibit Met signalling are of interest in the medicinal field.

Thus, the problem solved by the present invention is to provide inhibitors allowing prevention or treatment of cancer, in particular allowing to inhibit scattering of cells, in particular of cancer cells during cancer metastasis.

Another problem to be solved by the present invention is the provision of methods allowing screening and obtaining of Met receptor inhibitors.

Yet another problem to be solved by the present invention is providing methods for designing and synthesising inhibitors by molecular modelling.

### Brief description of the invention

The present invention relates to inhibitors which bind to the Met receptor at a new Inhibitor binding site, said binding site comprises at least one or all amino acid (aa) residues of the Met receptor corresponding to residues F590, R592, N593, D597, K599, K600, R602, L604, E639, S641, G643, H644, G645, and T646 of Seq-ID No.1.

In a preferred embodiment, said Inhibitor binding site does not comprise at least one or all of amino acid residues corresponding to aa residues 124-128, 148, 167, 190-192, 218, 220-224, 229, 230, 286, 414 of the Met receptor of Seq-ID No. 1.

In another aspect, the present invention relates to inhibitors of the Met receptor being a polypeptide having a LRR domain preferably, said inhibitor of the Met receptor is derived from mammalian LRR peptides, in particular, human molecules containing LRR domains.

In a further aspect, the present application relates to inhibitors based on the InIB molecule of *Listeria monocytogenes,* in particular the InIB 321 or InIB 241 fragment, which may be altered by replacing at least parts of the amino acids by analogous parts of a different specificity. Said alteration is also known as "humanising" of said peptide in case of altering the InIB peptide to be administered to humans, thus, altering the molecule in a way to decrease any negative side effects of said molecules, like activating the immune system etc.

Another aspect of the present invention concerns the use of a fragment of the Met receptor comprising amino acids 590 to 604 and 639 to 646, but not containing amino acids 124-128, 148, 167, 190-192, 218, 220-224, 229, 230, 286, 414 of Seq-ID No. 1 as a screening tool for an agent for treating or preventing cancer. Moreover, another aspect of the present invention relates to a method for screening agents for preventing or treating cancer comprising using a three dimensional structure of the newly identified binding site of the Met receptor and employing said three dimensional structure to design or select potential inhibitors, preparing said inhibitor and assaying the inhibitor for having an inhibitory activity for the Met receptor.

Finally, the present invention concerns a crystalline form of the Met receptor molecule as well as a method for selecting, testing and/or regionally or semi-regionally designing a chemical compound which bind covalently or non-covalently to the Met- receptor using said crystalline form of the Met receptor.

### Brief description of the drawings

Figure 1 shows the mapping of the Met domains required for binding of InIB:
   (A) Domain organization of InIB. The intemalin domain (residues 36 to 321) co-crystallized with Met is shown in grayscale.
   (B) Domain organization of Met and constructs of the ectodomain used in this study.
   (C) Domain organization of HGF/SF. N: N-domain; K1 - K4: kringle1 - 4. SPH: serine protease homology domain. Experimentally proven interactions of individual HGF/SF domains with Met are indicated by arrows.
   (D) SDS-PAGE of purified Met constructs. Under non-reducing conditions (left) the proteins run as a single, disulfide-linked species. Under reducing conditions (right) they split into alpha- and β-chain. The alpha-chain doublet is due to heterogenous glycosylation.
   (E) InIB₃₂₁ does not bind immobilized Met₅₆₇ in a solid phase binding assay.
   (F) Immobilized Met₉₂₈, Met₈₃₈, and Met₇₄₁ bind soluble InIB₃₂₁ with identical apparent affinities.
Figure 2 shows the structure of the Met₇₄₁ - InIB₃₂₁ complex revealing two Interfaces that are both distinct from that for the HGF/SF β-chain.
   (A) Cartoon representation of the complex. Colouring as in Figure 1A and 1B.
   (B) Side view of the complex. Ig2 is not present in the final model, the position of which was determined by molecular replacement.
   (C) Open book view of the complex in surface representation (Ig2 is omitted). Residues in the interface were coloured according to the degree of burial upon complex formation. Light-gray: up to 30% reduction of accessible surface area (ASA), gray: 31-80% reduction of ASA. Dark gray: >80% reduction of ASA. Atoms forming intermolecular hydrogen bonds are shown in light gray. Interface analysis was carried out with the PISA server (Krissinel and Henrick, 2005).
   (D) The binding site of the HGF/SF β-chain (left side) is distinct from the InIB binding sites. Overlay of the Met - InIB complex with that of the HGF/SF β-chain in complex with Met₅₆₇ (Stamos et al., 2004). The overlay was performed on the Sema domain. The β-propeller is viewed from the bottom and the blades are numbered. The Sema domain of the Met - InIB complex is shown in on the bottom right for the Met alpha- and in right for the Met β-chain. Met from the HGF/SF - Met complex is on the left side of the Met molecule.. The different positions of two spheres representing the Calpha positions of the C-terminal cysteine in the PSI domain indicate the large rearrangement of this domain relative to Sema.

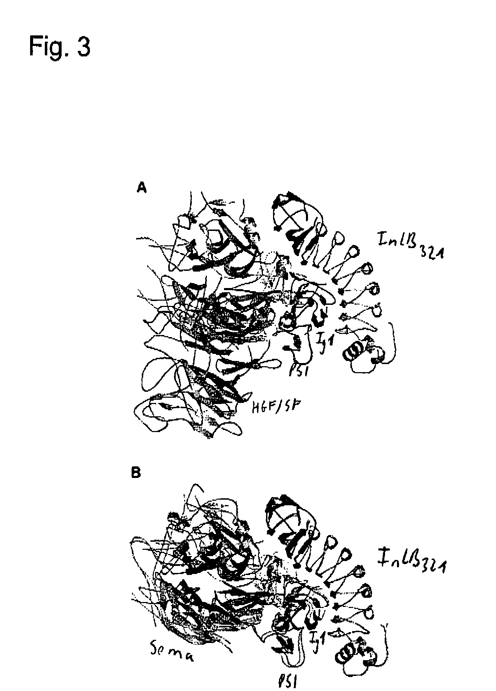

Figure 3 demonstrates the flexibility of the Free Met Ectodomain *versus* Rigidity of the Complex with InIB₃₂₁.
   (A) Overlay of the Met₇₄₁ - InIB₃₂₁ complex with Met₅₆₇ from the complex with the HGF/SF ββ-chain (bottom left). The structures were aligned on the PSI domain to visualize the large relative rearrangement of the Sema domain.
   (B) Overlay of the Met - InIB complex from crystal form I and from crystal form II. The structures were aligned on InIB. The Met Ig1 and PSI domains and the InIB-proximal side of the Sema domain align very well. The secondary interface between the InIB IR and the Met Sema domain is preserved.

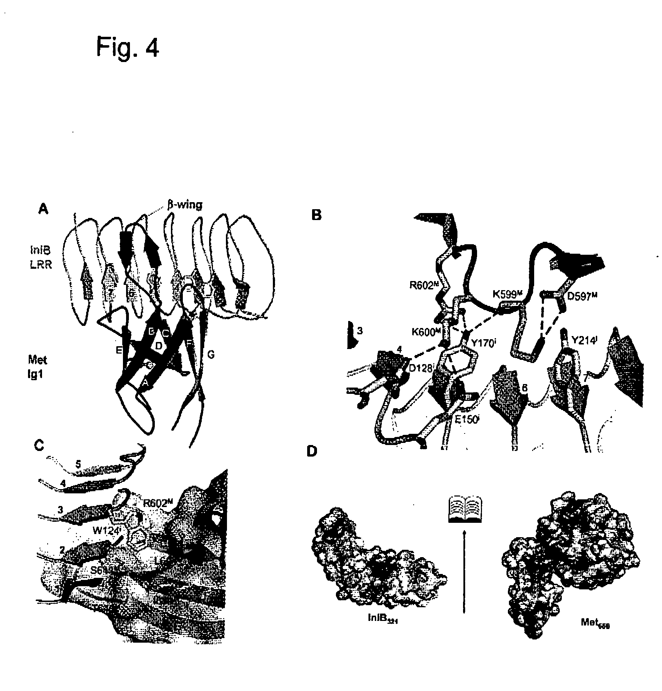

Figure 4 shows the Primary Interface Between InIB LRR and Met Ig1.
   (A) The InIB LRR embraces only the top of Met Ig1 with the unusual β-wing of the long B-C loop. The LRRs are numbered and the strands in Ig1 are labelled. Exposed aromatic side chains at the concave face of the InIB LRR and the disulfide bond connecting strands D and E of Met Ig1 are shown as sticks.
   (B) Close up showing InIB Y170ⁱ and Y214ⁱ interacting with K599^{M} and K600^{M} of Met. Y170ⁱ makes hydrogen bonds (dotted lines) to the carbonyl of K599^{M} and the R602^{M} side chain. The side-chains of K599^{M} and K600^{M} are held in place by an intra- and inter-molecular salt-bridge (dotted lines), respectively.
   (C) Side chains of residues from β-strands C, F, and G of the Met Ig1 domain form a hydrophobic pocket into which W124ⁱ from the concave face of the InIB LRR binds.
   (D) Electrostatic potential of InIB₃₂₁ (left) and Met (right; Ig2 omitted). In the open book view, the surfaces involved in binding are shown for both proteins. The negative charges on the InIB LRR face positive charges on Met Ig1.

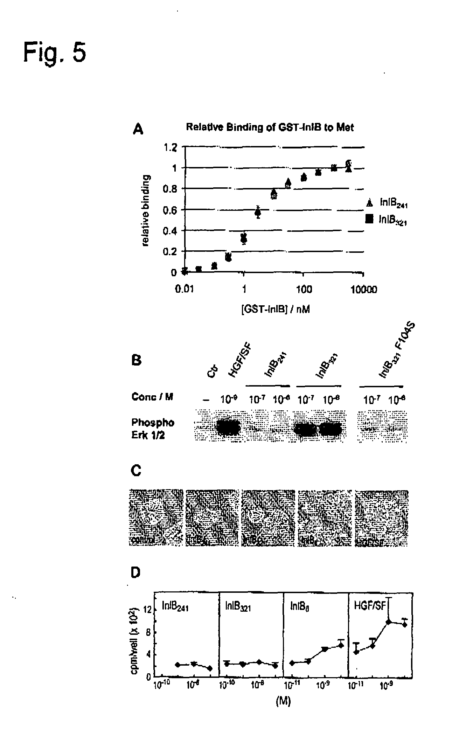

Figure 5 shows that the Secondary Interface is Required for Activation but not for Binding.
   (A) InIB₂₄₁ and InIB₃₂₁ bind to Met₉₂₈ with identical apparent affinity in a solid phase binding assay.
   (B) InIB₃₂₁, but not InIB₂₄₁ induces phosphorylation of ERK, a downstream target of Met. HGF/SF was used as positive and the binding deficient F104S mutant of InIB₃₂₁ (Machner et al., 2003) as negative control.
   (C) Like HGF/SF, InIB_{fl} induces scattering of MDCK colonies. The shorter InIB variants do not.
   (D) Like HGF/SF, InIB_{fl} induces DNA synthesis in MK keratinocytes but the shorter InIB variants are inactive.

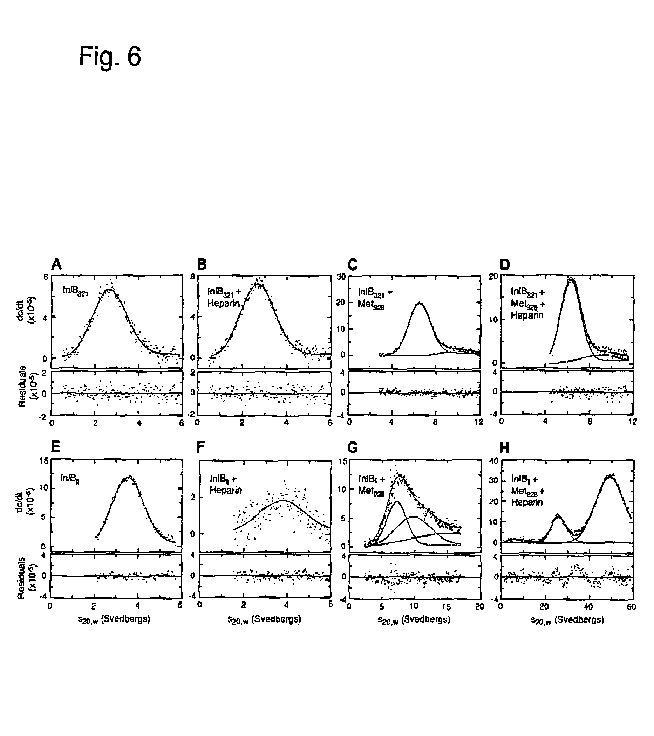

Figure 6 demonstrates that Heparin Induces Clustering of Met in Complex with InIB_{fl}, but not with InIB₃₂₁.
   Velocity sedimentation analysis with data expressed as a plot of dcldt *vs* _{s20,w}. Note the differences in x- and y-scale between different panels.
   (A) Velocity sedimentation analysis of InIB₃₂₁ (3 µM).
   (B) Velocity sedimentation analysis of InIB₃₂₁ (3 µM) with a two-fold excess of dp12 heparin.
   (C) Velocity sedimentation analysis of the Met₉₂₈ - InIB₃₂₁ complex (3 µM).
   (D) Velocity sedimentation analysis of the Met₉₂₈ - InIB₃₂₁ complex (3 µM) with a two-fold excess of dp12 heparin.
   (E) Velocity sedimentation analysis of InB_{fl} (3 µM).
   (F) Velocity sedimentation analysis of InB_{fl} (3 µM) with a two-fold excess of dp12 heparin.
   (G) Velocity sedimentation analysis of the Met₉₂₈ - InIB_{fl} complex (2 µM).
   (H) Velocity sedimentation analysis of the Met₉₂₈ - InIB_{fl} complex (2 µM) with a two-fold excess of dp12 heparin.

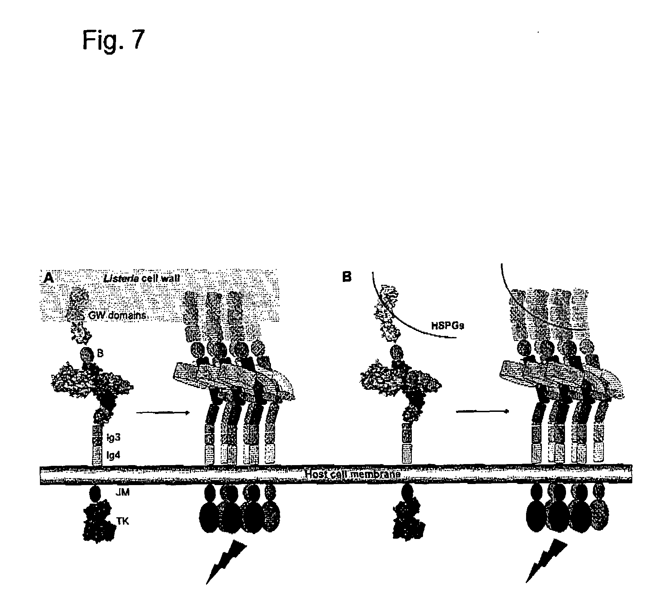

Figure 7 shows a model of InIB Mediated Met Activation by Receptor Clustering.
   (A) Model of Met activation by full-length InIB. All domains shown in surface representation are drawn to scale. InIB₃₂₁ is shown in light green (Cap), light blue (LRR) and dark blue (IR). The position of the GW domains was derived by aligning InIB_{fl} (Marino et al., 2002) with InIB₃₂₁ in the complex. InIB_{fl} bound to the bacterial surface could activate Met by clustering.
   (B) The GW domains of soluble InIB_{fl} induce clustering via interaction with heparan sulphate proteoglycans (HSPGs) present on the host cell.

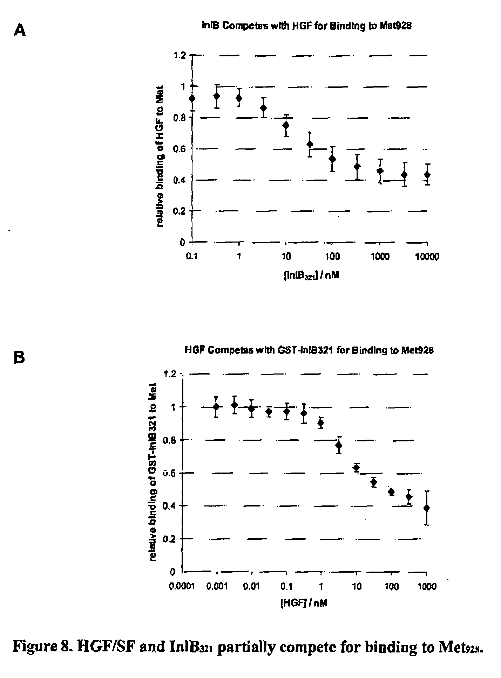

Figure 8 shows that HGF/SF and InIB₃₂₁ partially compete for binding to Met₉₂₈.
   **(A)** Met₉₂₈ was immobilized on NuncMaxiSorp plates. HGF/SF at 1 nM was mixed with a concentration series of InIB₃₂₁ as competitor. Binding of HGS/SF to Met₉₂₈ was detected by ABTS staining. **(B)** GST-InIB₃₂₁ at 3 nM was mixed with a concentration series of HGF/SF as competitor for binding to Met₉₂₈. Binding of InIB₃₂₁ to Met₉₂₈ was detected with an anti-GST-HRP conjugate and ABTS staining.

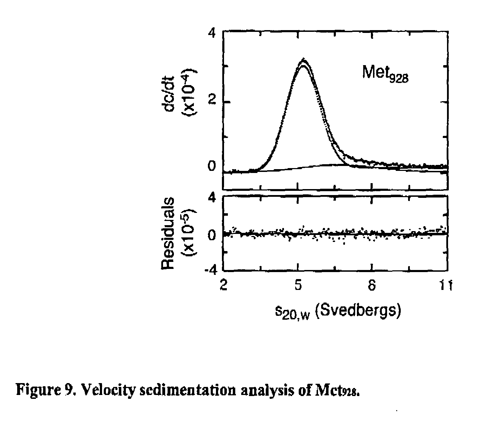

Figure 9 provides the velocity sedimentation analysis of Met928.

### Detailed description of the present invention

The present invention relates in a first aspect to an inhibitor of the Met receptor whereby said inhibitor is able to bind to a binding site for said inhibitor of the Met-receptor comprising at least one or all amino acid (aa) residues corresponding to residues F590, R592, N593, D597, K599, K600, R602, L604, E639, S641, G643, H644, G645, T464 of Seq-ID No. 1.

In a preferred embodiment, the inhibitor does not interact with a binding site of the Met-receptor comprising at least one or all of the amino acid residues corresponding to aa residues 124-128, 148, 167, 190-192, 218, 220-224, 229, 230, 286, 414 of the Met receptor of Seq-ID No.1.

The Met receptor is preferably the human Met receptor of Seq-ID No. 1. Of course, Met receptors from other species, in particular mammalian species, may be used.

### In this connection, the following definitions apply herein:

In the following, AA or aa is used as abbreviations for Amino acids; Amino acids are represented by single letter coder or three letter code.

The PSI domain is a small domain, which follows the Sema domain of Met, and spans about 50 residues and contains 4 disulfide bonds. "InIB derivative" as used herein refers to polypeptide that has a different sequence than a reference polypeptide. In some embodiments a derivative has at least 80% amino acid identity with the InIB derivatives amino acid sequence of Table Seq-ID No. 2 or Table or 3. The derivatives include those polypeptides that have substitutions, additions or deletions. The derivative also include those polypeptides that have a least one conservative amino acid substitutions, preferably all of the substitutions are conservative. In some embodiments, the InIB derivative has about 1-25 conservative amino acid substitutions, more preferably about 1-20 conservative amino acids substitutions, more preferably about 1-10 conservative amino acid substitutions, more preferably about 1-5 conservative amino acid substitutions, and more preferably about 1-2 conservative amino acid substitutions. The derivatives have the biological activity of binding to the Met receptor and attributing it. Ordinarily an InIB derivative polypeptide will have at least 80% sequence identity. More preferably will have at least 81% sequence identity, more preferably will have at least 82% sequence identity, more preferably will have at least 83% sequence identity, more preferably will have at least 84% sequence identity; more preferably will have at least 85% sequence identity, more preferably will have at least 86% sequence identity, more preferably will have at least 87% sequence identity, more preferably will have at least 88% sequence identity, more preferably will have at least 89% sequence identity, more preferably will have at least 90% sequence identity, more preferably will have at least 91% sequence identity, ore preferably will have at least 92% sequence identity, ore preferably will have at least 93% sequence identity, more preferably will have at least 94% sequence identity, more preferably will have at least 95% sequence identity, more preferably will have at least 96% sequence identity, more preferably will have at least 96% sequence identity, more preferably will have at least 98% sequence identity, more preferably will have at least 99% sequence identity with a InIB polypeptide having an amino acid sequence comprising Seq-ID No. 2 or Seq-ID No. 3.

The term "Met receptor" or "Met", as used herein, refers to any native or derivative (whether native or synthetic) Met polypeptide that is capable of binding to and/or being activated by HGF/SF. The term "wild-type Met receptor" generally refers to a polypeptide comprising an amino acid sequence found in a naturally occurring Met receptor and includes naturally occurring truncated or secreted forms, variant forms (e.g. alternatively spliced forms) and naturally occurring allelic variants. The Met Sema domain" comprises the N terminal amino acid residues 25 to 516 of a wild type Met receptor. A PSI domain follows the Sema domain and comprises 50 amino acid residues and has 4 disulfide bonds. Following the PSI domain are four IPT domains. IPT domains are related to immunoglobulin like domains. An embodiment of the Met receptor comprises an amino acid sequence of Seq-ID No. 1 including the Sema, PSI, Ig1, Ig2, Ig3, Ig4, transmembrane ( TM ), juxtamembrane (JM) and tyrosine kinase (TK) domains.

The term "Met receptor derivative", as used herein, refers to a polypeptide that has a different sequence than a reference polypeptide, wherein the reference polypeptide is the Met receptor that comprises an amino acid sequence SEQ ID NO 1 or sequence ID NO 5. Derivatives include those polypeptides that have substitutions, deletions, and/or deletions. Variants also include those polypeptides that have at least one conservative amino acid substitutions; preferably, all of the substitutions are conservative. In some embodiments, the Met receptor derivative has about 1-25 conservative amino acid substitutions, more preferably about 1-20 conservative amino acid substitutions, more preferably about 1-10 conservative amino acid substitions, more preferably about 1-5 conservative amino acid substitutions, and more preferably about 1-2 conservative amino acid substitutions. Ordinarily, a Met receptor derivative will have at least 80% sequence identity to a polypeptide having SEQ ID NO:1 In some embodiments, Met receptor polypeptide variants have at least 80% sequence identity, more preferably 81% sequence identity, more preferably 82% sequence identity, more preferably 83% sequence identity, more preferably 84% sequence identity, more preferably 85% sequence identity, more preferably 86% sequence identity, more preferably 87% sequence identity, more preferably 88% sequence identity. More preferably 89 sequence identity, more preferably 90% sequence identity, more preferably 91 sequence identity, more preferably 92% sequence identity, more preferably 93% sequence identity, more preferably 94% sequence identity, more preferably 95% sequence identity, more preferably 96% sequence identity, more preferably 97% sequence identity, more preferably 98% sequence identity, more preferably 99% sequence identity or greater, to a polypeptide having a sequence of Seq-ID No. 1 or Seq-ID No. 5.

The term "binding site", as used herein, refers to a region of a molecule or molecular complex that, as a result of its shape, distribution of electrostatic charge and/or distribution of non-polar regions favourably associates with a ligand. Thus, a binding site may include or consist of features such as cavities, surfaces, or interfaces between domains. Ligands that may associate with a binding site include, but are not limited to, cofactors, substrates, receptors, agonists, and antagonists. The term binding site includes a functional binding site and/or a structural binding site. A structural binding site includes "in contact" amino acid residues as determined from examination of a three-dimensional structure. Contact can be determined using Van der Waals radii of atoms or by proximity sufficient to exclude solvent, typically water, from the space between the ligand and the molecule or molecular complex. Some of the "in contact" amino acid residues may not cause any change in a biochemical assay, a cell-based assay, or an in vivo assay used to define a functional binding site but may contribute to the formation of a three dimensional structure. A functional binding site includes amino acid residues that are identified as binding site residues based upon loss or again of function, for example, loss of binding to ligand upon mutation of the residue. In some embodiments, the amino acid residues of a functional binding site are subset of the amino acid residues of the structural binding site.

The term "HGF/SF binding site" or "binding site for HGF/SF" includes all or a portion of a molecule or molecular complex whose shape is sufficiently similar to at least a portion of a binding site on Met as to be expected to bind HGF/SF or related structural analogs thereof. A structurally equivalent ligand binding site is defined by a root mean square deviation from the structure coordinates of the backbone atoms of the amino acids that make up binding sites for HGF/SF β on Met of at most about 0.7 Ǻ, preferably about 0.5Ǻ.

The term "Inhibitor binding site", "InIB241 binding site" or "binding site of the inhibitor" includes all, or a potion, of a molecule whose shape is sufficiently similar to the binding site of Met for InIB241. A structurally equivalent "Inhibitor binding site" is defined by root mean square deviation form the structure coordinates of the amino acids that make up the bindings sites in Met of at most about 0.70Ǻ. preferably about 0.5 Ǻ.

A binding site for the HGF/SF β-chain on the Met comprises, consists essentially of, or consists of at least one amino acid residue corresponding to a residue 124-128, 148, 167, 190-192, 218, 220 to 224, 227, 229, to 230, 286 or 414 or mixtures thereof of Seq-ID No 1. Numbering of amino acids is that of the native receptor. Whereas, the Met binding site for an inhibitor according to the present invention comprises aa residues corresponding to residues F590, R592, N593, D597, K599, K600, R602, L604, E639, S641, G643, H644, G645, and T646 of Seq-ID No: 1.

The term "corresponding" or "corresponds" refers to an amino acid residue or amino acid sequence that is found at the same positions or positions in a sequence aligned with a reference sequence. In some embodiments, the reference sequence is the Ig1 domain of the Met receptor comprising a sequence of SEQ ID NO: 5: It will be appreciated that when the amino acid position or sequence is aligned with the reference sequence the numbering of the amino acids may differ from that of the reference sequence or a different numbering system may be utilized.

"Structural homolog" of Met receptor as used herein refers to a protein that contains one or more amino acid substitutions, deletions, additions, or rearrangements with respect to the amino acid sequence of Met receptor, but that, when folded into its native conformation, exhibits or is reasonably expected to exhibit at least a portion of the tertiary (three-dimensional) structure of the Met receptor. In some embodiments, a portion of the three dimensional structure refers to structural domains of the Met receptor including the Sema domain, PSI domain, IPT domains, transmembrane domain and/or intracellular domain, and combinations thereof. For example, structurally homologous molecules of Met receptor include Met receptor derivatives, preferably derivatives with one or more conservative amino acid substitutions. In some embodiments, a Met receptor derivative has only conservative amino acid substitutions. Homolog tertiary structure can be probed, measured, or confirmed by known analytic or diagnostic methods, for example, X-ray, NMR, circular dichroism, a panel of monoclonal antibodies that recognize native Met receptor, and like techniques. For example, structurally homologous molecules can have substitutions, deletions or additions of one or more contiguous amino acids, such as loop or a domain. Structurally homologous molecules also include "modified" Met receptor molecules that have been chemically or enzymatically derivatized at one or more constituent amino acid, including side chain modifications, backbone modifications, and N- and C-terminal modifications including acetylation, hydroxylation, methylation, amidation, and the attachment of carbohydrate or lipid moieties, cofactors, and like modifications.

"X-ray diffraction pattern" means the pattern obtained from X-ray scattering of the periodic assembly of molecules or atoms in a crystal. X-ray crystallography is a technique that exploits the fact that X-rays are diffracted by crystals. X-rays have the proper wavelength (in the Ǻngström (Ǻ) range, approximately 10⁻⁸ cm) to be scattered by the electron cloud of an atom of comparable size. Based on the diffraction pattern obtained from X-ray scattering of the periodic assembly of molecules or atoms in the crystal, the electron density can be reconstructed. Additional phase information can be extracted either from diffraction data or from supplementing diffraction experiments to complete the reconstruction (the phase problem in crystallography). A model is then progressively built into the experimental electron density, refined against the data to produce an accurate molecular structure. X-ray structure coordinates define a unique configuration of points in space. Those of skill in the art understand that a set of structure coordinates for a protein of a protein/ligand complex, or a configuration in three dimensions. A similar or identical configuration can be defined by an entire set of coordinates, provided the distance and angles between coordinates remain essentially the same. In addition, a configuration of points can be defined by increasing or decreasing the distances between coordinates by a scalar factor, while keeping the angles essentially the same.

"Crystal structure" generally refers to the three-dimensional or lattice spacing arrangement of repeating atomic or molecular units in a crystalline material. The crystal structure of a crystalline material can be determined by X-ray crystallographic methods, see for example, "Principles of Protein X-ray Crystallography", by Jan Drenth, Springer Advanced Text in Chemistry, Springer Verlag; 2nd ed., February 1999, ISBN: 0387985875, and "Introduction to Macromolecular Crystallography", by Alexander McPherson, Wiley-Liss, October 18, 2002, ISBN: 0471251224.

The present disclosure also includes derivatives of the Met receptor and the peptide having a inhibitory activity on the Met receptor. Derivatives include those polypeptides that have amino acid substitutions, deletions, and additions. Amino acid substitutions can be made for example to replace cysteines and eliminate formation of disulfide bonds. Amino acid substitutions can also be made to change proteolytic cleavage sites. The derivatives also include those polypeptides that have at least one conservative amino acid substitution. In some embodiments a derivative only has conservative amino acid substitutions. In some embodiments, the Met receptor variant has about 1-25 conservative amino acid substitutions, more preferably about 1-20 conservative amino acids substitutions, more preferably about 1-10 conservative amino acid substitutions, more preferably about 1-5 conservative amino acid substitutions, and more preferably about 1-2 conservative amino acid substitutions. In some embodiments, said derivatives has at least 70%, like 80% such as 90% sequence identity to an extracellular domain fragment of the Met receptor, such as SEQ ID NO: 1 or 5 and the inhibitor of SEQ ID NO: 3 or 4, and has changes at amino acids other than those associated with the binding site for the inhibitor on Met, preferably the amino acid changes are only conservative substitutions. Other derivative can be made at the Met binding site for HGF/SF β.

Amino acid substitutions include one or more conservative amino acid substitutions. The term "conservative" amino acid substitution as used herein refers to an amino acid substitutions which substitutes a functionally equivalent amino acid. Conservative amino acid changes typically result in silent changes in secondary structure of the amino acid sequence of the resulting polypeptide. For example, one or more amino acids of a similar polarity act as functional equivalents and results in a silent alteration within the amino acid sequence of the peptide. In general, substitutions within a group can be considered conservative with respect to structure and function. However, the skilled artisan will recognize that the role of a particular residue is determined by its context within the three-dimensional structure of the molecule in which it occurs. For example, Cys residues may occur in the oxidized (disulfide) form, which is less polar than the reduced (thiol) form. The long aliphatic portion of the Arg side chain can constitute a feature of its structural of functional role, and this may be best conserved by substitution of a nonpolar, rather than another basic residue. Also, it will be recognized that side chains containing aromatic groups (Trp, Tyr, and Phe) can participate in ionic-aromatic or "cation-pi" interactions. In these cases, substitution of one of these side chains with a member of the acidic or uncharged polar group may be conservative with respect to structure and function. Residues such as Pro, Gly, and Cys (disulfide form) can have direct effects on the main chain conformation, and often may not be substituted without structural distortions.

Amino acid substitutions can be the result of replacing one amino acid with another amino acid having similar structural and/or chemical properties such as the replacement of a leucine with a isoleucine, i.e., conservative amino acid replacement. Examples of conservative substitutions are shown in Table 2. the variation allowed can be determined by systematically making insertions, deletions or substitutions of amino acids in the sequence and testing the resulting variants for activity exhibited by the native sequence.

**Table 2**

| **Original Residue** | **Exemplary Substitutions** | **Preferred Substitutions** |
|---|---|---|
| Ala (A) | Val; Leu; Ile | Val |
| Arg ( R ) | Lys; Gln; Asn | Lys |
| Asn (N) | Gln; His; Asp; Lys; Arg | Gln |
| Asp (D) | Glu; Asn | Glu |
| Cys ( C ) | Ser; Ala | Ser |
| Gln (Q) | Asn; Glu | Asn |
| Glu ( E ) | Asp; Gln | Asp |
| Gly (G) | Ala | Ala |
| His (H) | Asn; Gln; Lys; Arg | Arg |
| | Leu; Val; Met; Ala; Phe; | |
| Ile (I) | Norleucine | Leu |
| | Norleucine, Ile; Val; Met; | |
| Leu (L) | Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| | Ile; Leu; Met; Phe; Ala; | |
| Val (V) | Norleucine | Leu |

### Homologous molecules, molecular complexes, and crystal

### Structures

Structure coordinates can be used to aid in obtaining structural information about another crystallized molecule or molecular complex. The method of the disclosure allows determination of at least a portion of the three-dimensional structure of molecules or molecular complexes that contain one or more structural features that are similar to structural features of at least a portion of Met receptor or InIB: Met complex. These molecules are referred to herein as "structurally homologous" to Met receptor. Similar structural features can include, for example, regions of amino acid identity conserved active site or binding site motifs, and similar arranged secondary structural elements (e.g. binding sites for HGF/SF β, PSI domain , Ig domain, and propeller blades of the Sema domain).

Optionally, homology is determined by aligning the residues of the two amino acid sequences; gaps in either or both sequences are permitted in making the alignment in order to optimize the number of identical amino acids, although the amino acids in each sequence must nonetheless remain in their proper order. Two amino acid sequences are compared using the BLAST program, version 2.0.9, of the BLAST 2 search algorithm available at http:www.ncbi.nlm.nih.gov/BLAST/. Preferably, the default values for all BLAST 2 search parameters are used, including matrix =BLOSUM62; open gap penalty = 1d, gap x_dropoff = 50, expect = 10, wordsize = 3, and filter on. In the comparison of two amino acid sequences using the BLAST search algorithm, structural similarity is referred to as "identity".

In some embodiments, a homologous molecule is a protein that has an amino acid sequence sharing at least 70%, e.g. at least 80% identity with a native or recombinant amino acid sequence of Met, preferably an extracellular fragment of the Met receptor comprising a sequence of SEQ ID NO:5 or SEQ ID NO:1. An extracellular fragment of Met receptor comprising a sequence of SEQ ID NO:5 or 1 has amino acid substitutions at position 304-308 to insert a thrombin cleavage site. In some embodiments, a Met receptor has a sequence of SEQ ID NO:1 and the structurally homologous molecule is a variant that ahs a % sequence identity to SEQ ID NO: 1 of at least 80% 81% 82% 83% 84% 85% 86% 87% 88% 89% 90%91% 92% 93% 94% 95% 96% 97% 98% 99% or greater. In some embodiment, the Met receptor derivative or structurally homologous molecule has one or more conservative amino acid substitutions, preferably only conservative amino acid substitutions and retains the structure of the binding site for the inhibitor, like InIB. In some embodiments the Met receptor derivative has about 1-25 conservative amino amino acid substitutions, more preferably about 1-20 conservative amino acids substitutions, more preferably about 1-5 conservative amino acid substitutions, and more preferably 1-2 conservative amino acid substitutions. Preferably, the derivative retains at least one or more domains including the binding site for the InIB. More preferably, a protein that is structurally homologous to Met includes at least one contiguous stretch of at least 50 amino acids that shares at least 80% amino acid sequence identity with the analogous portion of the native or recombinant Met. Methods for generating structural information about the structurally homologous molecule or molecular comply are well known and include, for example, molecular replacement techniques.

Therefore, in another embodiment this disclosure provides a method of utilizing molecular replacement to obtain structural information about a molecule or molecular complex whose structure is unknown comprising:
a) generating an X-ray diffraction pattern from a crystallized molecule or molecular complex of unknown or incompletely known structure; and/or
b) applying at least a portion of the structural coordinates of the Met receptor or InIB:Met complex to the X-ray diffraction pattern to generate a three-dimensional electron density map of the molecule or molecular complex whose structure is unknown or incompletely known.

By using molecular replacement, all or part of the structure coordinates of the Met receptor and/InIB: Met complex as provided by this disclosure can be used to determine the unsolved structure of a crystallized molecule or molecular complex more quickly and efficiently than attempting to determine such information experimentally. Coordinates of structural features of the Met receptor can be utilized including the Sema domain, PSI domain and the Ig1 domain.

Molecular replacement can provide an accurate estimation of the phases for an unknown or incompletely known structure. Phases are one factor in equations that are used to solve crystal structures, and this factor cannot be determined directly. Obtaining accurate values for the phases, by methods other than molecular replacement, can be a time-consuming process that involves iterative cycles of approximations and refinements and greatly hinders the solution of crystal structures. However, when the crystal structure of a protein containing at least a structurally homologous portion has been solved, molecular replacement using the known structure provide a useful estimate of the phases for the unknown structure.

### Homology model

Using homology modeling, a computer model of an Inhibitor Met complex or Met receptor homolog can be built or refined without crystallizing the homolog. First, a preliminary model of the homolog is created by sequence alignment with InIB321: Met or an extracellular fragment of Met, secondary structure prediction, the screening of structural libraries, or any combination of those techniques. Computational software may be used to carry out the sequence alignments and the secondary structure predictions. Programs available for such an analysis include Protein Explorer (eg available at molvissdsc.edu.protexpl.frontdoor.htm), Swiss Model (eg available at swismodel.expasy.org) and RASMOL. Structural incoherencies, e.g., structural fragments around insertions and deletions, can be modeled by screening a structural library for peptides of the final homology model can be used to solve the crystal structure of the homolog by molecular replacement, as described above. Next, the preliminary model is subjected to energy minimization to yield an energy-minimized model. The energy-minimized model may contain regions where stereochemistry restraints are violated, in which case such regions are remodeled to obtain a final homology model. The homology model is positioned according to the result of molecular replacement, and subject to further refinement including molecular dynamics calculations.

Computational techniques can be used to screen, identify, select, design ligands, and combinations thereof, capable of associating with Met or structurally homologous molecules. Candidate modulators InIB useful as an inhibitor and/or Met may be identified using functional assays, such as binding to Met, and novel modulators designed based on the structure of the candidate molecules so identified. Knowledge of the structure coordinates for Met or InIB: Met permits, for example, the design, the identification of synthetic compounds, and like processes, and the design, the identification of other molecules and like processes, that have a shape complementary to the conformation of the InIB binding sites.

A ligand designed or selected as binding to or interfering with an inhibitor binding site may be further computationally optimized so that in its bound state it would preferably lack repulsive electrostatic interaction with the target enzyme and with the surrounding water molecules. Such non-complementary electrostatic interactions include repulsive charge-charge, dipole-dipole, and charge-dipole interactions. Specific computer software is available to evaluate compound deformation energy and electrostatic interactions and are known to those skilled in the art.

The term "a blade of a propeller" refers to a structural feature of the Met receptor. A blade is formed by four anti-parallel strands with strand A in the centre of the blade. The 7 blades are arranged in a circular fashion with the N terminal strand forming strand D of the last blade. The AB and CD loops of each blade of the Met Sema domain form the flat bottom face of the propeller and the BC and DA loops form the top face of the propeller.

Crystal as used herein refers to one form of a solid state of matter in which atoms are arranged in a pattern that repeats periodically in three-dimensions, typically forming a lattice.

As used herein, the term "inhibitor" refers to compounds that are able to suppress or partly or totally inhibit the activation of cells via the Met receptor, said activation is defined in the ability to induce DNA-synthesis and/or scattering of cells expressing the Met receptors. Said cells being activated otherwise in the presence of sufficient amounts of HGF/SF.

The term "homology" as used herein means a value obtained by a BLAST (Basic local alignment search tool; Altschul, S. F. et al., J. Mol. Biol., 215, 403-410, (1990)) search. The homology in the amino acid sequence may be calculated by a BLAST search algorithm. More particularly, it may be calculated using b12seq program (Tatjana A. Tatusova and Thomas L. Madden, FEMS Microbiol. Lett., 174, 247-250, 1999) in a BLAST package (sgi32bit edition, version 2.0-12; obtained from NCBI) in accordance with a default parameter. As a pairwise alignment parameter, a program "blastp" is used. Further, "0" as a Gap elongation cost value, "SEG" as a filter for a Query sequence, and "BLOSUM62" as a Matrix are used, respectively.

It was found that the bacterial protein InIB binds to the Met extracellular Ig1 domain with high affinity. Further as shown in the examples, specific truncated forms of InIB, like InIB241 or InIB321 bind to Met with high affinity but do not activate the receptor in the sense that no scattering or DNA synthesis can be observed. InIB and the natural ligand HGF/SF use different binding sites on Met, although a partial competition between the two ligands for binding to Met can be observed.

Based on the structure of a complex between InIB and Met, as disclosed herein, it is possible to engineer InIB derivatives that are inhibitors of the Met receptor and/or are HGF/SF antagonists.

Said inhibitor binds to the binding site of the inhibitor on Met comprising at least one or all amino acid residues corresponding to residues F590, R592, N593, D597, K599, K600, R602, L604, E639, S641, G643, H644, G645, and T646 of the Met receptor of Seq-ID No. 1. However, no activation in the sense of scattering or DNA synthesis should occur.

The InIB peptide identified as having inhibitory activity on the Met receptor belongs to the large class of structurally conserved leucine rich repeat (LRR) proteins.

These proteins are typical eukaryotic proteins and only rarely found in bacteria. Many LRR proteins are found in humans and a number of crystal structures have been solved so far. Therefore, it is envisaged that a human LRR protein can be used as template for humanising the Met binding site of the InIB peptide.

That is, in a further aspect of the present invention, the inhibitor of the Met receptor is an altered non-immunogenic polypeptide having a LRR domain. Preferably, said LRR domains have aromatic side chains on the surface, in particular side chains of the tyrosine amino acid residue.

In another embodiment, the Inhibitor of a Met receptor is a compound comprising structural elements corresponding to at least two of the residues F104, W124, Y170 and Y214 of Seq. ID No. 2 and having a spatial configurartion of said aa residues as present in InIB as defined in table 1. Preferably, the Inhibitor has a spatial configuration of at least five or preferabley all aa residues Q80, N84, F104, N106, W124, F126, D128, E129, E150, S168, Y170, N173, T190, E194, Q211, N212, Y214, D233 of Seq ID No. 2 as defined in table 1.

Thus, preferably, the inhibitor is an InIB fragment comprising amino acids 36 to 241 of Seq-ID No. 2 or a functional derivative thereof exhibiting binding to Met receptor. In particular, the inhibitor is a polypeptide of Seq-ID No. 3 or 4, a polypeptide comprising an amino acid sequence of Seq-ID No. 3 or 4 and exhibiting i) binding to the Ig1 domain of the Met receptor and ii) are not activating said receptor by inducing DNA synthesis in and/or scattering of cells, a functional fragment or derivative of the above polypeptide exhibiting binding to the Ig1 domain of the Met receptor while not activating said receptor by inducing DNA synthesis in and/or scattering of cell, the polypeptide may also be a polypeptide having a 70% or more homology with an amino acid sequence of Seq-ID No. 3 or 4 or a functional fragment or derivative thereof as defined above, or, alternatively, an altered polypeptide in which at least parts of the amino acids have been replaced by analogous parts of a different specificity, in particular human, while maintaining the ability to bind the Ig1 domain of the Met receptor but not activating said receptor for inducing DNA synthesis in and/or scattering of cells. In particular, said alteration is a humanisation as described above and which is well known to the skilled person.

Said inhibitor may be an inhibitor having at least six β-strands having aromatic residues on its surface, in particular side chains of tyrosine residues.

The inhibitor is able to bind to the inhibitor binding site comprising the Ig1 domain of the Met receptor, in particular said Met inhibitor bind to the binding site formed by a peptide comprising at least amino acid residues 590-604 and 639-646 of Seq-ID No. 1. In a further embodiment, said inhibitor does not bind to the binding site for HGF/SF on the Sema domain of the Met receptor.

As shown in the following, at least the Ig1 domain of Met is crucial for binding of the model inhibitor InIB₃₂₁.

To map the InIB binding site on Met four recombinant variants of the Met ectodomain (Figure 1B and 1D ) have been used, purified from conditioned medium of stably transfected, gylcosylation-deficient CHO Lec cells. In solid phase binding assays, InIB₃₂₁ did not show high affinity binding to Met₅₆₇, a construct comprising only the Sema and PSI domains (Figure 1E). In contrast, InIB₃₂₁ bound with high and virtually identical affinity to longer variants of the Met ectodomain (Met₇₄₁, Met₈₃₈ and Met₉₂₈) that contain two, three or four immunoglobulin (Ig)-like domains, respectively (Figure 1 F). Thus, the binding site for InIB₃₂₁ is fully included in Met₇₄₁ but not in Met₅₆₇, implying a critical role of the Ig1 and/or Ig2 domains of Met for binding the bacterial ligand.

In a further aspect, the present invention relates to the use of a fragment of the Met receptor comprising the newly identified binding site as described herein. In particular, in a further aspect, the present invention concerns the use of a fragment of the Met receptor comprising amino acids 590 to 604 and 639 to 646 of Seq-ID No. 1 as a screening tool for an agent for treating or preventing cancer.

The term "screening tool" as used herein means a tool used for screening, more particular, a polypeptide or a cell expressing a polypeptide used for screening. The term "screening tool for an agent for treating or preventing cancer" as used herein means a cell or a polypeptide as a subject to be brought into with a test compound in the method of the present invention for screening an agent for treating or preventing cancer, for screening an agent for treating or preventing cancer.

Preferably, said agent for treating or preventing cancer screened by using said fragment of the Met receptor is an agent exhibiting an activity of binding to said Met receptor fragment but exhibits no property of activating the Met receptor for inducing DNA synthesis in and/or scattering of cells. For example, said agent binds to the binding site described above but does not bind to the know HGF/SF binding site of the Met receptor, comprising at least one or all of amino acids 124-128, 148, 167, 190-192, 218, 220 to 224, 227, 229 to 230, 286 or 414 of Seq-ID No. 1.

In a preferred embodiment, said fragment of the Met receptor is a peptide according to Seq-ID No. 5 or a functional derivative thereof able to bind the InIB 241 fragment according to Seq-ID No. 4 or InIB 321 fragment according to Seq-ID No. 3.

It is also possible to use a cell which is transfected with an expression vector comprising a polynucleotide encoding a polypeptide comprising a fragment of the Met receptor having amino acids 590 to 604 and 639 to 646 of Seq-ID No. 1 as a screening tool for an agent for treating or preventing cancer. Said polypeptide is preferably the peptide according to Seq-ID No. 4.

Another aspect of the present invention relates to a method for screening an agent for preventing or treating cancer comprising a step of contacting a Met receptor molecule as defined above with a compound to be tested and determining binding of said compound to be tested to said Met receptor and, in a second step, contacting another peptide not containing the residues F590, R592, N 593, D597, K599, K600, R602, L604, I639, S641, G643, H644, G645, and T646 with a compound to be tested and determining binding of the compound to be tested.

An inhibitor identified by the method for screening said agent is a molecule which does bind to a fragment of the Met receptor comprising amino acids 590 to 604 and 639 to 646 but does not bind to the HGF/SF binding site comprising amino acids 124-128, 148, 167, 190-192, 218, 220 to 224, 227, 229 to 230, 286 or 414 of Seq-ID No. 1.

Further, the present invention concerns a method for identifying a potential Met receptor inhibitor compound for the Met receptor which does not activate said Met receptor, said method comprising steps of a) using a three dimensional structure of the Met receptor as defined by atomic coordinates according to table 1 below, b) employing said three dimensional structure to design or select potential inhibitor such that said potential inhibitor is capable of binding to at least one amino acid of the InIB 321 binding site of the Ig1 domain of the Met receptor, c) synthesising said potential inhibitor, d) in an essay contacting said potential inhibitor with said Met receptor and, e) determining the inhibitory activity of said potential inhibitor.

The inhibitory activity can be determined by detecting the ability of said molecule to induce DNA synthesis in cells expressing the Met receptor or by determining the ability to induce scattering of cells expressing the Met receptor. The skilled person is well aware of suitable methods for measuring DNA synthesis or determining scattering effects of said potential inhibitors.

In one aspect, said potential Met receptor inhibitor may be designed or selected using computer modelling. In another aspect, the potential Met receptor inhibitor is designed de novo. It is further possible that the potential Met receptor inhibitor is designed based on a known inhibitor. In particular, said potential Met receptor inhibitor may be designed based on the InIB 241 or InIB 321 peptides as described herein. Said design may be a humanisation of the bacterial InIB peptide by altering the peptide sequence, in particular, substituting amino acids by conservative substitution, thus, rendering said known inhibitor less immunogenic without deteriorating the inhibitory activity.

A further embodiment relates to a method of using a crystal of the Met receptor for screening for a novel drug, wherein said crystal effectively diffracts X-rays for the determination of the atomic coordinates of said Met-receptor of greater than 4 Ǻ and wherein the structure of said Met-receptor comprises aa residues 590 to 604 and 639 to 646 of Seq-ID No. 1 and wherein said structure of said Met-receptor comprises a root mean square deviation of less than or equal to 1 Ǻ in the positions of C-alpha atoms as defined by the atomic coordinates of Met-receptor according to table 1 and wherein said method comprises a) selecting a potential ligand by performing rational drug design with a three dimensional structure determined for the crystal; b) in an essay, contacting the potential ligand with the ligand binding domain of the enzyme; and c) detecting the binding potential of the potential ligand for the ligand binding domain, wherein the potential ligand is selected as a novel drug based on the potential binding activity. In one aspect, no binding to the binding site of the HGF/SF compound, comprising aa residues 124-128, 148, 167, 190-192, 218, 220-224, 229, 230, 286, 414 of the Met-receptor of Seq-ID No. 1 is present while in another embodiment, the inhibitor also binds to the HGF/SF binding site on the Met receptor.

Further, the present invention leads to a crystalline form of the Met receptor molecule, which has a crystal structure for which structural coordinates of the back bone nitrogen, α-carbon and carbonyl carbon atoms of said Met receptor molecule having a root mean square deviation from the structural coordinates of the equivalent back bone atoms of the Met receptor as defined in table 1 of less than 1.0 Ǻ following structural alignment of equivalent back bone atoms, and wherein said Met receptor protein consists of the Ig1, PSI and Sema domains, said domains fold up as a seven bladed β-propeller for the Sema domain and a immunoglobulin-like domain for the Ig1 domain connected via the PSI domain, said Met receptor molecule has an overall amino acid sequence that is at least 70% identical to the amino acid sequence of human Met receptor set forth in Seq-ID No. 1.

Said crystalline form may be used in a method for selecting, testing and/or regionally or semi-regionally designing a chemical compound which binds covalently or non-covalently to Met receptors, e.g. by applying in a computational analysis structure the coordinates of said crystalline form.

Said crystalline form may also be used in a method for selecting, testing and/or regionally or semi-regionally designing a modified Met receptor molecule by applying in a computational analysis structure coordinates of said crystalline form.

Finally, the present invention concerns a method for identifying a potential inhibitor of Met receptor molecules comprising the steps: a) using the atomic coordinate of said crystalline form to define the inhibitor binding sites and the HGF/SF binding site of said receptor, b) identifying a compound that fits the inhibitor binding site but not the HGF/SF binding site; c) obtaining the compound, and d) contacting the compound with the Met receptor molecule determining the binding properties and/or effects of said compounds on and/or the inhibition of the Met receptor molecule by said compound. Said determination of the binding properties is effected e.g. by determining the capacity to induce DNA synthesis or to induce scattering of cells.

The inhibitors are useful for preventing or treating different types of cancer. In particular, the inhibitors according to the present invention are useful for suppressing or preventing metastasis of the tumor and proliferation of the tumor cells. Thus, the inhibitors may be administered to individuals at risk of tumor metastasis.

In the following, the invention will be illustrated further by way of examples without limiting the invention thereto.

### EXPERIMENTAL PROCEDURES

### Protein Purification for Crystallization

Human Met constructs (Gherardi et al., 2003) were produced as secreted, C-terminally His-tagged protein in CHO Lec8 (Met₉₂₈ and Met₈₃₈) or in CHO Lec3.2.8.1 cells (Met₇₄₁ and Met₅₆₇). Cells were grown in SMIF6 medium with 0.5% (Met₈₃₈) or without FCS (Met₉₂₈ and Met₅₆₇) or in serum-free ProCHO5 medium (Met₇₄₁). The yield was about 1 to 2 mg of purified protein from 1 liter of culture. So far, we have not been able to produce Met₆₅₆, the variant with only one Ig domain. During the production phase, cells were cultured at 32 °C. Conditioned medium was concentrated and exchanged against 25 mM Na-phosphate pH 7.4, 150 mM NaCl using cross-flow and a cartridge with 30 kDa cutoff. The protein was purified by affinity chromatography over NiNTA superflow (Qiagen) followed by MonoS (GE Healthcare) using a NaCl gradient (0.1 M to 1 M) in 50 mM Mes, pH 6.0. The protein was deglycosylated over night at 20 °C using ca. 5 mU of EndoH (Calbiochem) per mg of protein in Na-acetate pH 5.5. Deglycosylated protein was re-purified over MonoS as above but using a shallow salt gradient. InIB₃₂₁ not containing the signal sequence aa 1 to 35 of Seq ID No. 2 was expressed as GST-fusion protein from the pETM30 vector in *E*. *coli* BL21-CodonPlus(DE3) cells and purified with a yield of about 20 mg per litre essentially as described (Schubert et al., 2001) except that TEV protease was used for tag removal. The complex was formed by mixing Met₇₄₁ with a molar excess of InIB₃₂₁ and purified using a Superdex200 HR 10/30 column (GE Healthcare) equilibrated in 10 mM Tris, pH 8.0, 50 mM NaCl. The complex was concentrated to 5 mg/ml and aliquots were frozen at -70 °C.

### Crystallization and Data Collection

Initial crystals of the deglycosylated protein were grown from the precipitant synergy screen (Majeed et al., 2003). Diffraction quality crystals were obtained at 20 °C in 96-well sitting-drop plates with 2 µl protein (5 mg/ml) + 1 µl reservoir. The reservoir (70 µl of 16.5% PEG 1500, 4.4% MPD, 0.1 M Tris, pH8.5) was covered with 20 µl of Al's oil to slow down vapour diffusion. Crystals grew over several weeks to a final size of about 100 x 100 x 50. µm. Crystals typically grew as clusters and had to be broken apart for data collection. Crystals were harvested in mother liquor supplemented with 15% glycerol and were flash frozen in liquid nitrogen. Data were collected at ESRF beamline ID23-2 in three wedges of 70-80 degrees from a single crystal with 1 sec exposure and 1 ° rotation per image. The crystal was translated between wedges because of radiation damage. All data were indexed, integrated and scaled with the XDS package (Kabsch, 1993). Data statistics are given in Table 3.

### Structure Determination and Refinement

Crystal form I was solved by molecular replacement in Phaser (McCoy et al., 2005). Crystal structures or homology models of individual domains of the complex were used as search models: InIB₃₂₁ (PDB ID 1h6t (Schubert et al., 2001)); Sema and PSI domain separately (1shy (Stamos et al., 2004)); homology models of Ig1 (1ux3) and Ig2 (2cew (Gherardi et al., 2003)). All domains could be located with confidence. A map calculated directly from the solution identified by Phaser was used for correcting Sema, PSI and InIB and for rebuilding the homology model of Ig1 in coot (Emsley and Cowtan, 2004). The model was completed by iterative building and refinement. The electron density for domain Ig2 was not continous and did not allow rebuilding of the homology model. Hence, Ig2 is not included in the final model. The C2 cell contains two complexes in the asymmetric unit with translational NCS ½ along a and c leading to pseudo-centering with a non-crystallographic two-fold parallel to the crystallographic two-fold axis. Tight NCS restraints on the individual domains were employed throughout refinement in CNS (Brunger et al., 1998) and Refmac5 (Murshudov et al., 1997). After each cycle of rebuilding, simulated annealing and individual B-factor refinement was carried out in CNS followed by TLS and restrained refinement in Refmac5. Final steps of refinement were carried out in Phenix (Adams et al., 2004) using simulated annealing and tight NCS. B factors were modelled solely via TLS refinement with 12 TLS groups, one for InIB₃₂₁ and one for each Met domain. We did not refine B-factors individually, as this caused only a marginal drop in Rfree along with a significant drop in Rwork. The same free-R set was kept in all programs. Refinement statistics are given in Table 4.

Interface analysis and calculation of buried surface areas were carried out using the PISA server (Krissinel and Henrick, 2005). The electrostatic potential was calculated using APBS (Baker et al., 2001). Domain motions were analysed with Dyndom (Hayward and Berendsen, 1998). Figures were prepared with PyMol (DeLano, 2002)

### Purification of full-length InIB

Mature InIB (residues 36-630 of Seq ID No 1) was expressed from the vector pETM30 in *E*. *coli* BL21-CodonPlus(DE3) cells at 37 °C for 4 hr and purified by affinity chromatography using glutathione-sepharose. InIB was cleaved from the tag with TEV in 50 mM Tris, pH 8.0, 150 mM NaCl, 1 mM DTT. The protein was then purified over MonoQ. To remove remaining nucleic acids, the protein was further purified over MonoS in 50 mM Hepes, pH7.9, 0.2 M NaCl, 1 mM DTT with a linear salt gradient. The yield was about 0.5 mg of purified protein per litre. Monodispersity of the protein was verified by dynamic light scattering.

### Analytical Ultracentrifugation

Sedimentation velocity analysis was performed using a Beckman An60Ti rotor in a ProteomeLab XL-A ultracentrifuge. All runs were at 20.0°C, at speeds of 30,000, 45,000, 50,000 and 60,000 rev.min⁻¹ (as appropriate to the sample) and scanning at 280nm at the shortest possible time intervals (∼1.5min). Protein samples were in 0.05 M Tris-Cl, 0.1 M NaCl, pH 8.0 (InIB_{fl} and Met₉₂₈) or in 0.02 M Tris-Cl, 0.1 M NaCl, pH 8.0 (InIB₃₂₁-Met₉₂₈ and InIB_{fl}-Met₉₂₈ complexes) and studied at the concentrations given in the legend to Figure 6. Data were analysed with DC/DT+ v.2.0.7 (Philo, 2006) to give sedimentation coefficients and, in simple cases, Mᵣ, using partial specific volumes and solvent parameters calculated (Laue et al., 1992) with SEDNTERP (Hayes, D. B., Laue, T. & Philo, J. from the RASMB software archive). Due to the complexity of the mixtures of aggregates in many cases, results are shown as plots of dc/dt against s_{20,w}, since these are directly derived from the data with no assumption about boundary shape.

### Met Activation

Phosphorylation of Erk1/2 was studied in Vero (African green monkey kidney) cells using established procedures (Holmes et al., 2007; Rubin et al., 2001) except that lysis buffer contained phosphatase inhibitor cocktail 2 (Sigma P5726). Activated and total Erk1/2 were detected with mouse monoclonal (Sigma M8159) or rabbit polyclonal (Promega V114A) antibodies followed by HRP-conjugated secondary antibodies and HRP substrate. Scattering of MDCK colonies (Stoker et al., 1987) in response to truncated or full length InIB or HGF/SF was assessed by phase contrast micrography18-24 hours after addition of test proteins using a Leitz IRB55 microscope equipped with a Hamamatsu C5810 3CCD digital camera. DNA synthesis assays were carried out on the MK keratinocyte cell line (Holmes et al., 2007).

### Gylcosylated Met: crystal form II

### Protein Crystallization and Data Collection and Processing

Initial crystals of the glycosylated complex were grown from the precipitant synergy screen (Majeed et al., 2003). Crystals diffracting to 4 Å at ID23-2 grew at 25 degree in a 96-well sitting-drop plate with 2 µl protein (8 mg/ml) + 2 µl reservoir (1.4 M Na/K phosphate, pH 6.5, 10% PEG 2000 mono-methyl-ether) to a size of about 20 x 20 x 80 µm. Data were merged from 3 crystals. From each crystal, three wedges of 15 images with 4 sec exposure or 50 images with 2 sec exposure were collected. The rod shaped crystals were translated between wedges to account for radiation damage. All data were indexed, integrated and scaled with the XDS package (Kabsch, 1993). Data statistics are given in Table 3.

### Structure solution and refinement

There are two complexes in the asymmetric unit. The Sema domain and InIB could be located by molecular replacement in Phaser for both complexes. No reasonable solution was obtained for the remaining domains. Possible locations for Ig1 and PSI were deduced by superposing the complex from crystal form I on the Sema domains. This model was rigid body refined in CNS allowing each domain to move individually. Refinement converged to yield plausible positions for all domains. The position of Met domain Ig2 as observed in crystal form I would lead to clashes due to crystal packing in crystal form II. However, there was clear difference density for Ig2. The Ig2 homology model (2cew (Gherardi et al., 2003)) was placed into this density at the graphics workstation followed by rigid body refinement in CNS. Refinement converged and resulted in a decrease in Rfree. The final position fitted very well the initial difference density used for placing Ig2. This model from CNS was further refined in Phenix using TLS and simulated annealing applying tight NCS restraints on the individual domains. B factors were refined by TLS refinement with 12 TLS groups, one group each for InIB and for the individual Met domains.

Refinement statistics from Phenix are given in Table 4. The same free-R set was kept for different refinement programs.

### Binding assays

Monomeric Met constructs (40 - 80 nM) were bound to Ni-coated HisGrab plates (Pierce) via their C-terminal His-tag. After blocking, wells were incubated with concentration series of GST-InIB₃₂₁ or GST-InIB₂₄₁ fusion protein. HRP-coupled anti-GST antibody was used for detection with an ABTS staining reaction. For competition experiments, Nunc MaxiSorp plates were coated with 80 nM Met₉₂₈ and then blocked. A dilution series of the competitor (HGF/SF or InIB₃₂₁) was prepared in a solution containing a fixed concentration (3 nM for GST-InIB₃₂₁ and 1 nM for HGF/SF) of the first ligand prior to addition to the Met coated plate. InIB binding was detected with an HRP coupled anti-GST antibody. HGF/SF binding was assayed with sheep polyclonal serum (1W53) and an HRP coupled secondary antibody.

**Table 3. Data Collection Statistics**

| | **Deglycosylated** | **Gylcosylated** |
|---|---|---|
| **Space group** | C2 | P2₁2₁2₁ |
| **a, b, c (Ǻ)** | 214.5 66.7 181.5 | 139.0 144.98 150.5 |
| **alpha, beta, gamma(°)** | 90.0 123.3 90.0 | 90.0 90.0 90.0 |
| **Solvent content (%)** | 48 | 63 |
| **Resolution (Ǻ)** | 20 - 2.8 (2.9 - 2.8) | 20 - 4.0 (4.1 - 4.0) |
| **Measured reflections** | 244595 (13718) | 253131 (18407) |
| **Unique reflections** | 51863 (4219) | 26274(1876) |
| **Completeness (%)** | 96.9 (79.9) | 99.5 (99.9) |
| **I / sig(I)** | 5.27 (1.68) | 5.99 (2.21) |
| **R-meas (%)** | 24.6 (79.4) | 34.4 (107.4) |
| **Rmrgd-F (%)** | 23.6(70.1) | 21.6 (57.4) |
| **Wilson B** | 53 | 94 |

**Table 4. Refinement Statistics**

| | **Deglycosylated** | **Gylcosylated** |
|---|---|---|
| | C2 | P2₁2₁2₁ |
| **Content of asymmetric unit** | 2 complexes | 2 complexes |
| **Resolution** | 15 - 2.8 | 15 - 4.0 |

| **Reflections** | | |
|---|---|---|
| **Working set** | 49046 | 24413 |
| **Testset** | 2471 | 1286 |
| **Protein atoms** | 13178 | 14391 |
| **Rwork %** | 26.78 | 25.13 |
| **Rfree %** | 30.73 | 30.10 |

| **R.m.s deviations** | | |
|---|---|---|
| **Bond lengths (Ǻ)** | 0.005 | 0.002 |
| **Bond angles (°)** | 0.665 | 0.454 |

### InIB Binds Met via two Interfaces Different from the Binding Site for the HGF/SF β-Chain

The complex between InIB₃₂₁ and Met₇₄₁, the shortest Met construct that retained high affinity binding was crystallised in two different crystal forms. Crystal form I with two complexes in the asymmetric unit contained enzymatically deglycosylated Met and diffracted to 2.8 Å. Crystal form II in a different space group contained Met with truncated N-linked carbohydrate side chains produced by the Lec3.2.8.1 mutant of CHO cells (Stanley, 1989). These crystals diffracted to 4 Å and also contain two complexes in the asymmetric unit. Both crystal forms were solved by molecular replacement. Crystal form II was used only to compare the overall domain arrangement with that of the better diffracting and more extensively refined crystal form I discussed below.

In crystal form I, the first three Met domains have been modelled: Sema, PSI and Ig1, all of which are in direct contact with InIB (Figures 2A and 2B). Ig2 does not contact InIB and there is no continuous electron density for this domain. It is, therefore, omitted from the final model. Nevertheless, it was possible to place by molecular replacement an Ig2 homology model (Gherardi et al., 2003) that is shown in Figures 2A and 2B. InIB interacts with Met *via* two interfaces. A contact between the concave face of the InIB LRR region and Met Ig1 forms the primary interface (Figure 2C), in agreement with the binding data generated with domain deletion constructs of Met (Figure 1E and 1F) or point mutants of InIB (Machner et al., 2003). The secondary, less extensive contact involves the InIB IR region and the Sema domain of Met (Figure 2C) and had not been predicted by binding studies.

Like the InIB IR region, the β-chain of HGF/SF contacts the Sema domain on the bottom face of the β-propeller (Stamos et al., 2004). However, the binding sites for the two ligands on the Sema domain are distant and do not overlap (Figure 2D). The HGF/SF β-chain contacts the Met β-chain at blade 2 and 3 of the Sema domain, whereas InIB contacts the Met β-chain at blades 4 to 6. This agrees with the observation that full length InIB and HGF/SF can bind Met simultaneously (Shen et al., 2000). Apart from the β-chain, HGF/SF has additional domains that interact with Met. To test for a potential overlap of other HGF/SF binding sites on Met with that of InIB, more extensive competition experiments had been performed. These revealed a concentration dependent, partial competition between InIB₃₂₁ and full-length HGF/SF, but neither ligand could displace the other completely (Figure 8).

### Flexibility in the Met Ectodomain

The structure of InIB₃₂₁ within the complex with Met is virtually identical to several structures of free ligand (Marino et al., 2002; Schubert et al., 2001). In contrast, Met undergoes major rearrangements in order to bind InIB as demonstrated by the fact that the relative orientation of the Met Sema and PSI domain is radically different in the Met₇₄₁ - InIB₃₂₁ complex and in the complex of Met₅₆₇ with the β-chain of HGF/SF (Stamos et al., 2004). Indeed, upon aligning the Sema domains of the two structures, the C-terminal end of the PSI domain is displaced by some 15 Å (Figure 2D). The movement can be described as a rigid body rotation of roughly 60° around an axis dose to the linker between the two domains (Figure 3A). Two closely spaced glycine residues (G517 and G519) provide sufficient flexibility to make this linker an effective hinge region.

As both of the crystal forms of the present invention contain two complexes per asymmetric unit, there are four crystallographically independent copies of the complex. Tight non-crystallographic symmetry (NCS) restraints throughout refinement had been applied, but each domain was treated as a separate NCS group. This allows to asses the interdomain flexibility within the Met₇₄₁ - InIB₃₂₁ complex. The two complexes in crystal form I are almost identical. This is not surprising as the NCS in crystal form I is very close to a crystallographic symmetry and may merely represent the break-down of this perfect symmetry. The two copies in crystal form II are truly independent of each other and of the complex from crystal form I. Nevertheless, the overall domain arrangement of the complexes from crystal form II is similar to that of crystal form I, and the secondary interface between the InIB IR region and the Met Sema domain is preserved. An overlay of the complexes performed on InIB shows that the Ig1 domain, the PSI domain and the InIB-proximal side of the Sema domain overlap very well and large movements are limited to Ig2 and the InIB-distal side of the Sema domain, due to a crystal lattice contact (Figure 3B).

### Details of the Primary Interface between the InIB LRR Region and Met Ig1

Met₇₄₁ is the largest fragment of the Met ectodomain crystallized to date, and the structure of the Met Ig1 domain has not been reported before, except for a homology model (Gherardi et al., 2003). Ig1 is an immunoglobulin-like domain with a disulfide bridge linking strands D and E. An unusually long B-C loop forms a unique β-hairpin extending from the core (Figure 4A). The hairpin, which is termed β-wing herein, is a key element for InIB binding. The β-wing lies at the centre of the primary interface between the concave face of the InIB LRR and Met Ig1 in which some 1700 Å² of surface area are buried.

The LRR - Ig1 contact is reminiscent of InIA that likewise binds the Ig-like domain EC1 of human E-cadherin in the void of its larger, horse-shoe shaped LRR (Schubert et al., 2002). However, unlike EC1, which is located centrally within the LRR of InIA, the core of Met Ig1 is offset by some 16 Å towards the loops connecting the 3₁₀-helices and β-strands of the InIB LRR (Figure 4A). Only the β-wing and the top of the Ig fold are in direct contact with the ligand. Two potential glycosylation sites are located at the bottom of Ig1 implying that the carbohydrate side chains present in native Met should neither interfere with nor participate in ligand binding.

Overall, the LRR - Ig1 interface has a mixed hydrophobic and polar character. A string of aromatic amino acid side chains is surface exposed on the concave face of the InIB LRR. Acting as paddles, these residues engage in receptor binding. The aromatic ring of the centrally located Y170ⁱ (i denoting residues from InIB and M from Met), stacks and packs, laterally, against the aliphatic side chains of lysines K600^{M} and K599^{M}, respectively. Through its hydroxyl group Y170^{I} forms additional polar contacts to the side chain of R602^{M} and the backbone carbonyl of K599^{M} from the base of the β-wing (Figure 4B). K599^{M} also stacks against Y214ⁱ. The side chains of K599^{M} and K600^{M} are held in place by an intra- and an inter-molecular salt-bridge, respectively. Side chains from the β-sheet formed by strands CFG of Ig1 shape a hydrophobic pocket for W124^{I} (Figure 4C). The side chains of Phe104ⁱ and Phe126ⁱ are completely buried upon complex formation, mainly by residues from the F-G loop, especially G643^{M} and G645^{M}. These findings are in good agreement with the results of previous mutational analysis highlighting the functional importance of these aromatic InIB residues for Met binding (Machner et al., 2003). Complementary electrostatics between R592^{M} and K600^{M} from the tip and the base of the β-wing, respectively, and a patch of three acidic residues in the LRR region (D128^{I}, E129ⁱ, E150ⁱ) further contribute to the interaction (Figure 4D). The PSI domain of Met contributes to the primary contact a single hydrogen bond to the LRR of InIB.

### The Secondary Interface Between the InIB IR and Met Sema Domain is Critical for Receptor Activation

The second, less prominent contact between InIB IR and Met Sema buries some 870 Å² and is mainly polar. The overall strength of the interaction between the IR and Sema domains is low, as no binding of InIB₃₂₁ to Met₅₆₇ was observed, which can form the secondary, but not the primary contact (Figure 1 E). Moreover, the secondary interface does not measurably contribute to the binding affinity of InIB₃₂₁ to Met, as the shorter InIB₂₄₁ (Figure 1A) that is only capable of binding Ig1 has an apparent affinity for Met similar to InIB₃₂₁ (Figure 5A). This underscores that LRR-Ig1 is the primary, affinity determining contact. However, InIB₂₄₁ and InIB₃₂₁ show clear differences in terms of Met activation. InIB₂₄₁ cannot induce Met phosphorylation or Met-dependent activation of Erk1/2 (Banerjee et al., 2004) (Figure. 5B), whereas InIB₃₂₁ is active in these assays highlighting the fact that the interaction between the IR and Sema domains is essential for the ability of InIB to activate Met.

### Full-Length InIB Induces Receptor Clustering in the Presence of Heparin

Full-length InIB (InIB_{fl}) is much more active than InIB₃₂₁ in receptor phosphorylation assays and, like HGF/SF it elicits full cell responses such as cell migration (Banerjee et al., 2004; Shen et al., 2000) or DNA synthesis in target cells, which InIB₃₂₁ and InIB₂₄₁ cannot induce (Figures 5C and 5D). Receptor dimerization, or more generally oligomerization, is important in the activation of most receptor tyrosine kinases (Hubbard and Till, 2000; Schlessinger, 2000). Therefore, the oligomerisation properties of InIB₃₂₁ and InIB_{fl} were analysed and their complexes with Met₉₂₈, a soluble form of the Met receptor encompassing the whole ectodomain (Figure 1 B) in the absence or presence of a heparin 12mer, in view of reports demonstrating that the GW domains of InIB bind heparin and HSPGs (Banerjee et al., 2004; Jonquieres et al., 2001). Analytical ultra centrifugation (AUC) was used as complexes of InIB_{fl} with its receptor(s) are hardly tractable by size exclusion chromatography due to strong interactions of InIB_{fl} with the matrix at physiological salt concentrations.

InIB₃₂₁ is monomeric (Figures 6A) and equimolar mixtures of InIB₃₂₁ and Met₉₂₈ produced a 1:1 complex and a very small faster boundary (Figure 6C) that is also visible on sedimentation of Met alone (Figure S2). Addition of heparin did not change the sedimentation behaviour of InIB₃₂₁ alone (Figure 6B) or in complex with Met₉₂₈ (Figure 6D). Hence, under none of the conditions tested, dimerization of Met upon binding of InIB₃₂₁ was observed.

InIB_{fl} alone sediments as a monomer (Banerjee et al., 2004) (Figure 6E). Addition of heparin caused extensive aggregation and pelleting of the protein with just 20% of the original material remaining in solution as a 1:1 InIB_{fl}-heparin complex (Figure 6F). InIB_{fl} and Met₉₂₈ at 1:1 molar ratio gave a broad peak containing the 1:1 and 2:2 complexes as well as smaller amounts of higher molecular weight species (Figure 6G). Addition of heparin had a striking effect. The low molecular weight species disappeared and most of the material shifted to large sedimentation coefficients, (Figure 6H). Thus, heparin induced massive clustering of the Met - InIB_{fl} complexes in striking contrast with the results obtained with mixtures of HGF/SF and Met₉₂₈ (Gherardi et al., 2003).

To conclude, InIB₃₂₁ acts as a "molecular clamp" to lock Met in a signalling-competent conformation. Electron microscopy has shown that in the absence of ligand, the Met ectodomain is highly flexible (Gherardi et al., 2006). The large relative rearrangement of the Sema and the PSI domain between the Met - InIB complex and the complex of Met and the HGF/SF β-chain confirms these findings. In the latter complex, the Met PSI domain is not constrained by interactions with the ligand. Therefore, the orientation of the PSI domain does not represent a specific HGF/SF-bound conformation. Rather, it will represent one of potentially many conformations that can be sampled by the free Met ectodomain.

In contrast, in the Melt - InIB complex the position of the PSI domain is restricted through extensive contacts of its two flanking domains Sema and Ig1 with the ligand InIB. InIB₃₂₁ itself is a rigid unit that hardly changes upon binding to Met. It thus presents a preformed binding site to which the receptor accommodates. We suggest that InIB acts as a "molecular clamp" that forces the otherwise flexible receptor into a rigid, signaling competent conformation. This interpretation provides an explanation for the fact that InIB₃₂₁ but not the shorter InIB₂₄₁ can induce Met and Erk phosphorylation. Both proteins can bind the Ig1 domain of the receptor, but the lack of the IR domain abrogates the ability of InIB₂₄₁ to clamp Met into a signaling competent conformation *via* the second interface. Currently no definite conclusions about how the intracellular signalling is initiated upon binding of InIB₃₂₁ to the extra-cellular part of Met can be drawn. However, it is plausible that the rigidification of the Met ectodomain allows receptor molecules in the membrane to pack more closely, which in turn may facilitate cross-phosphorylation of the cytoplasmic tyrosine kinase domains.

Further, there is evidence that receptor dimerization/oligomerization is a key event in Met activation (Banerjee et al., 2004; Kong-Beltran et al., 2004; Prat et al., 1998). However, no dimerization of the Met ectodomain upon binding of InIB₃₂₁ in solution was observed. Furthermore, although both crystal form I and II contain two Met₇₄₁ -InIB₃₂₁ complexes in the asymmetric unit, these dimeric assemblies appear to be caused by crystal packing because the arrangement differs markedly between the two crystal forms and the contacts supporting them bear little specificity, arguing against a potential physiological significance. Finally, it was found that mutant forms of InIB in which the major contact stabilizing the more plausible dimeric assembly from crystal form I was disrupted by insertion of an extra LRR retained the ability to activate the Met receptor.

Full receptor activation requires the C-terminal GW domains of the full-length protein in addition to the Met binding N-terminal domain. The present structure, the biological activities of InIB₃₂₁ and InIB_{fl} and the solution behaviour of these proteins in complex with Met₉₂₈ provide a framework for Met activation by InIB in which receptor clustering plays the pivotal role. InIB exists in two forms, non-covalently associated with the bacterial surface, and free in solution (Braun et al., 1997). It is currently unknown, which of these forms normally mediates bacterial invasion, as both induce Met signaling (Bieme and Cossart, 2002). The structures of the Met₇₄₁ - InIB₃₂₁ complex and full length InIB (Marino et al., 2002) (Fig. 7A) have been superimposed. This structure-based model seems sterically plausible, as the proteins' C-termini associated with the respective cell-surfaces point into almost opposite directions and suggests that several InIB molecules could activate Met by receptor clustering while being attached to *Listeria via* interaction between the GW domains and lipoteichoic acid (Jonquieres et al., 1999). Soluble InIB_{fl} could activate Met by receptor clustering as well, brought about by interaction of the GW domains with HSPGs on the host cell surface (Fig. 7B).

Two processes contribute to InIB-mediated Met activation, namely clamping of the receptor ectodomain and receptor clustering. Using engineered variants of InIB, these processes can be experimentally separated. Clamping on its own is sufficient for partial activation of Met as evident from receptor phosyphorylation studies using InIB₃₂₁, which can clamp but not cluster Met ((Shen et al., 2000) and Figure 5C).

Likewise, clustering of Met alone can promote receptor activation in the absence of clamping. This is apparent from experiments in which InIB constructs consisting of only Cap and LRR are artificially clustered. For example, immobilization of Cap-LRR constructs on latex beads allows for efficient Met mediated entry of beads into cells that do not take up control beads (Braun et al., 1999). Fusion of GW domains 2 and 3 to a Cap-LRR fragment also yields a protein that can induce Met phosphorylation, although requiring approximately 20-fold higher concentrations compared with a version that additionally harbors the IR region (Banerjee et al., 2004). This strongly suggests that in the physiological context of the full-length protein both processes cooperate to turn InIB into such a potent Met agonist. In summary, activation of the Met receptor by InIB occurs in three steps: (i) a first, high-affinity binding event involving the LRR and Met Ig1, (ii) receptor rigidification *via* the secondary contact involving the IR and the Met Sema domain and finally, (iii) receptor oligomerisation *via* the GW domains, a process greatly enhanced by heparin and, presumably, by heparan sulphate on the surface of target cells.

The structural aspects of the interaction between Met and its natural ligand HGF/SF are still far from being completely understood, certainly owing, at least in part, to problems in the amount and homogeneity of the sample available. Proteins derived from bacteria are generally easier to produce and handle than those of eukaryotic or even mammalian origin. Bacterial virulence factors that interact with eukaryotic host proteins, therefore, often can be turned into useful tools to study not only the process of infection but also the signalling pathway of the host that is targeted. The structural data in combination with the data from competitive binding studies allow to draw according to the present invention conclusions pertinent to the interaction of the natural ligand HGF/SF with the Met receptor.

The active form of HGF/SF is a two-chain protein produced by proteolytic cleavage of an inactive, single-chain precursor. The C-terminal β-chain forms a single domain homologous to serine proteases (SPH domain). The N-terminal β-chain contains five additional domains (an N-terminal (N) and four kringle (K1 - K4) domains) (Birchmeier et al., 2003). Out of these six domains, three (N, K1 and SPH) are responsible for HGF/SF binding to the Sema domain of Met (Holmes et al., 2007; Stamos et al., 2004). No competition for binding to Met would be expected between InIB₃₂₁ and the SPH domain of HGF/SF, as the binding sites are distant. The fact that no partial competition between InIB₃₂₁ and HGF/SF was shown herein suggests that the N and/or K1 binding sites overlap, at least partially, with that for InIB IR on the bottom and side faces around blade 5 of the β-propeller.

The results of the present invention highlight two important differences in the mechanism of Met binding and activation by InIB and the physiological ligand HGF/SF: (i) In the case of HGF/SF, both the N-terminal (NK1) and the C-terminal moiety (the SPH domain) display Met binding and both ends of the molecule bind the Sema domain (Holmes et al., 2007; Stamos et al., 2004). Binding of InIB to Met, in contrast, involves the N-terminal Cap-LRR-IR fragment only (Banerjee et al., 2004; Shen et al., 2000) and crucially depends on the Ig1 domain of Met; (ii) Heparin has a massive effect on the oligomerisation of the InIB_{fl}-Met₉₂₈ complex (Fig 6H) but not on the HGF/SF- Met₉₂₈ complex (Gherardi et al, 2003).

The ability of intracellular pathogenic bacteria to exploit receptors on the host cell membrane for cell invasion involves, in a number of instances, bacterial proteins which are structural mimics of the physiological ligand (Stebbins and Galan, 2001). The intracellular responses elicited by InIB and HGF/SF are similar (Bierne and Cossart, 2002; Shen et al., 2000) and HGF/SF can even substitute for InIB in inducing bacterial uptake (Banerjee et al., 2004). However, the present invention clearly shows that InIB is not a structural mimic of NGF/SF, that the two ligands bind to different regions of the receptor, and that they employ different molecular mechanisms for receptor activation. *L. monocytogenes* thus relies on a robust, less regulated approach to achieve maximum receptor activation allowing for efficient bacterial uptake during invasion. Finally, the results demonstrate a striking promiscuity of Met towards different ligands. Over evolutionary periods of time *L*. *monocytogenes* has adapted to bind and activate Met by a novel mechanism that may lead to an alternative way to therapeutic approaches against cancer or infection that take into account the Met stalk rather than to focus merely on the Sema domain.

### Accession Numbers

Coordinates and structure factors of crystal form I and crystal form II have been deposited in the Protein Data Bank with accession number 2uzx and 2uzy, respectively.

### REFERENCES

Adams, P.D., Gopal, K., Grosse-Kunstleve, R.W., Hung, L.W., loerger, T.R., McCoy, A.J., Moriarty, N.W., Pai, R.K., Read, R.J., Romo, T.D., et al. (2004). Recent developments in the PHENIX software for automated crystallographic structure determination. J. Synchrotron Radiat. 11, 53-55.
Baker, N.A., Sept, D., Joseph, S., Holst, M.J., and McCammon, J.A. (2001). Electrostatics of nanosystems: application to microtubules and the ribosome. Proc. Natl. Acad. Sci. USA 98, 10037-10041.
Banerjee, M., Copp, J., Vuga, D., Marino, M., Chapman, T., van der Geer, P., and Ghosh, P. (2004). GW domains of the Listeria monocytogenes invasion protein InIB are required for potentiation of Met activation. Mol. Microbiol. 52, 257-271.
Bieme, H., and Cossart, P. (2002). InIB, a surface protein of Listeria monocytogenes that behaves as an invasin and a growth factor. J. Cell Sci. 115, 3357-3367.
Birchmeier, C., Birchmeier, W., Gherardi, E., and Vande Woude, G.F. (2003). Met, metastasis, motility and more. Nat. Rev. Mol. Cell Biol. 4, 915-925.
   Braun, L., Dramsi, S., Dehoux, P., Bierne, H., Lindahl, G., and Cossart, P. (1997). InIB: an invasion protein of Listeria monocytogenes with a novel type of surface association. Mol. Microbiol. 25, 285-294.
   Braun, L., Ghebrehiwet, B., and Cossart, P. (2000). gC1q-R/p32, a C1q-binding protein, is a receptor for the InIB invasion protein of Listeria monocytogenes. EMBO J. 19, 1458-1466.
   Braun, L., Nato, F., Payrastre, B., Mazie, J.C., and Cossart, P. (1999). The 213-amino-acid leucine-rich repeat region of the Listeria monocytogenes InIB protein is sufficient for entry into mammalian cells, stimulation of PI 3-kinase and membrane ruffling. Mol. Microbiol. 34, 10-23.
   Brunger, A.T., Adams, P.D., Clore, G.M., DeLano, W.L, Gros, P., Grosse-Kunstleve, R.W., Jiang, J.S., Kuszewski, J., Nilges, M., Pannu, N.S., et al. (1998). Crystallography & NMR system: A new software suite for macromolecular structure determination. Acta Crystallogr. D Biol. Crystallogr. 54, 905-921.
   DeLano, W.L. (2002). The PyMOL Molecular Graphics System. on World Wide Web http://www.pymol.org.
   Emsley, P., and Cowtan, K. (2004). Coot: model-building tools for molecular graphics. Acta Crystallogr. D Biol. Crystallogr. 60, 2126-2132.
   Gherardi, E., Sandin, S., Petoukhov, M.V., Finch, J., Youles, M.E., Ofverstedt, L.G., Miguel, R.N., Blundell, T.L., Vande Woude, G.F., Skoglund, U., et al. (2006). Structural basis of hepatocyte growth factor/scatter factor and MET signalling. Proc. Natl. Acad. Sci. USA 103, 4046-4051.
   Gherardi, E., Youles, M.E., Miguel, R.N., Blundell, T.L., lamele, L., Gough, J., Bandyopadhyay, A., Hartmann, G., and Butler, P.J. (2003). Functional map and domain structure of MET, the product of the c-met protooncogene and receptor for hepatocyte growth factor/scatter factor. Proc. Natl. Acad. Sci. USA 100, 12039-12044.
   Hayward, S., and Berendsen, H.J. (1998). Systematic analysis of domain motions in proteins from conformational change: new results on citrate synthase and T4 lysozyme. Proteins 30, 144-154.
   Holmes, O., Pillozzi, S., Deakin, J.A., Carafoli, F., Kemp, L., Butler, P.J., Lyon, M., and Gherardi, E. (2007). Insights into the Structure/Function of Hepatocyte Growth Factor/Scatter Factor from Studies with Individual Domains. J. Mol. Biol. 367, 395-408.
   Hubbard, S.R., and Till, J.H. (2000). Protein tyrosine kinase structure and function. Annu. Rev. Biochem. 69, 373-398.
   Jonquieres, R., Bieme, H., Fiedler, F., Gounon, P., and Cossart, P. (1999). Interaction between the protein InIB of Listeria monocytogenes and lipoteichoic acid: a novel mechanism of protein association at the surface of gram-positive bacteria. Mol. Microbiol. 34, 902-914.
   Jonquieres, R., Pizarro-Cerda, J., and Cossart, P. (2001). Synergy between the N- and C-terminal domains of InIB for efficient invasion of non-phagocytic cells by Listeria monocytogenes. Mol. Microbiol. 42, 955-965.
   Kabsch, W. (1993). Automatic processing of rotation diffraction data from crystals of initially unknown symmetry and cell constants. J. Appl. Crystallogr. 26, 795-800.
   Kong-Beltran, M., Stamos, J., and Wickramasinghe, D. (2004). The Sema domain of Met is necessary for receptor dimerization and activation. Cancer Cell 6, 75-84.
   Krissinel, E., and Henrick, K. (2005). Detection of Protein Assemblies in Crystals. In CompLife, M.R. Berthold, ed. (Berlin Heidelberg, Springer-Verlag), pp. 163-174.
   Laue, T.M., Shah, B.D., Ridgeway, T.M., and Pelletier, S.L. (1992). In Analytical Ultracentrifugation in Biochemistry and Polymer Science, S.E. Harding, A.J. Rowe, and J.C. Horton, eds. (Cambridge, UK, Royal Society of Chemistry), pp. 90-125.
   Machner, M.P., Frese, S., Schubert, W.D., Orian-Rousseau, V., Gherardi, E., Wehland, J., Niemann, H.H., and Heinz, D.W. (2003). Aromatic amino acids at the surface of InIB are essential for host cell invasion by Listeria monocytogenes. Mol. Microbiol. 48, 1525-1536.
   Majeed, S., Ofek, G., Belachew, A., Huang, C.C., Zhou, T., and Kwong, P.D. (2003). Enhancing protein crystallization through precipitant synergy. Structure 11, 1061-1070.
   Marino, M., Banerjee, M., Jonquieres, R., Cossart, P., and Ghosh, P. (2002). GW domains of the Listeria monocytogenes invasion protein InIB are SH3-like and mediate binding to host ligands. EMBO J. 21, 5623-5634.
   McCoy, A.J., Grosse-Kunstleve, R.W., Storoni, L.C., and Read, R.J. (2005). Likelihood-enhanced fast translation functions. Acta Crystallogr. D Biol. Crystallogr. 61, 458-464.
   Murshudov, G.N., Vagin, A.A., and Dodson, E.J. (1997). Refinement of macromolecular structures by the maximum-likelihood method. Acta Crystallogr. D Biol. Crystallogr. 53, 240-255.
   Philo, J.S. (2006). Improved methods for fitting sedimentation coefficient distributions derived by time-derivative techniques. Anal. Biochem. 354, 238-246.
   Prat, M., Crepaldi, T., Pennacchietti, S., Bussolino, F., and Comoglio, P.M. (1998). Agonistic monoclonal antibodies against the Met receptor dissect the biological responses to HGF. J. Cell Sci. 111 (Pt 2), 237-247.
   Rubin, J.S., Day, R,M., Breckenridge, D., Atabey, N., Taylor, W.G., Stahl, S.J., Wingfield, P.T., Kaufman, J.D., Schwall, R., and Bottaro, D.P. (2001). Dissociation of heparan sulfate and receptor binding domains of hepatocyte growth factor reveals that heparan sulfate-c-met interaction facilitates signaling. J. Biol. Chem. 276, 32977-32983.
   Schlessinger, J. (2000). Cell signaling by receptor tyrosine kinases. Cell 103, 211-225.
   Schubert, W.D., Gobel, G., Diepholz, M., Darji, A., Kloer, D., Hain, T., Chakraborty, T., Wehland, J., Domann, E., and Heinz, D.W. (2001). Internalins from the human pathogen Listeria monocytogenes combine three distinct folds into a contiguous internalin domain. J. Mol. Biol. 312, 783-794.
   Schubert, W.D., and Heinz, D.W. (2003). Structural aspects of adhesion to and invasion of host cells by the human pathogen Listeria monocytogenes. Chembiochem. 4, 1285-1291.
   Schubert, W.D., Urbanke, C., Ziehm, T., Beier, V., Machner, M.P., Domann, E., Wehland, J., Chakraborty, T., and Heinz, D.W. (2002). Structure of internalin, a major invasion protein of Listeria monocytogenes, in complex with its human receptor E-cadherin. Cell 111, 825-836.
   Shen, Y., Naujokas, M., Park, M., and Ireton, K. (2000). InIB-dependent internalization of Listeria is mediated by the Met receptor tyrosine kinase. Cell 103, 501-510.
   Stamos, J., Lazarus, R.A., Yao, X., Kirchhofer, D., and Wiesmann, C. (2004). Crystal structure of the HGF beta-chain in complex with the Sema domain of the Met receptor. EMBO J. 23, 2325-2335.
   Stanley, P. (1989). Chinese hamster ovary cell mutants with multiple glycosylation defects for production of glycoproteins with minimal carbohydrate heterogeneity. Mol. Cell. Biol. 9, 377-383.
   Stebbins, C.E., and Galan, J.E. (2001). Structural mimicry in bacterial virulence. Nature 412, 701-705.
   Stoker, M., Gherardi, E., Perryman, M., and Gray, J. (1987). Scatter factor is a fibroblast-derived modulator of epithelial cell mobility. Nature 327, 239-242.

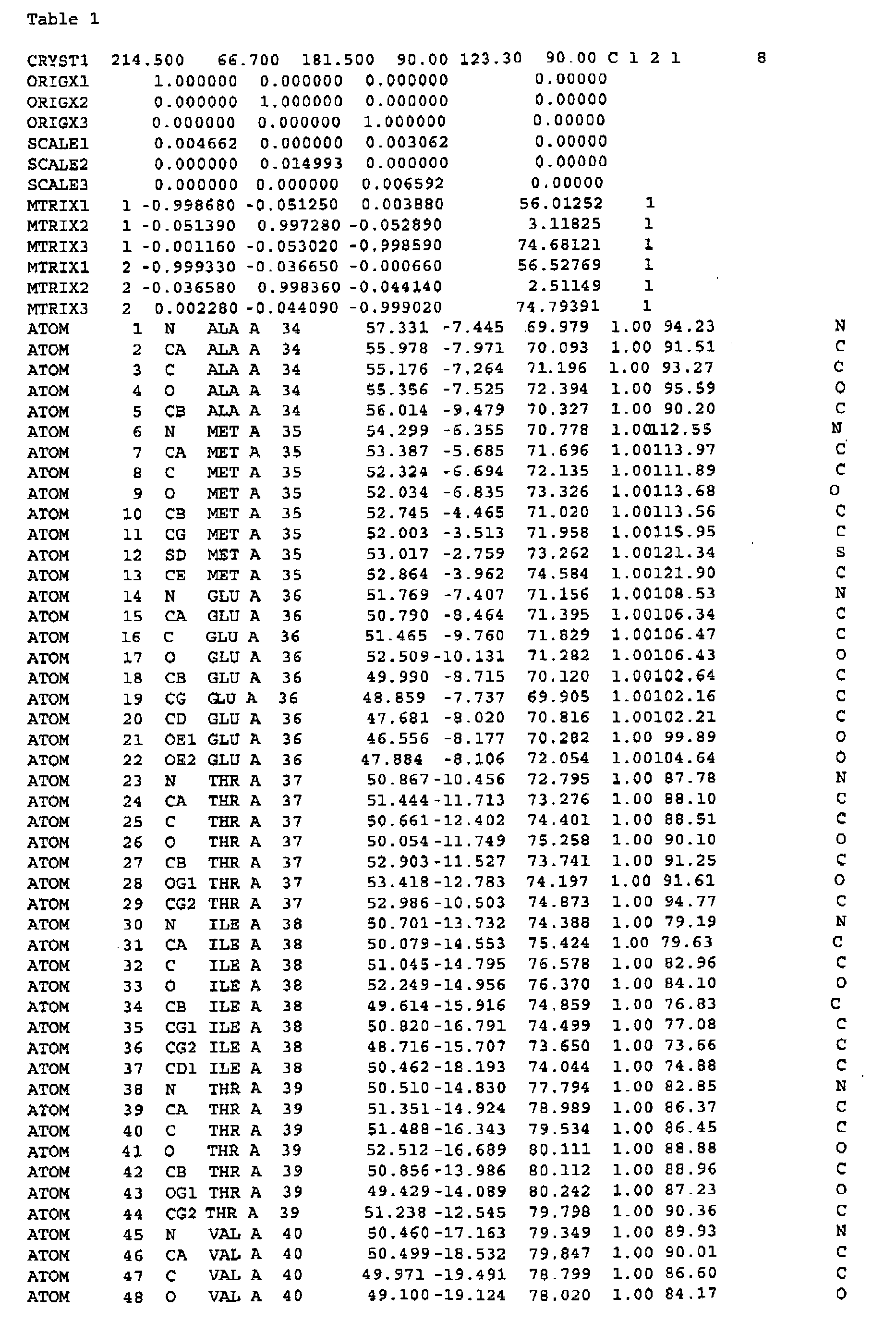

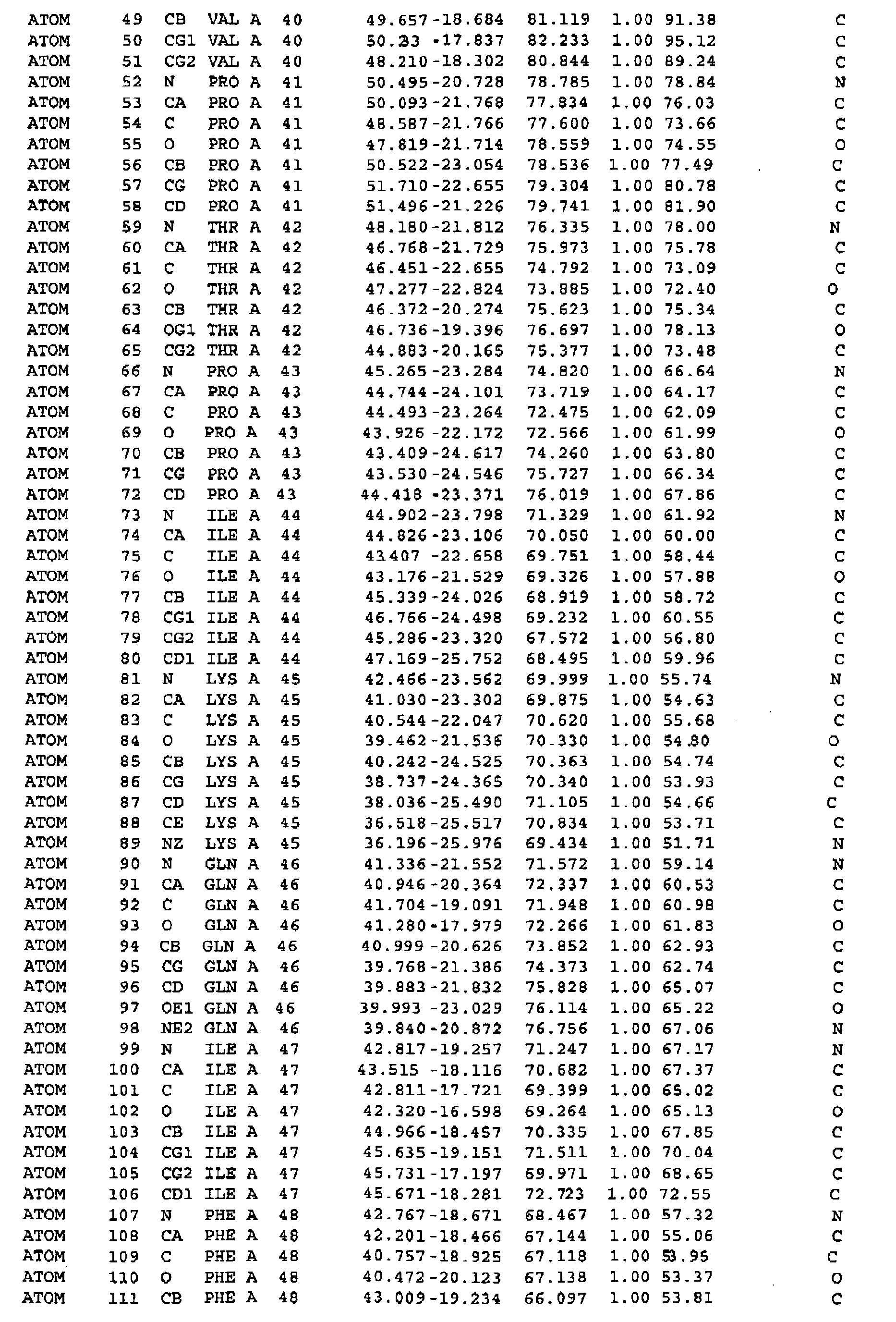

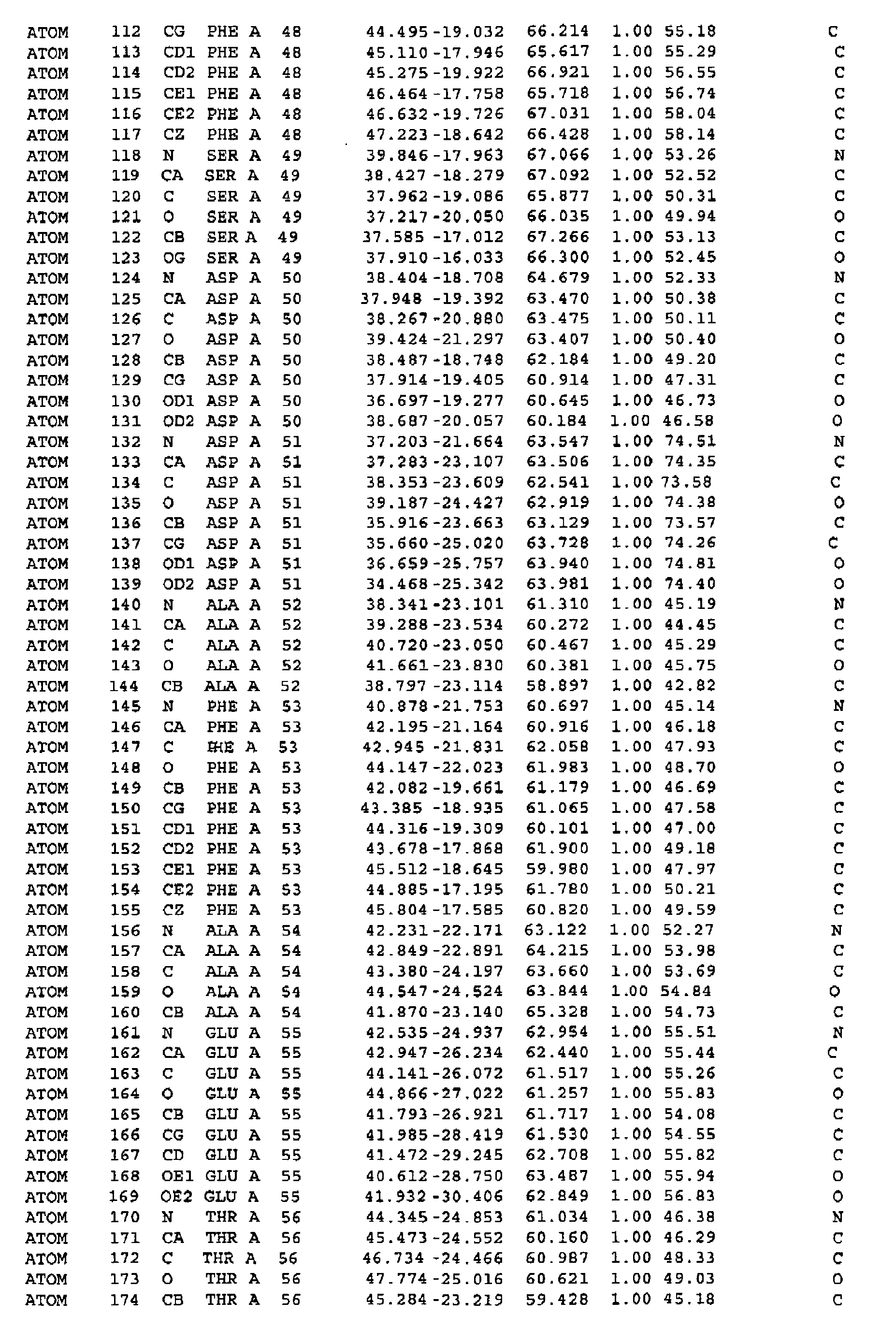

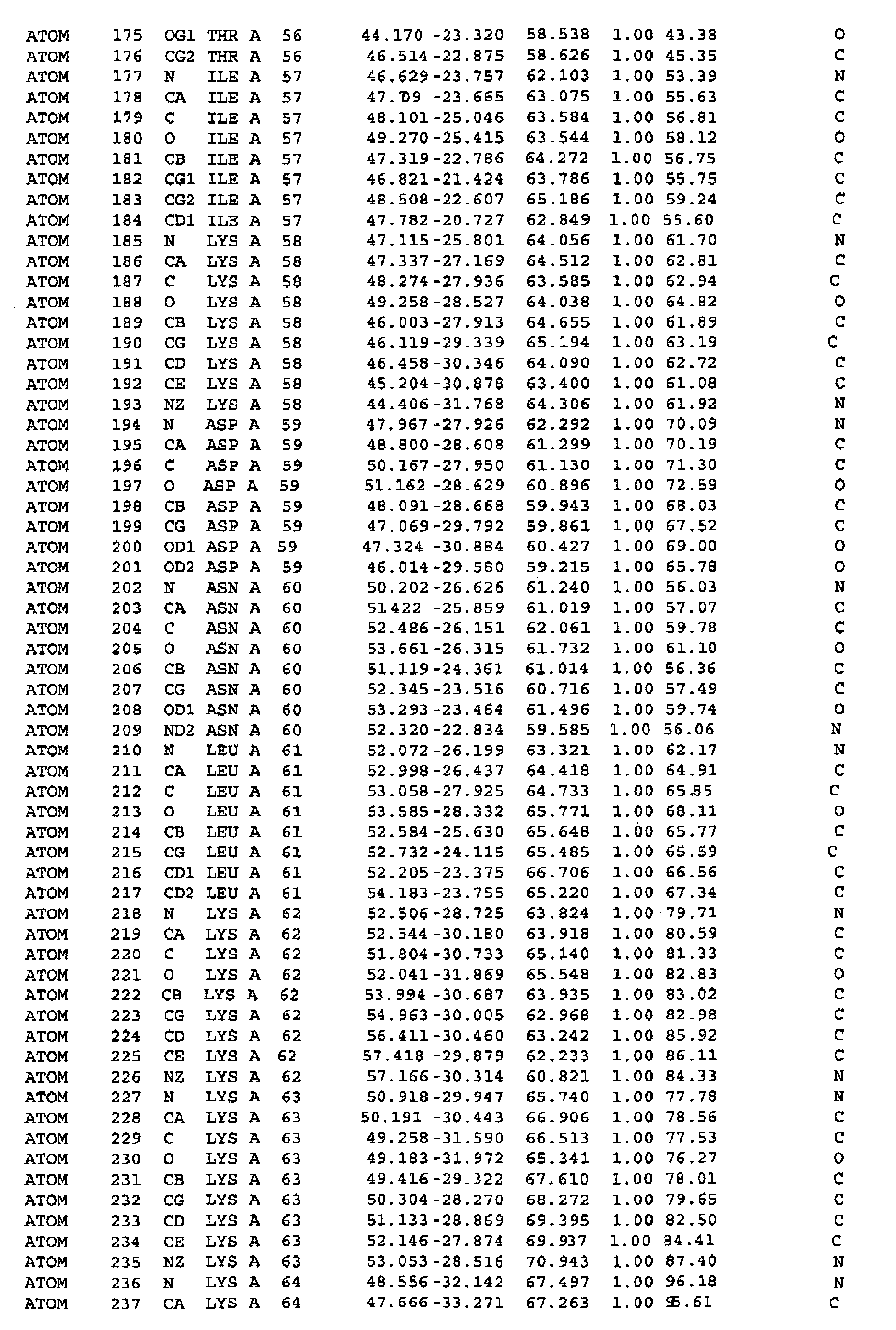

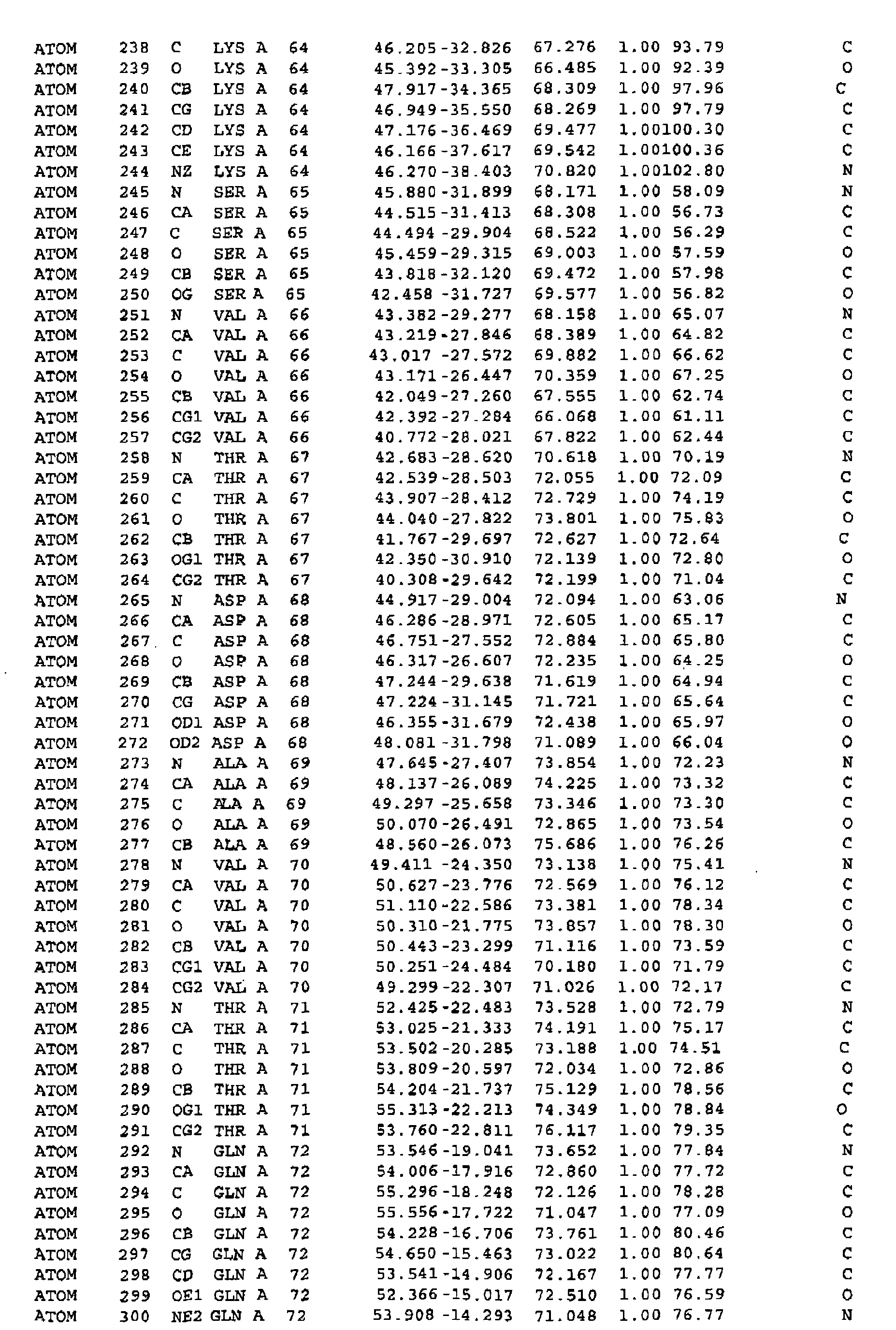

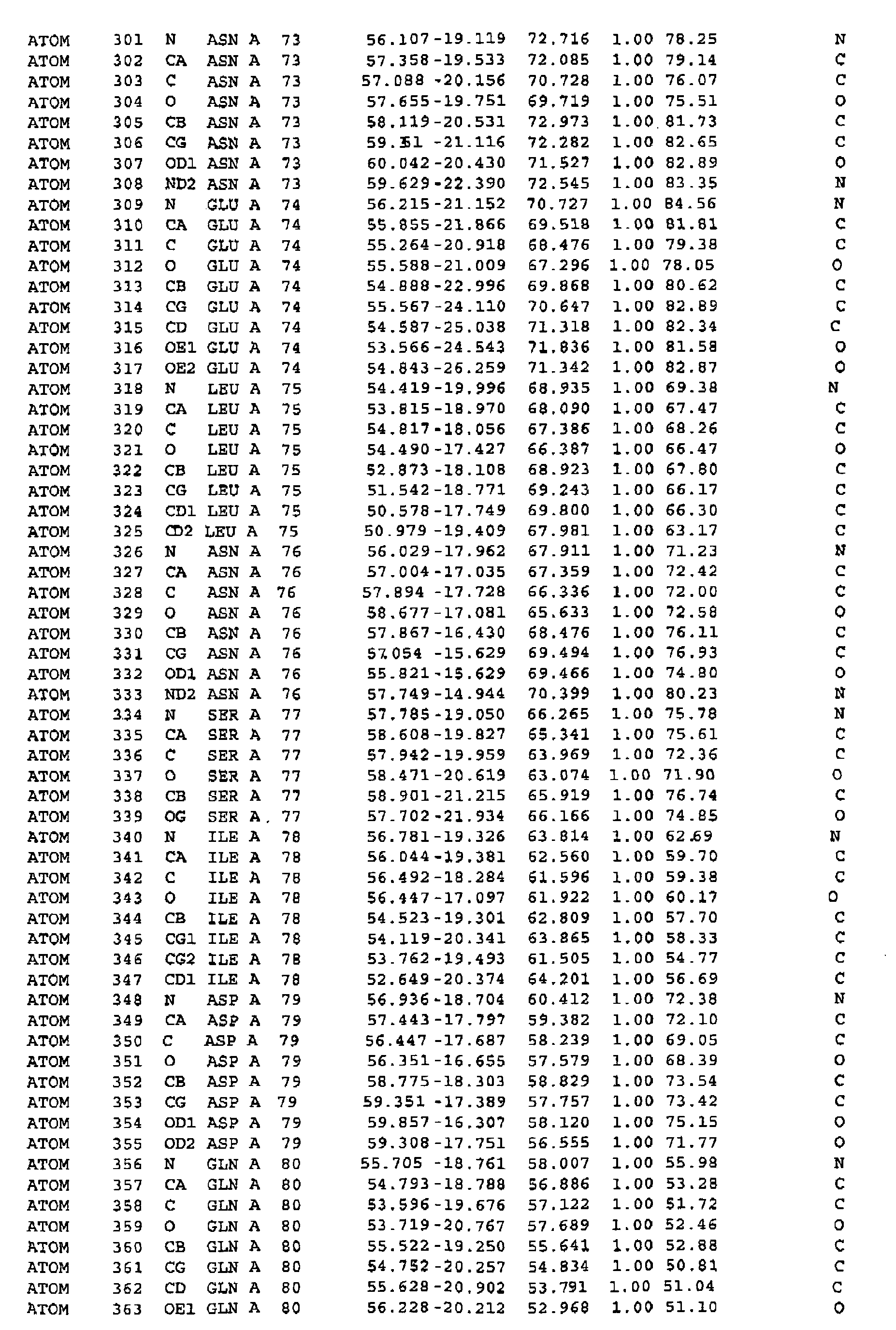

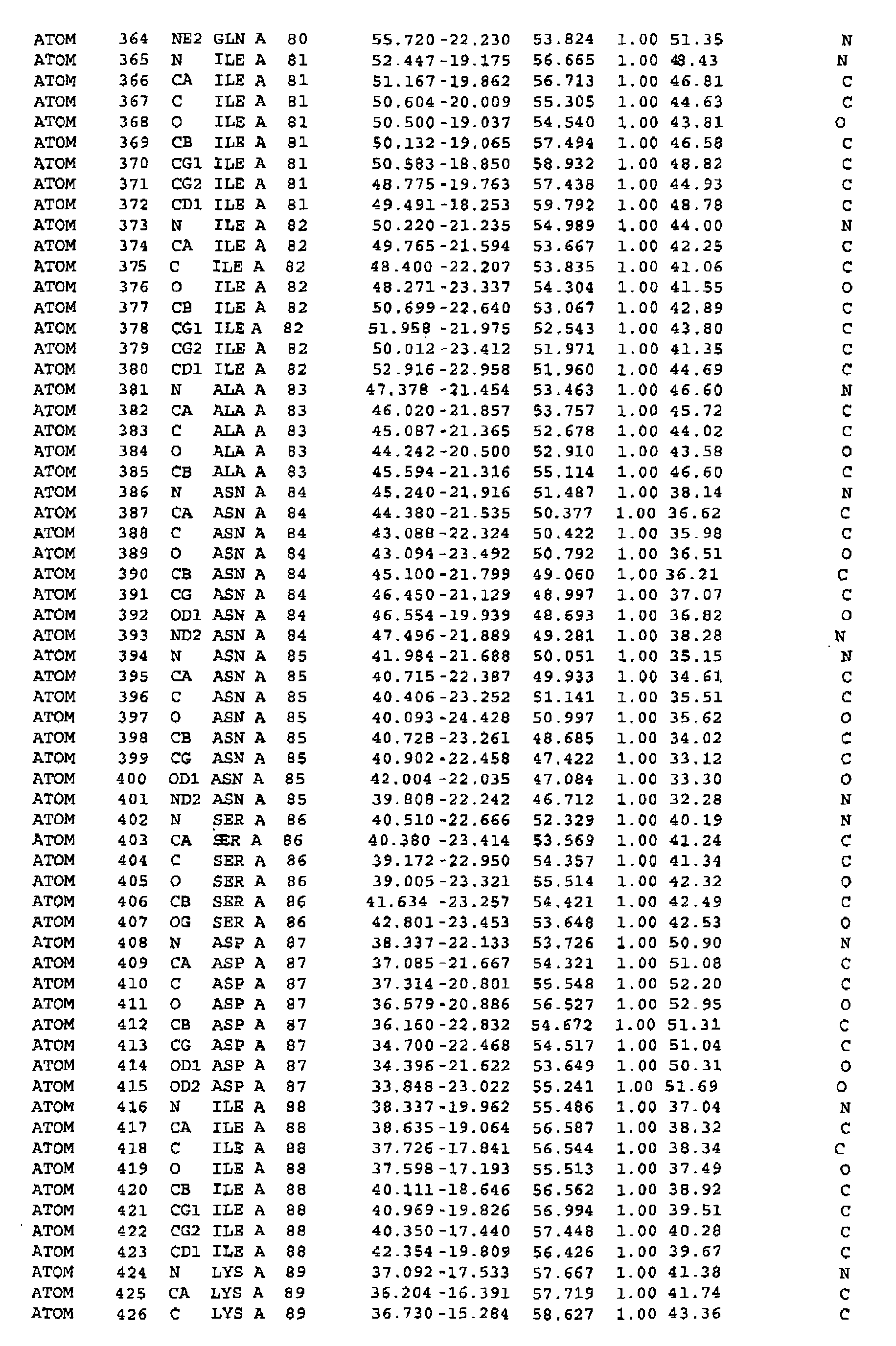

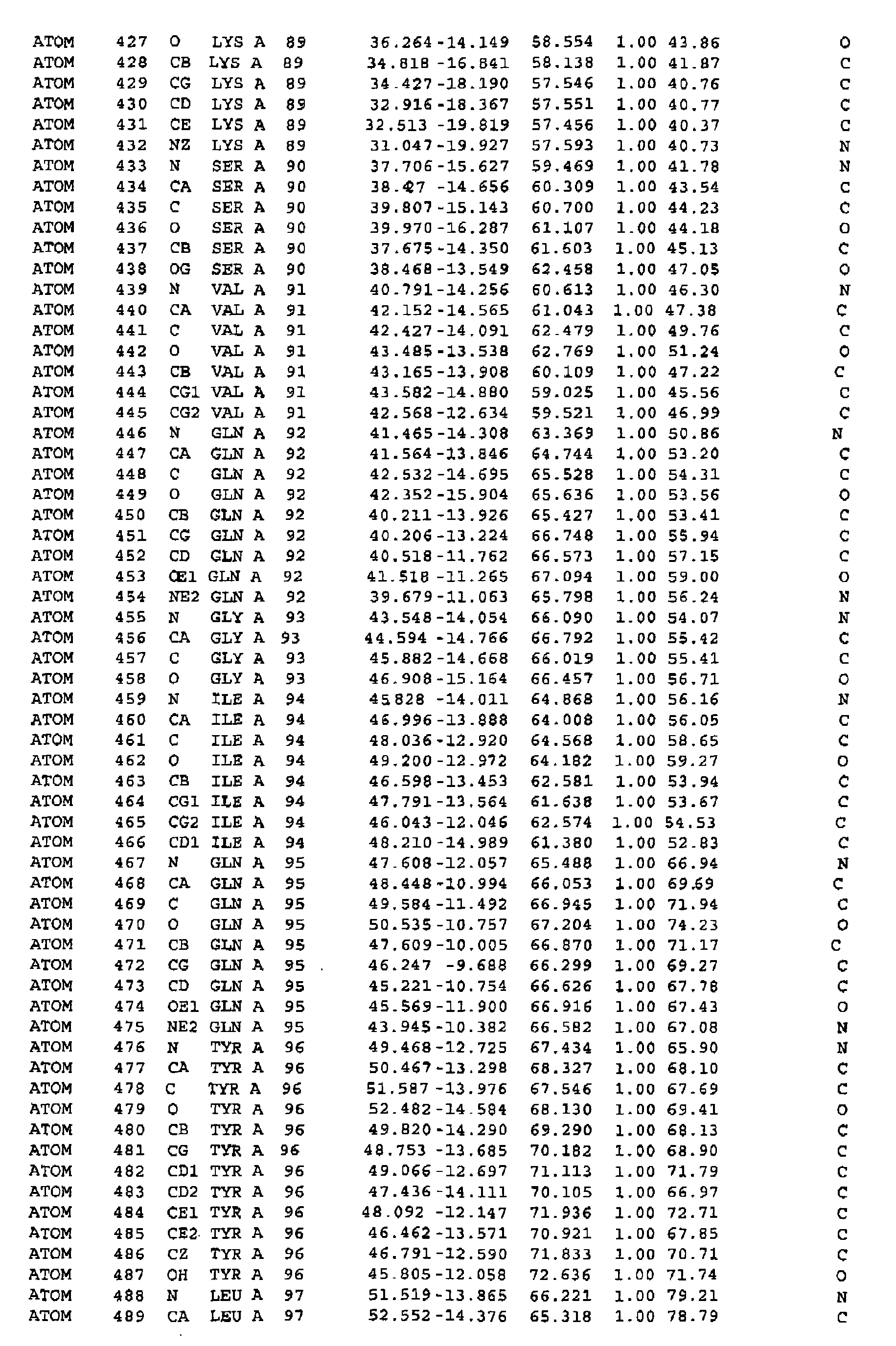

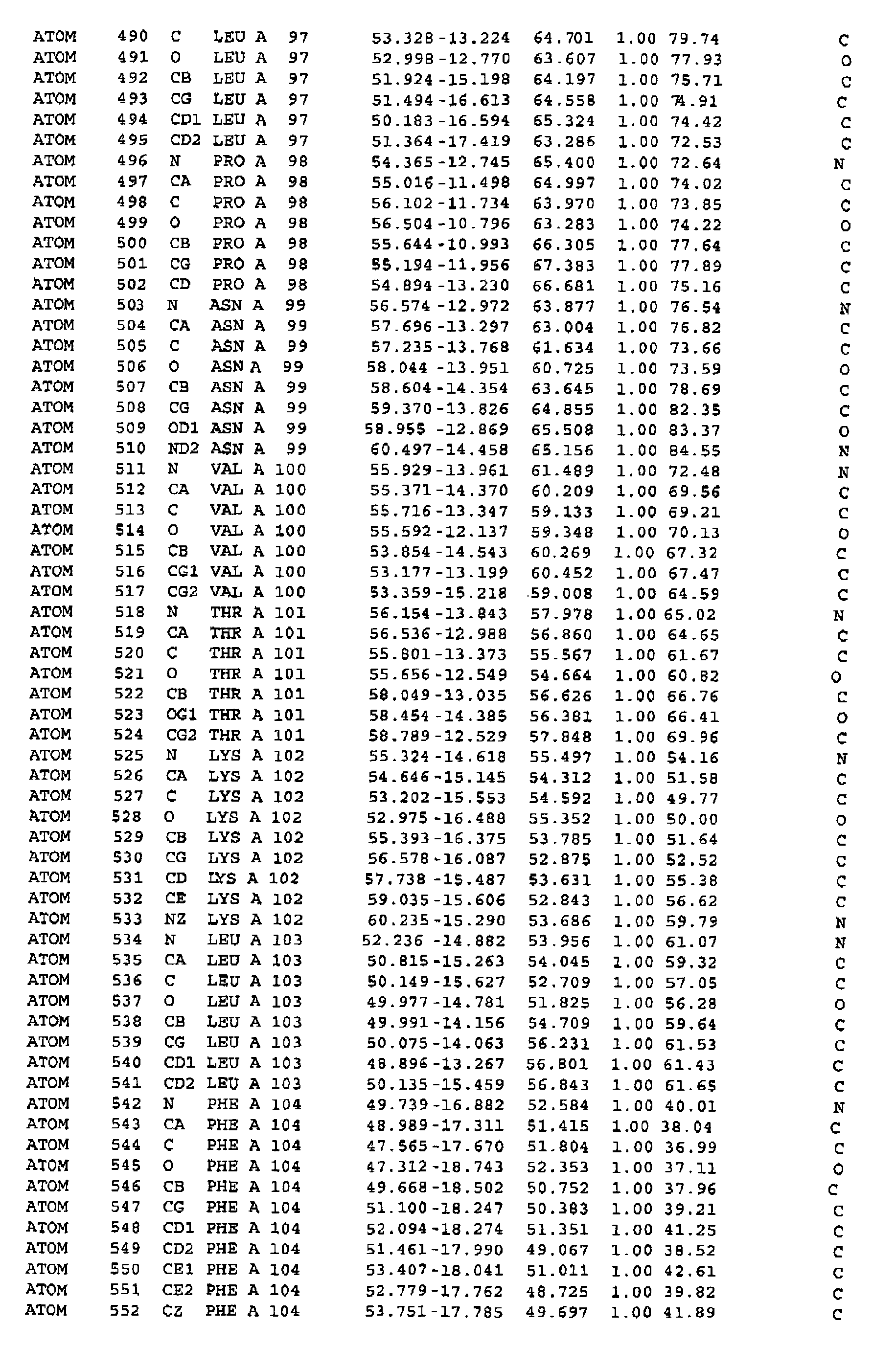

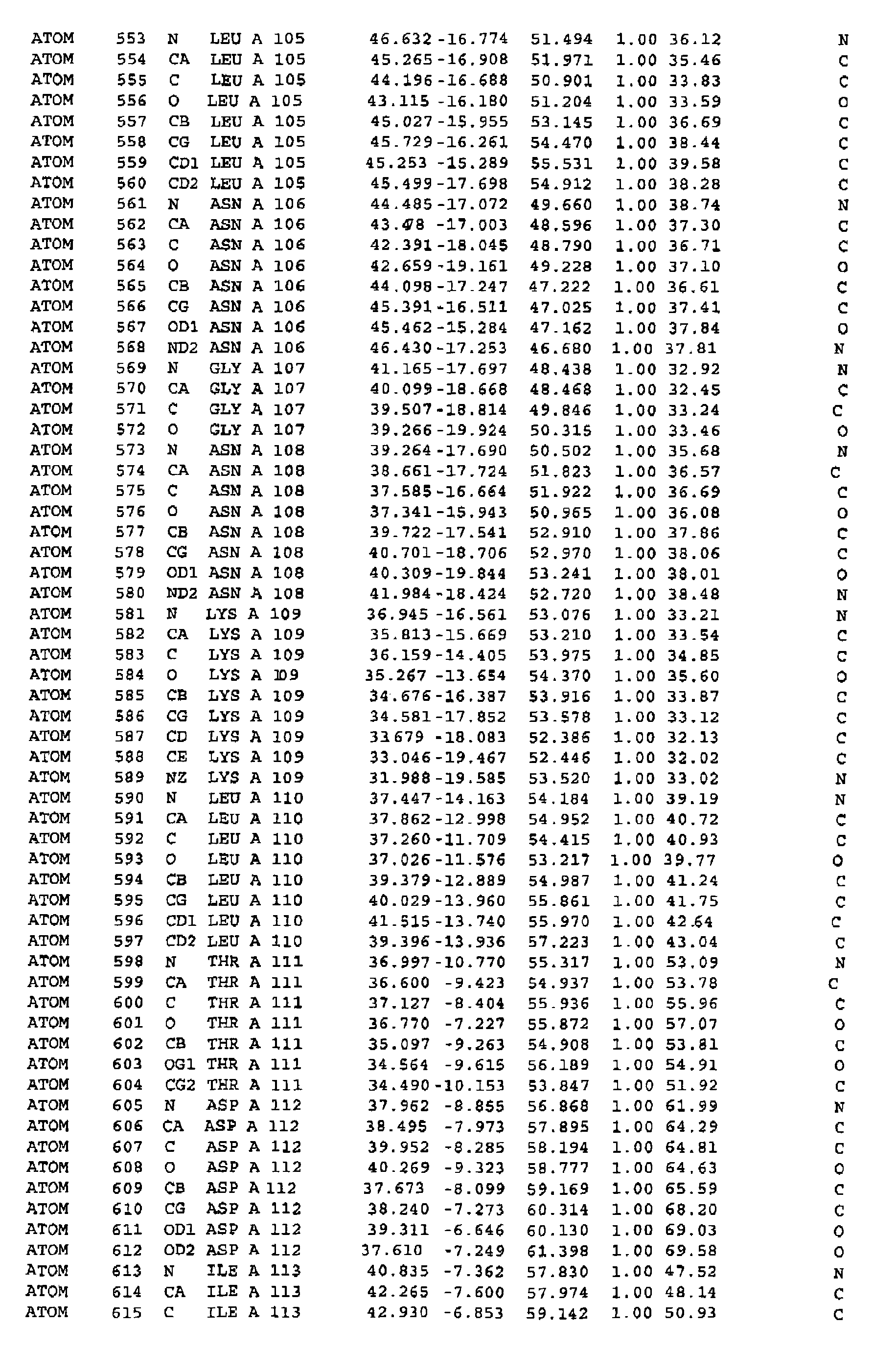

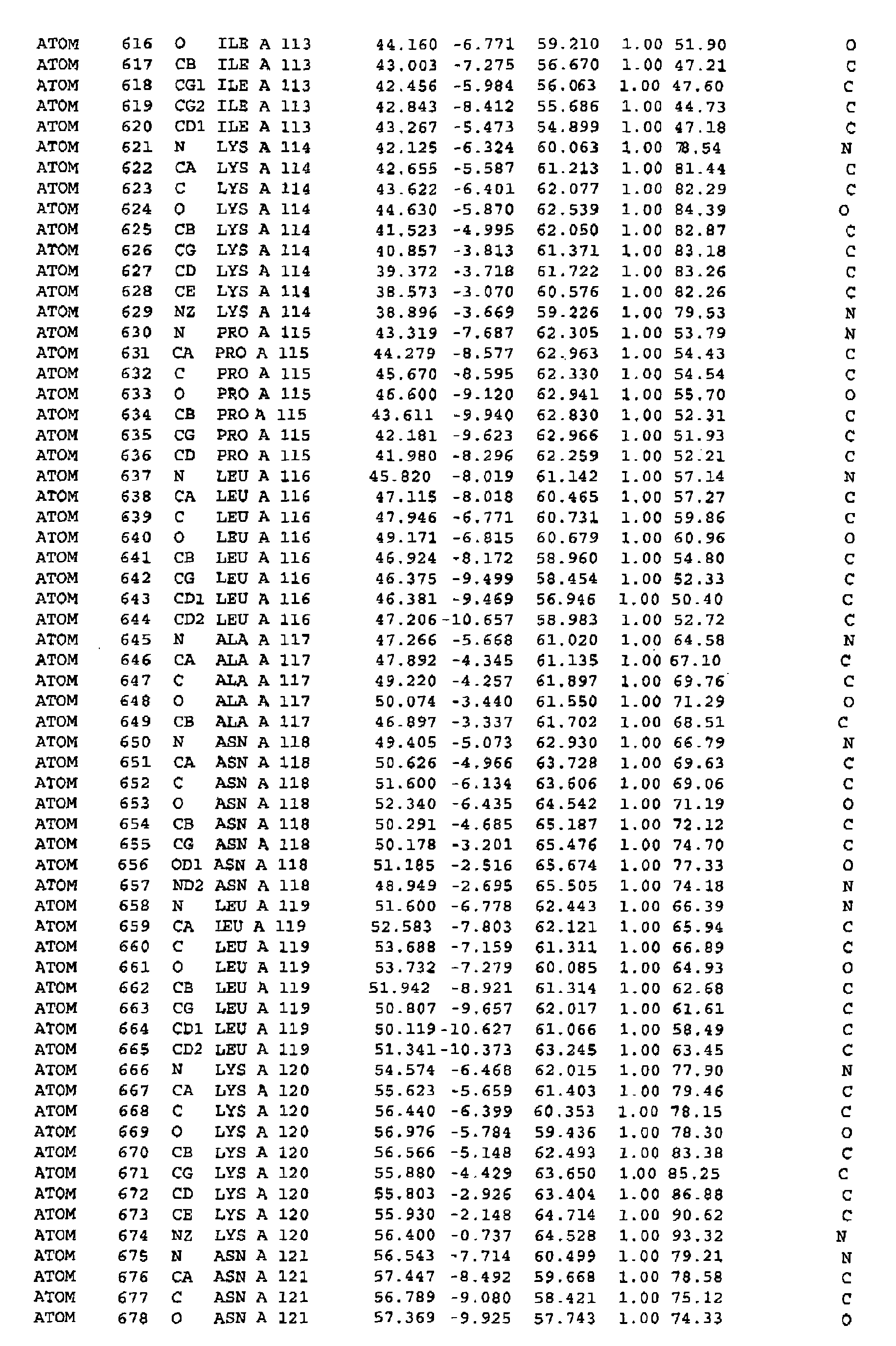

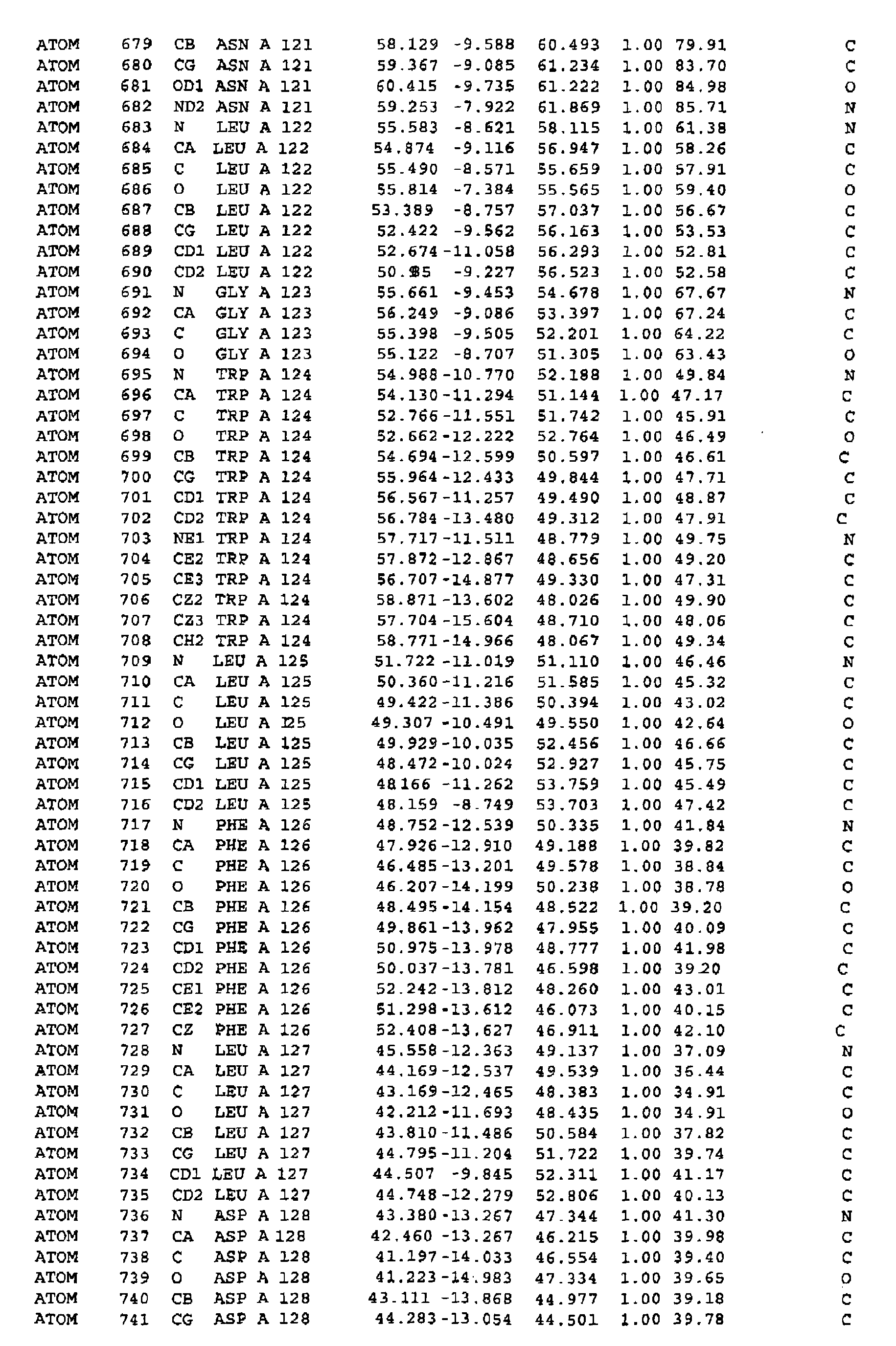

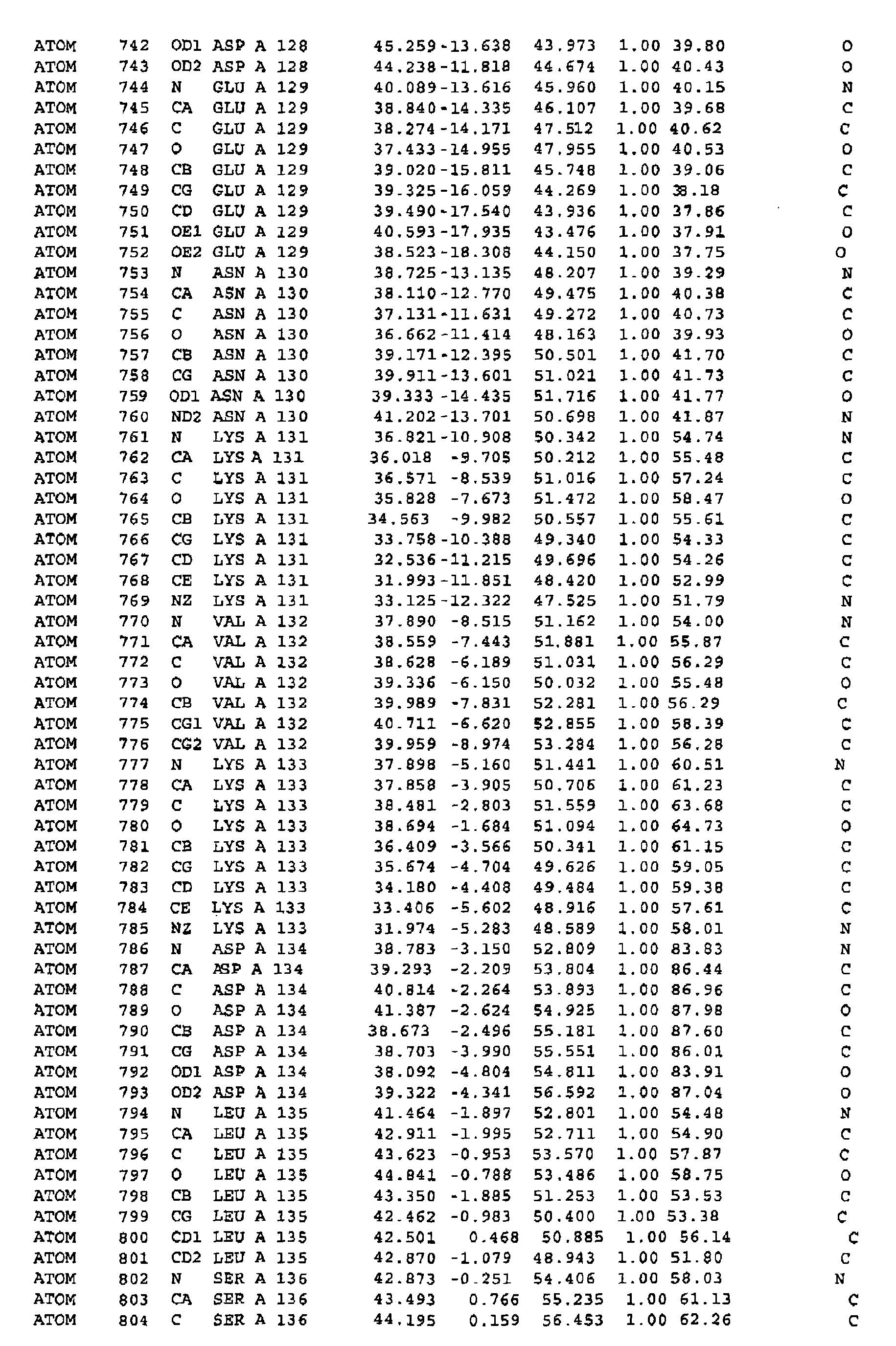

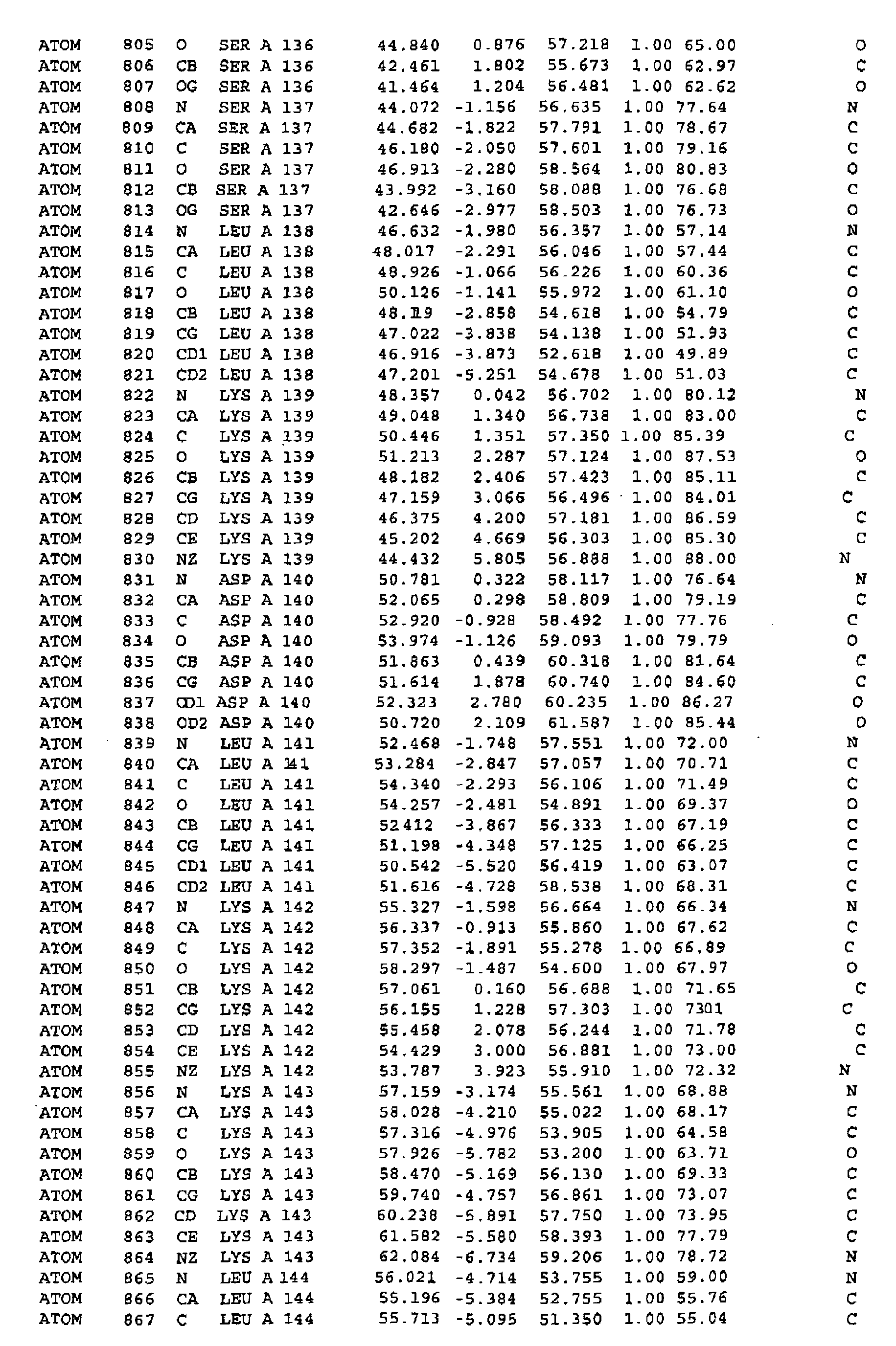

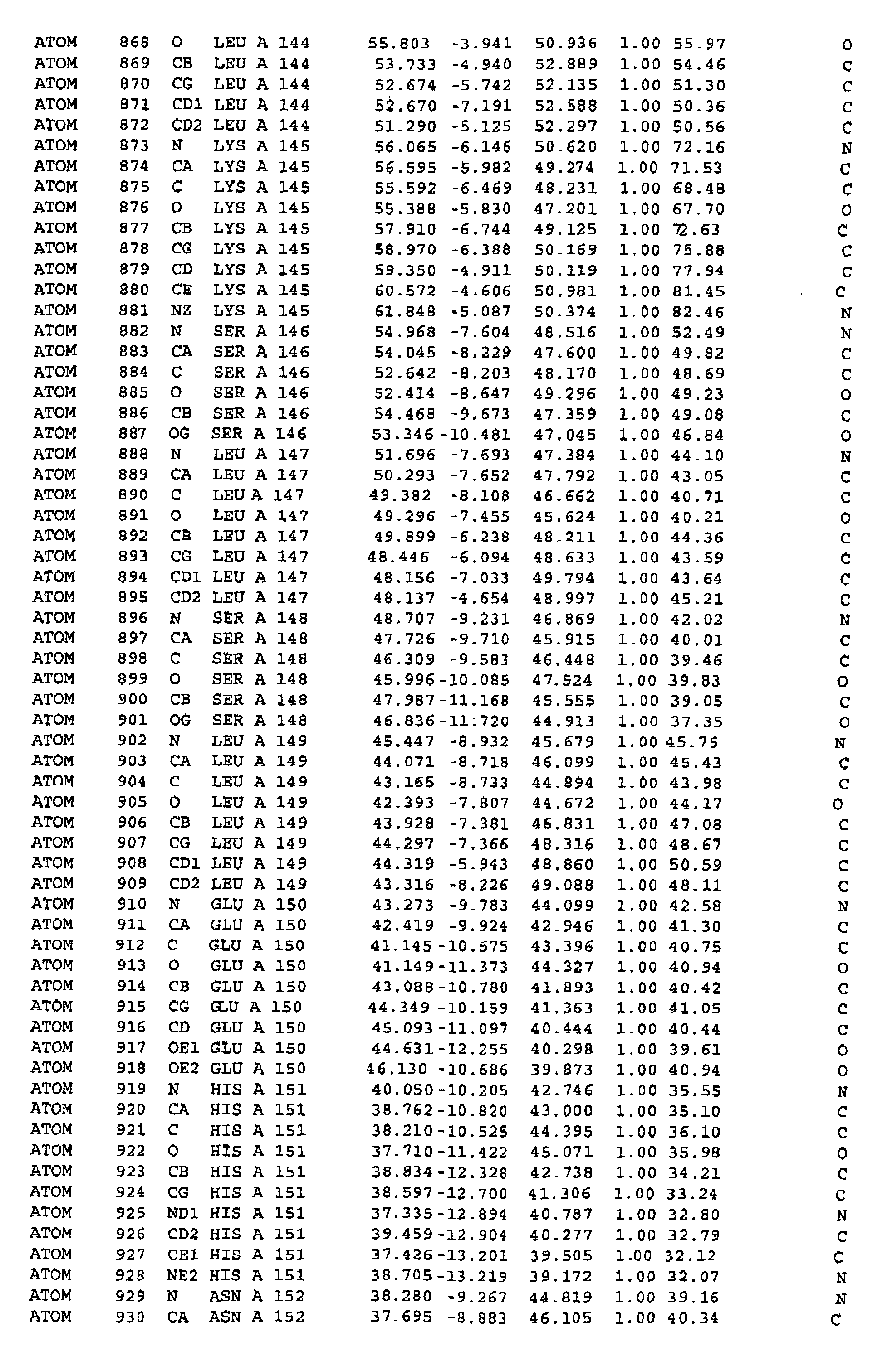

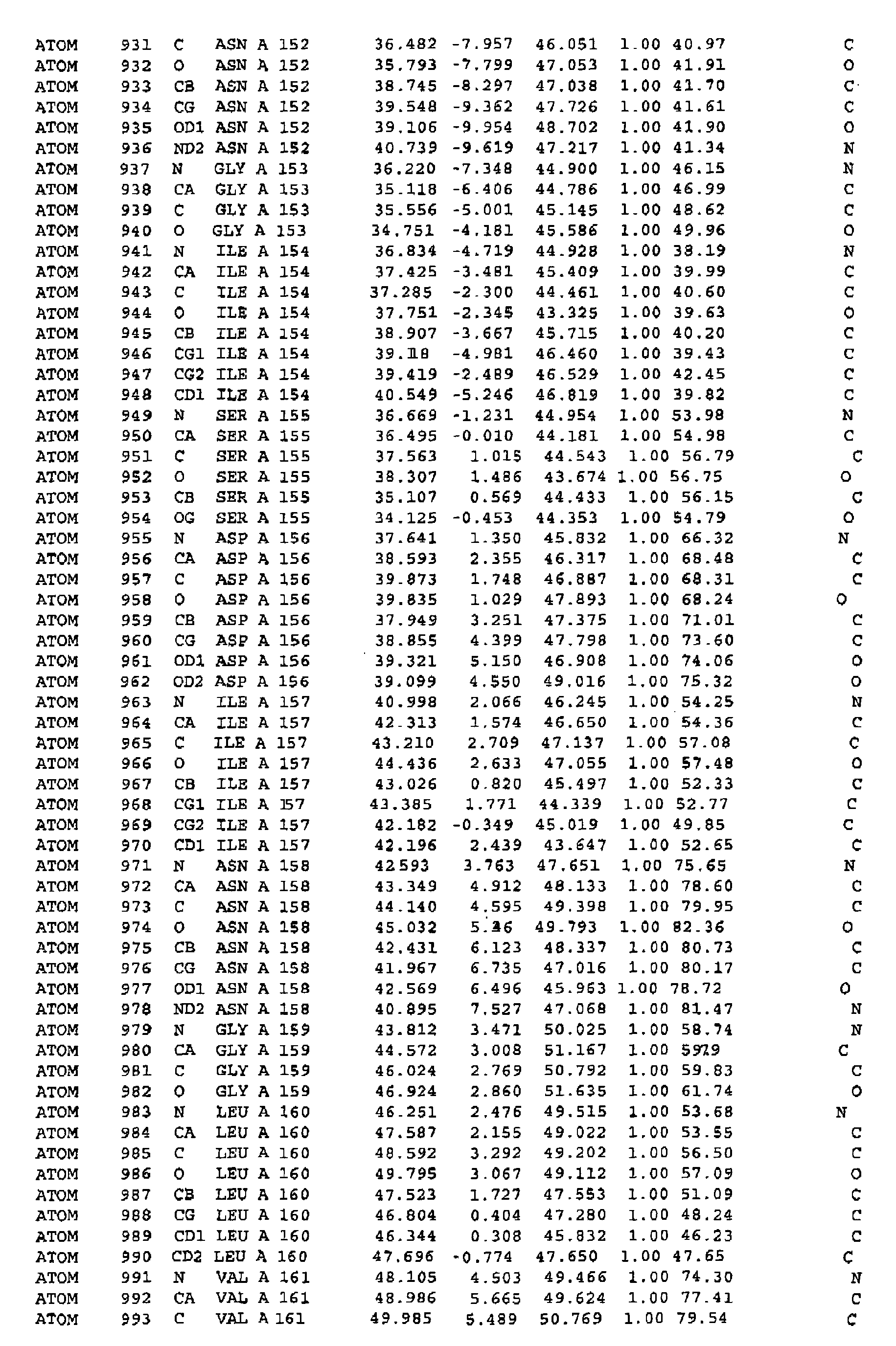

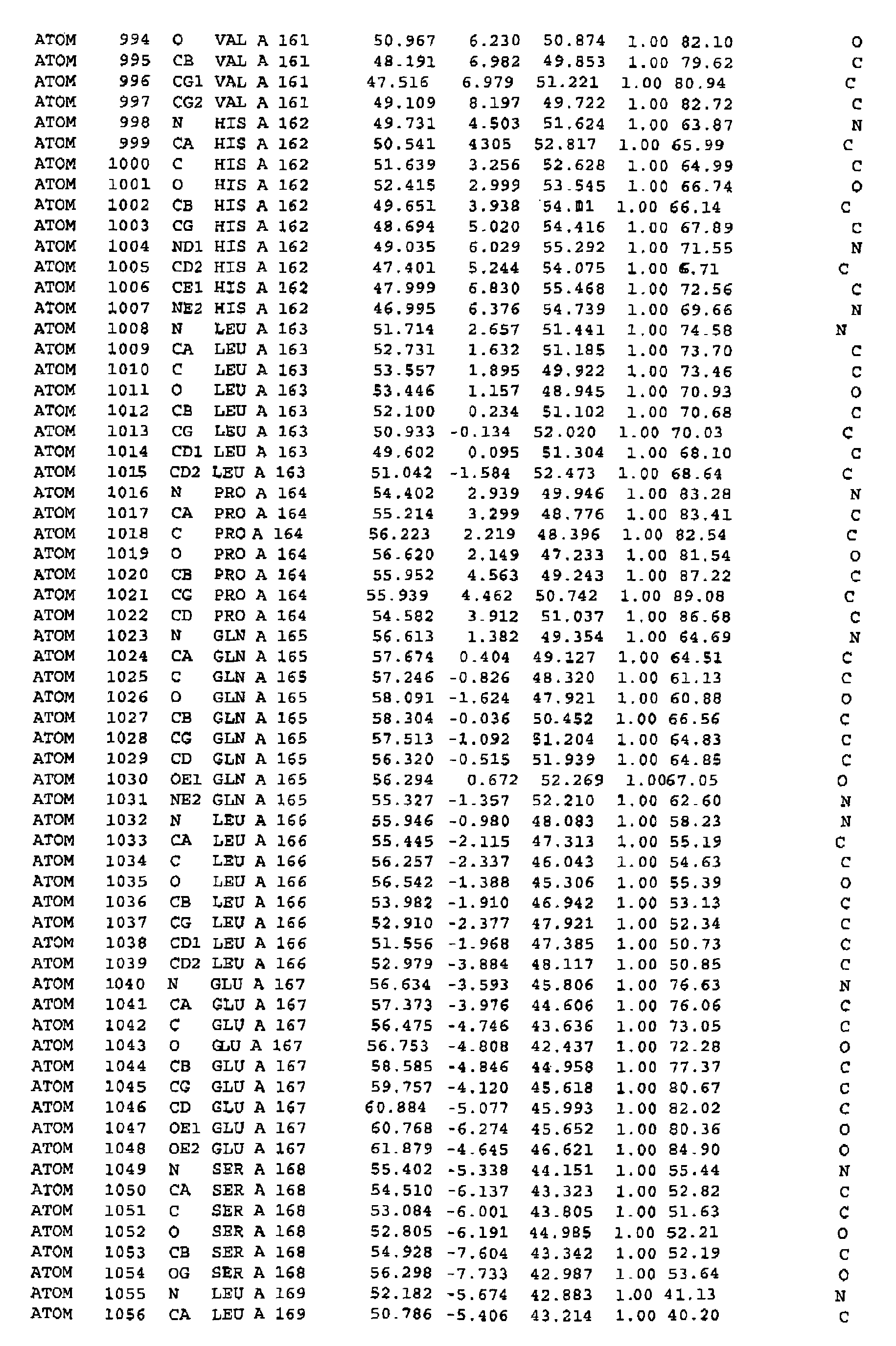

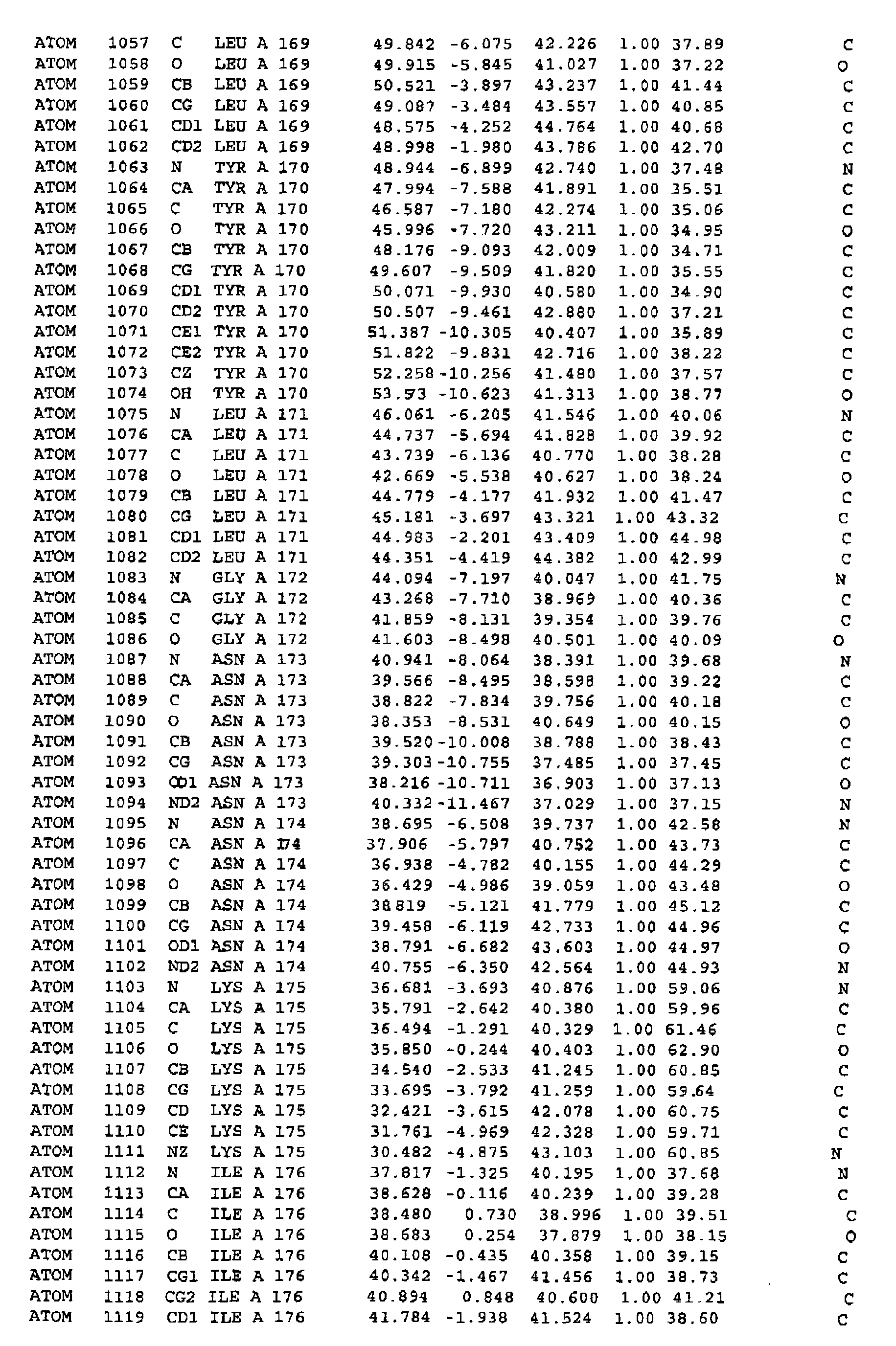

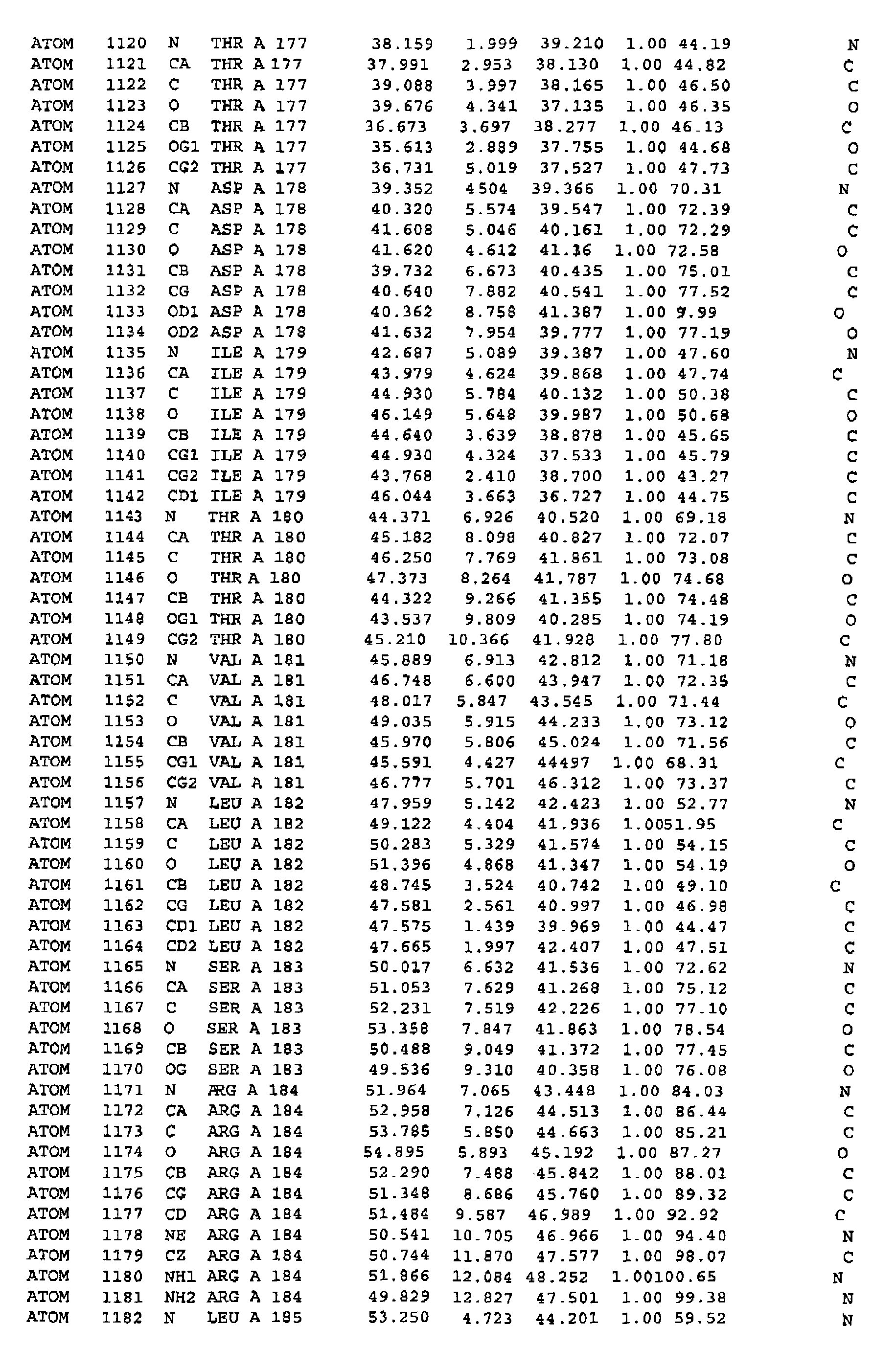

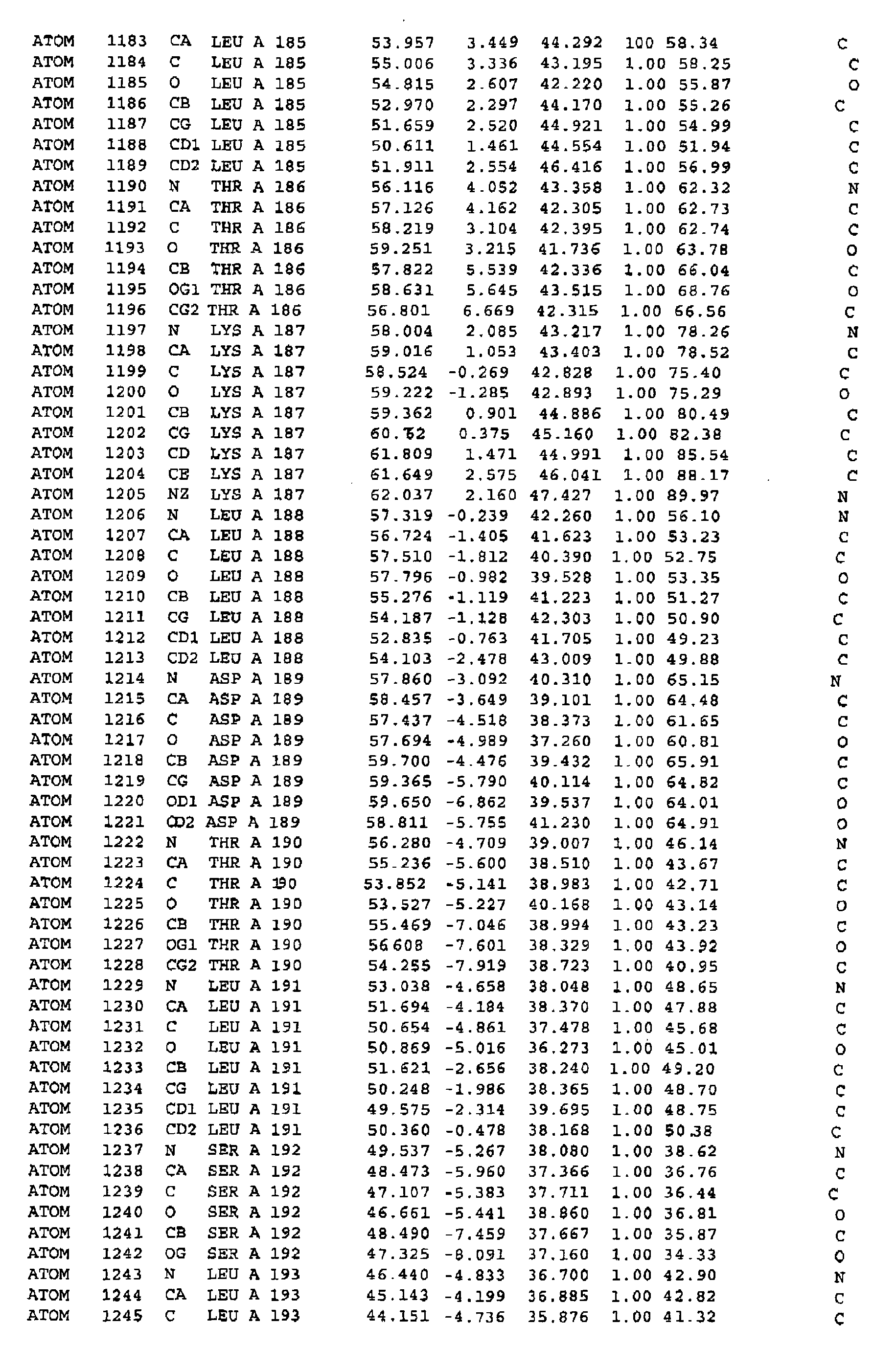

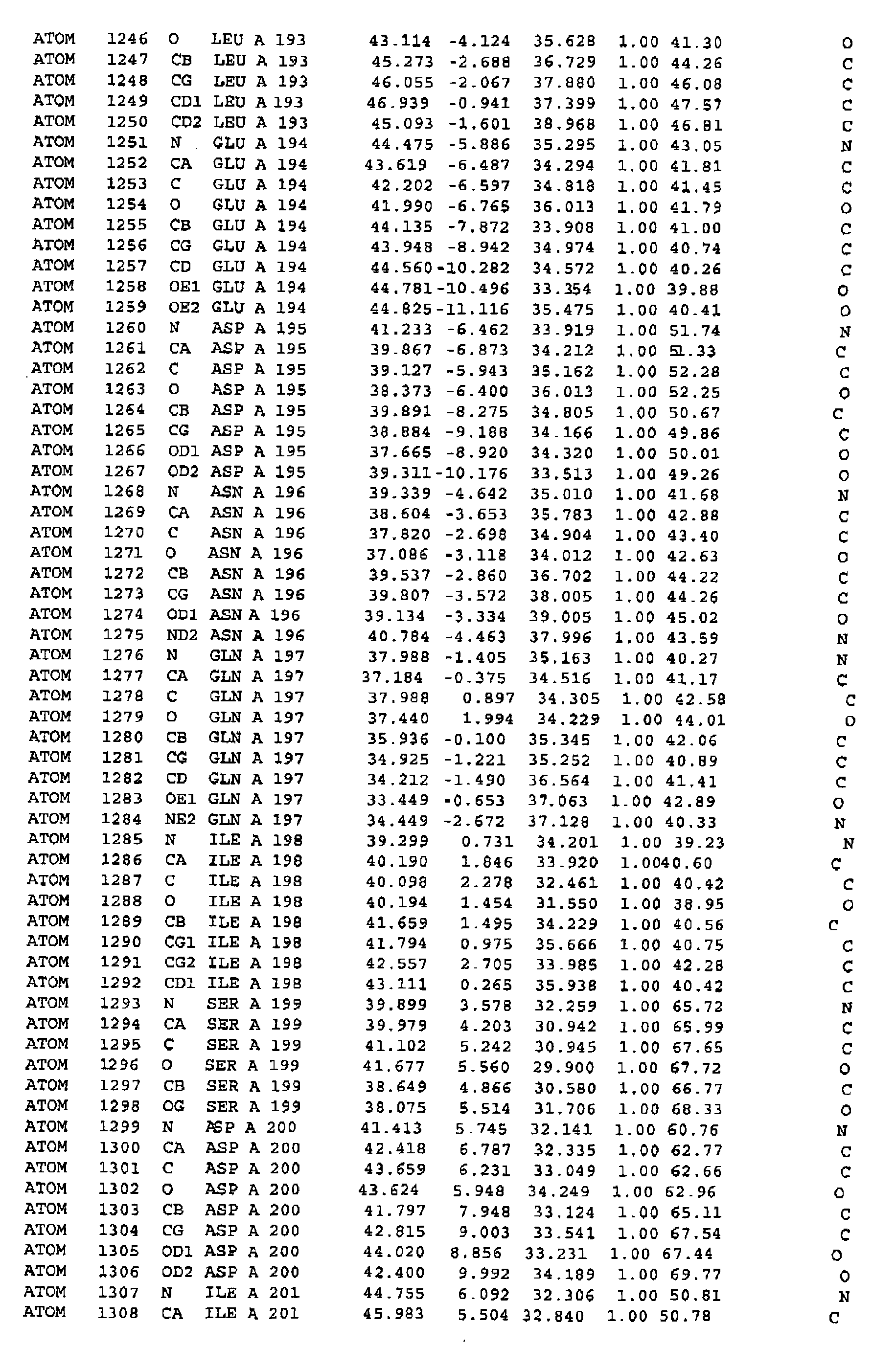

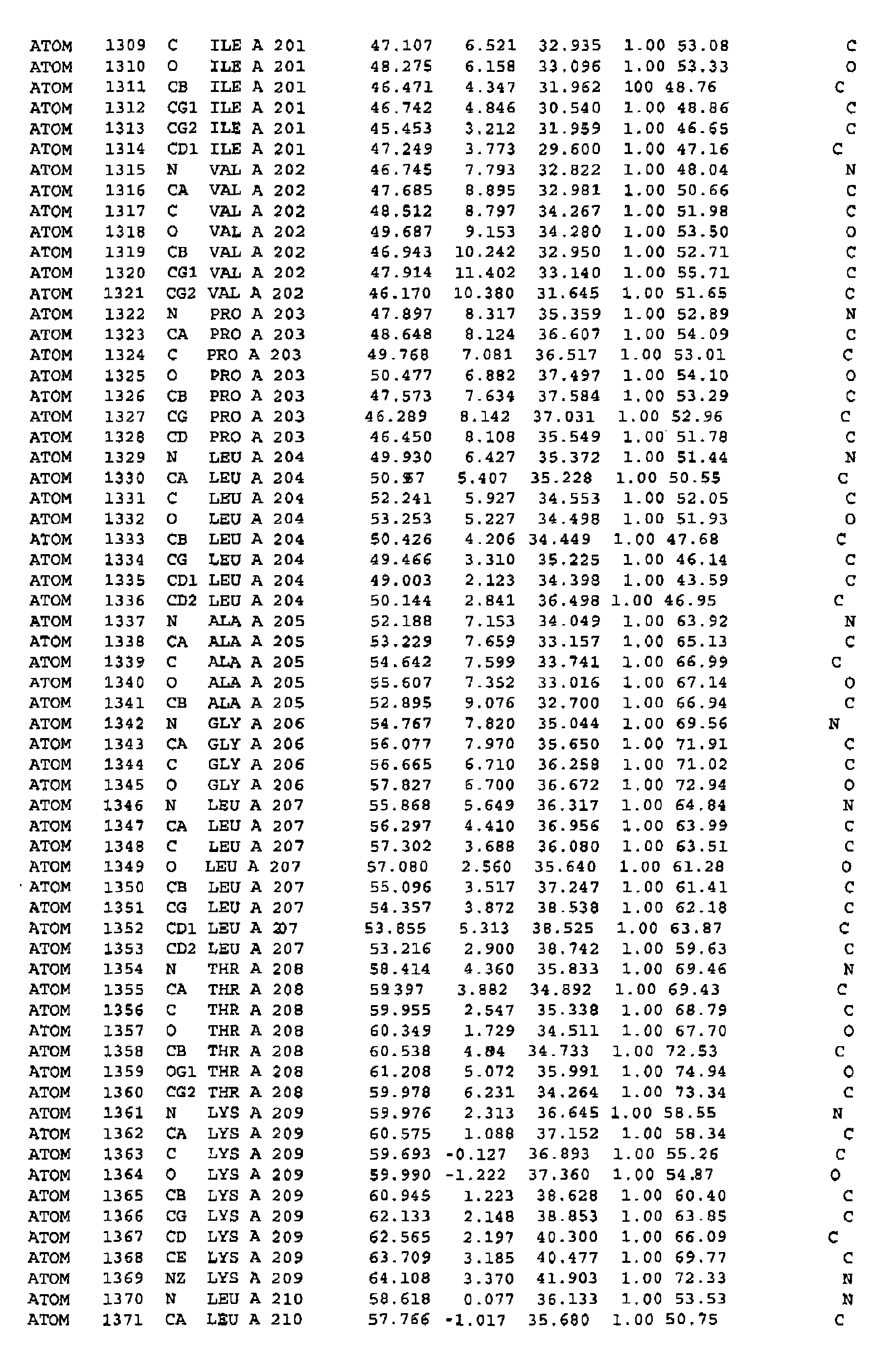

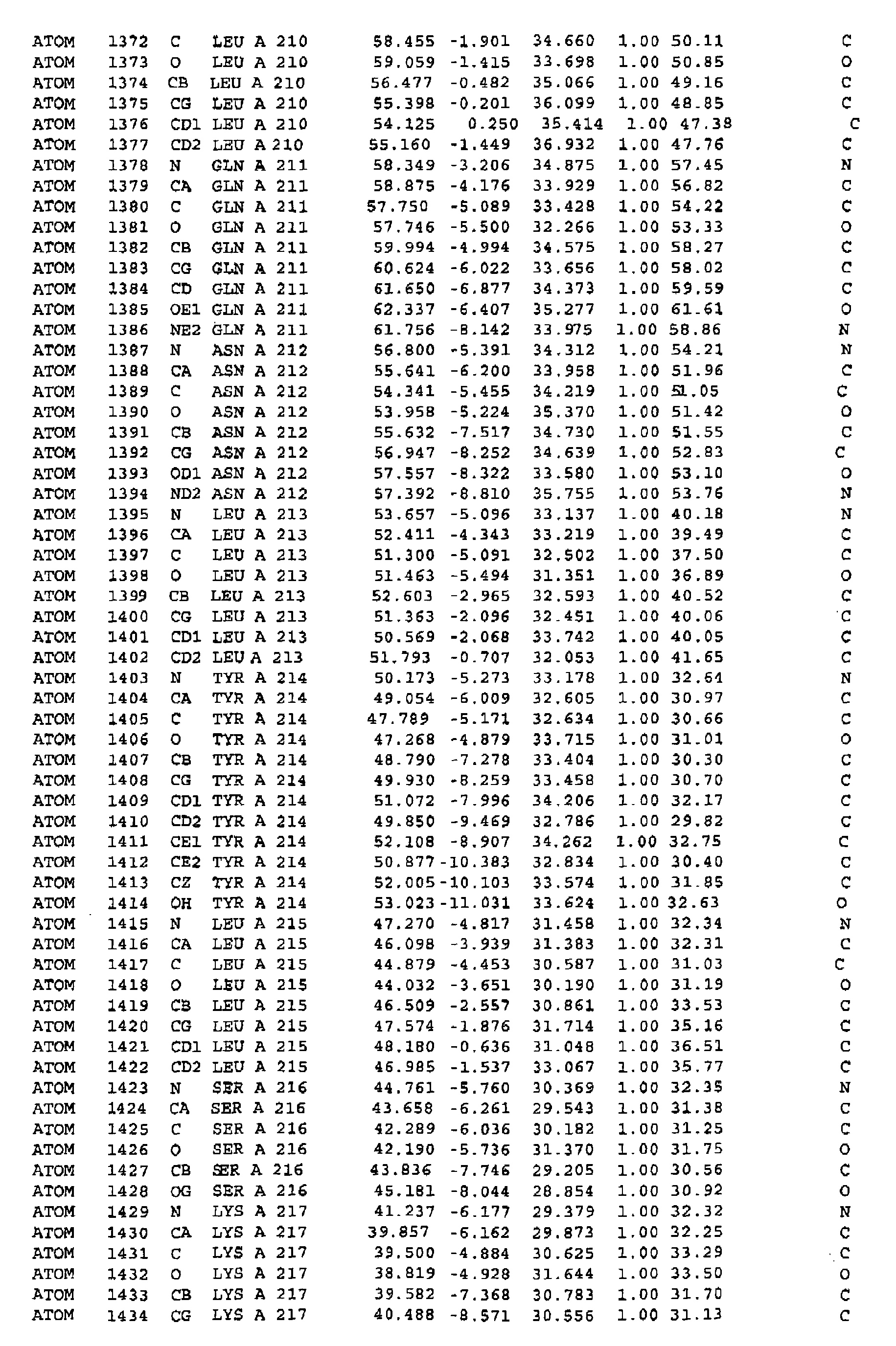

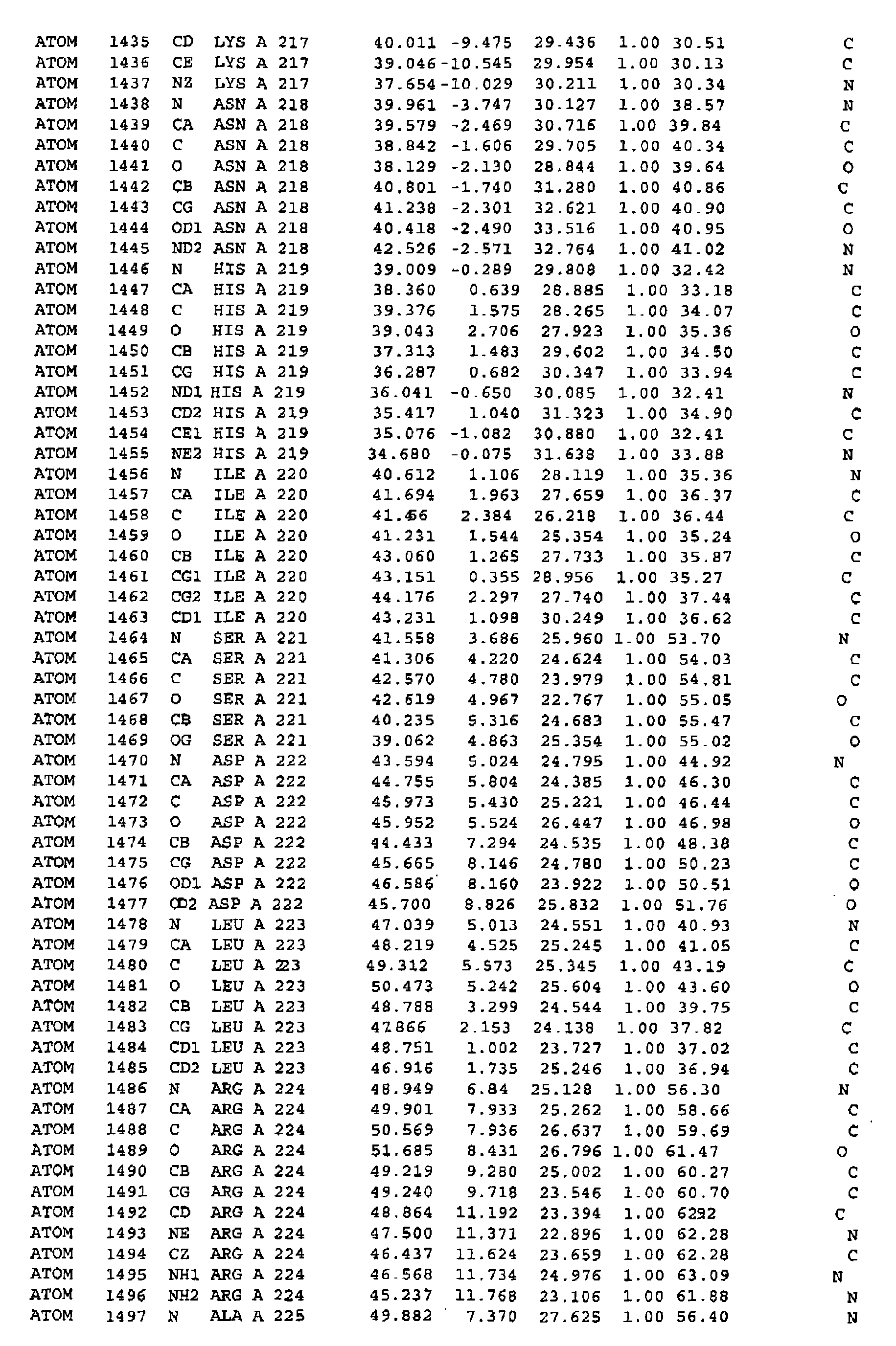

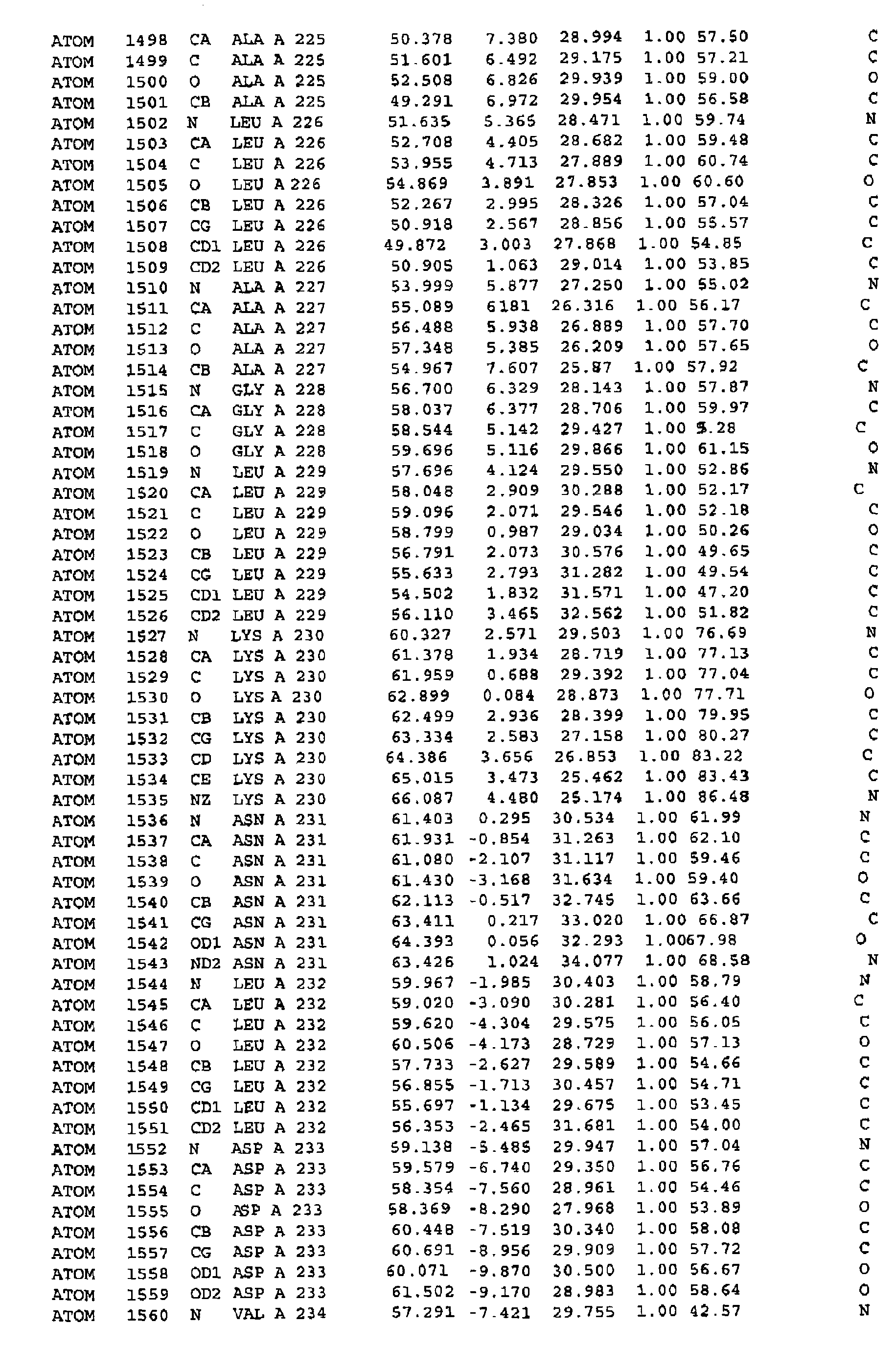

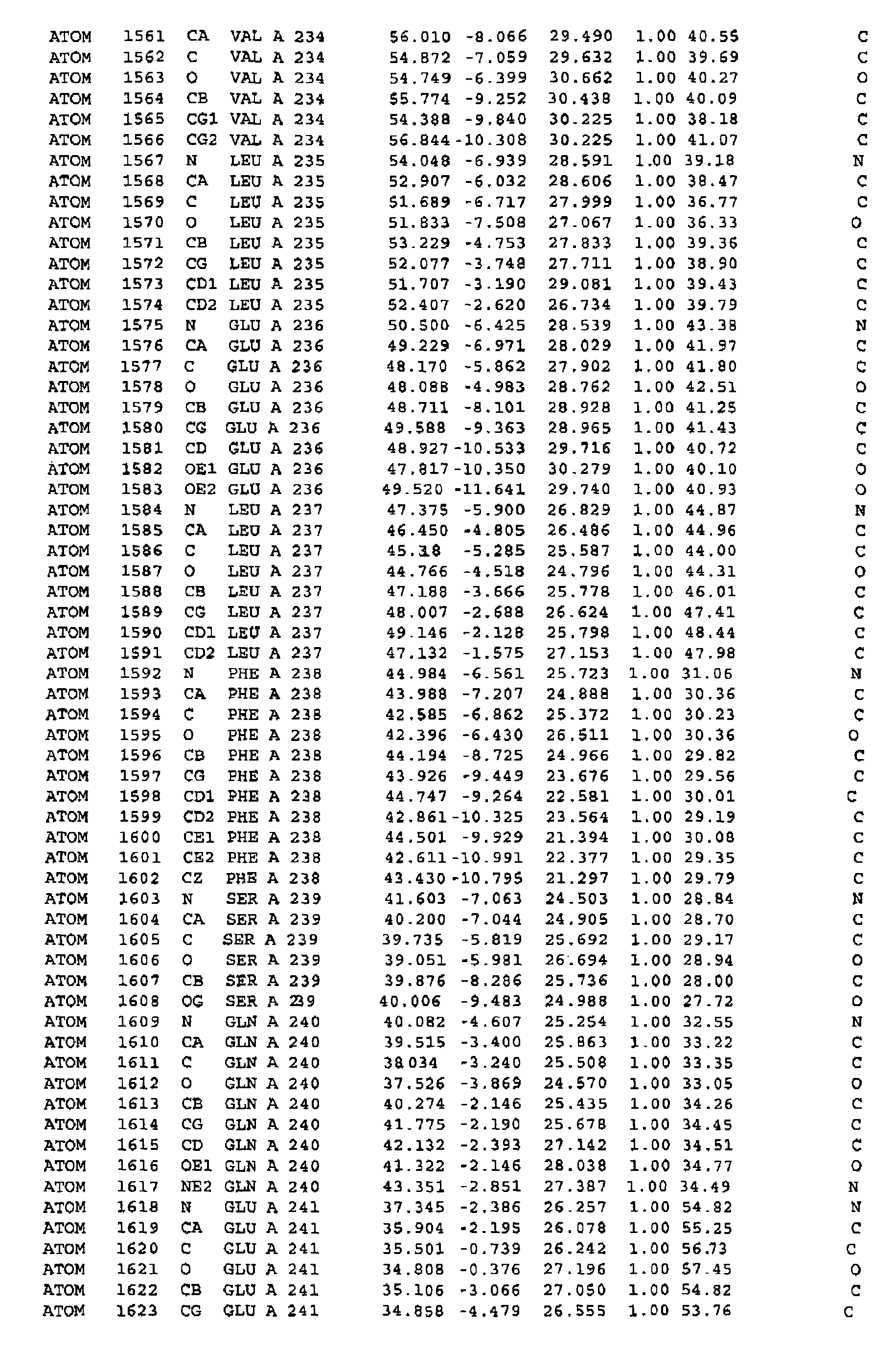

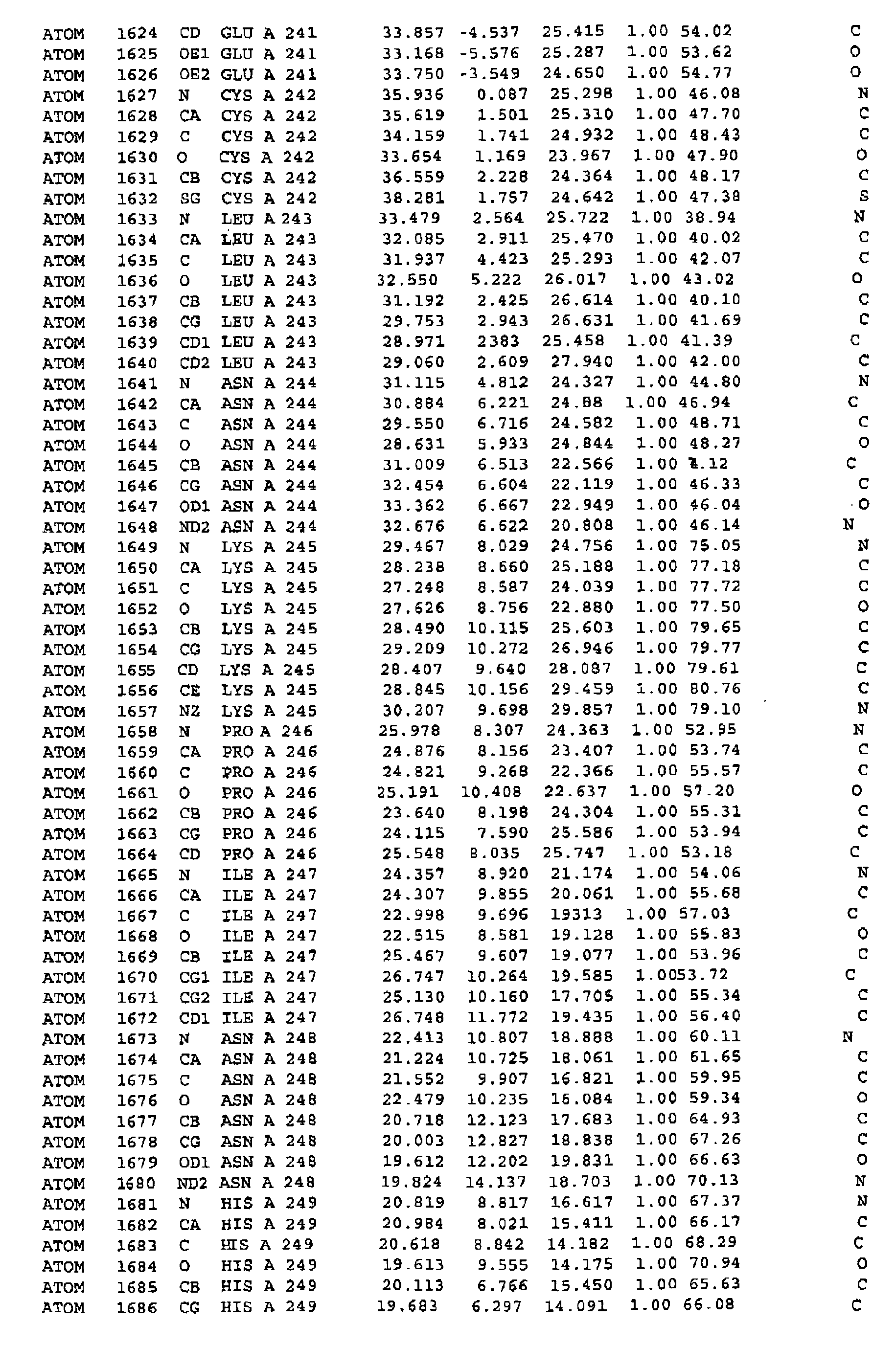

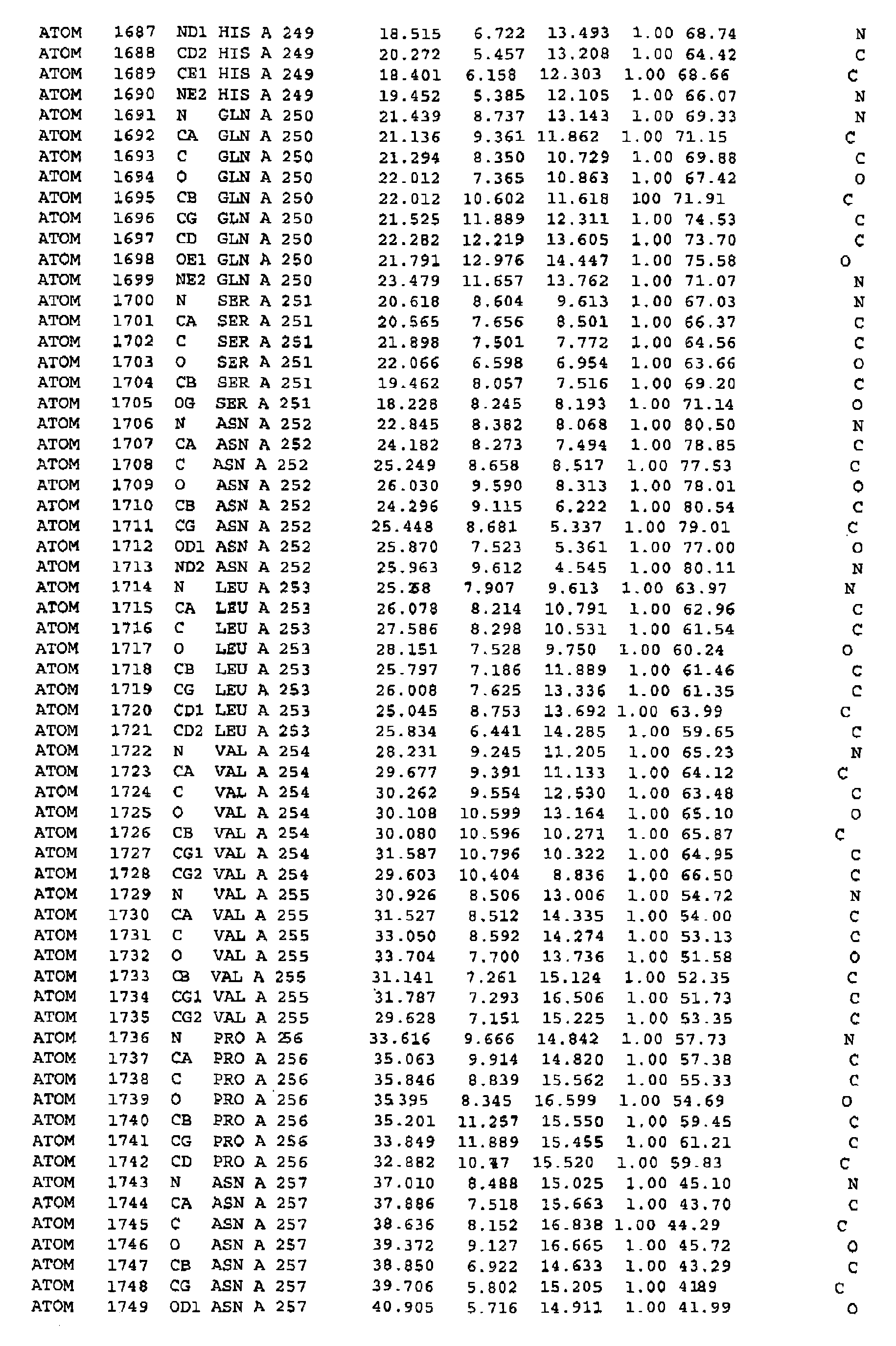

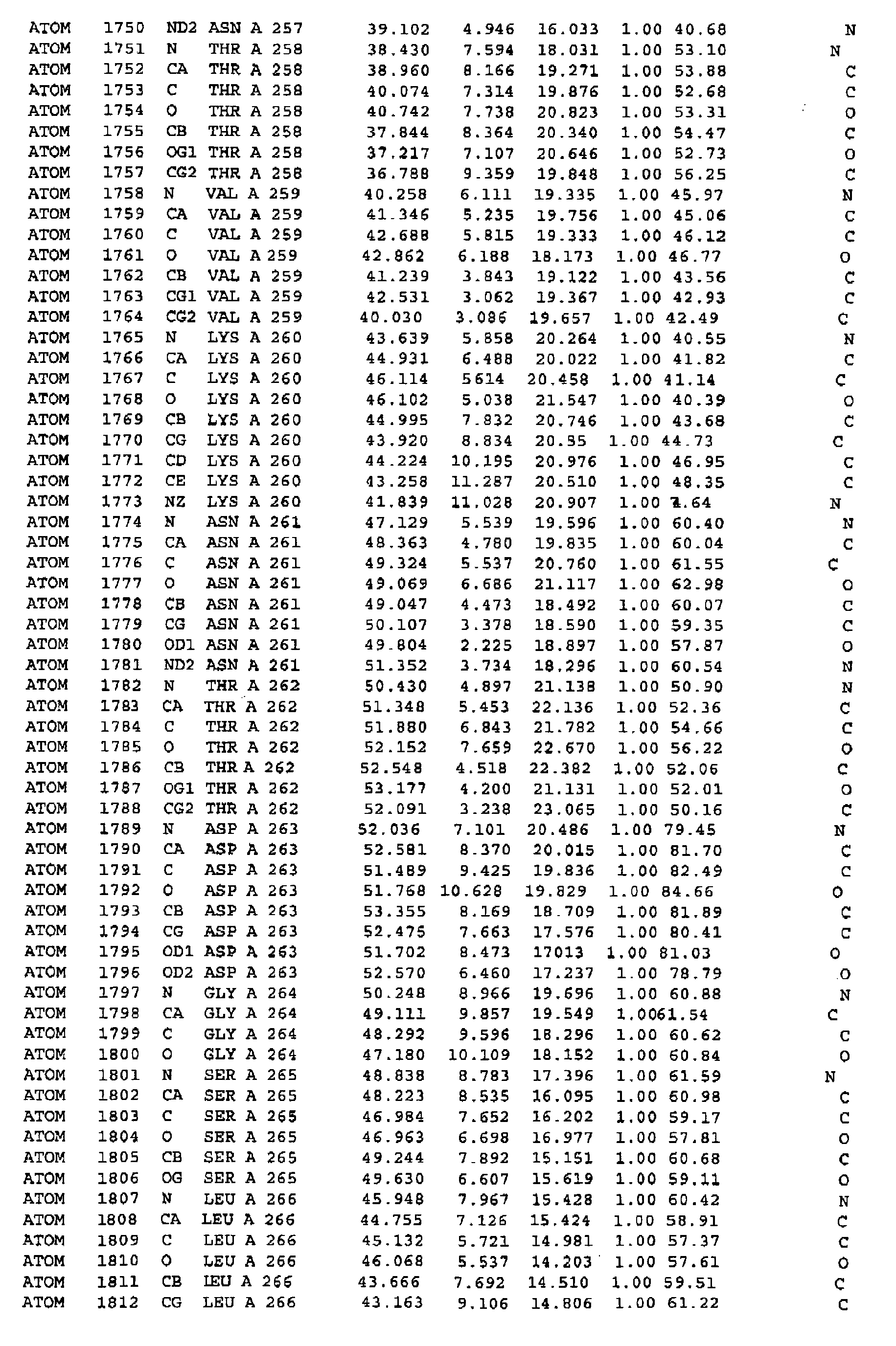

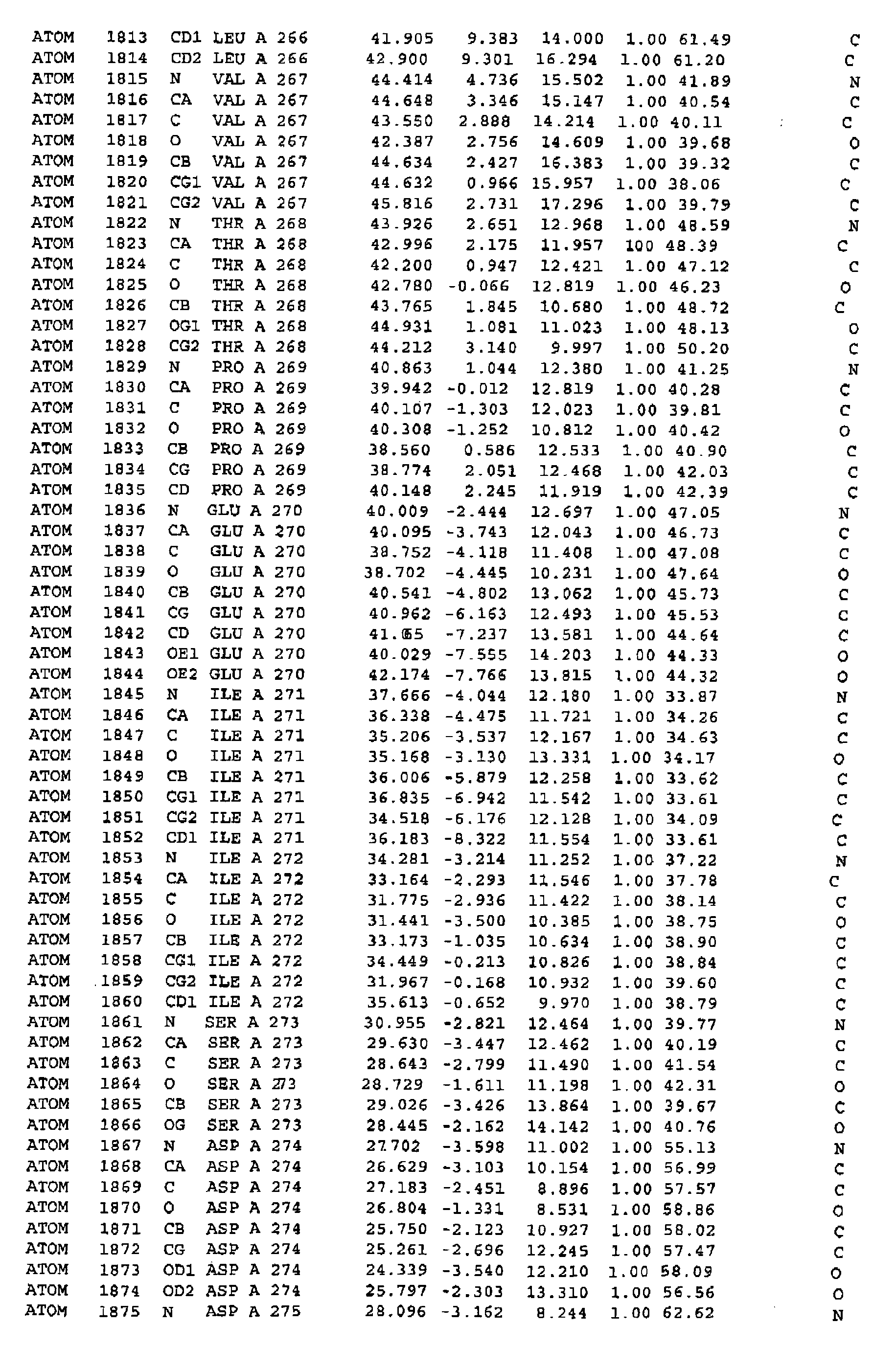

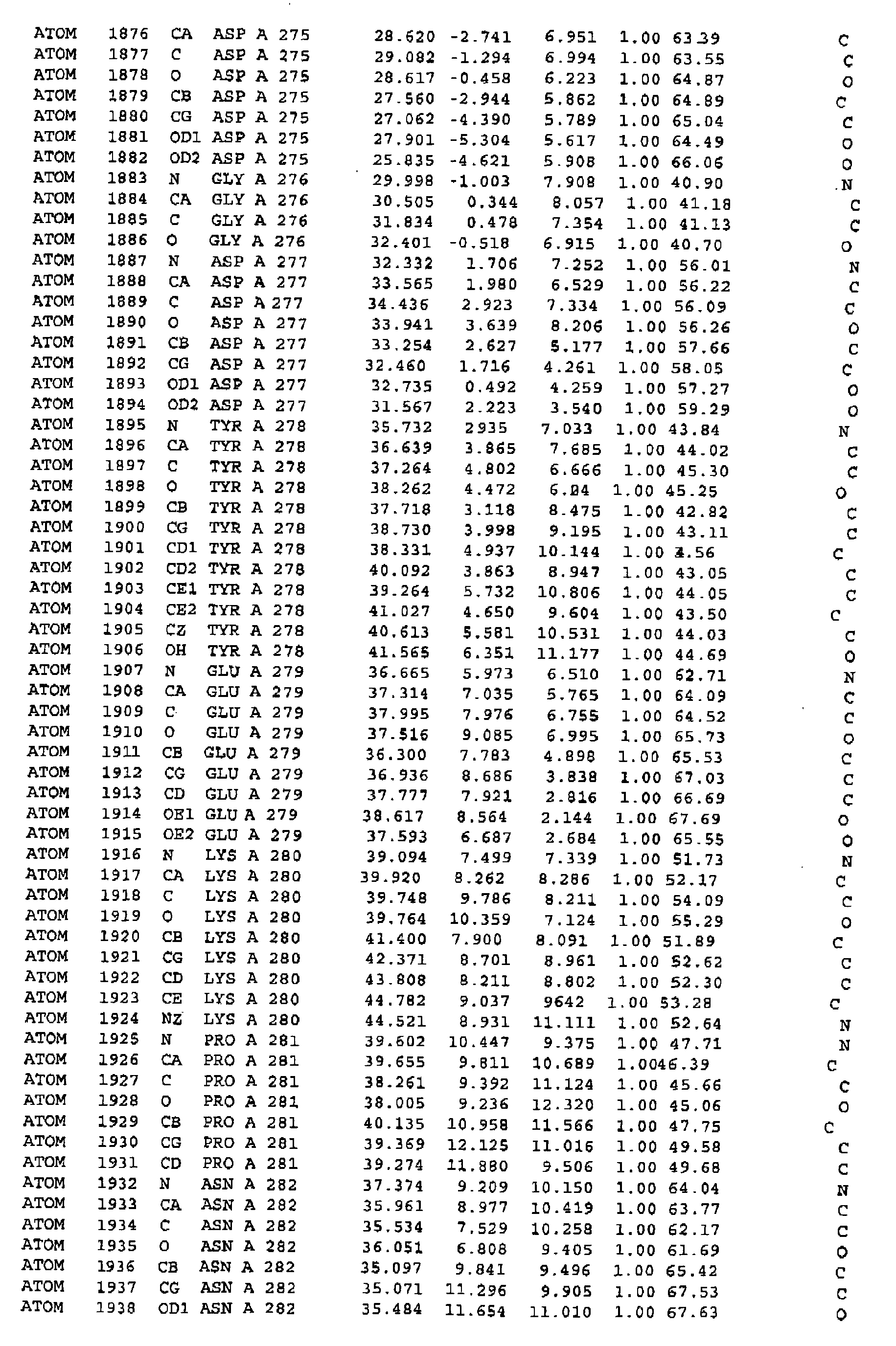

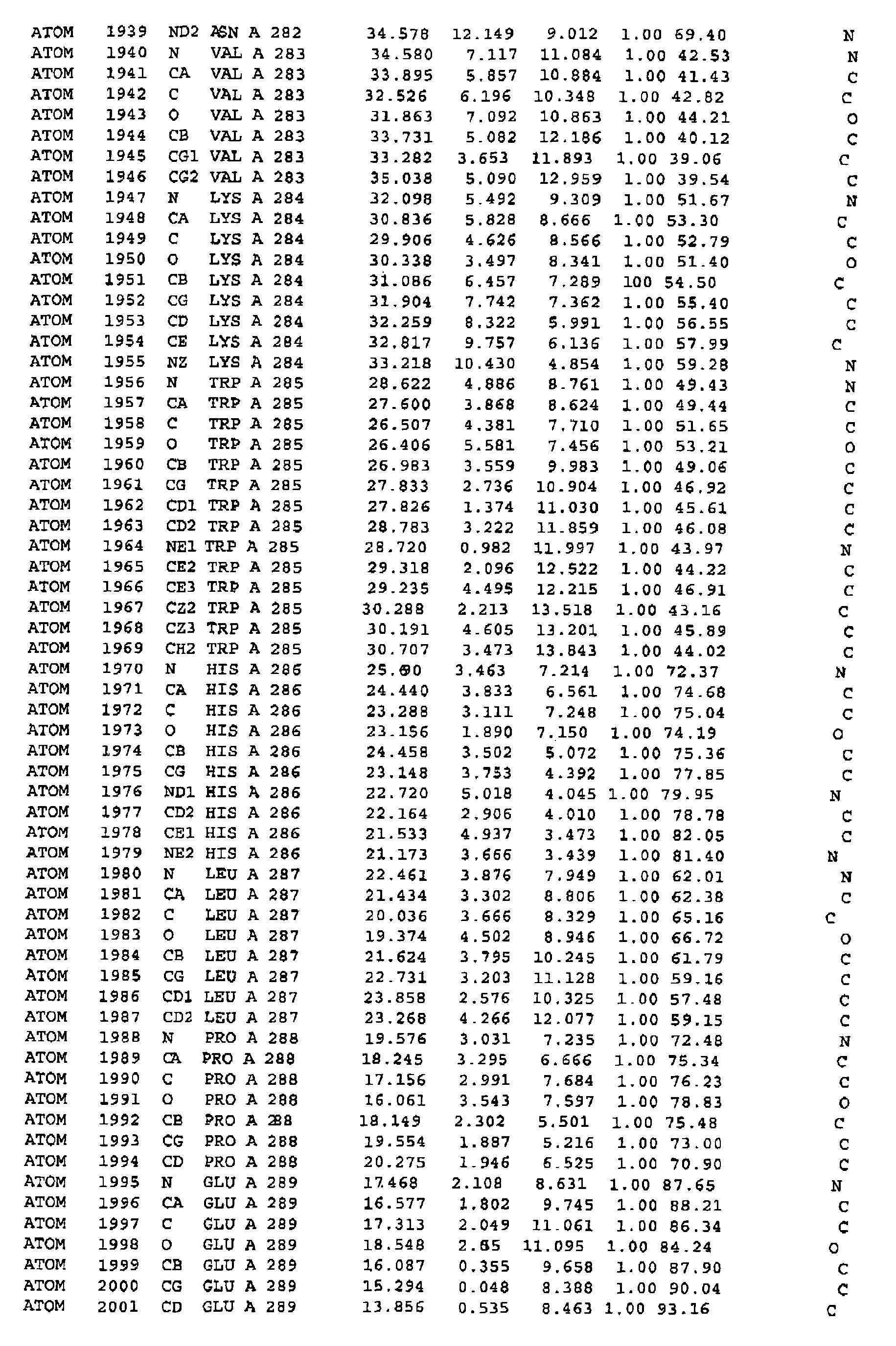

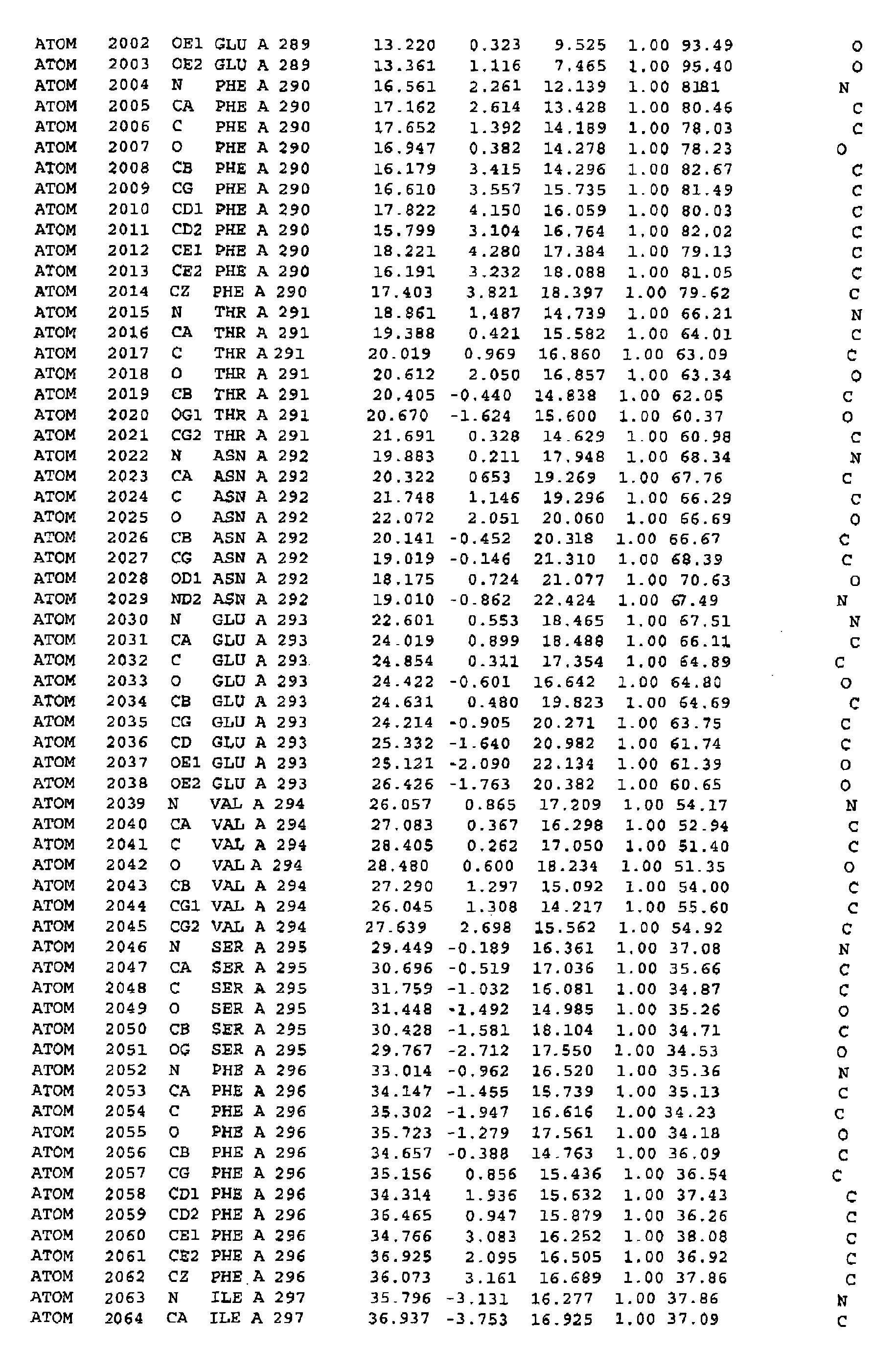

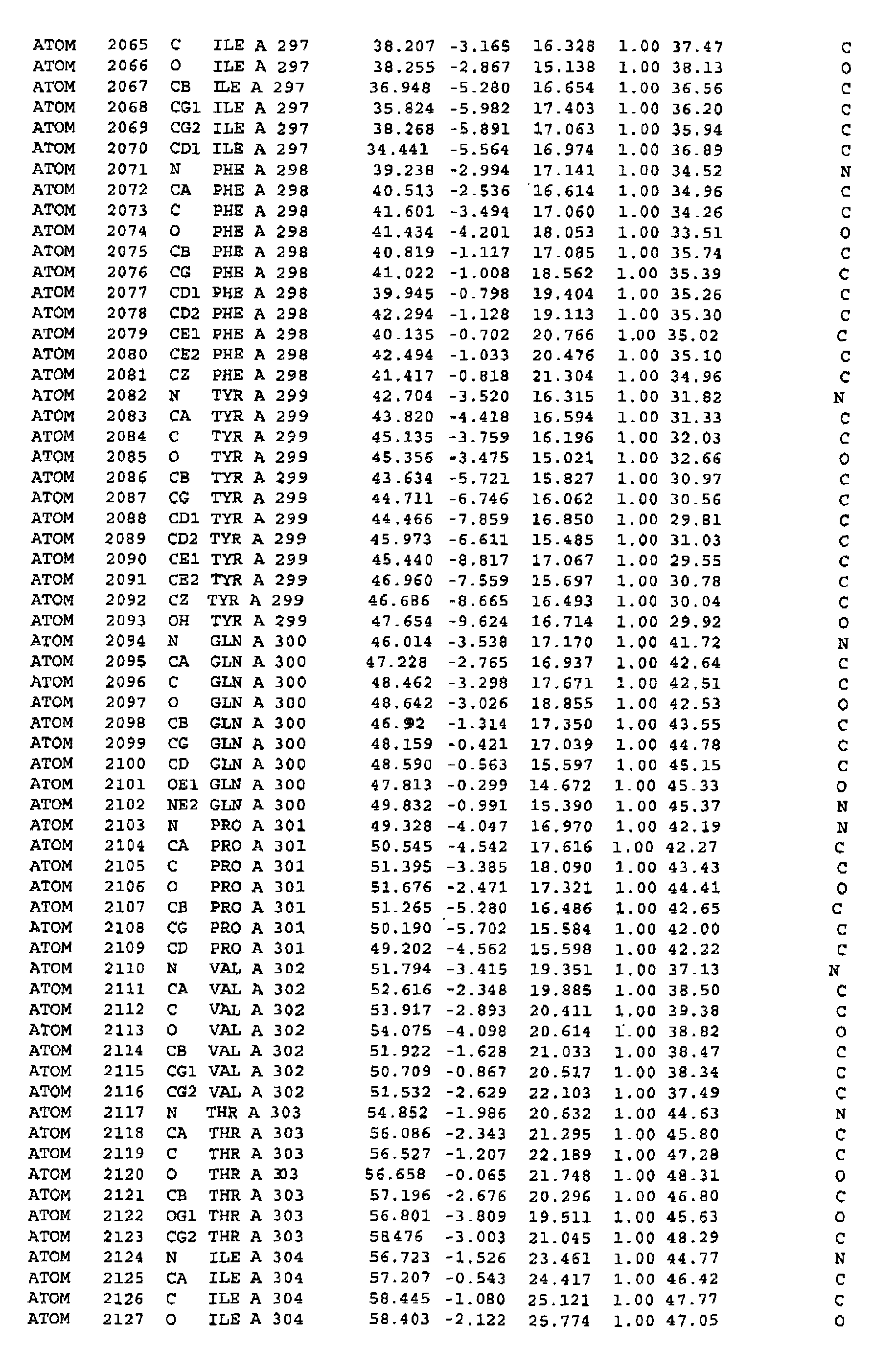

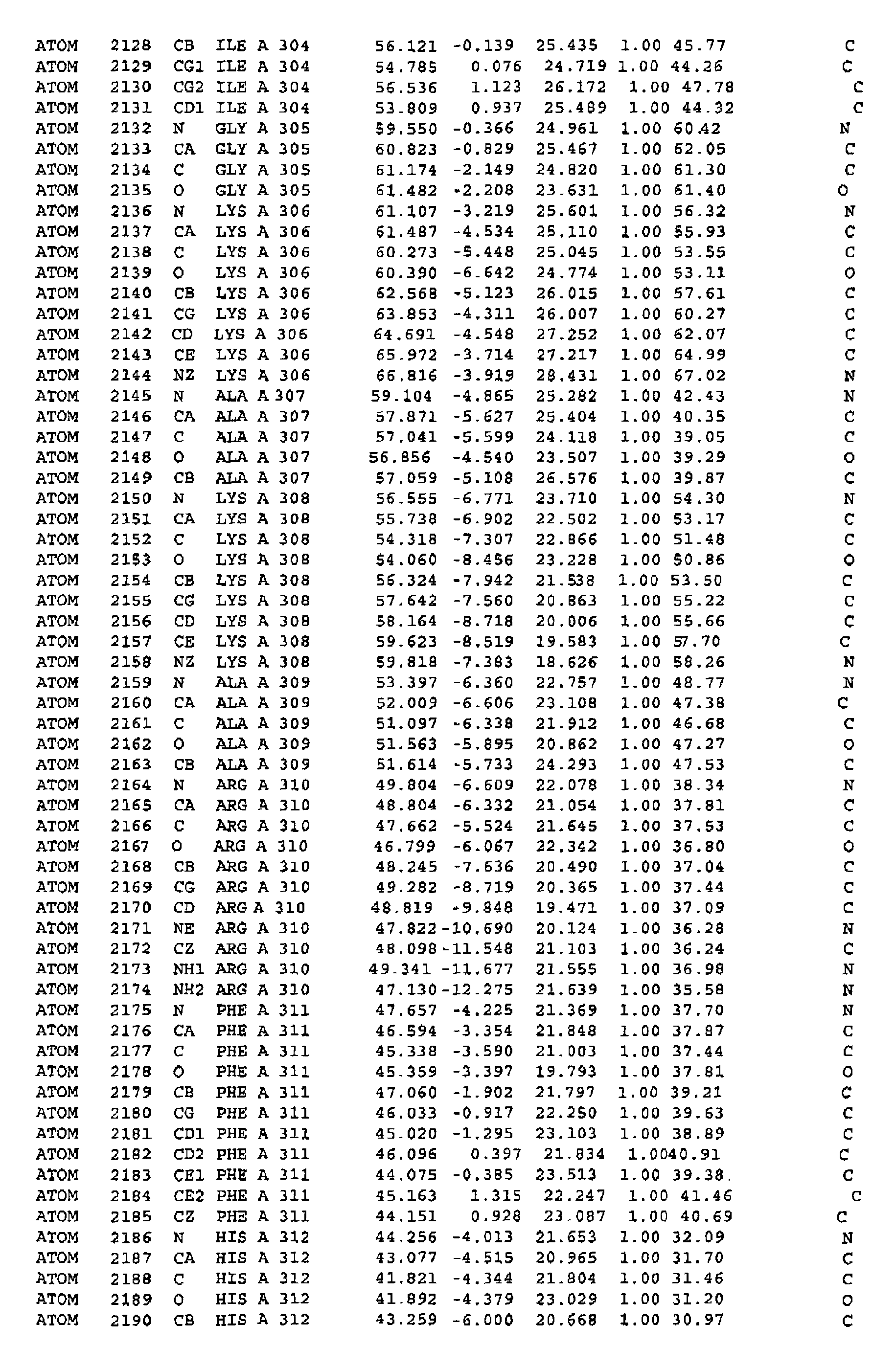

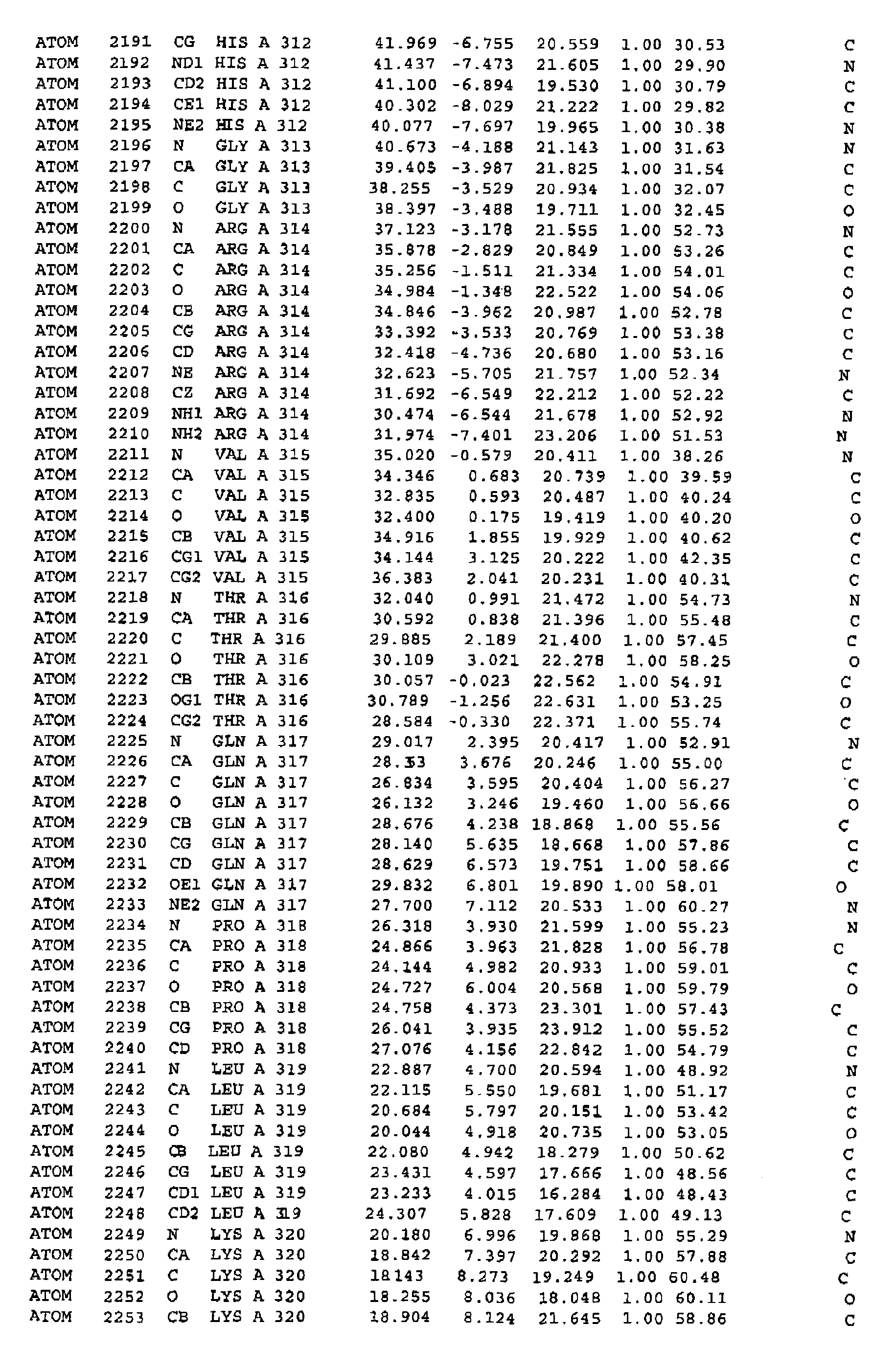

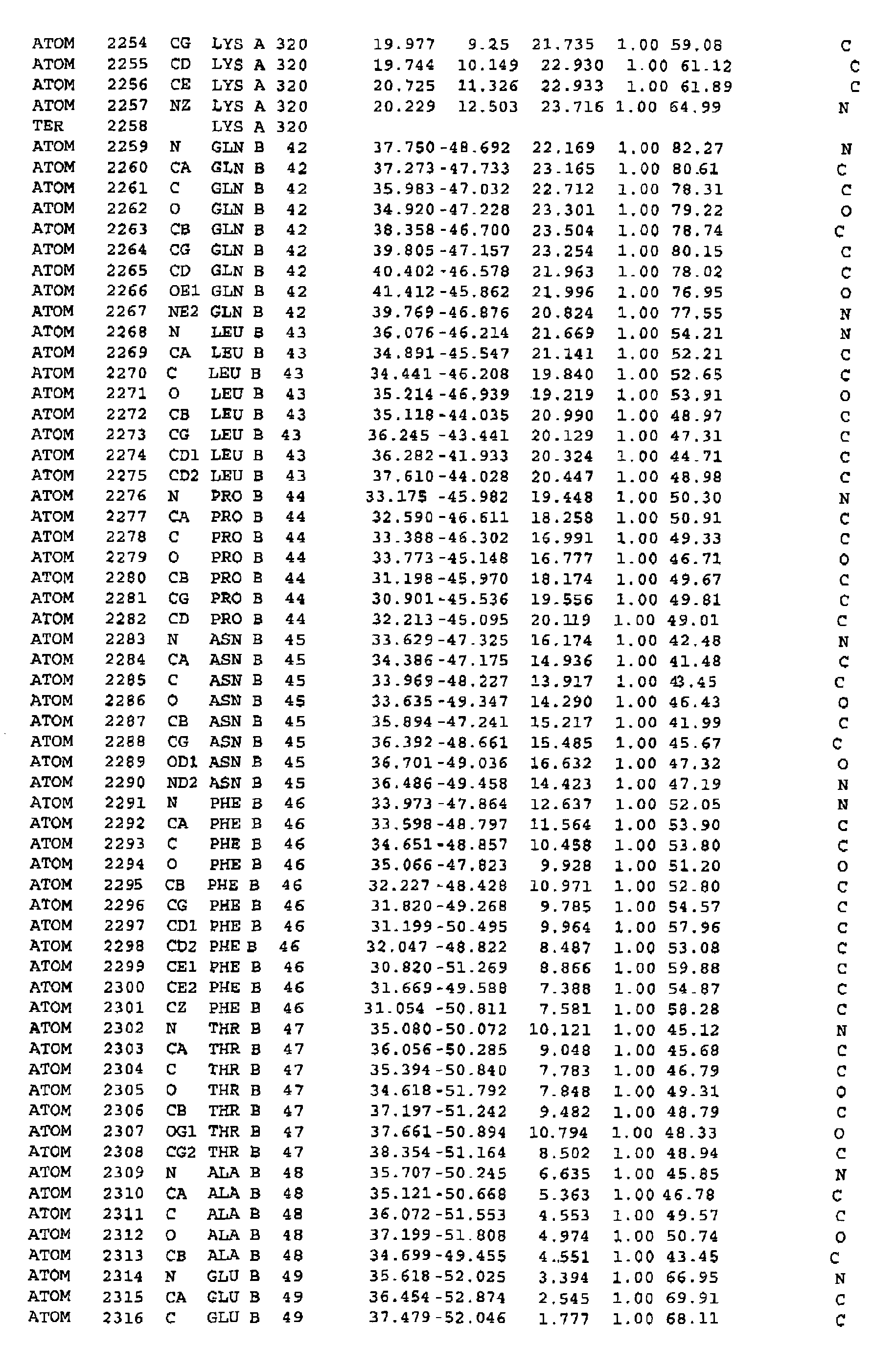

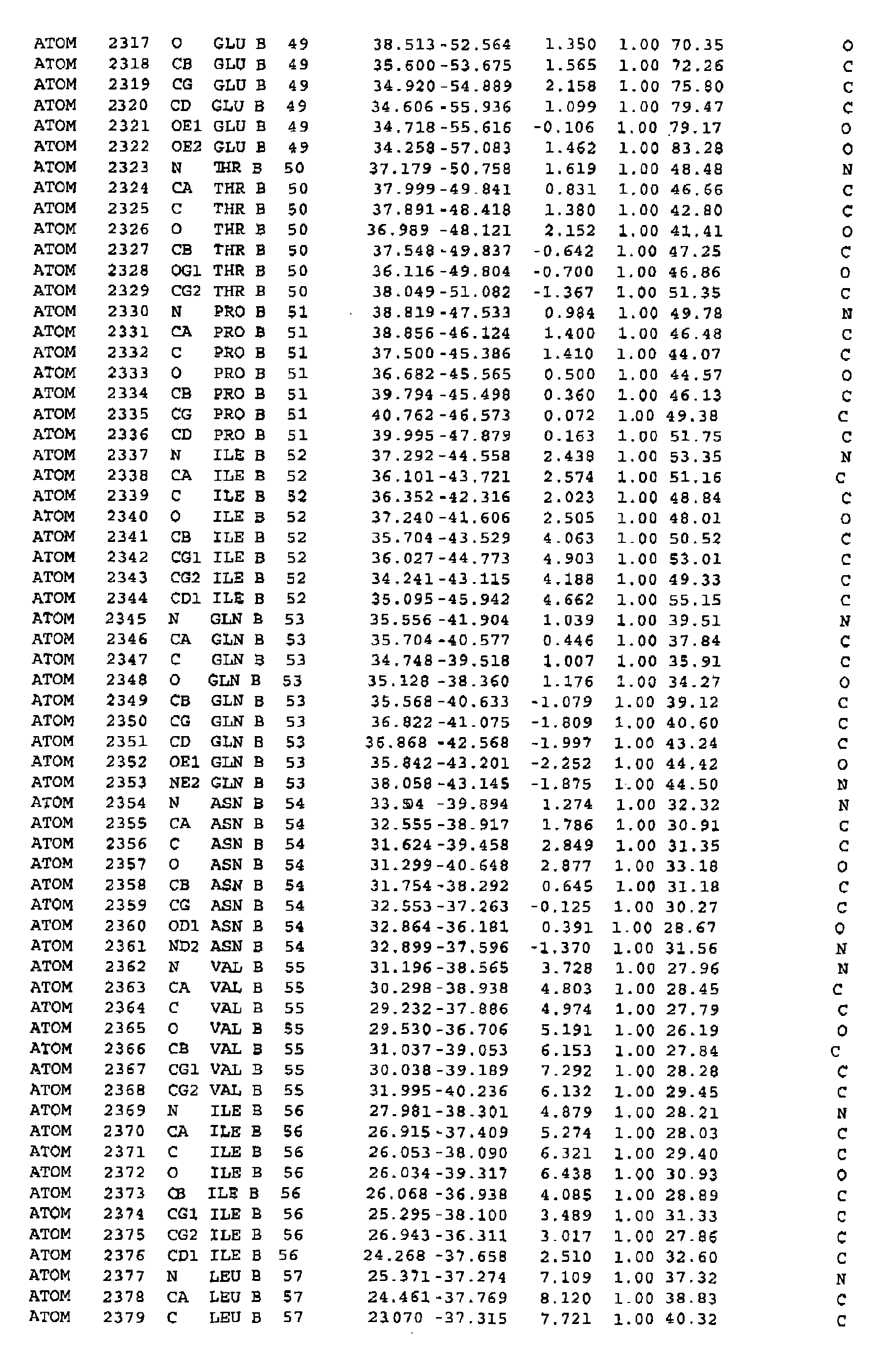

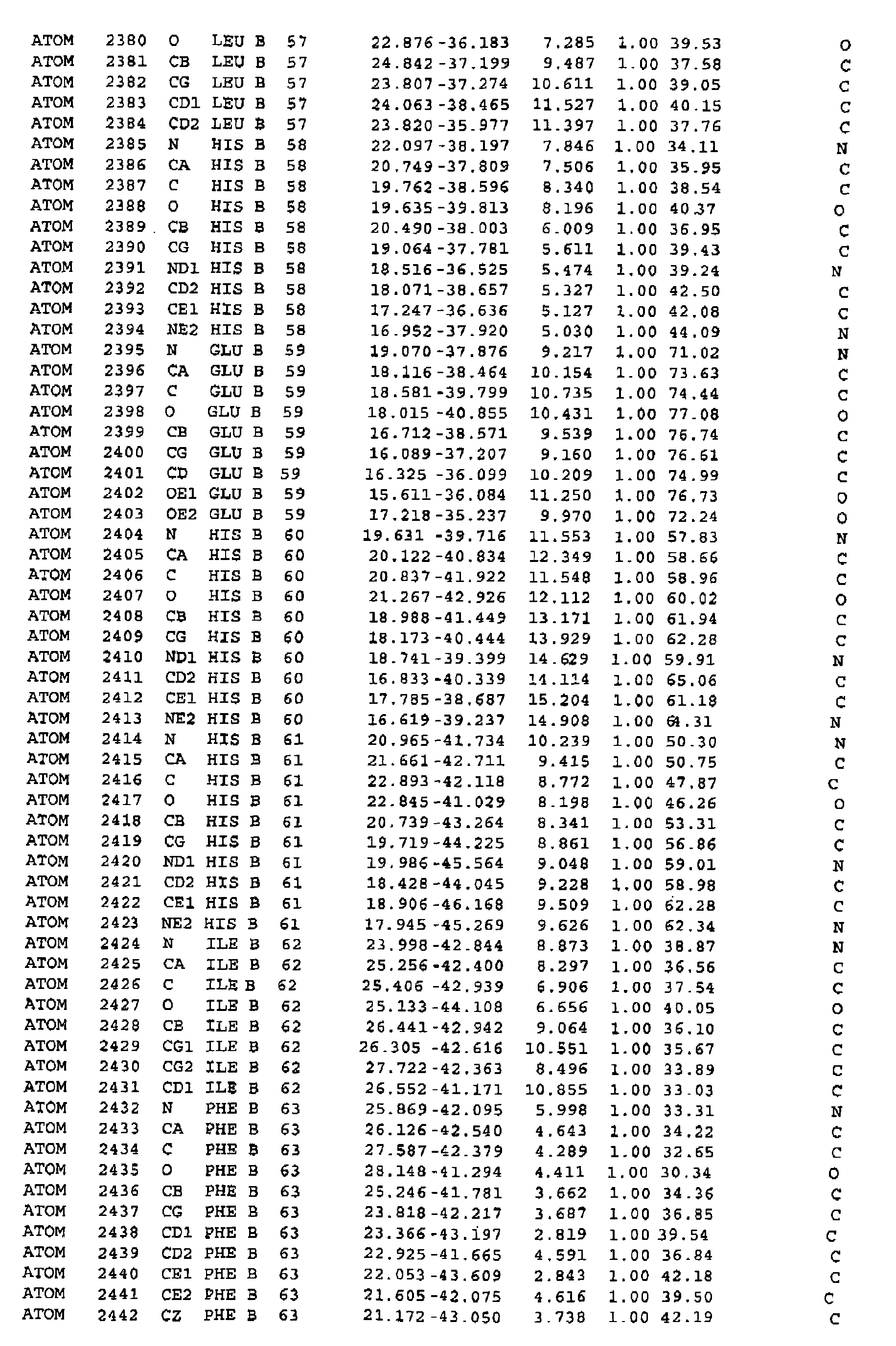

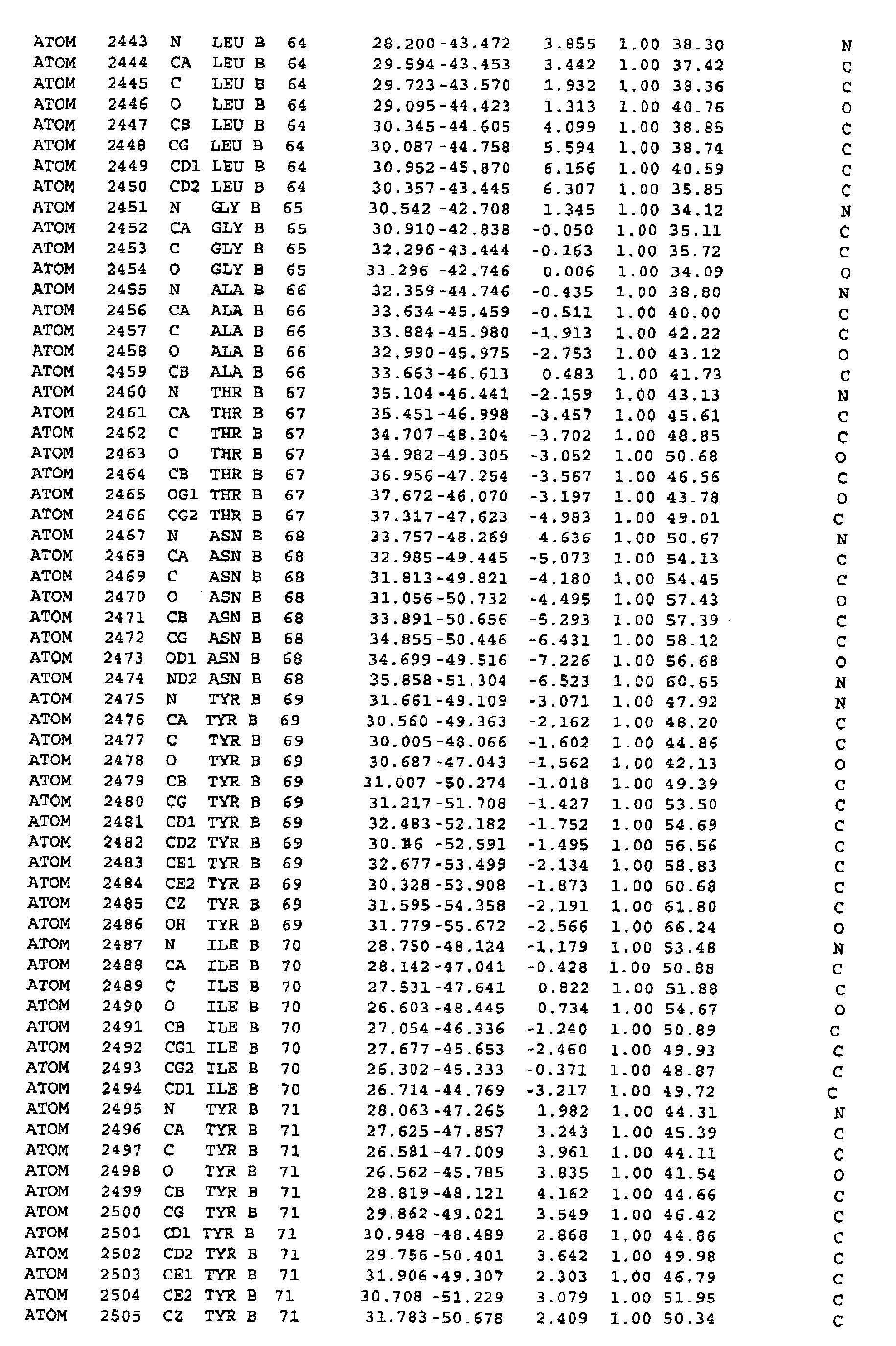

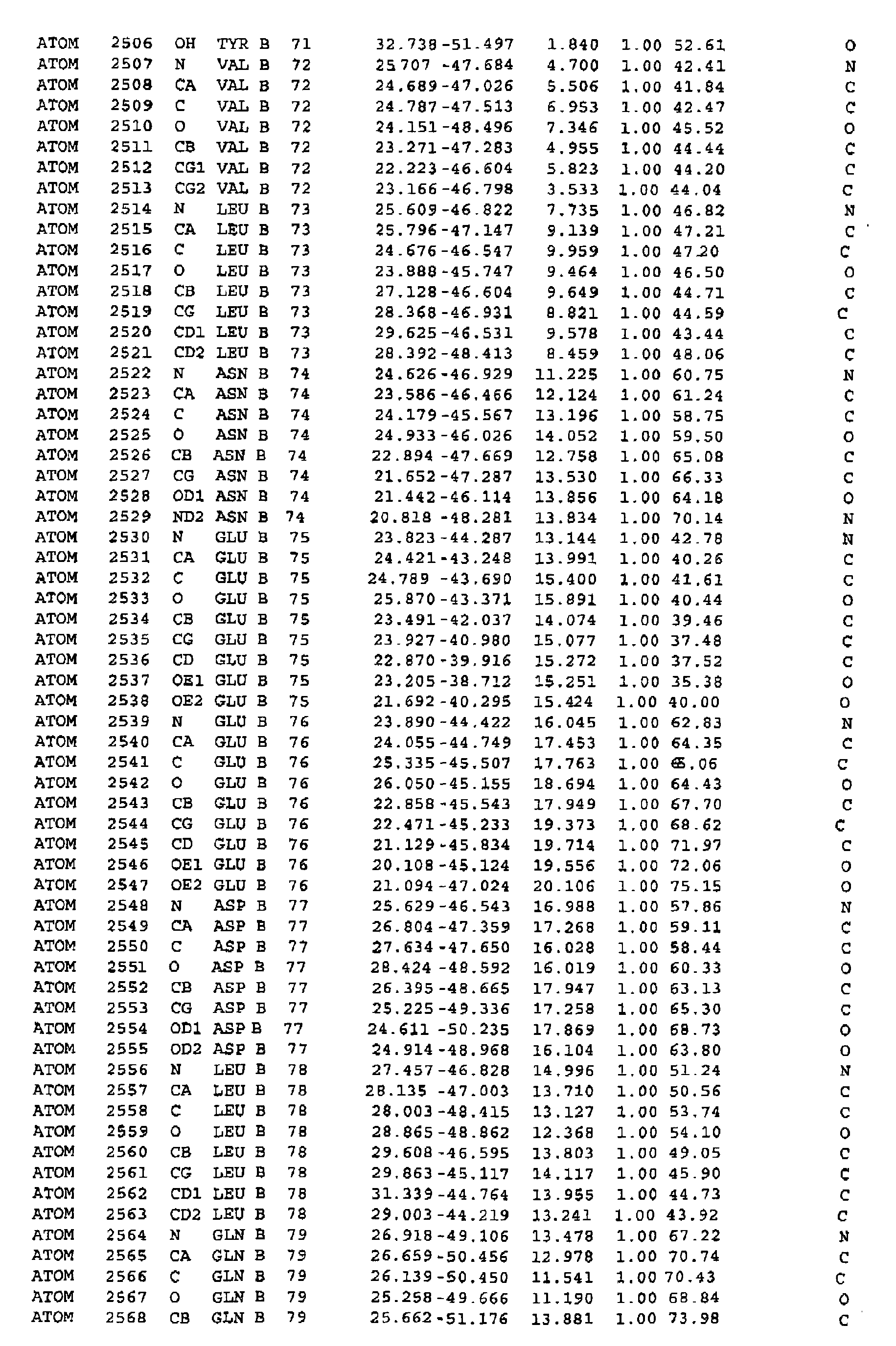

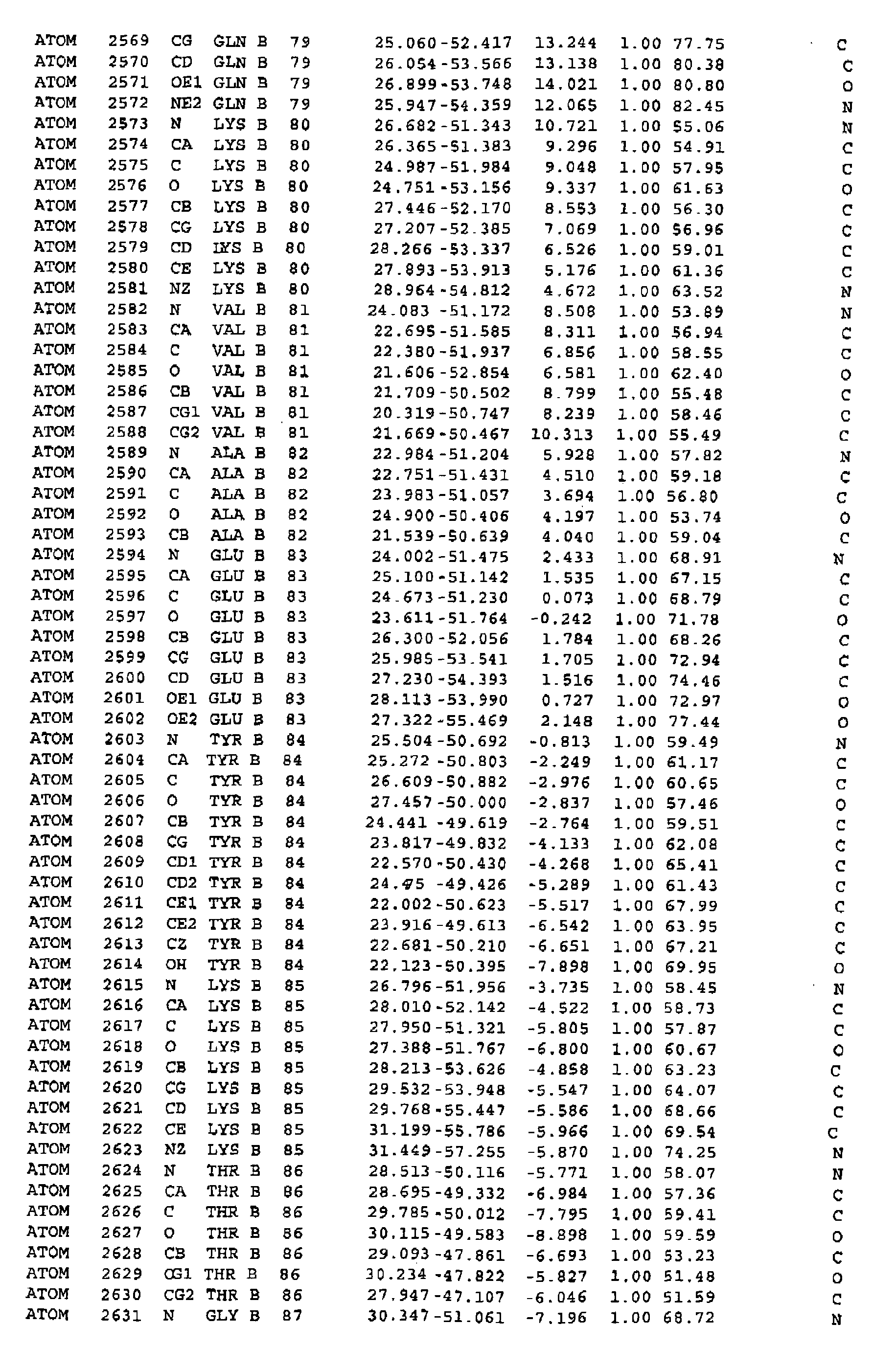

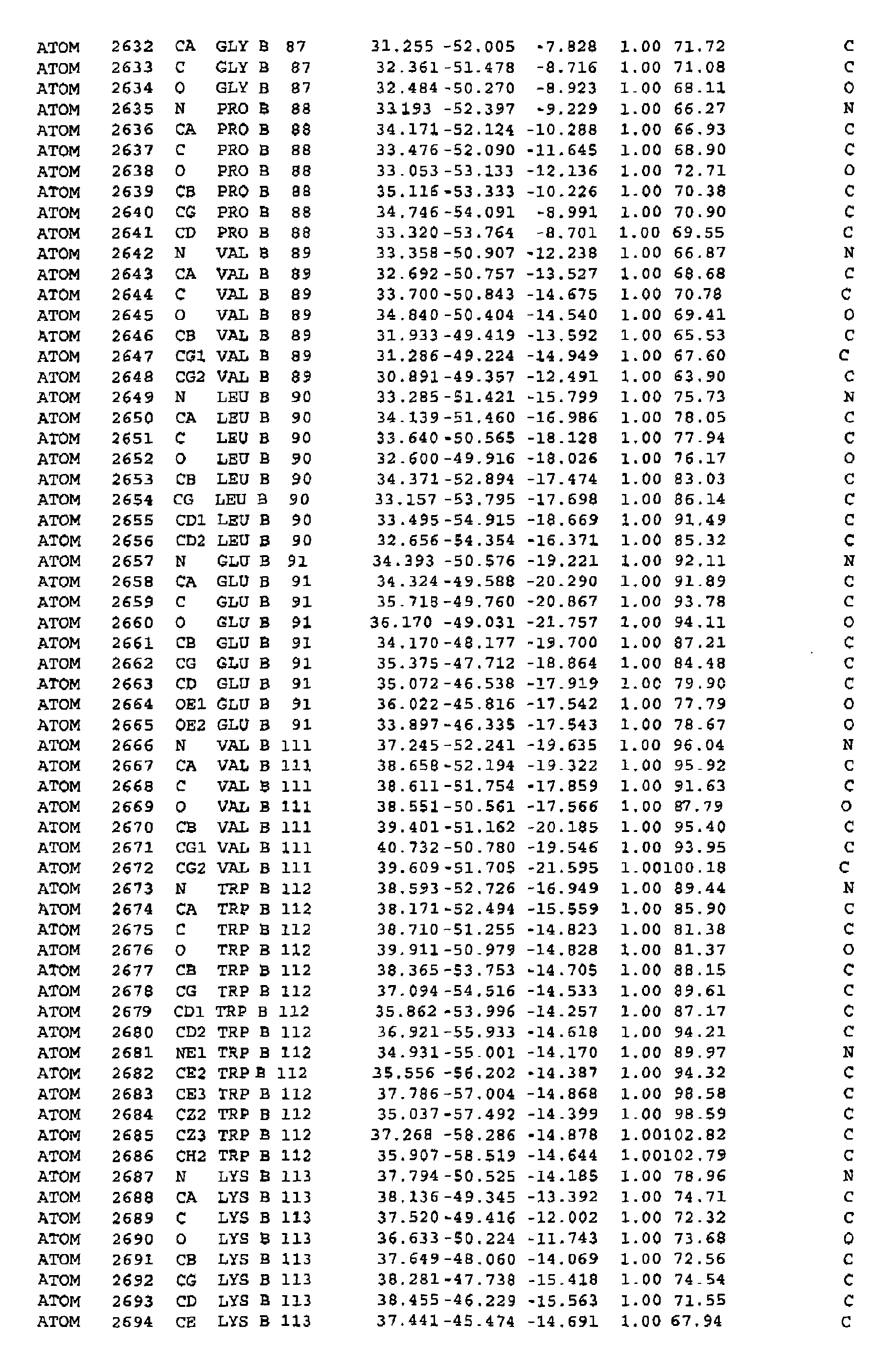

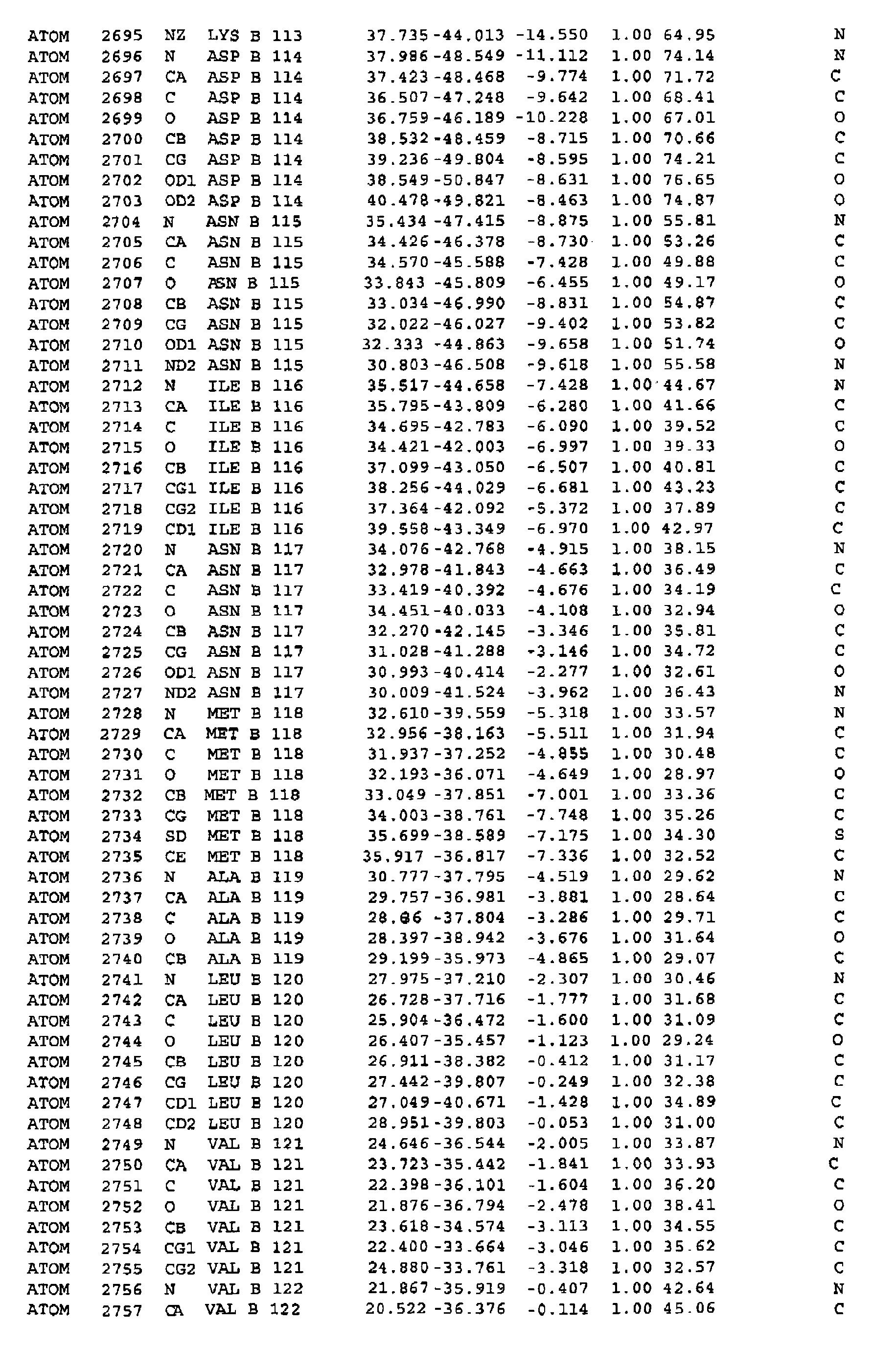

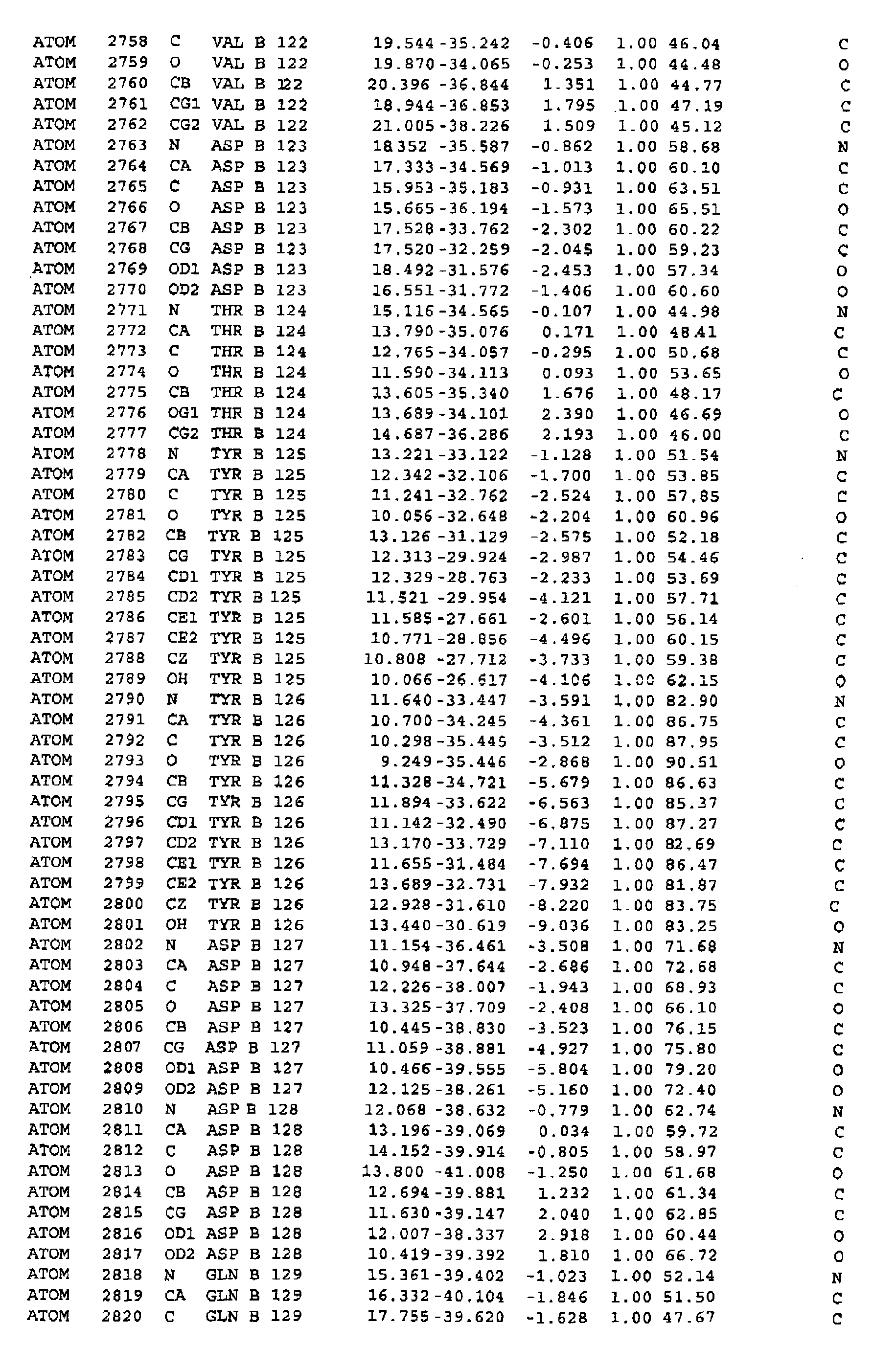

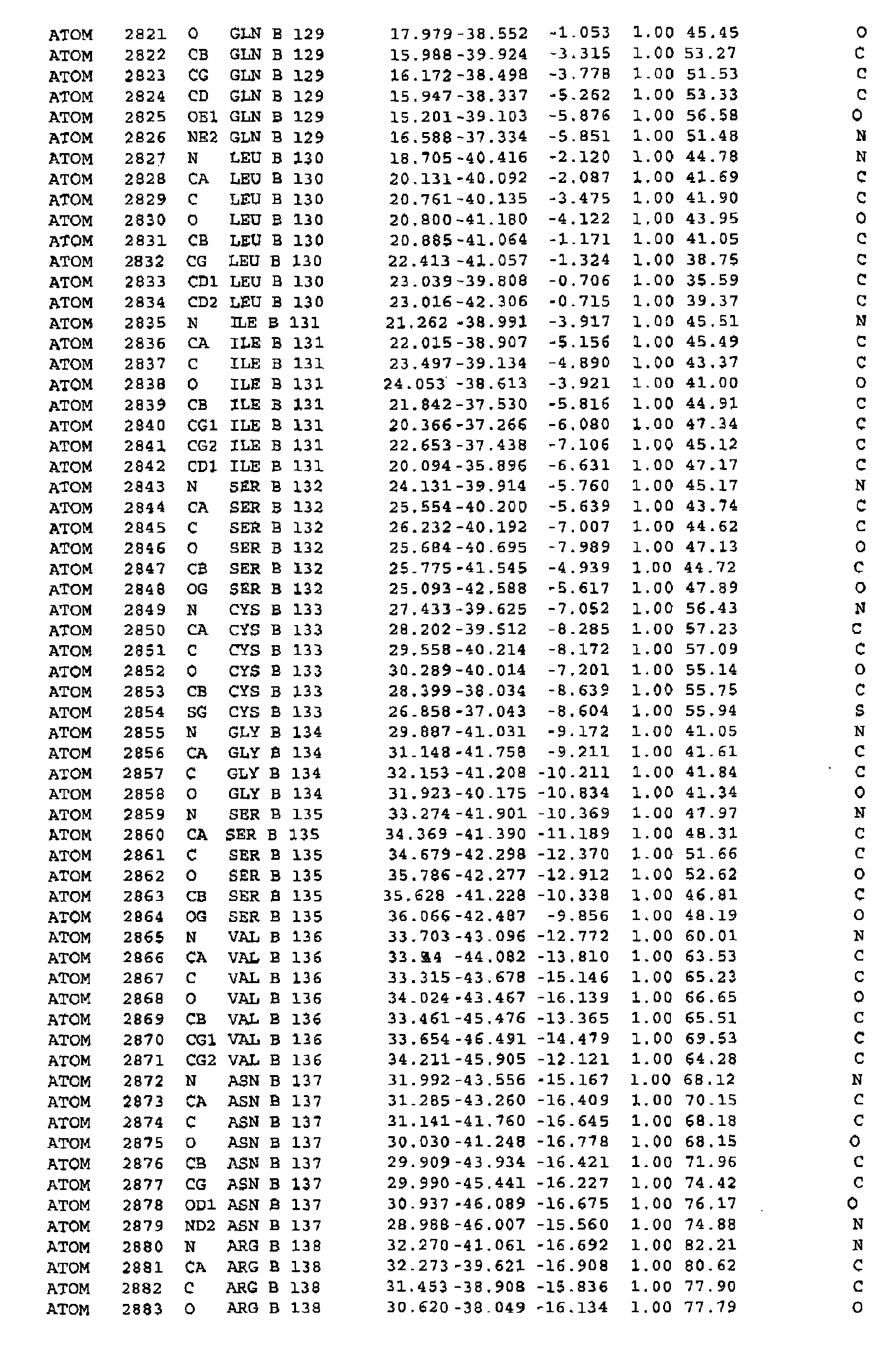

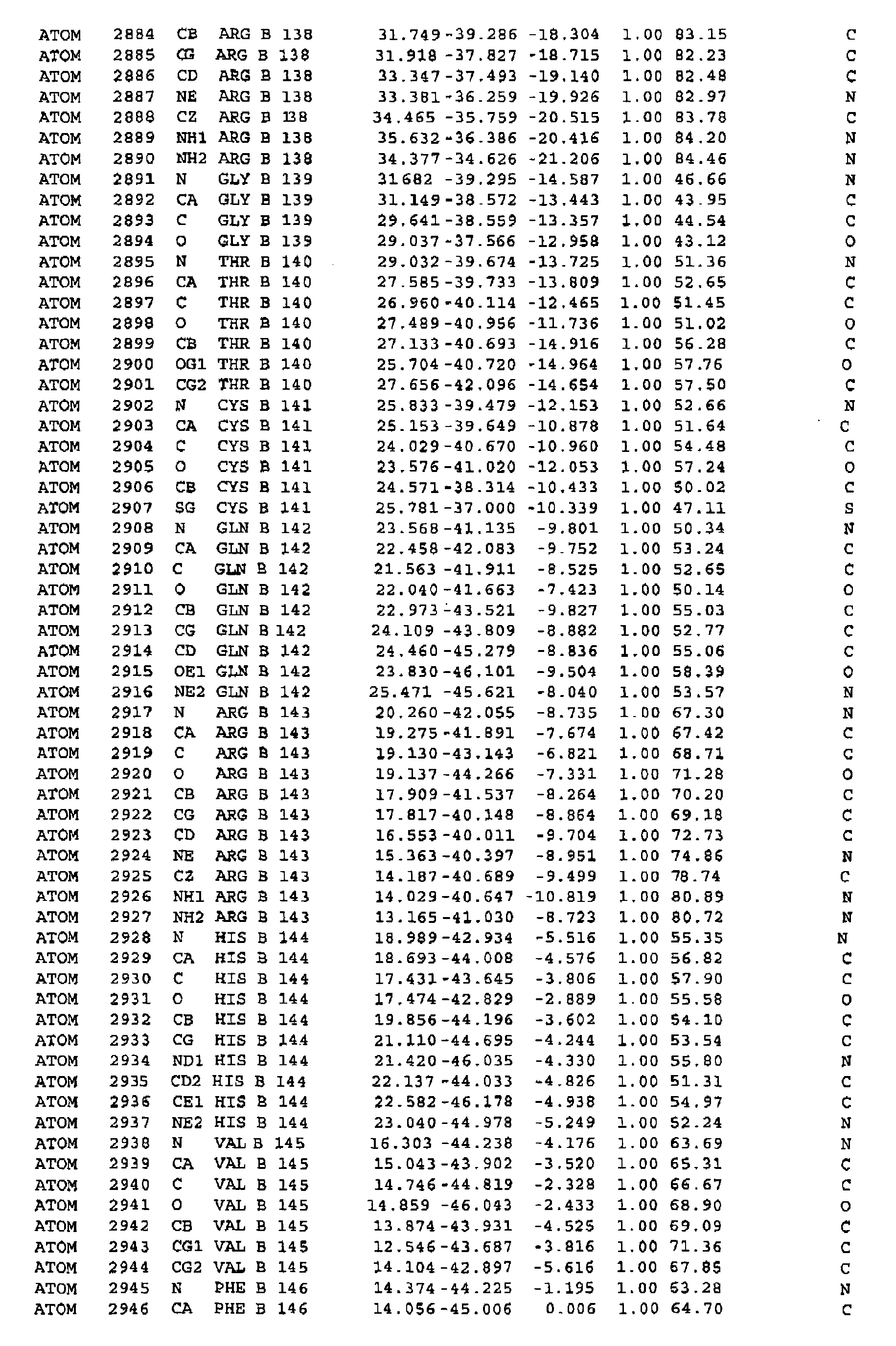

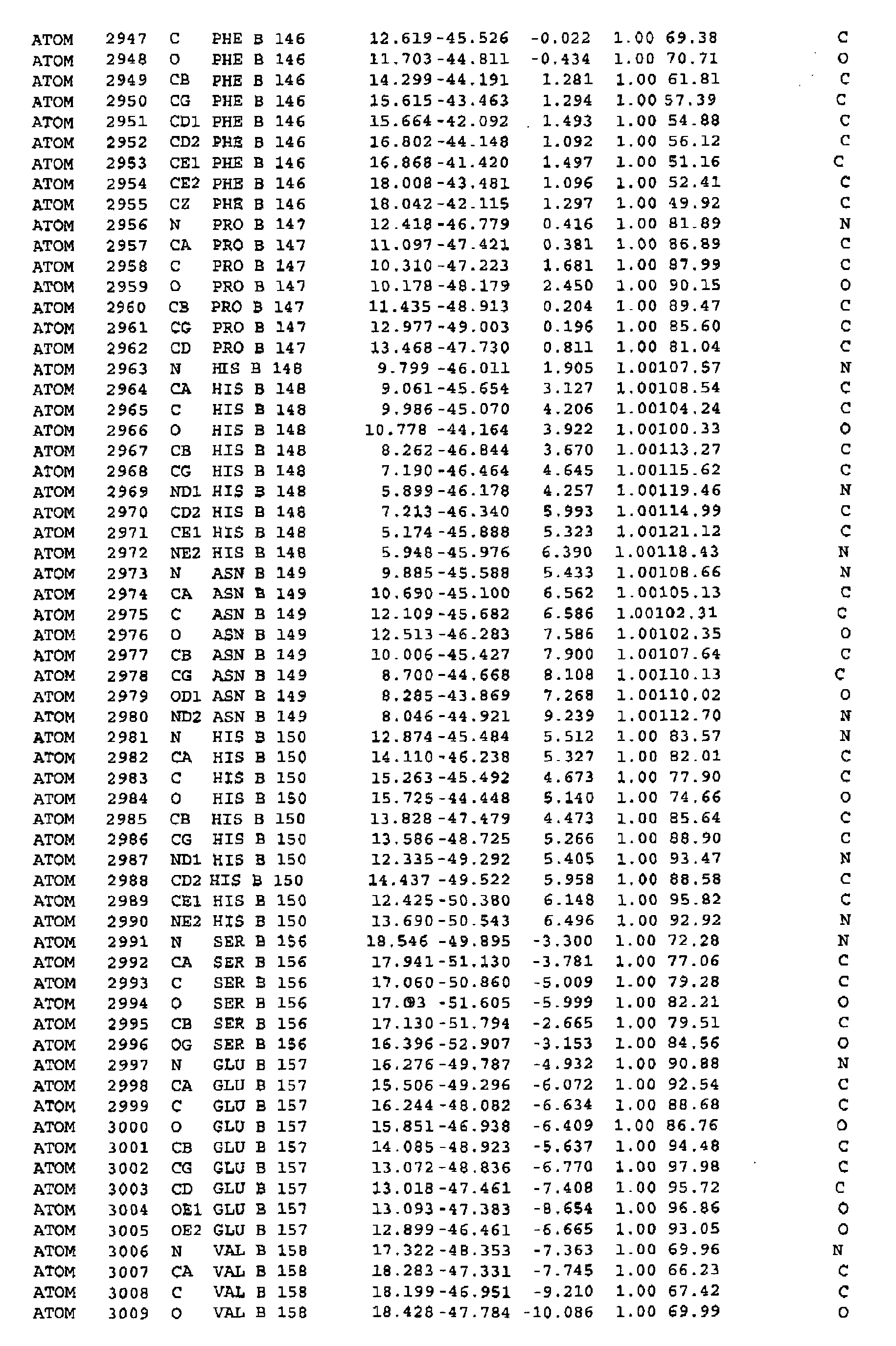

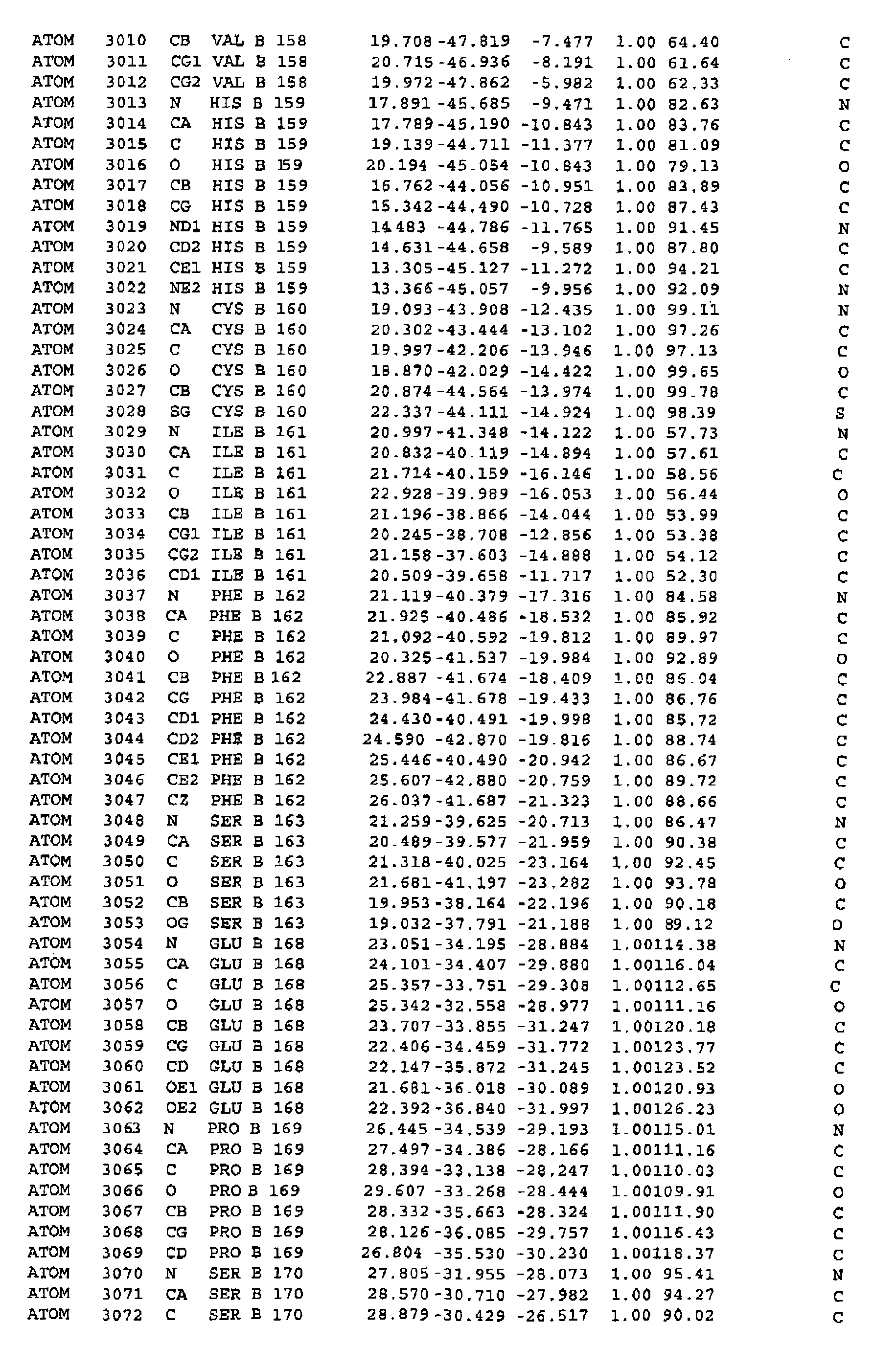

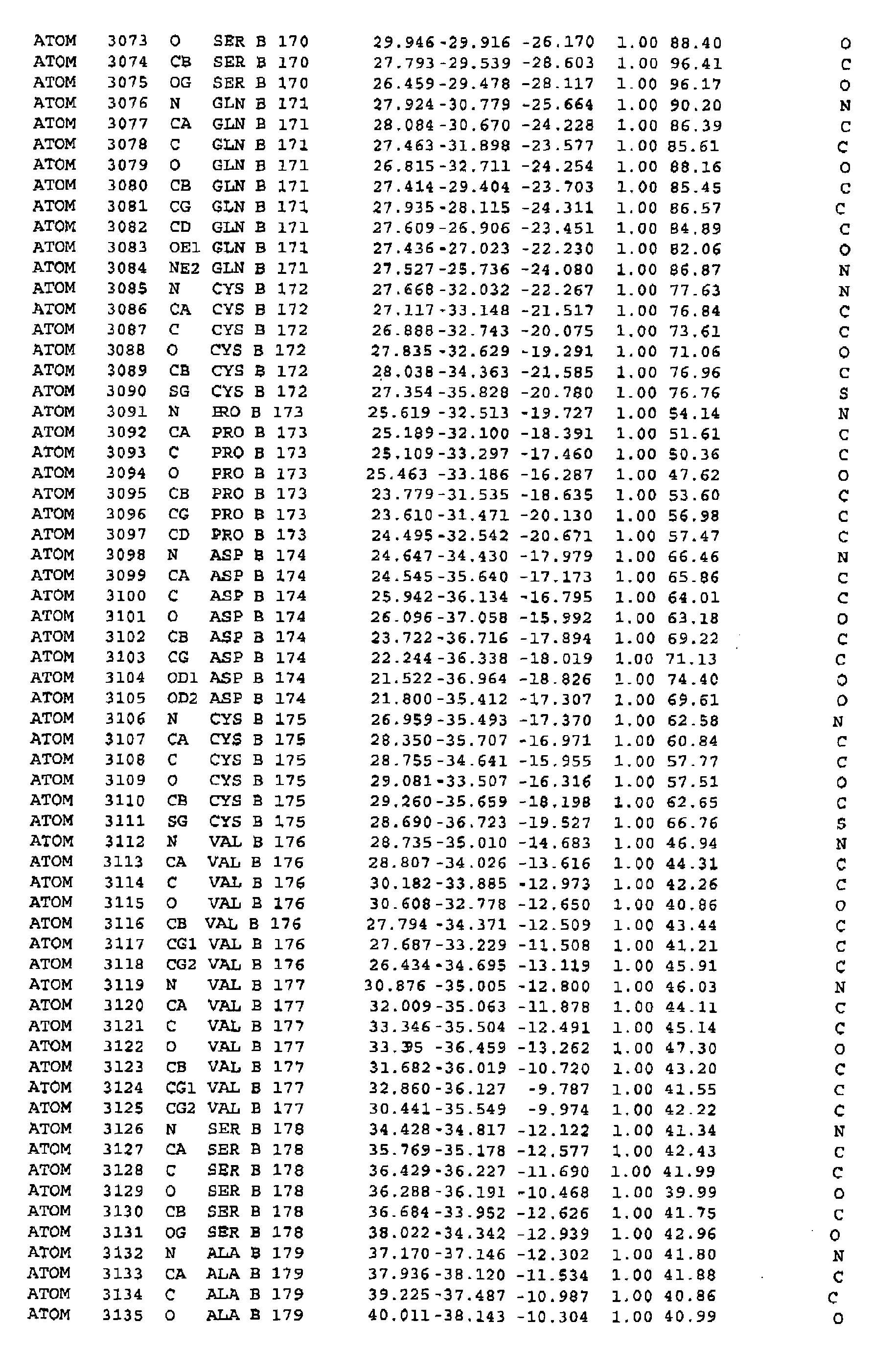

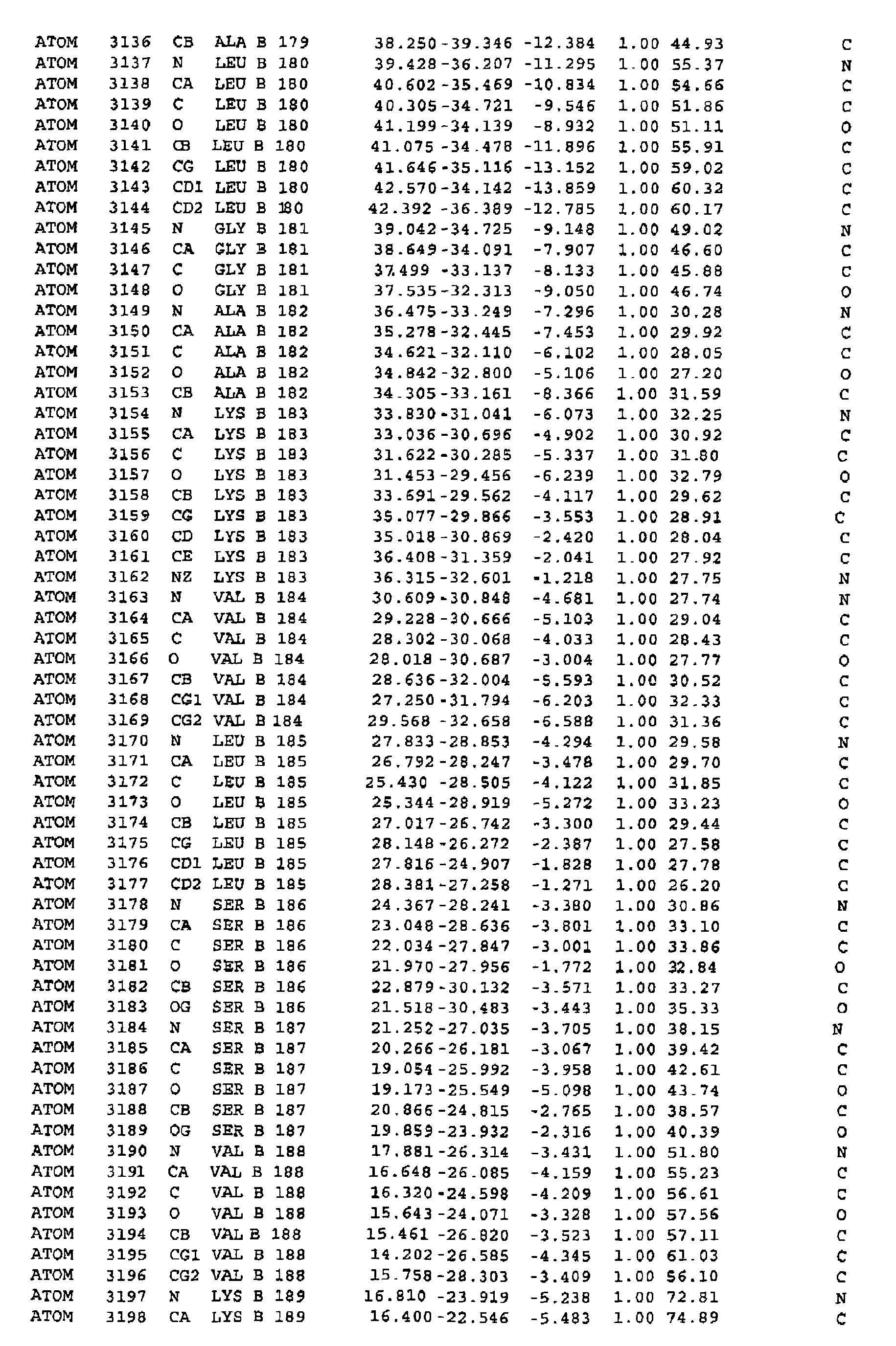

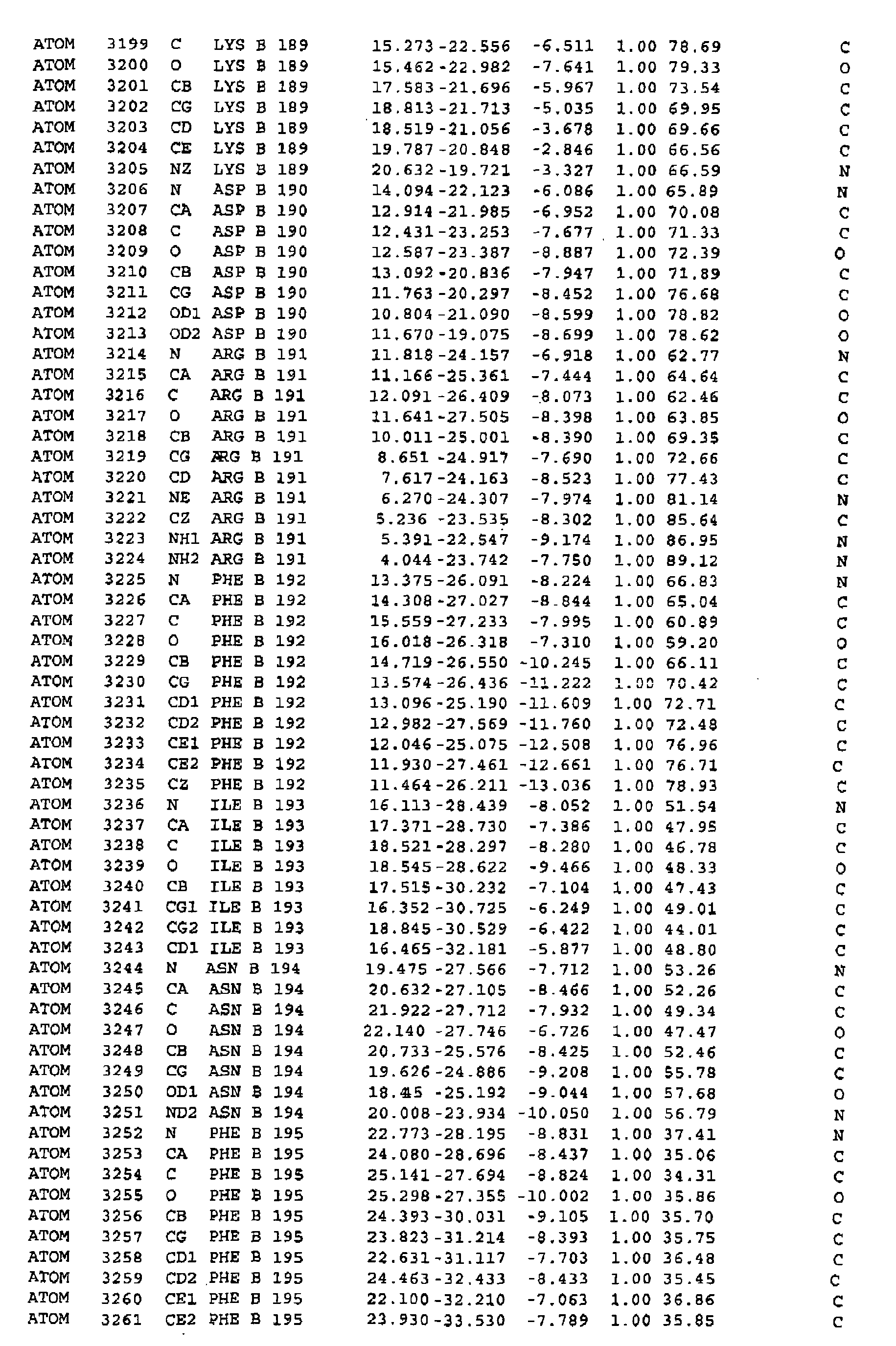

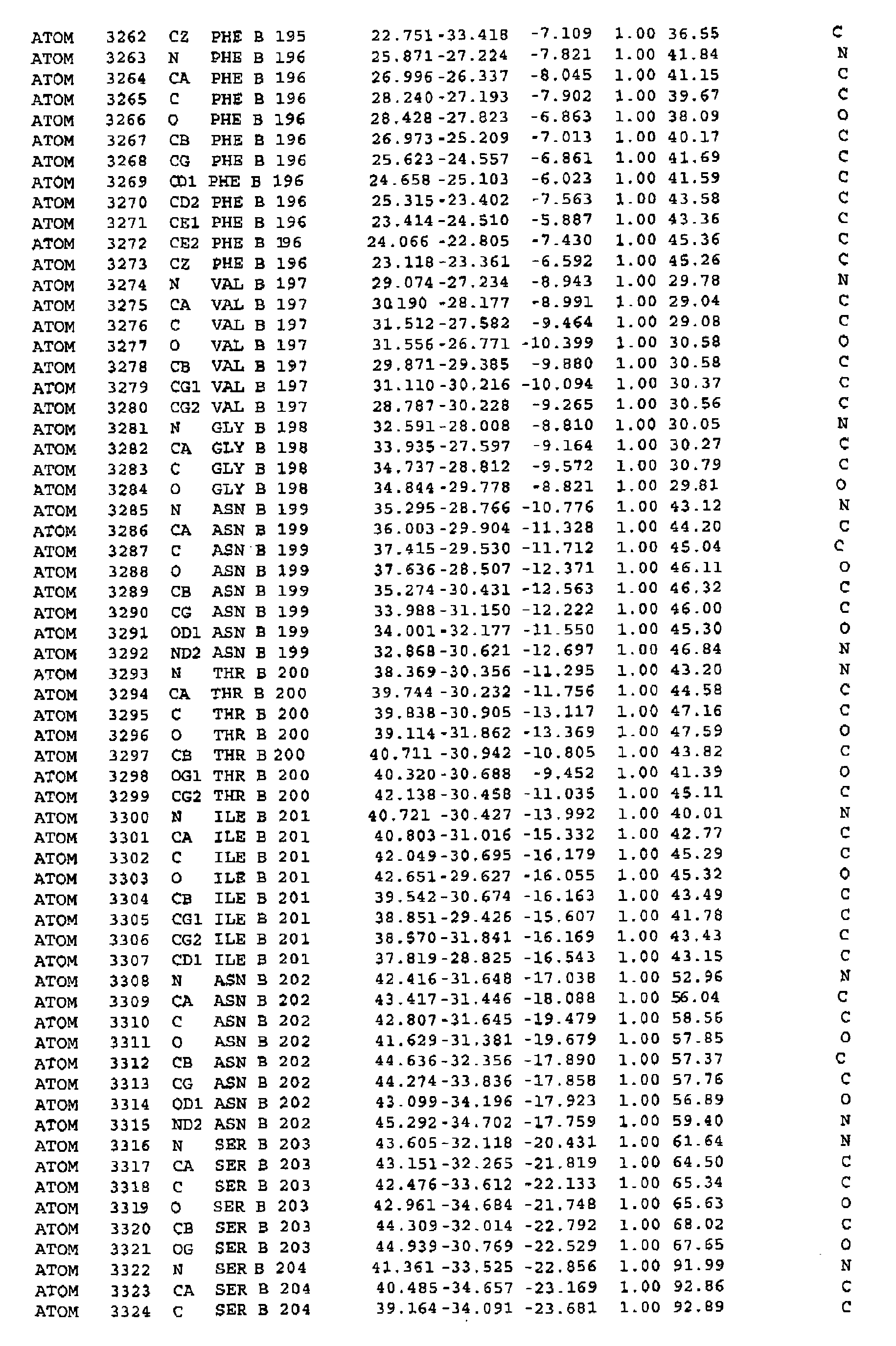

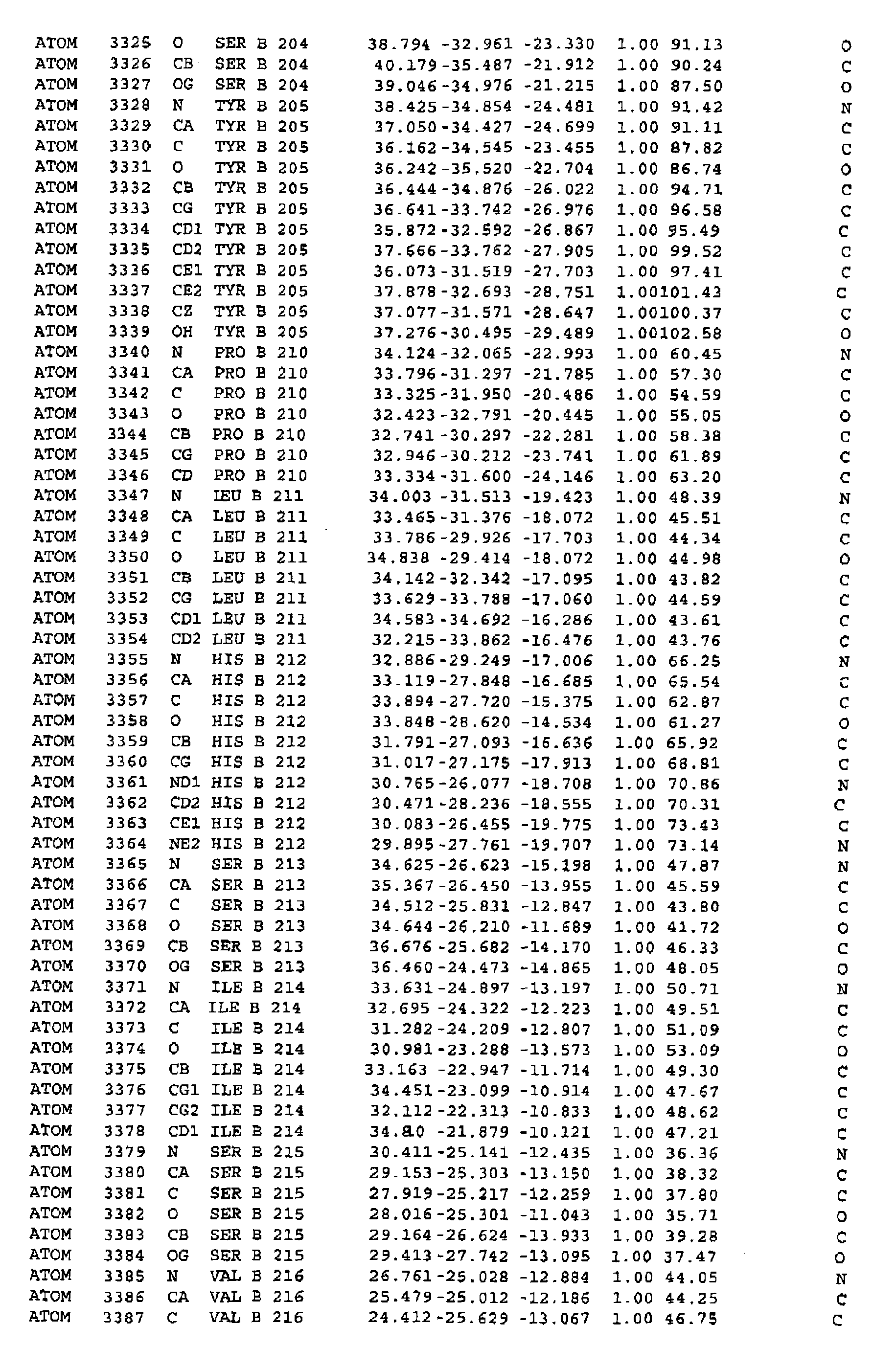

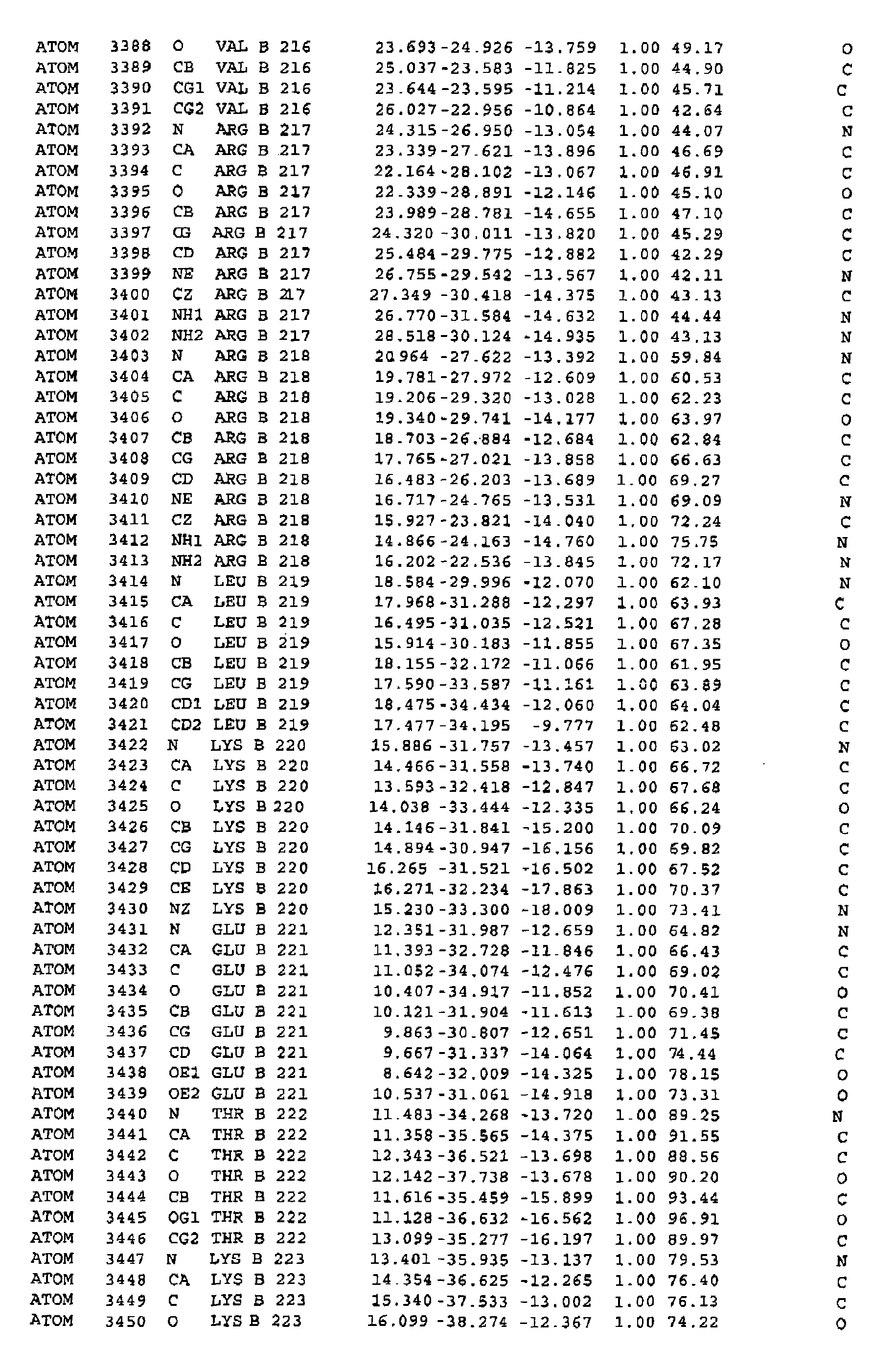

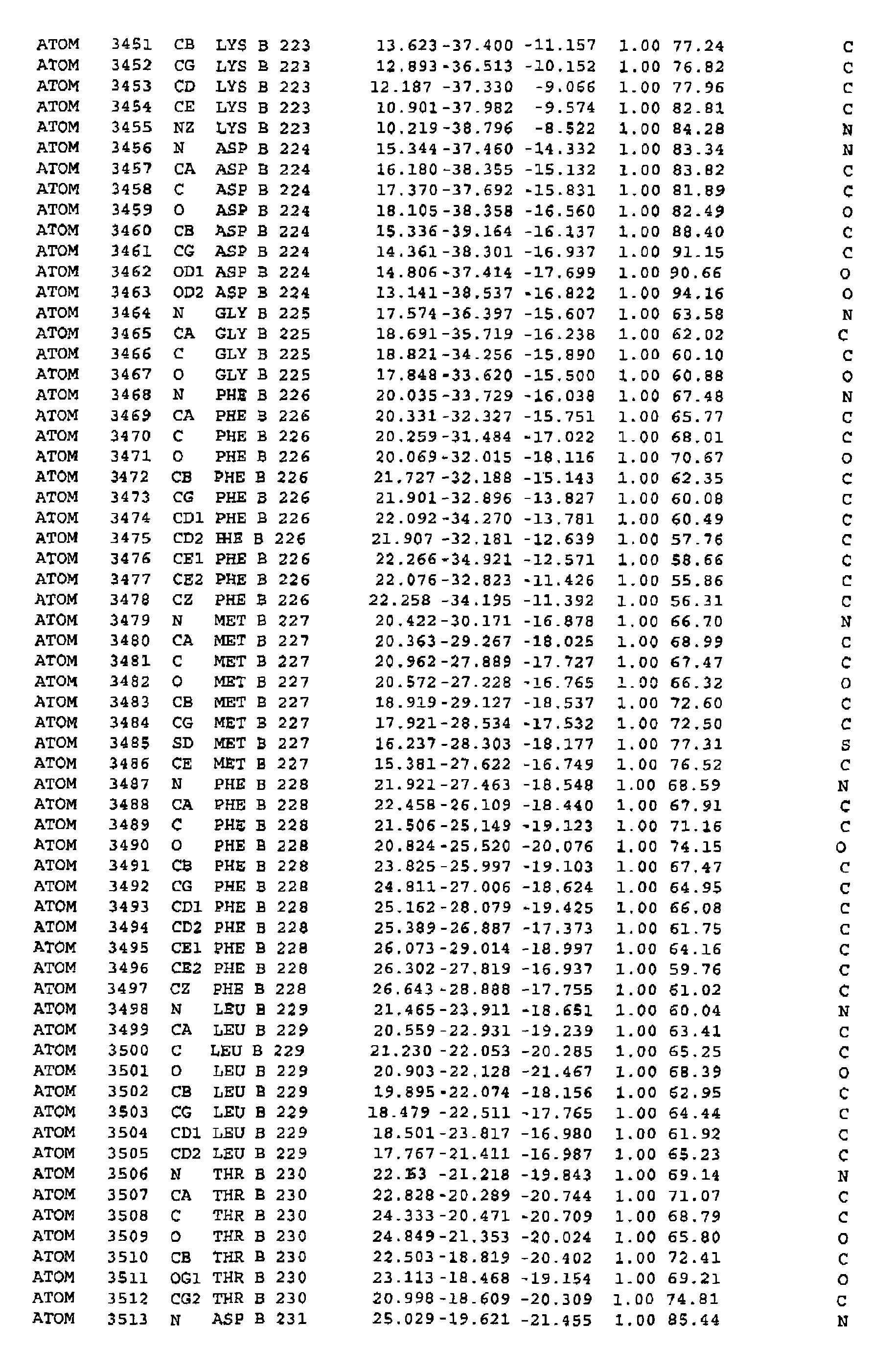

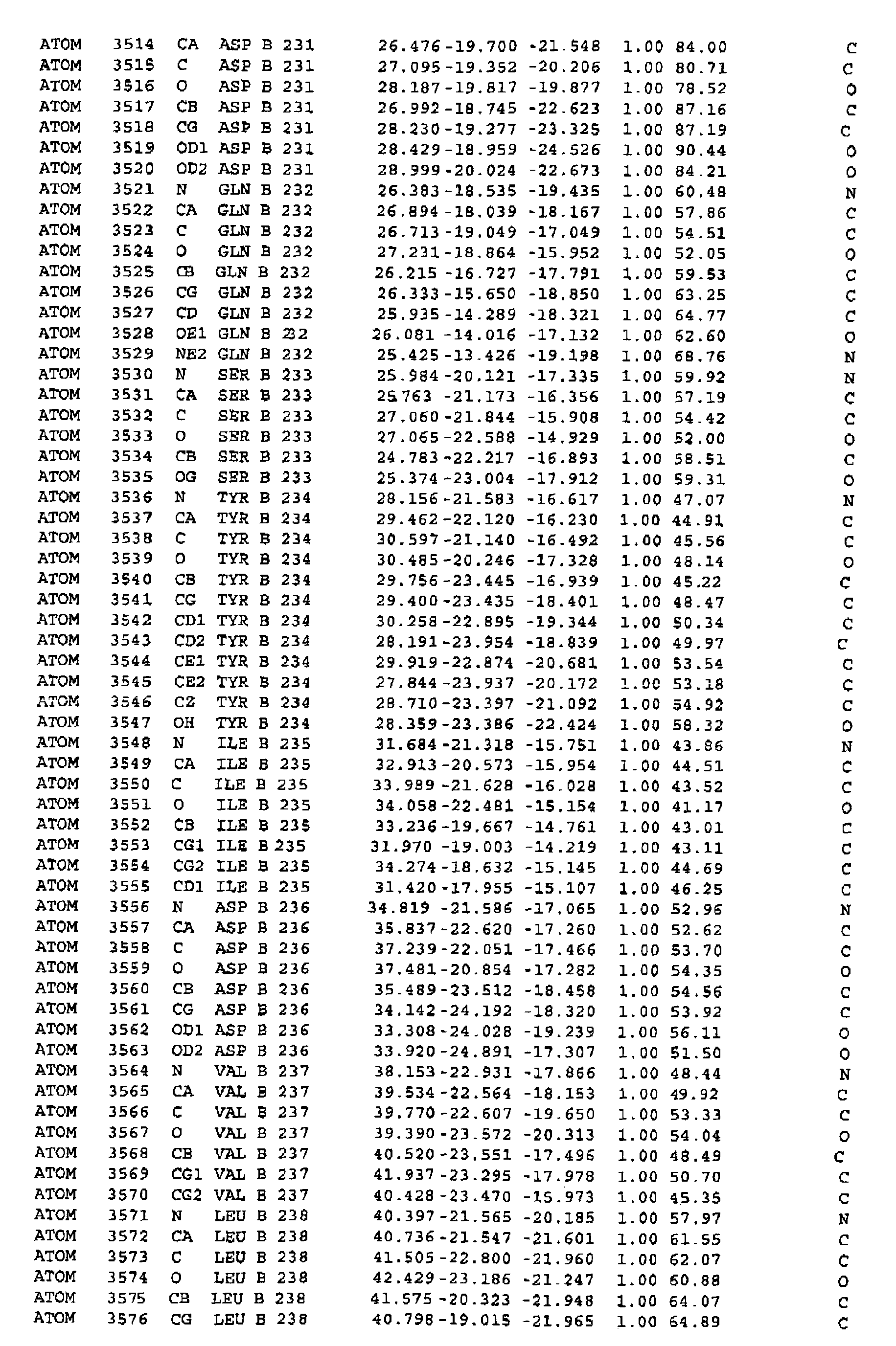

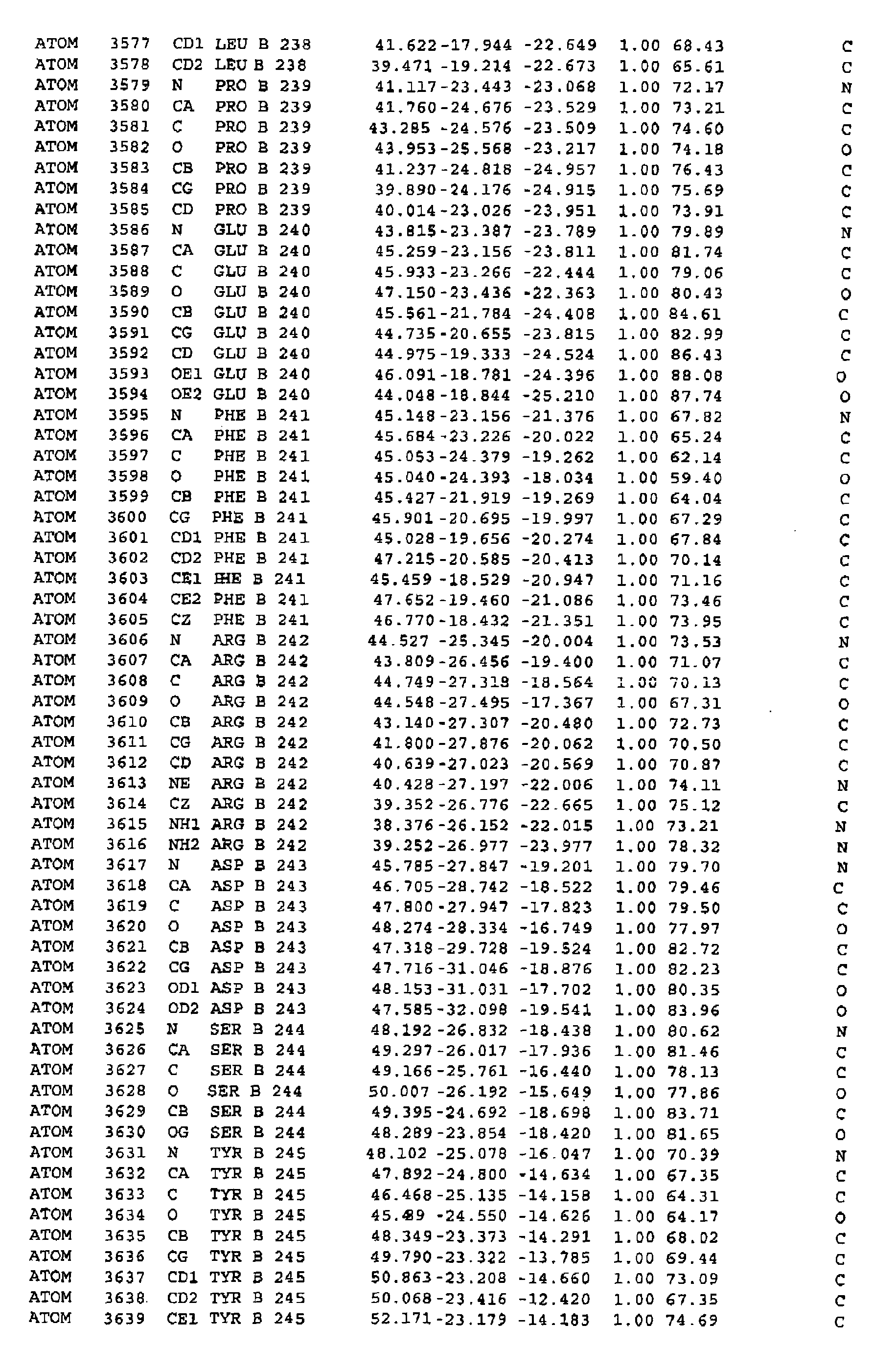

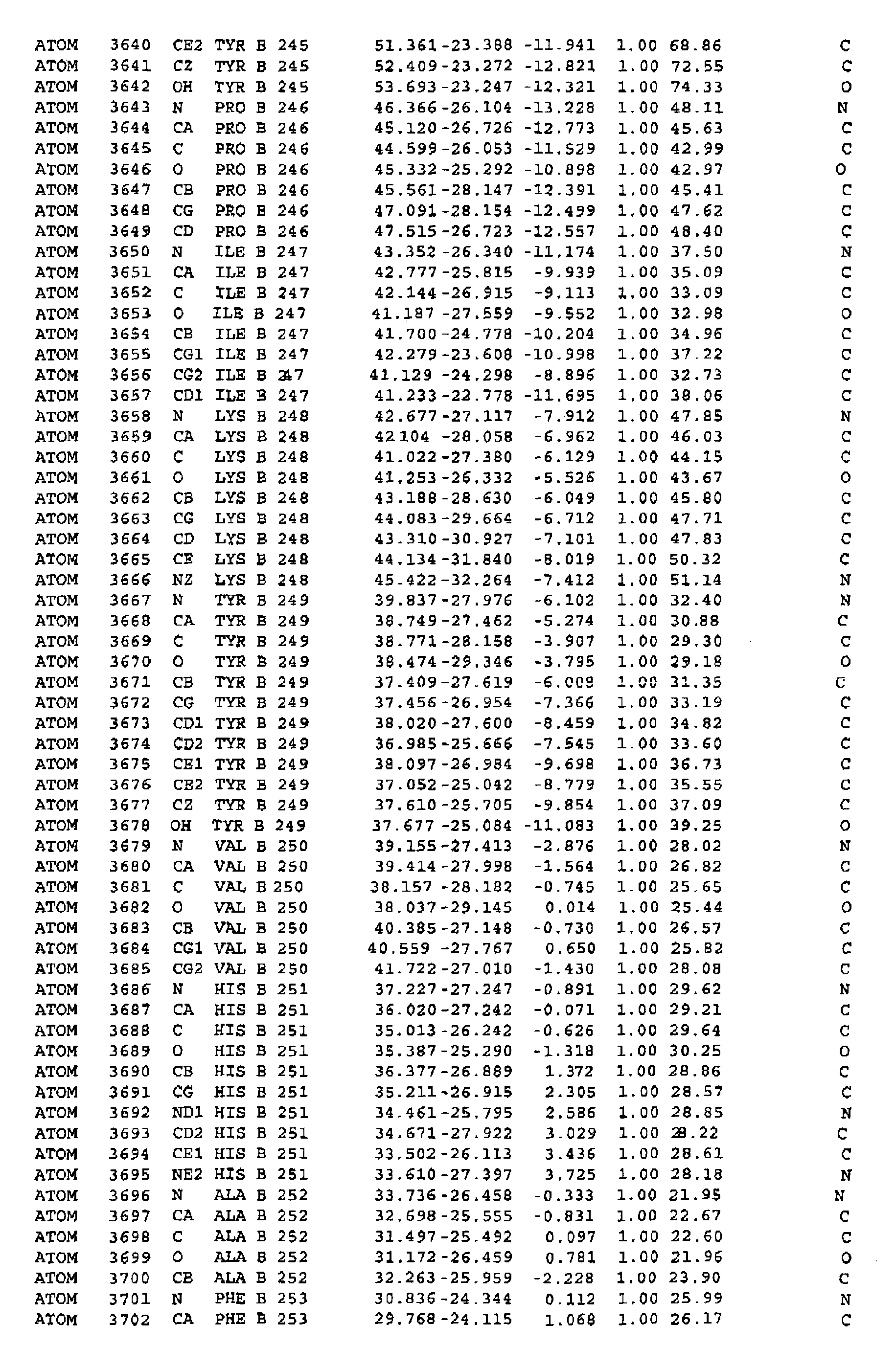

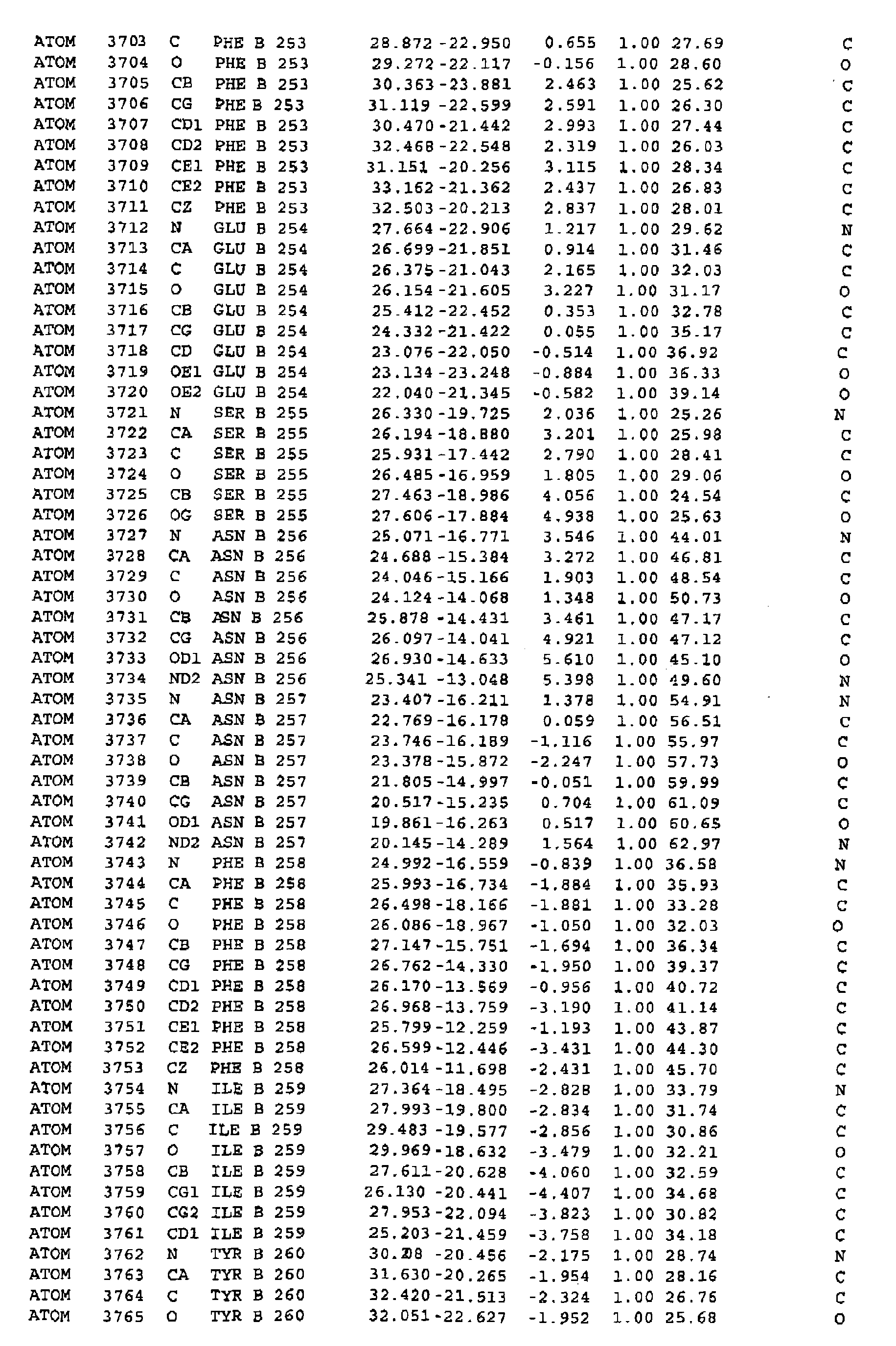

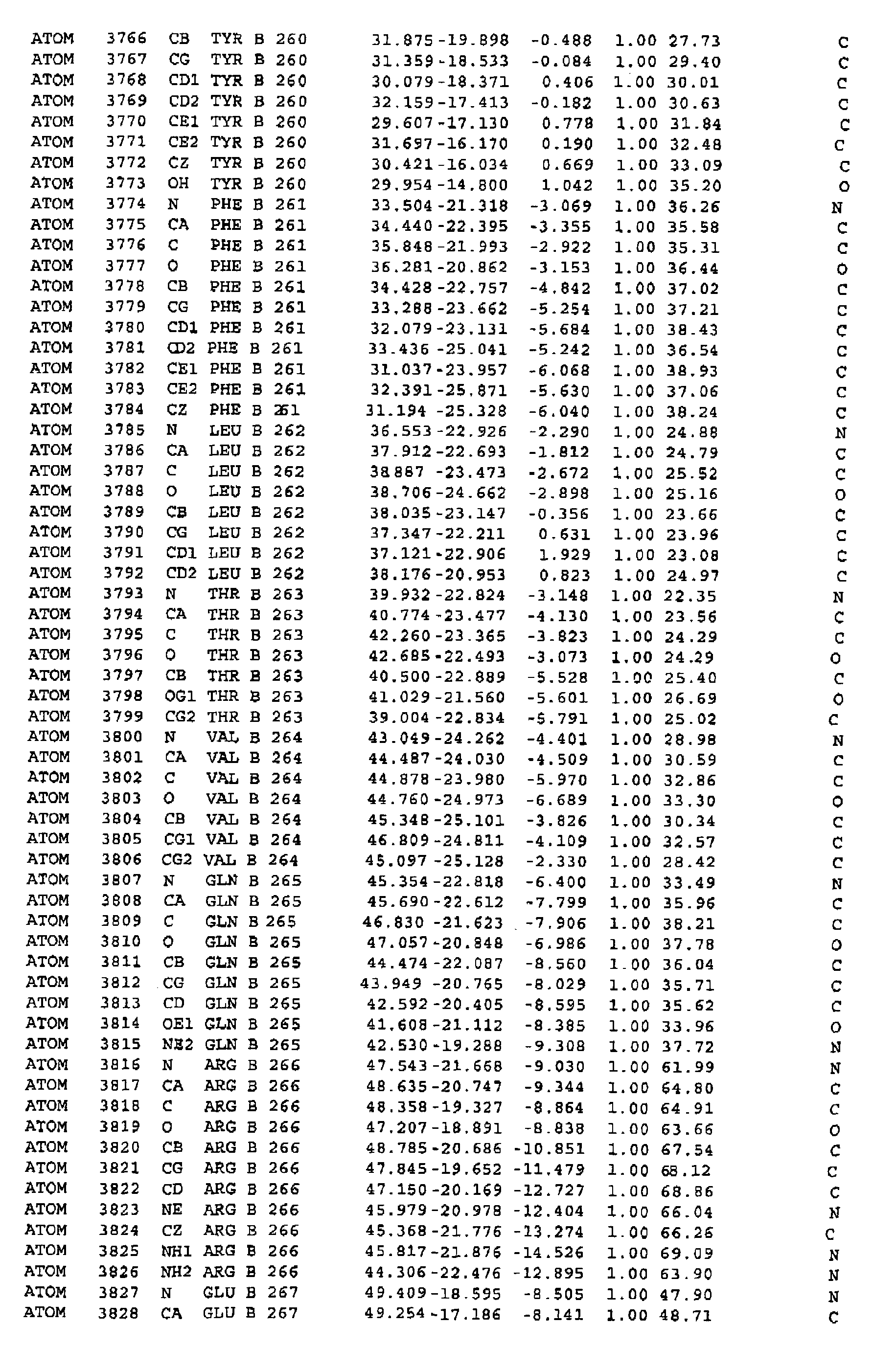

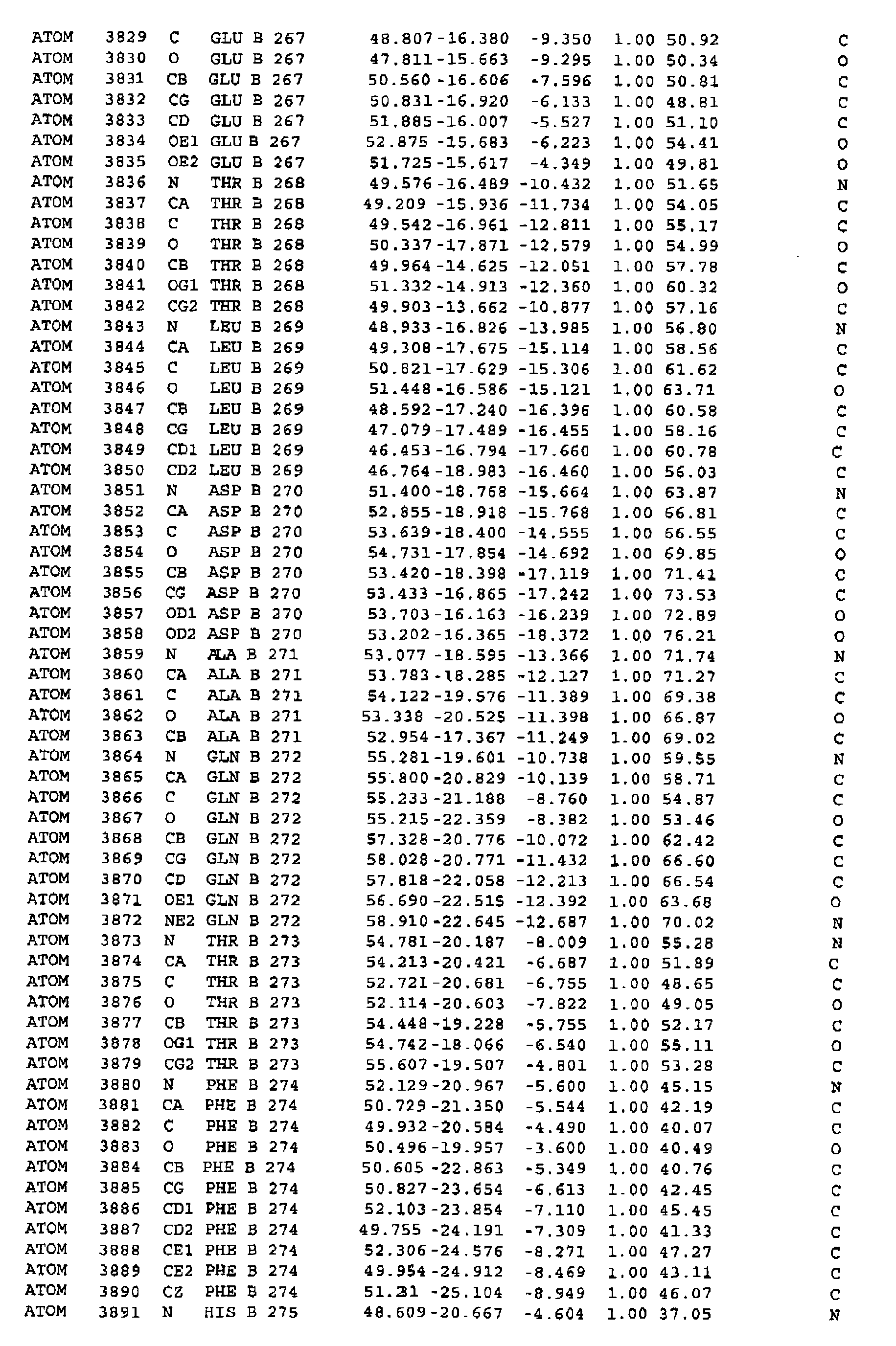

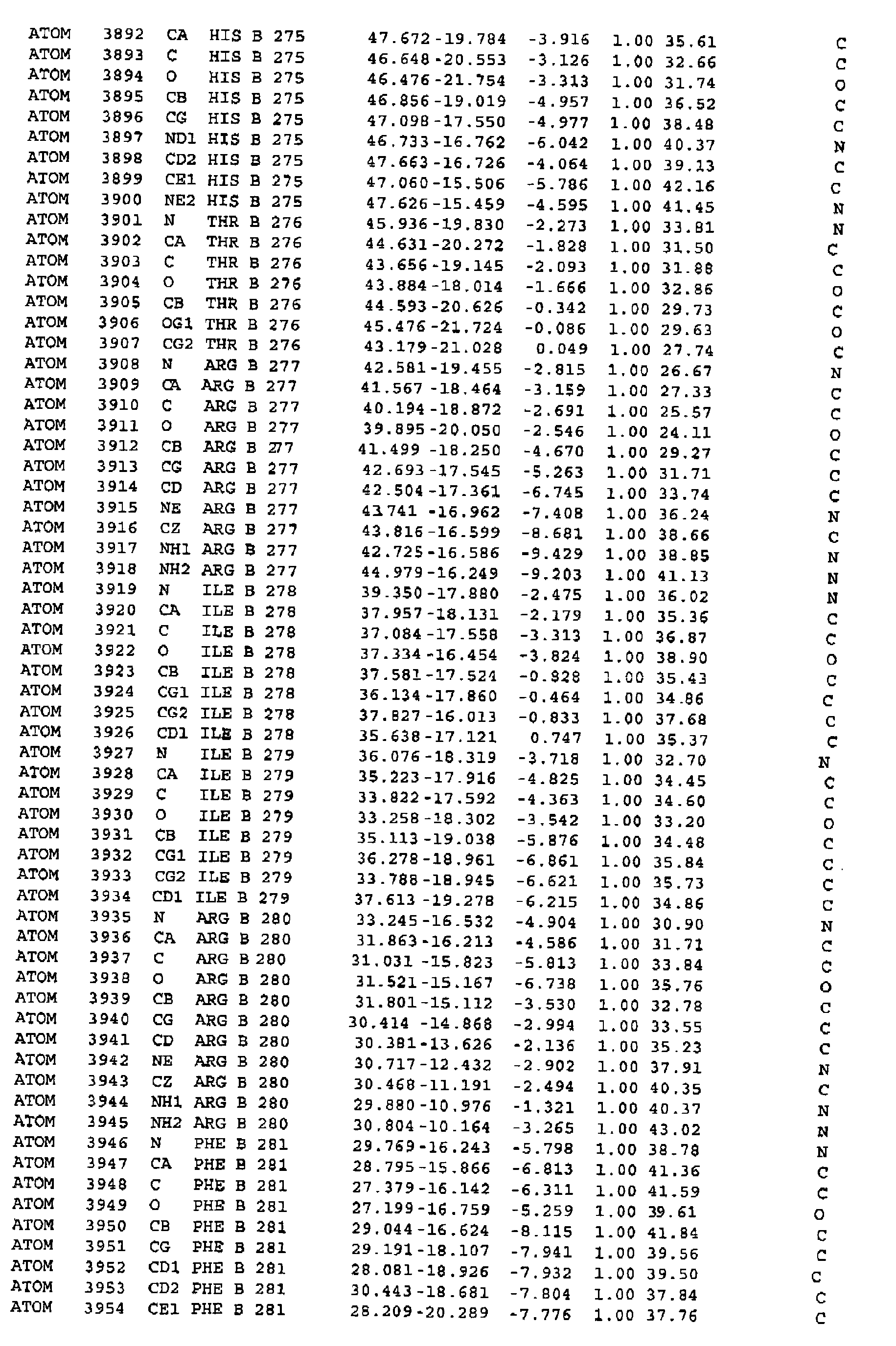

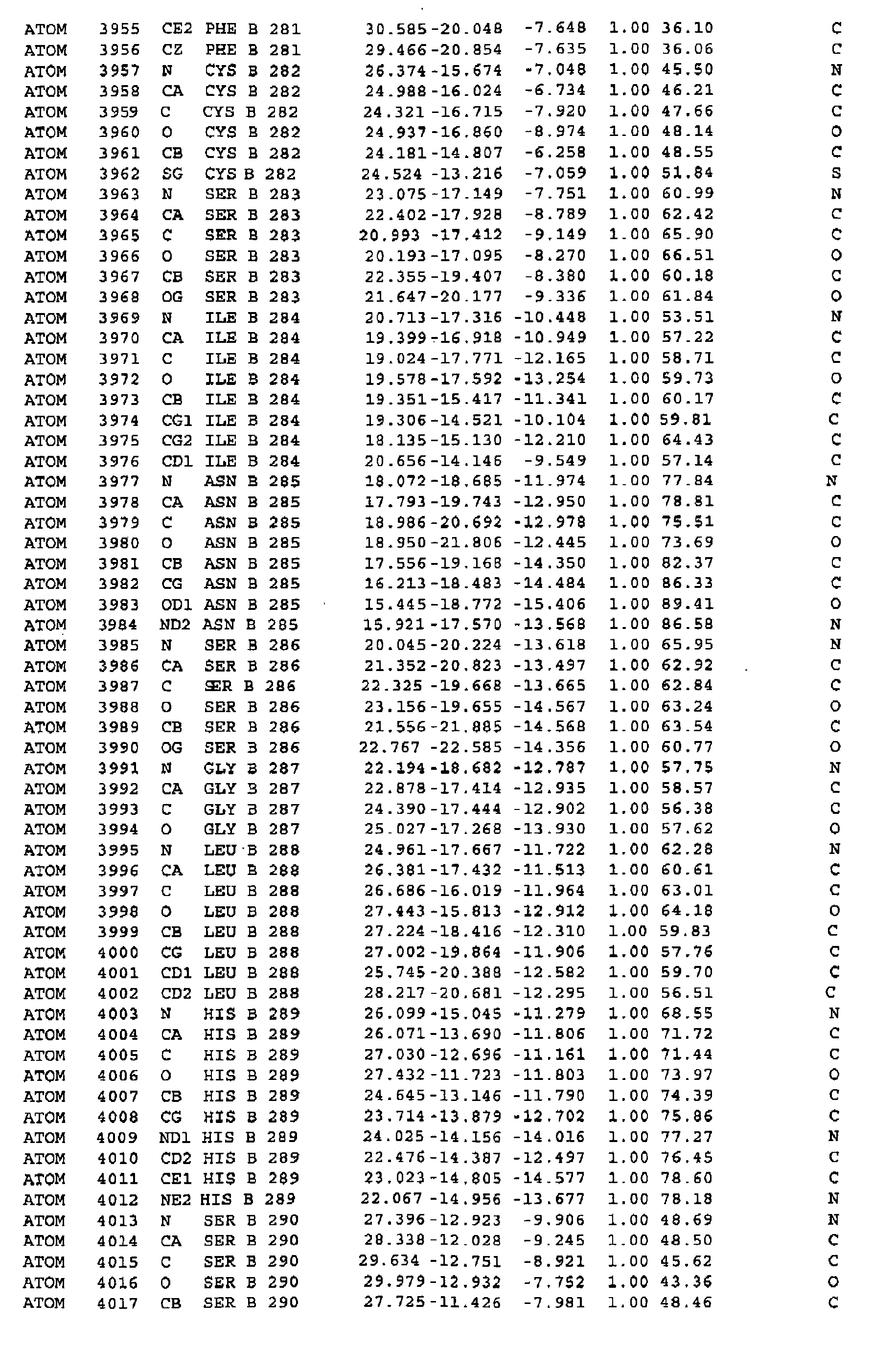

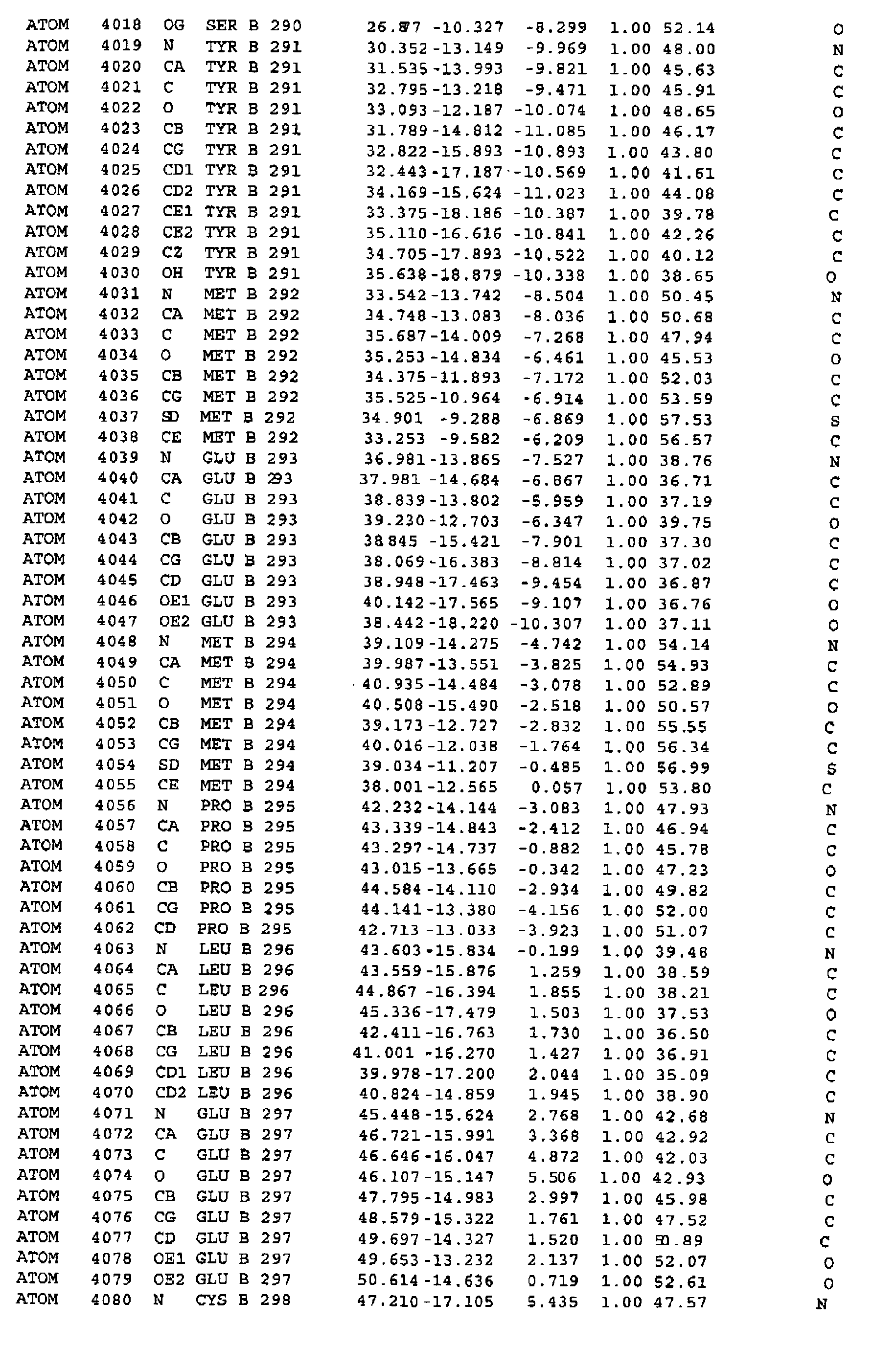

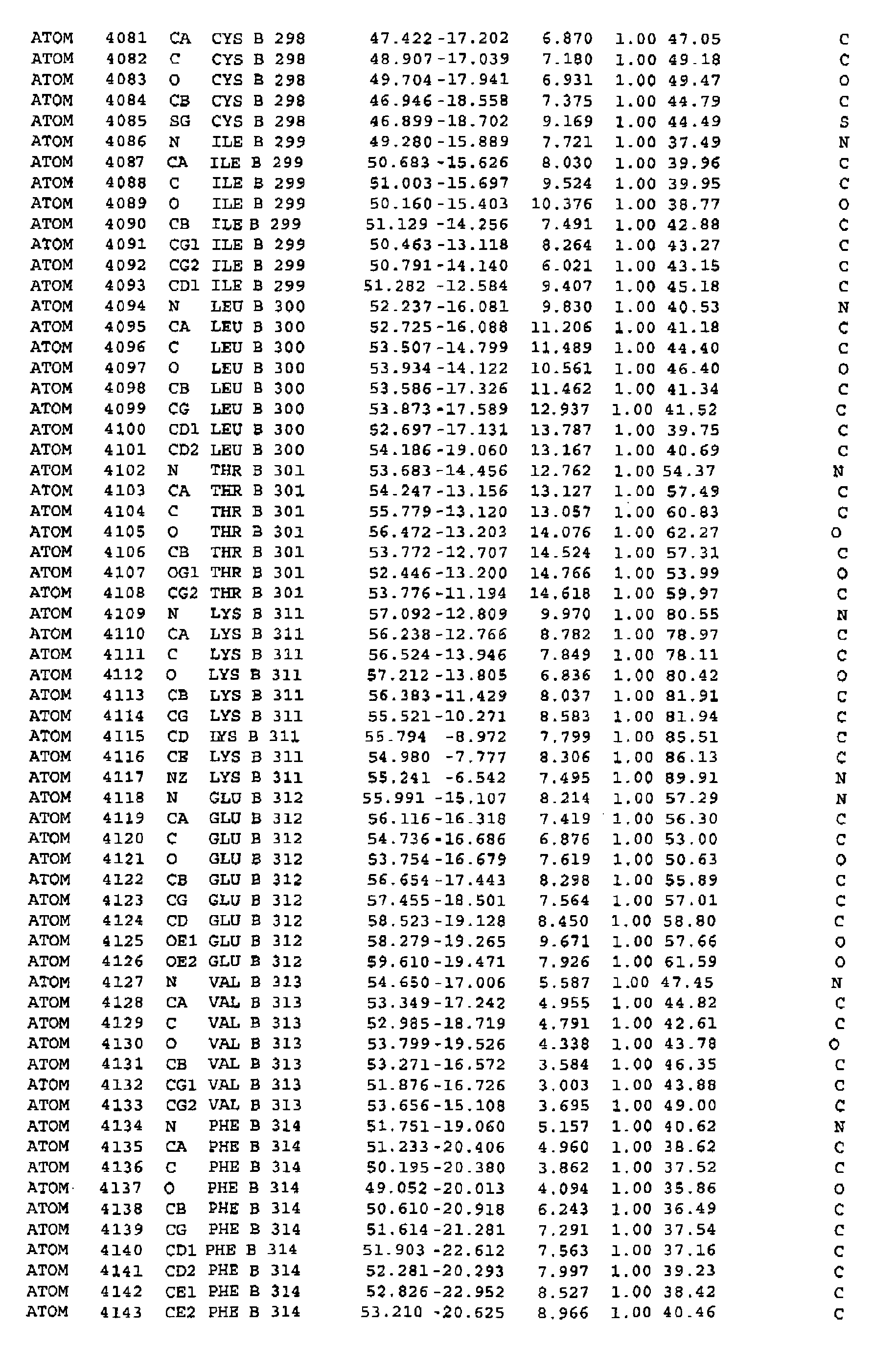

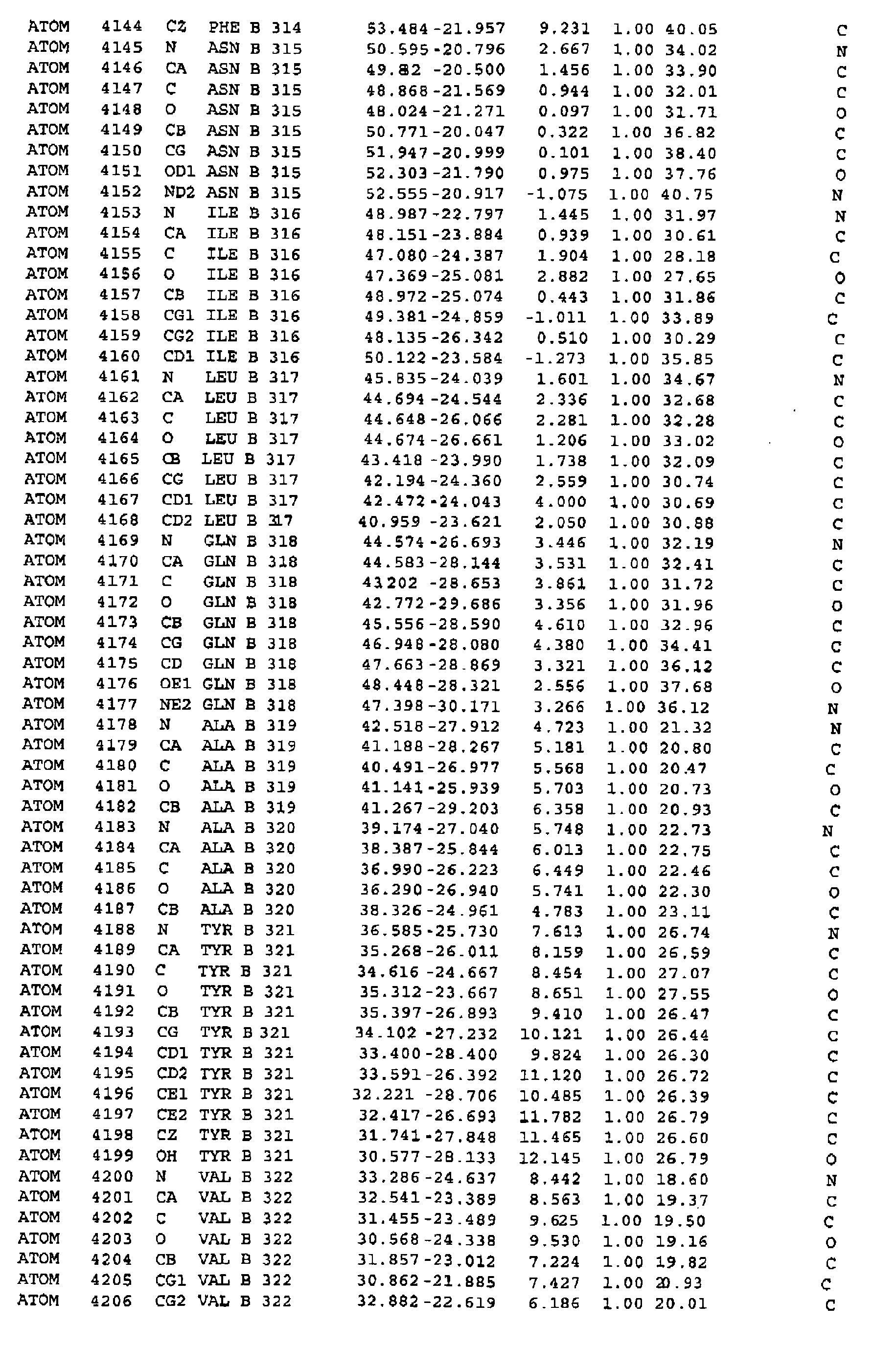

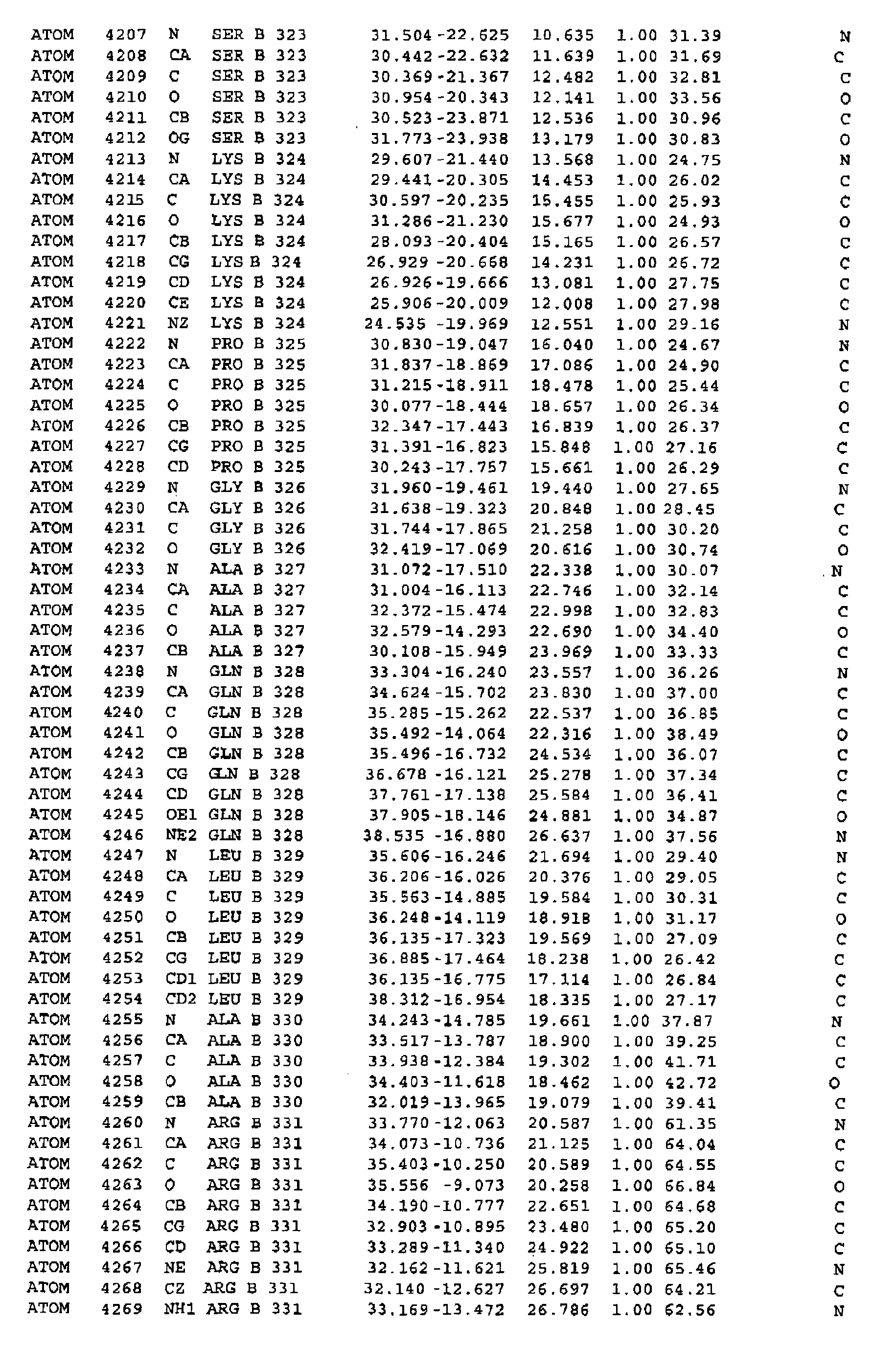

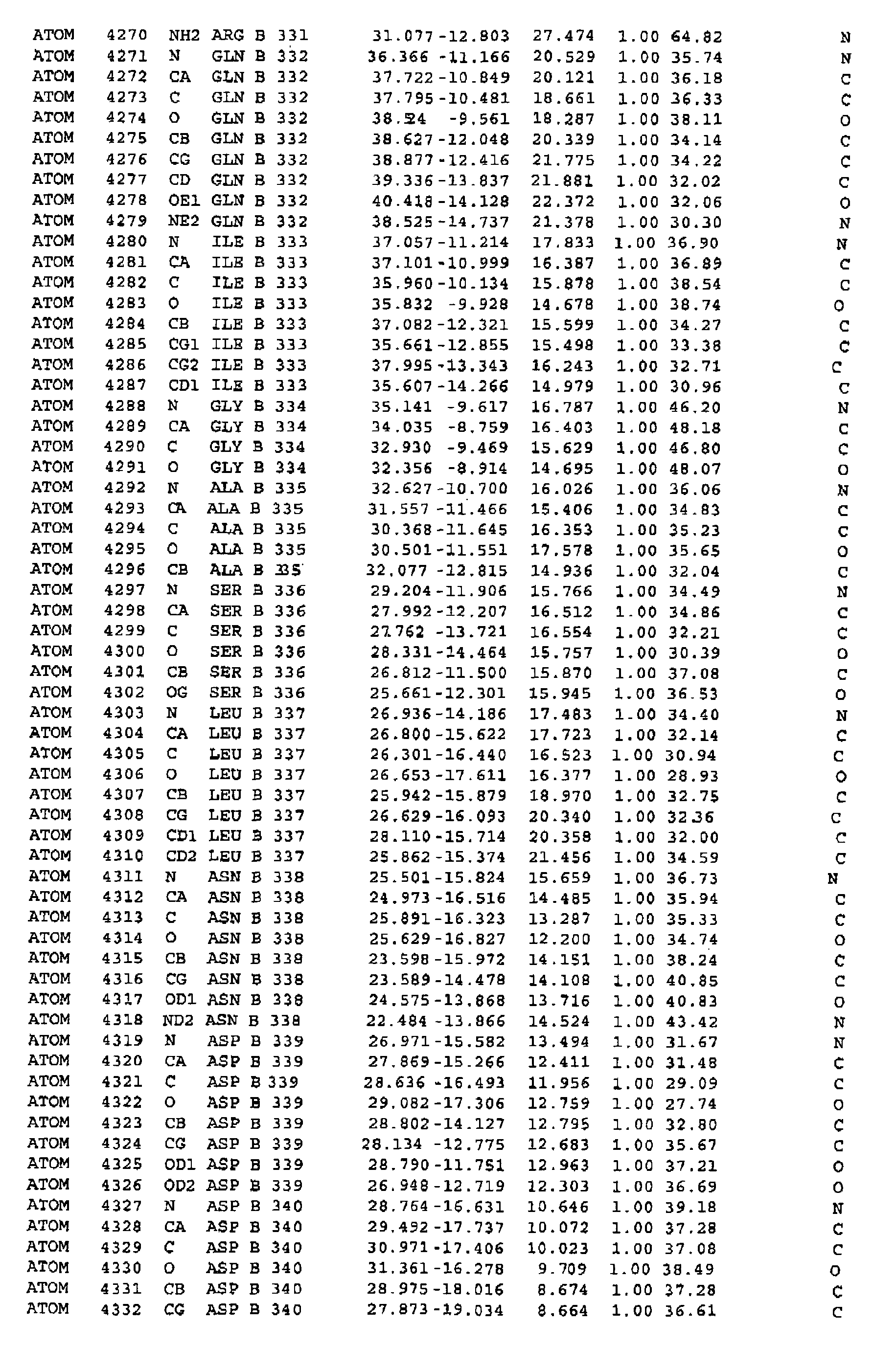

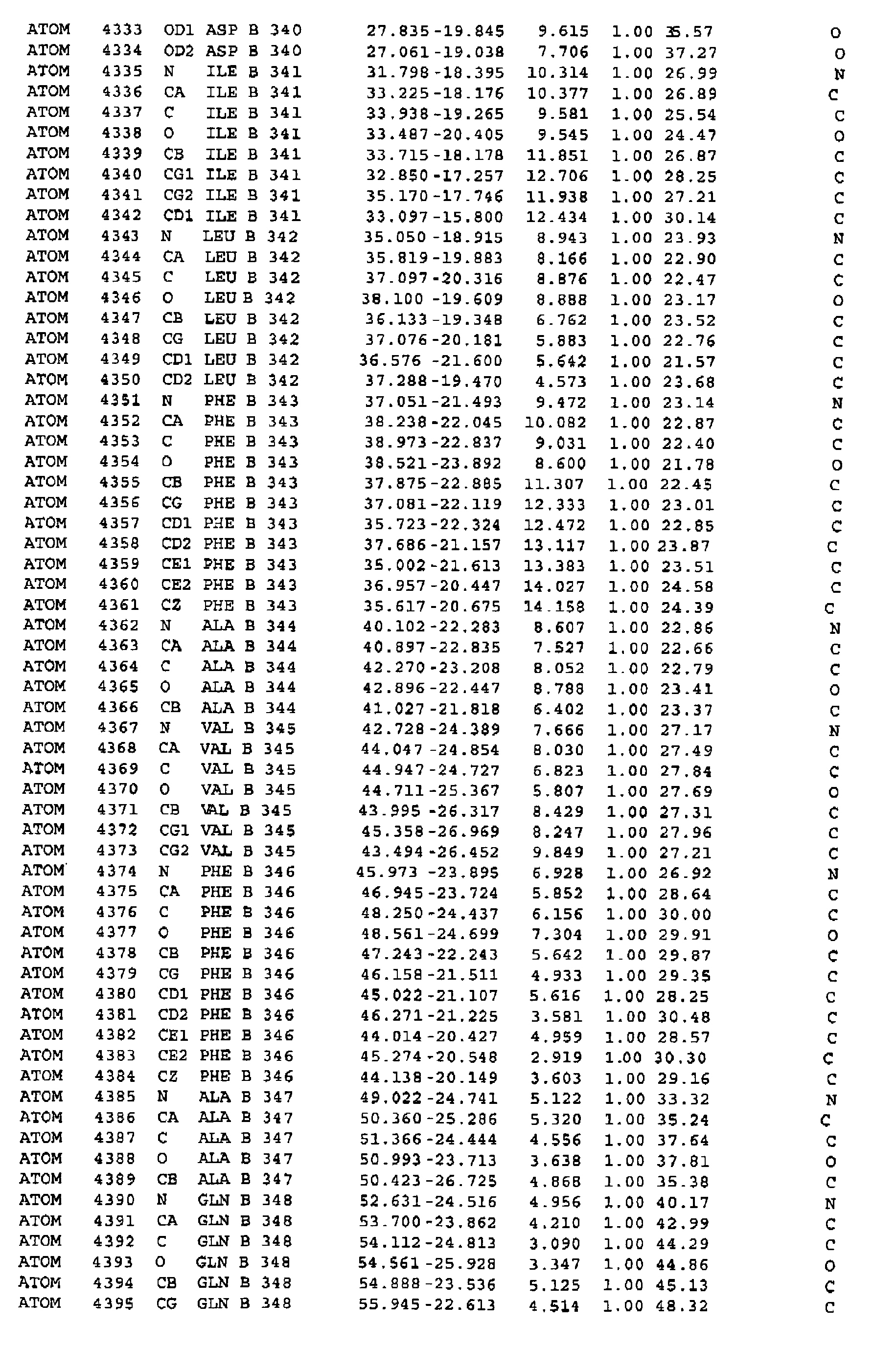

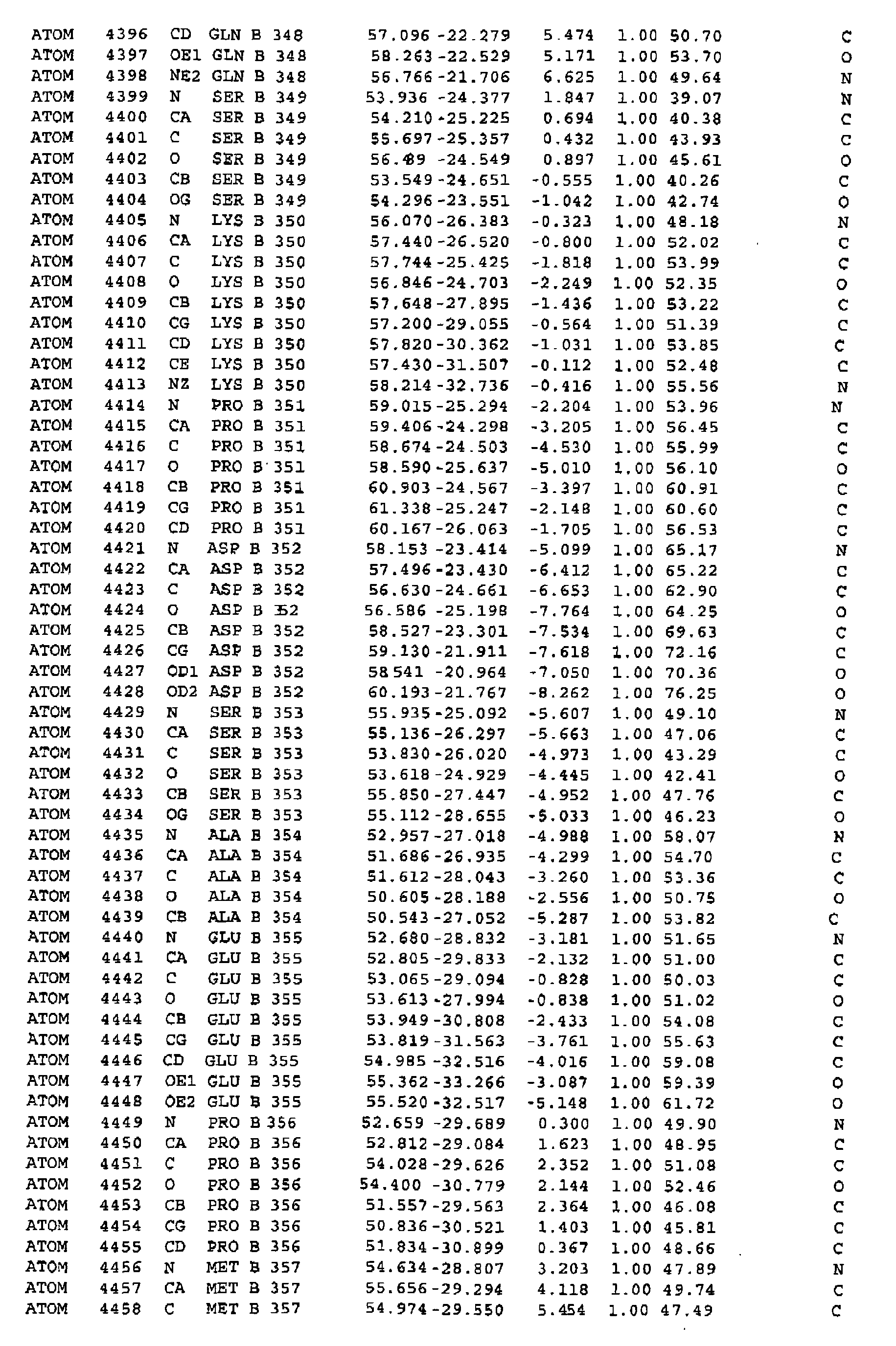

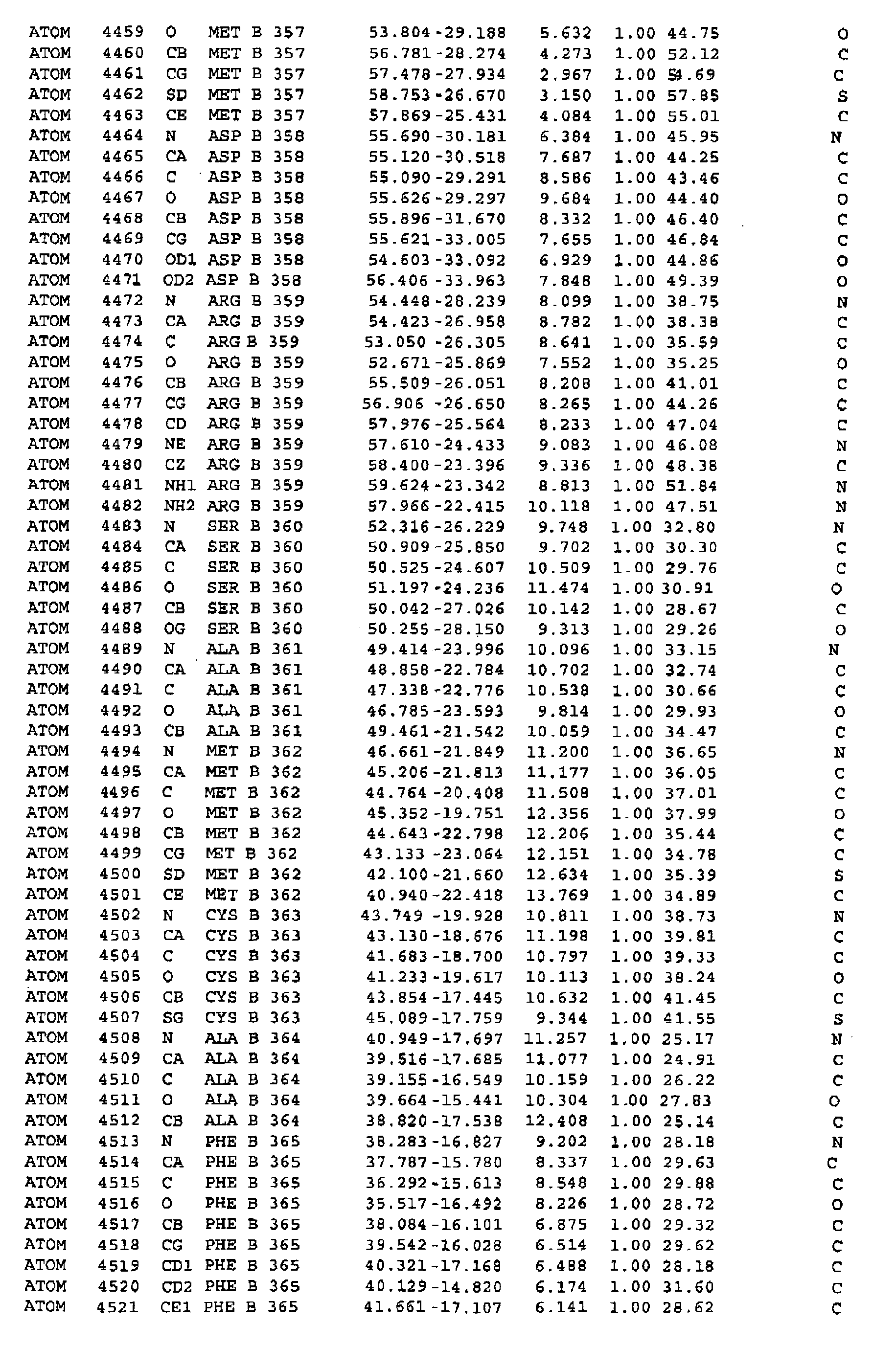

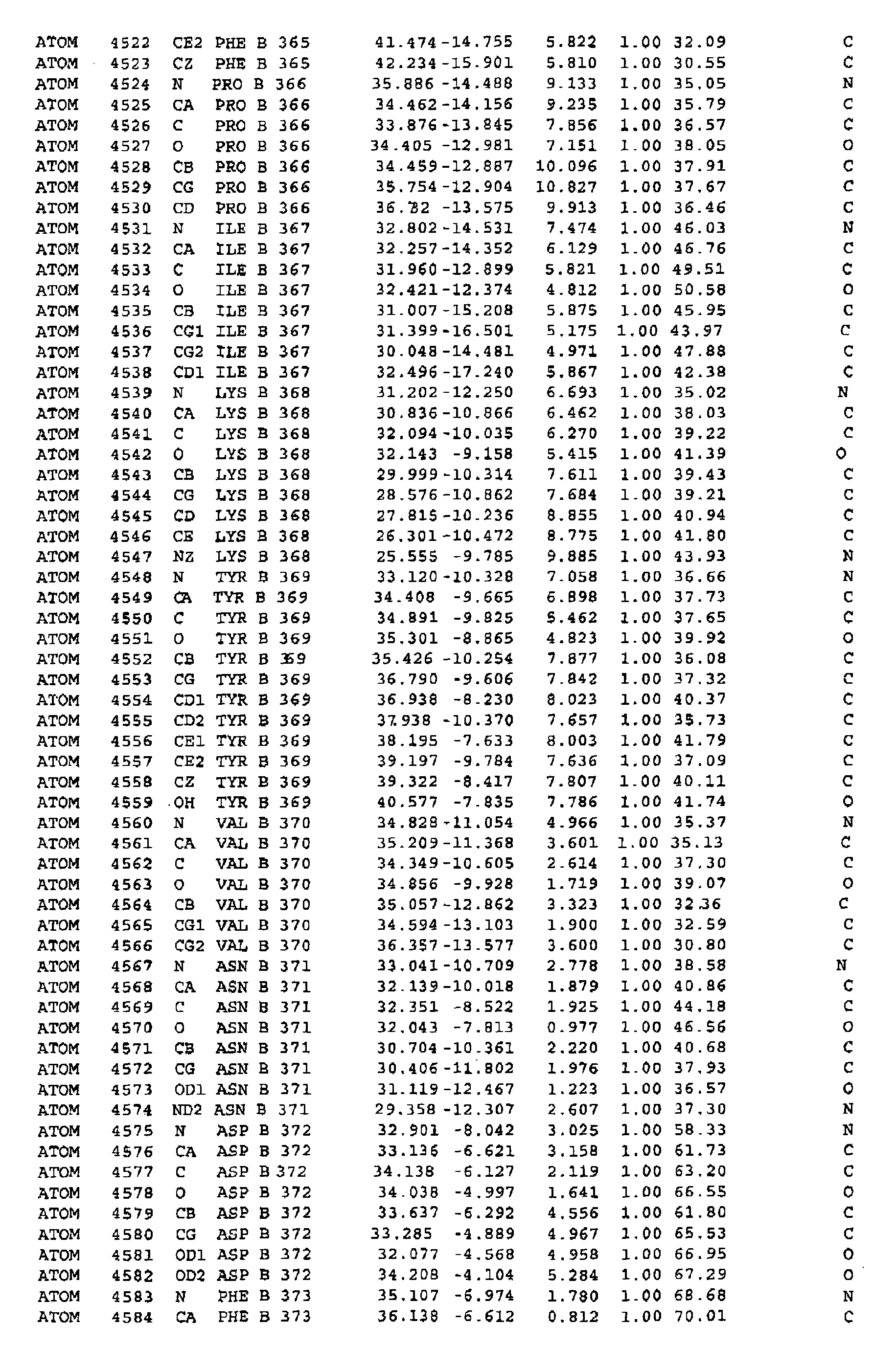

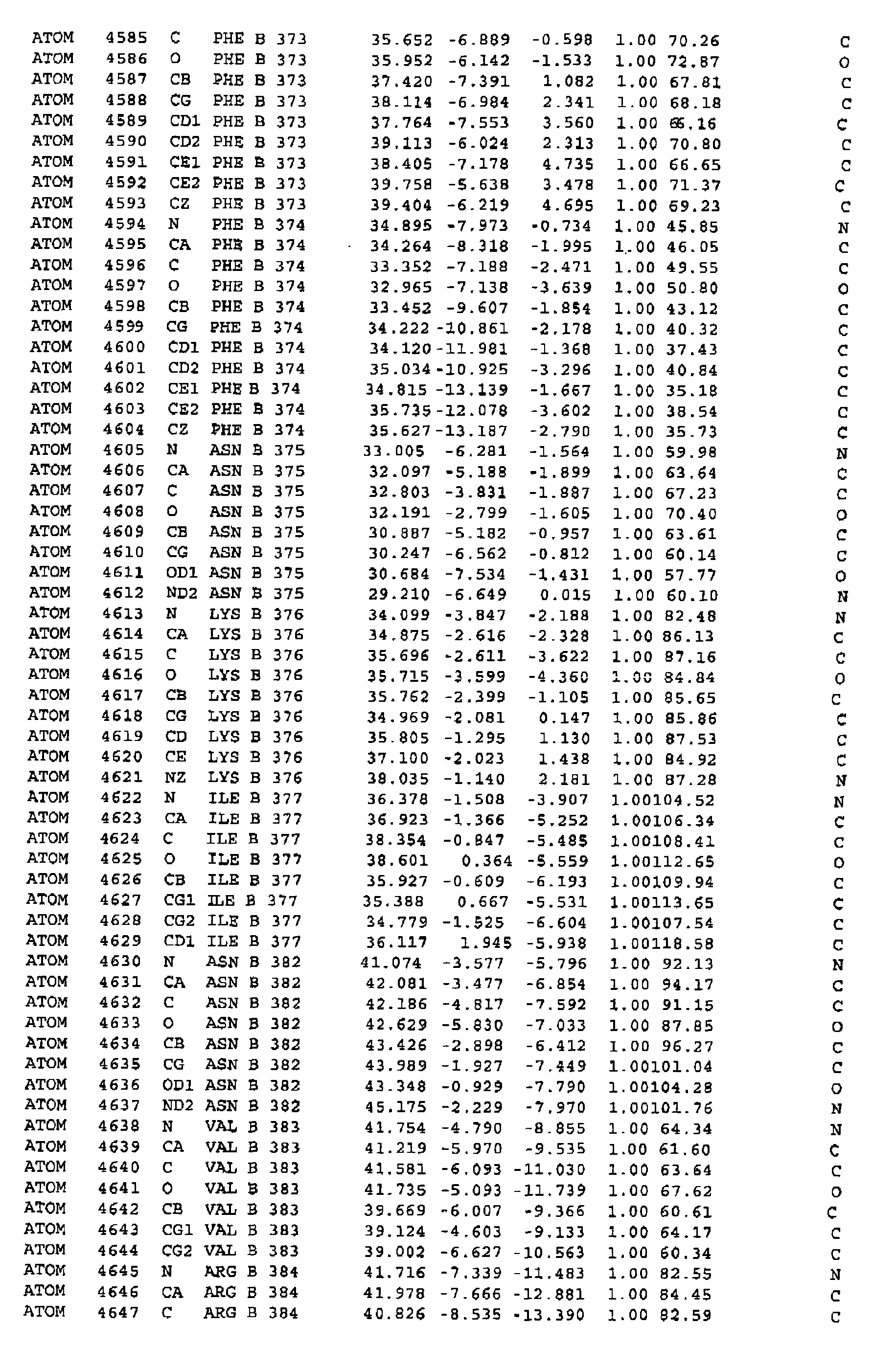

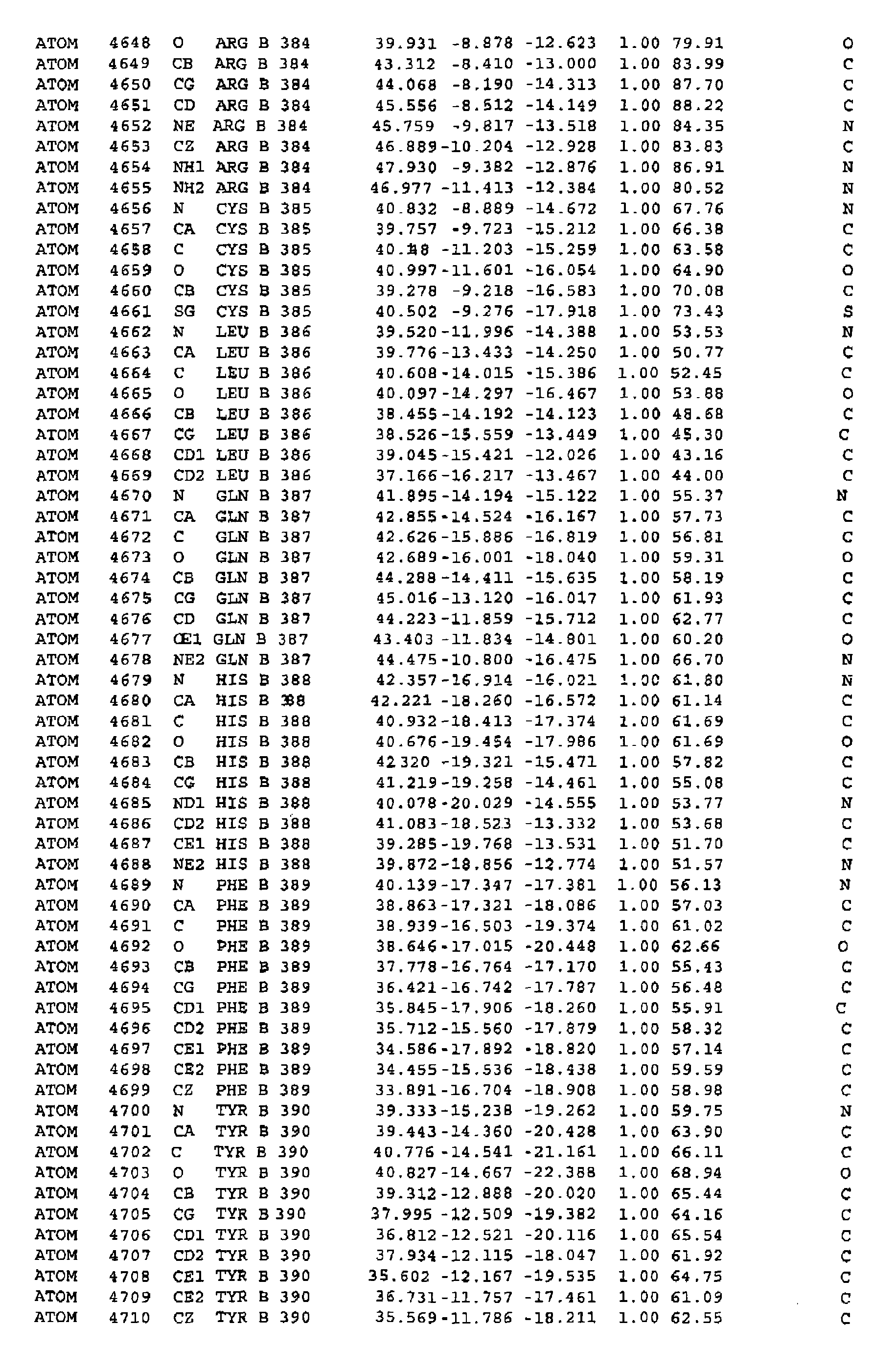

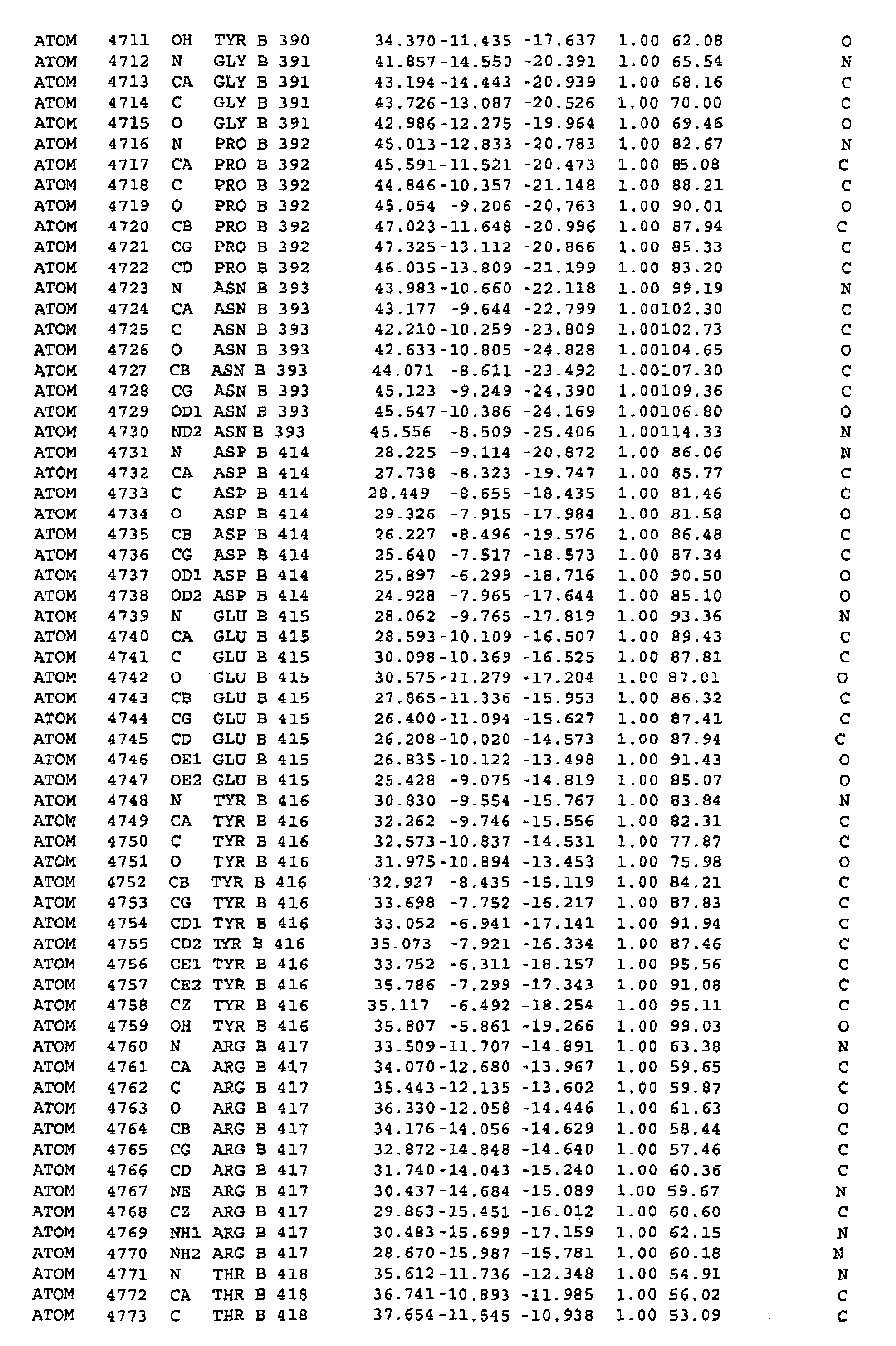

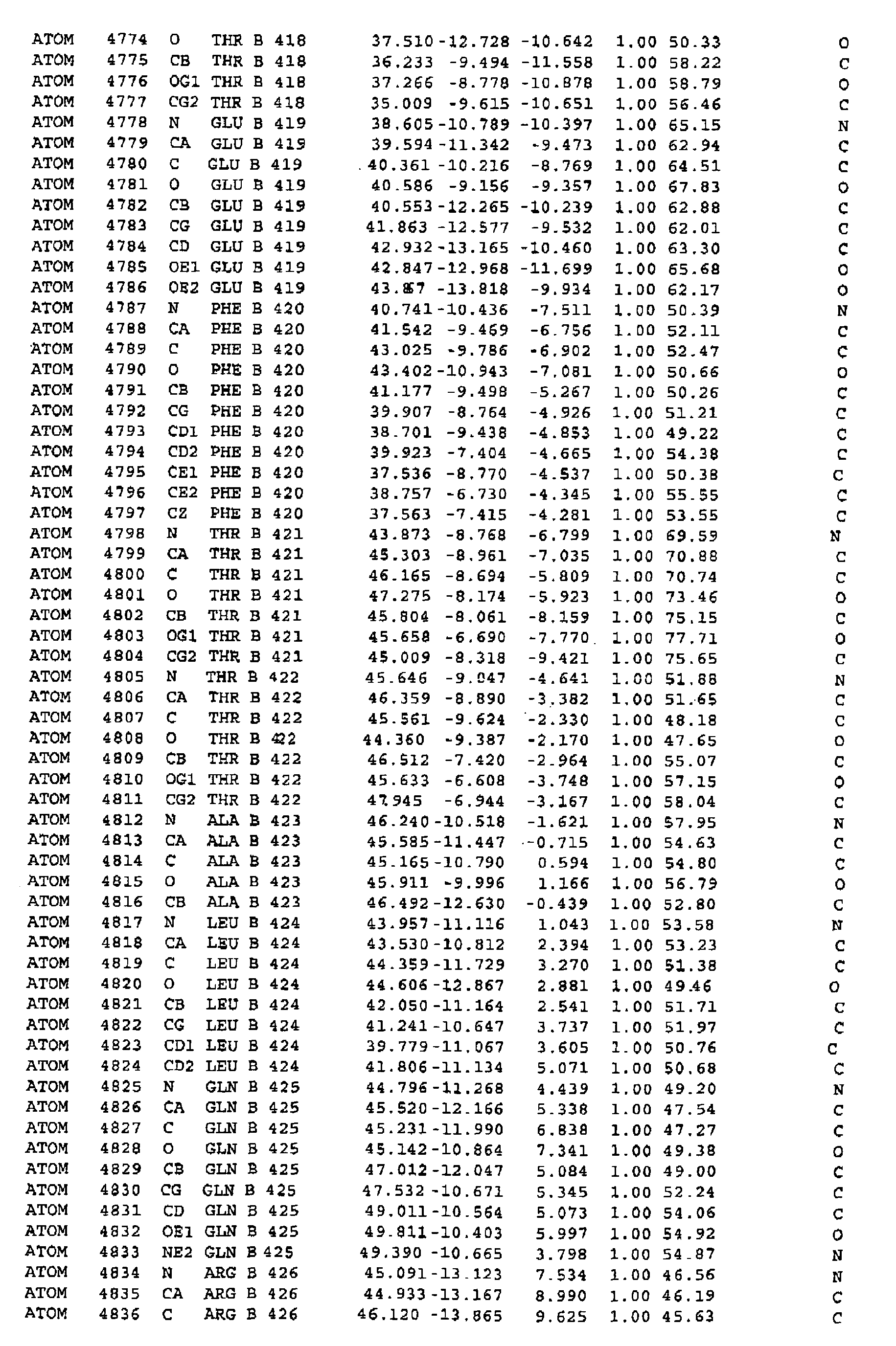

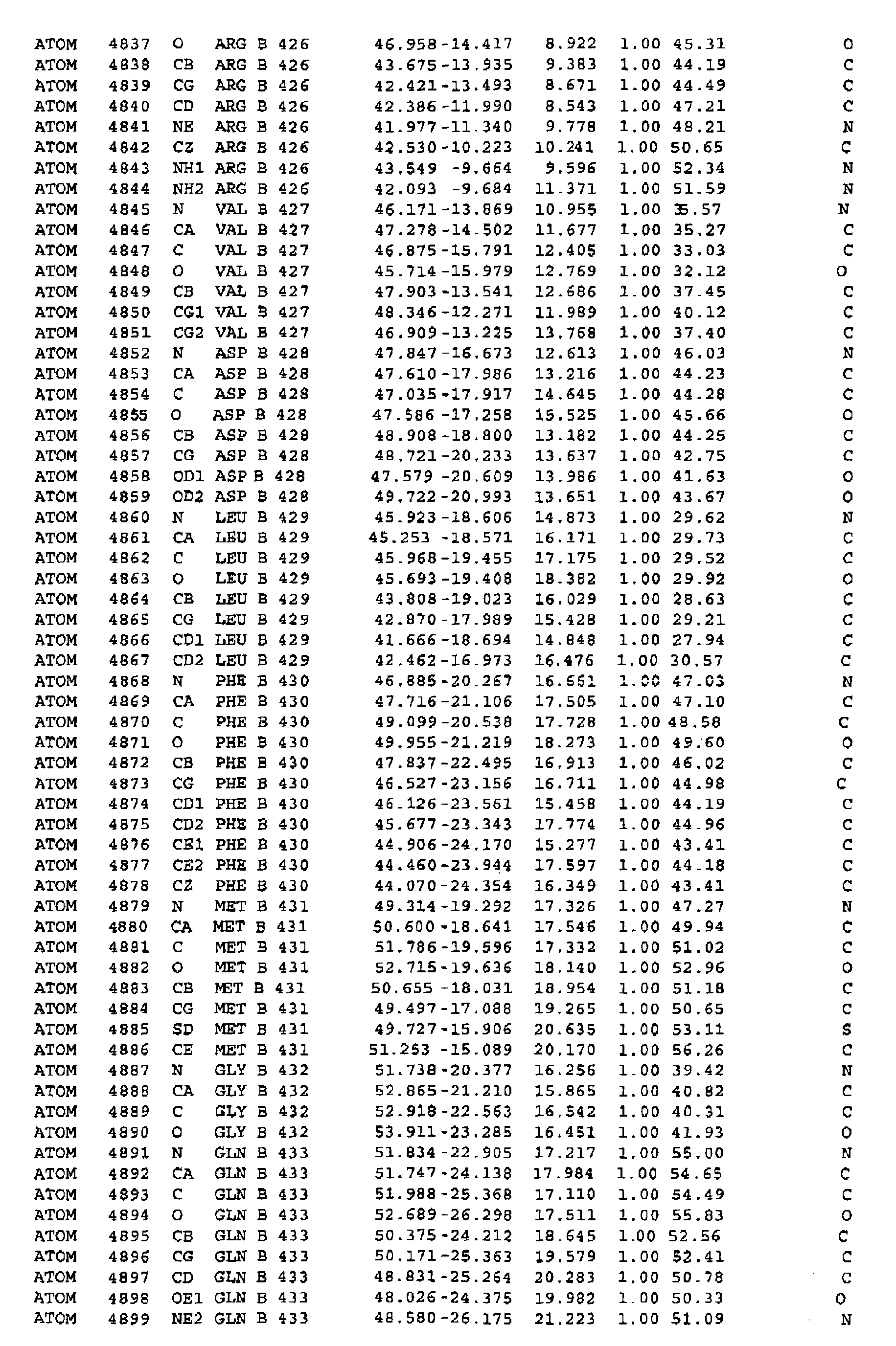

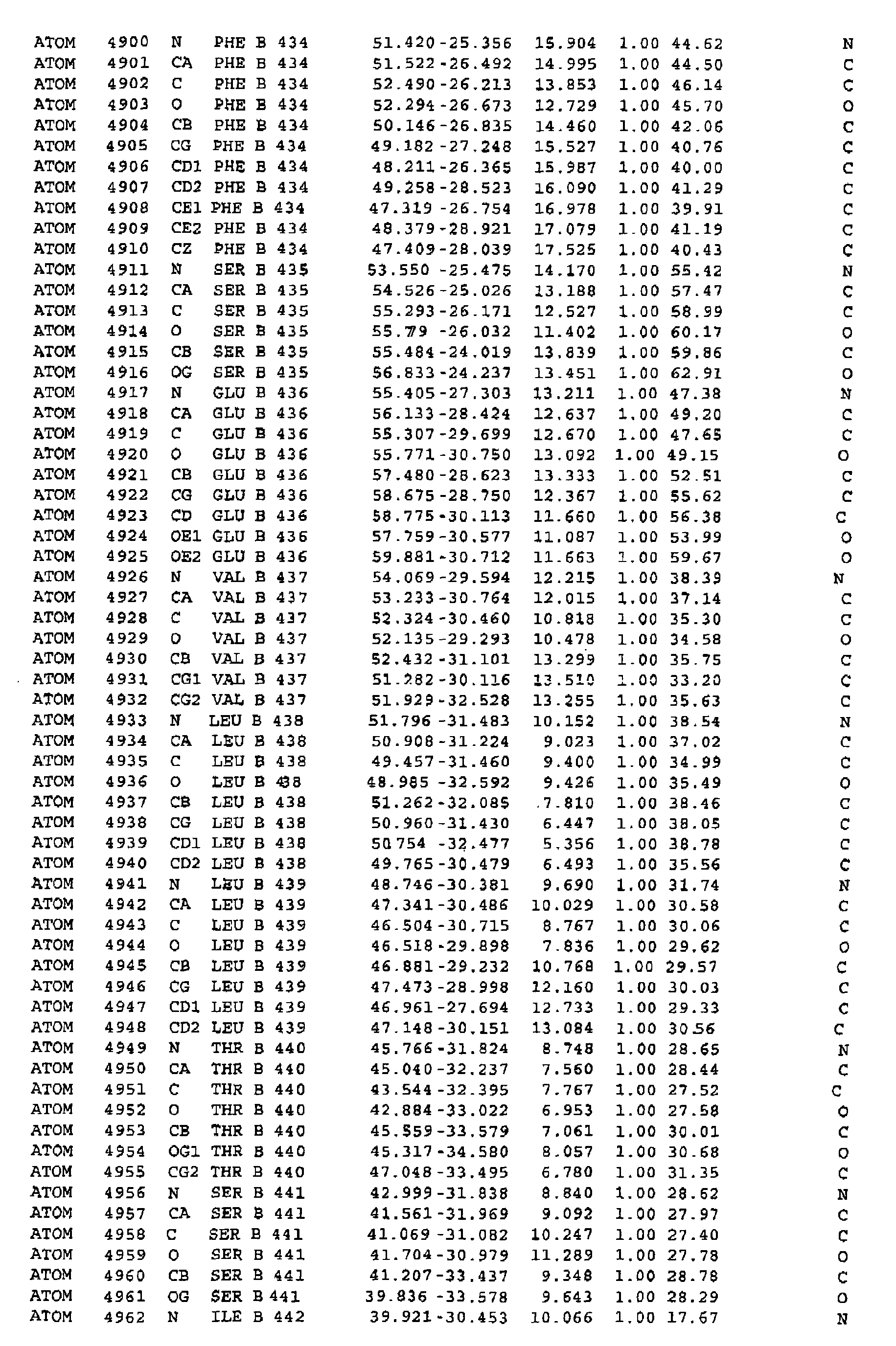

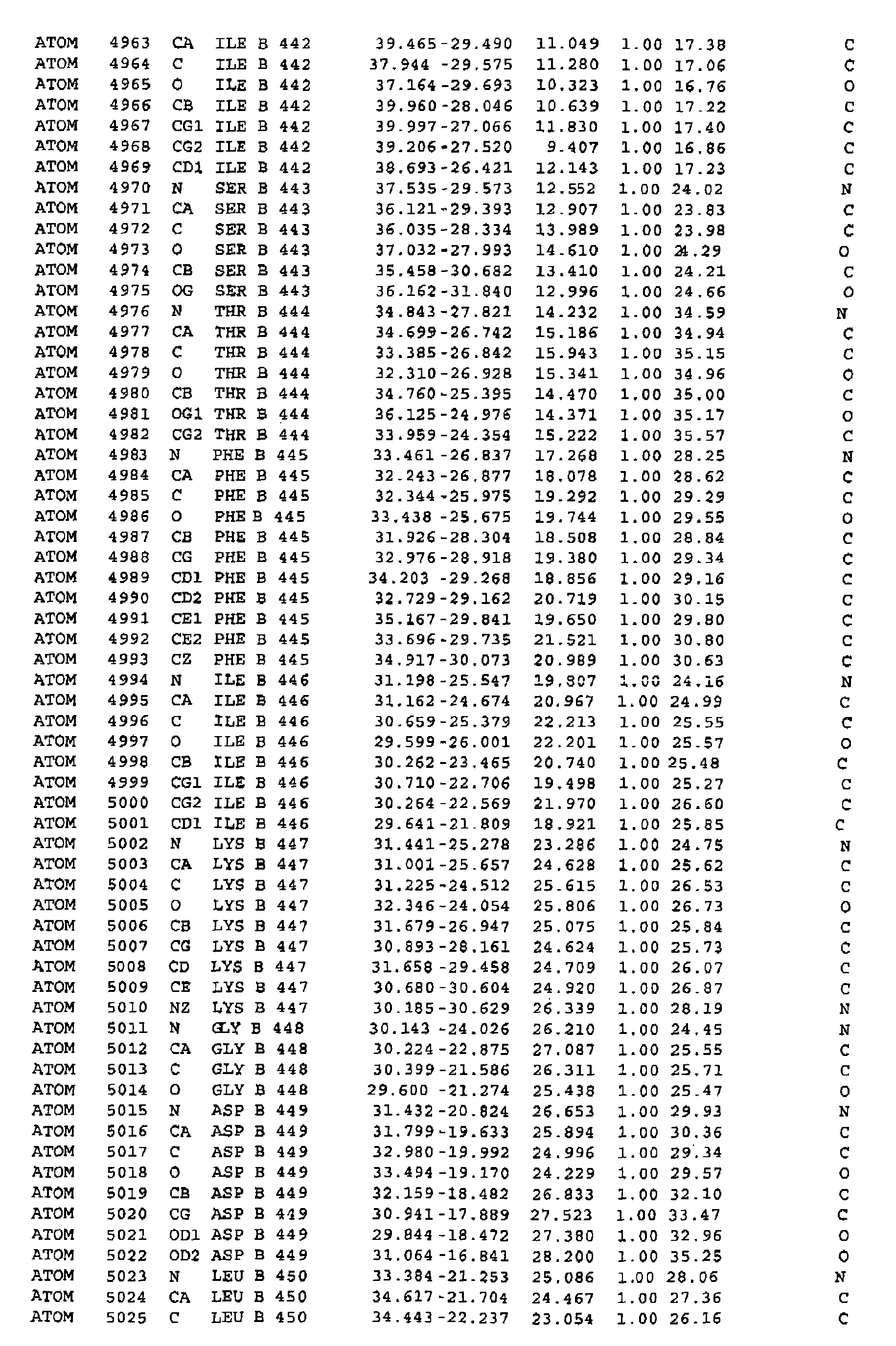

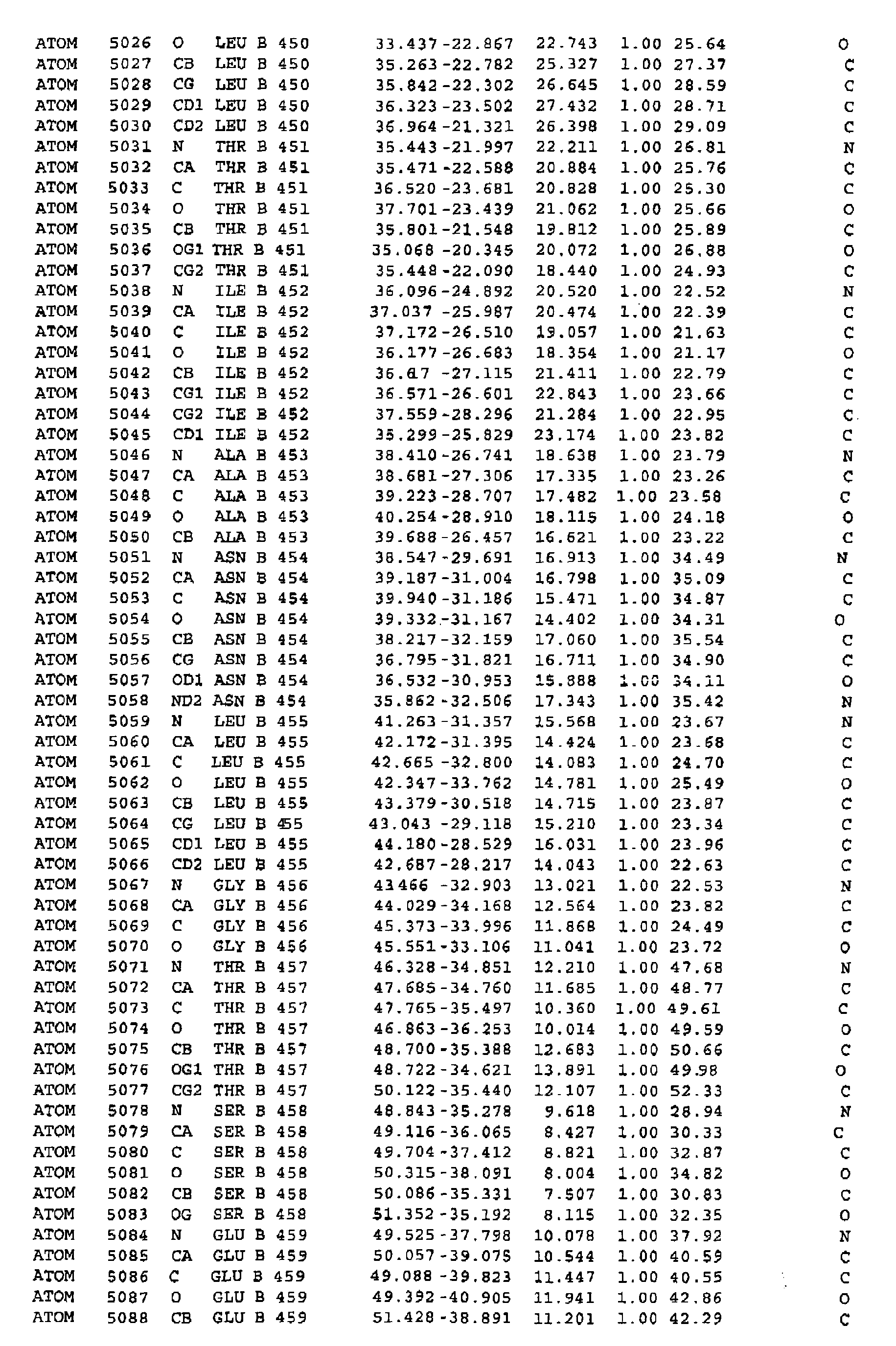

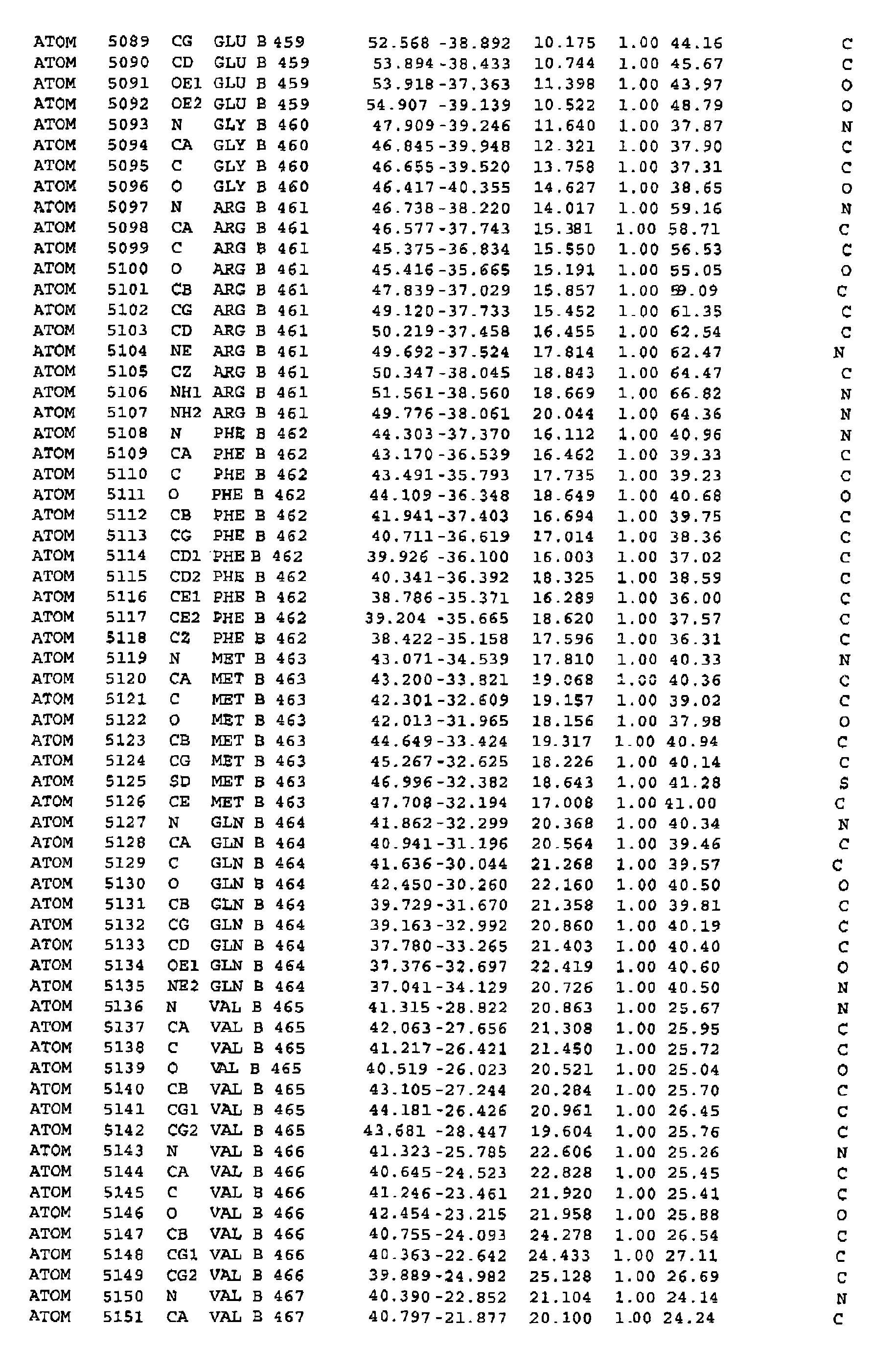

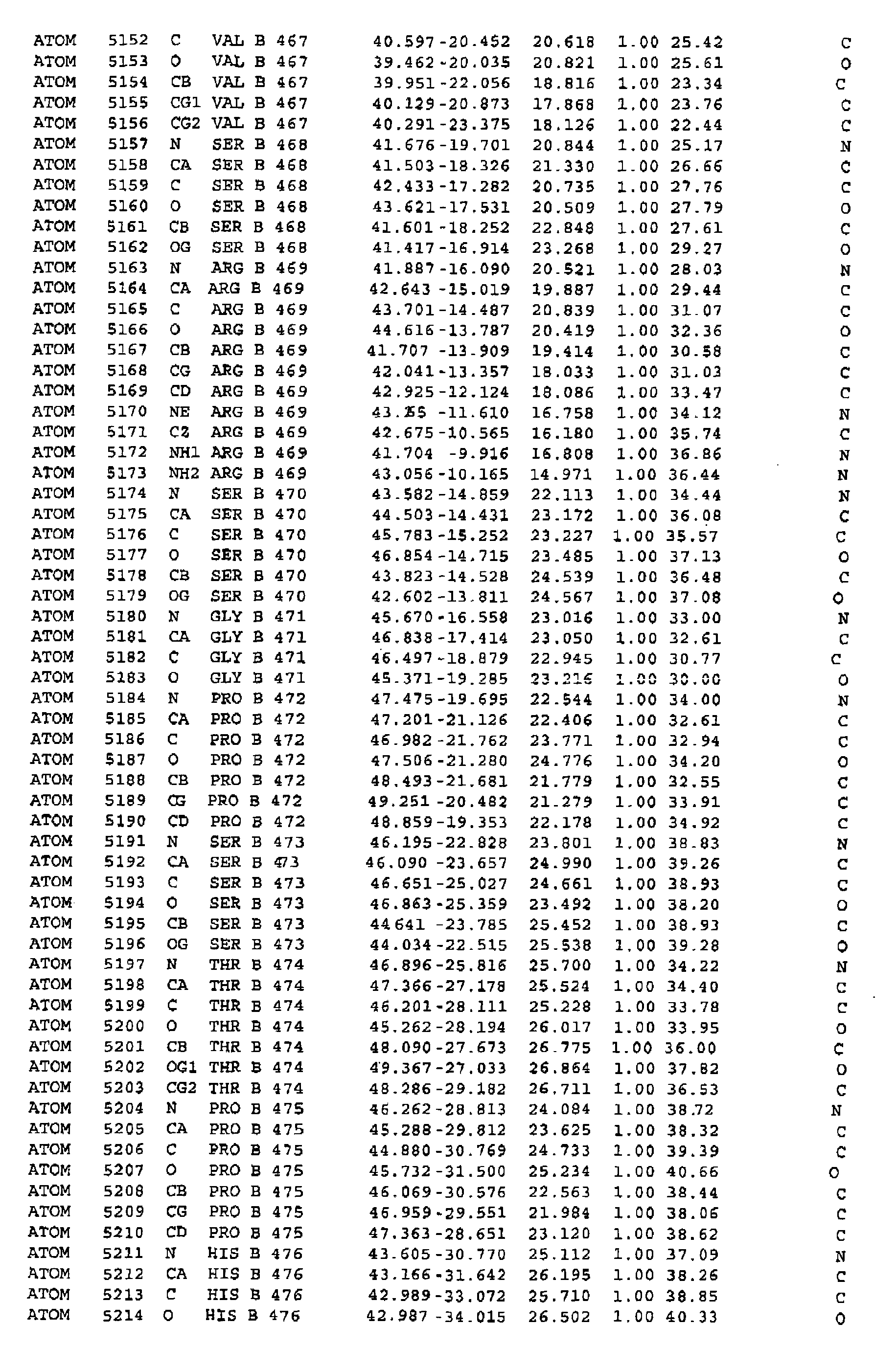

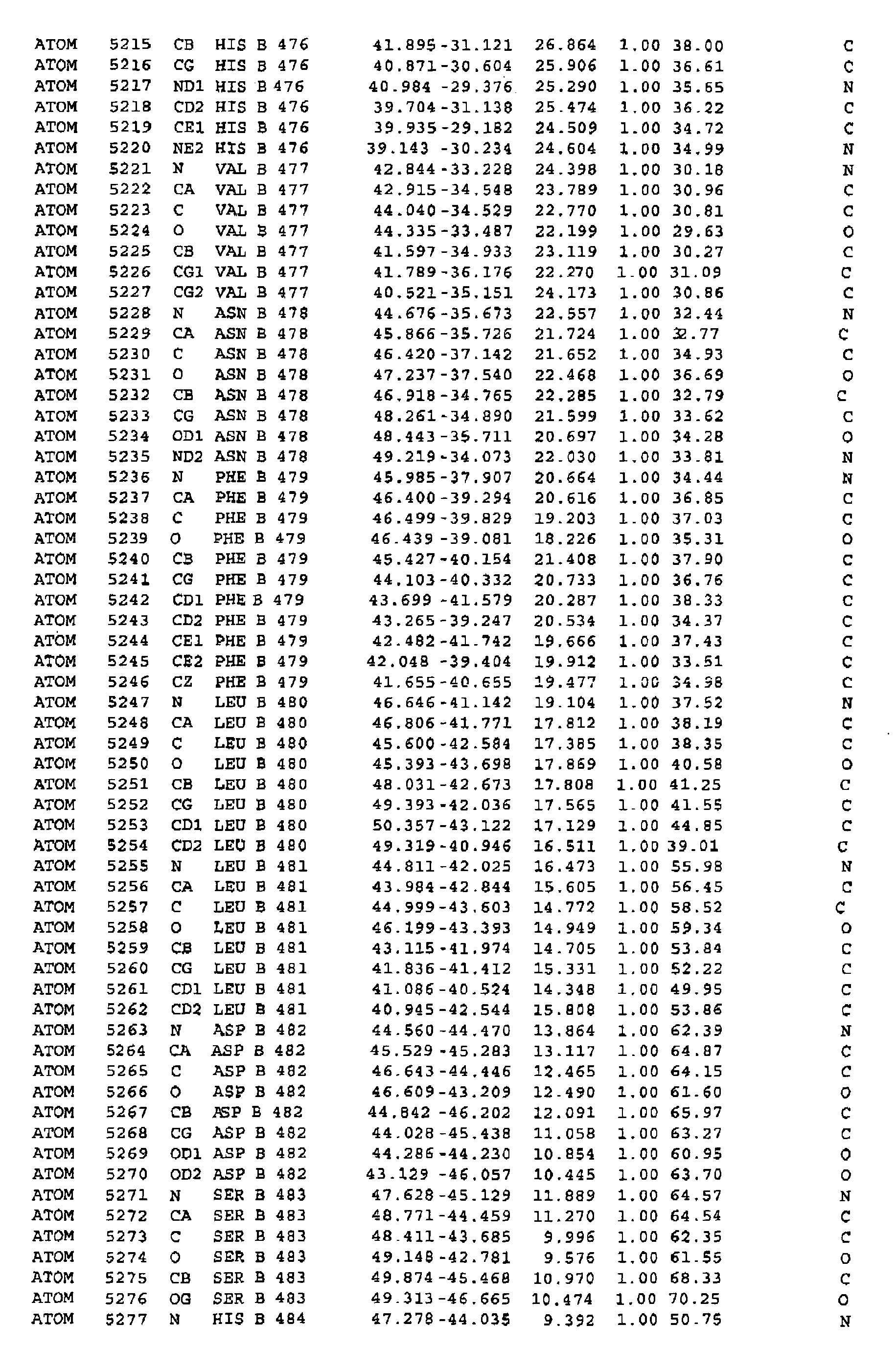

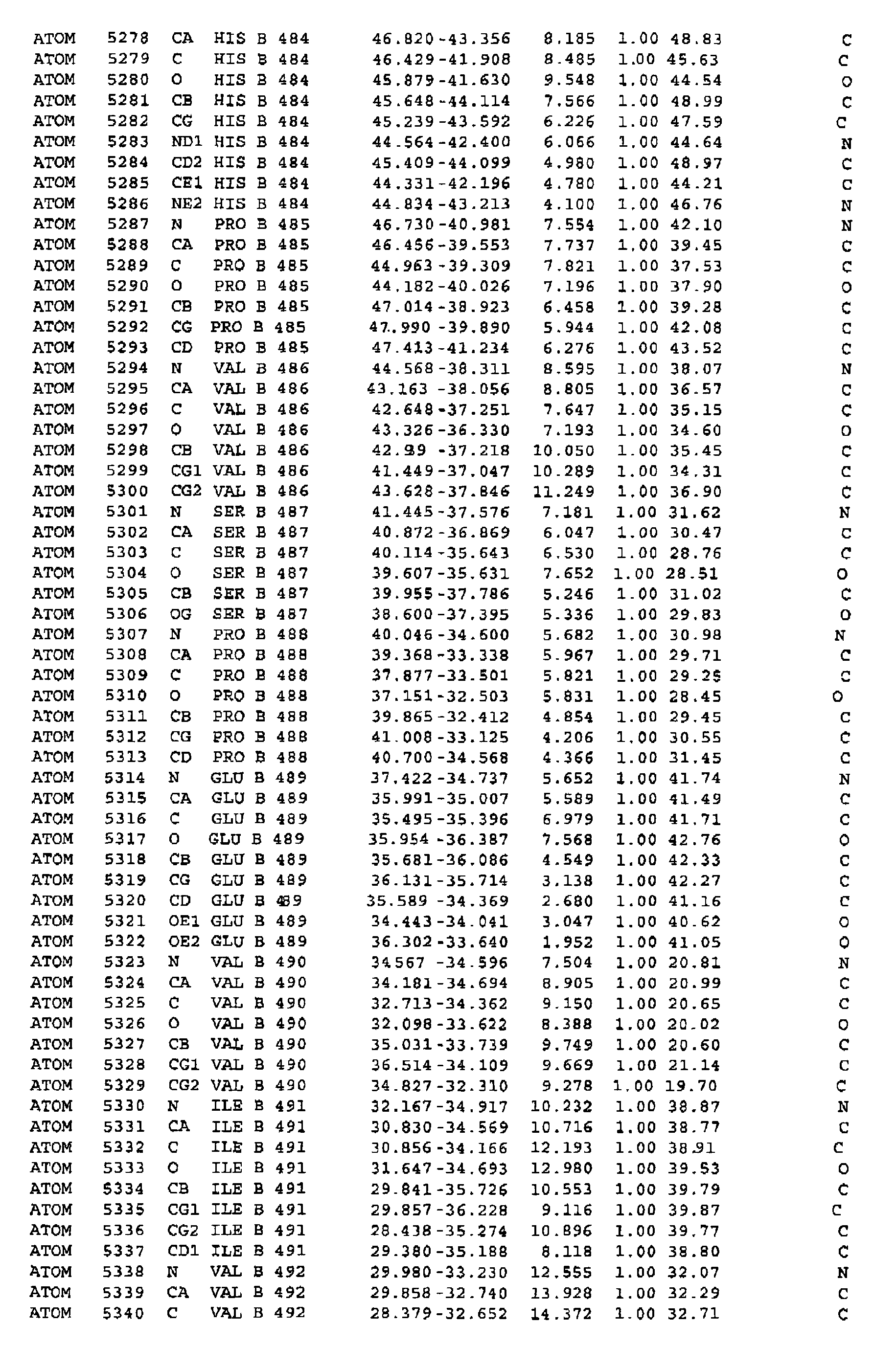

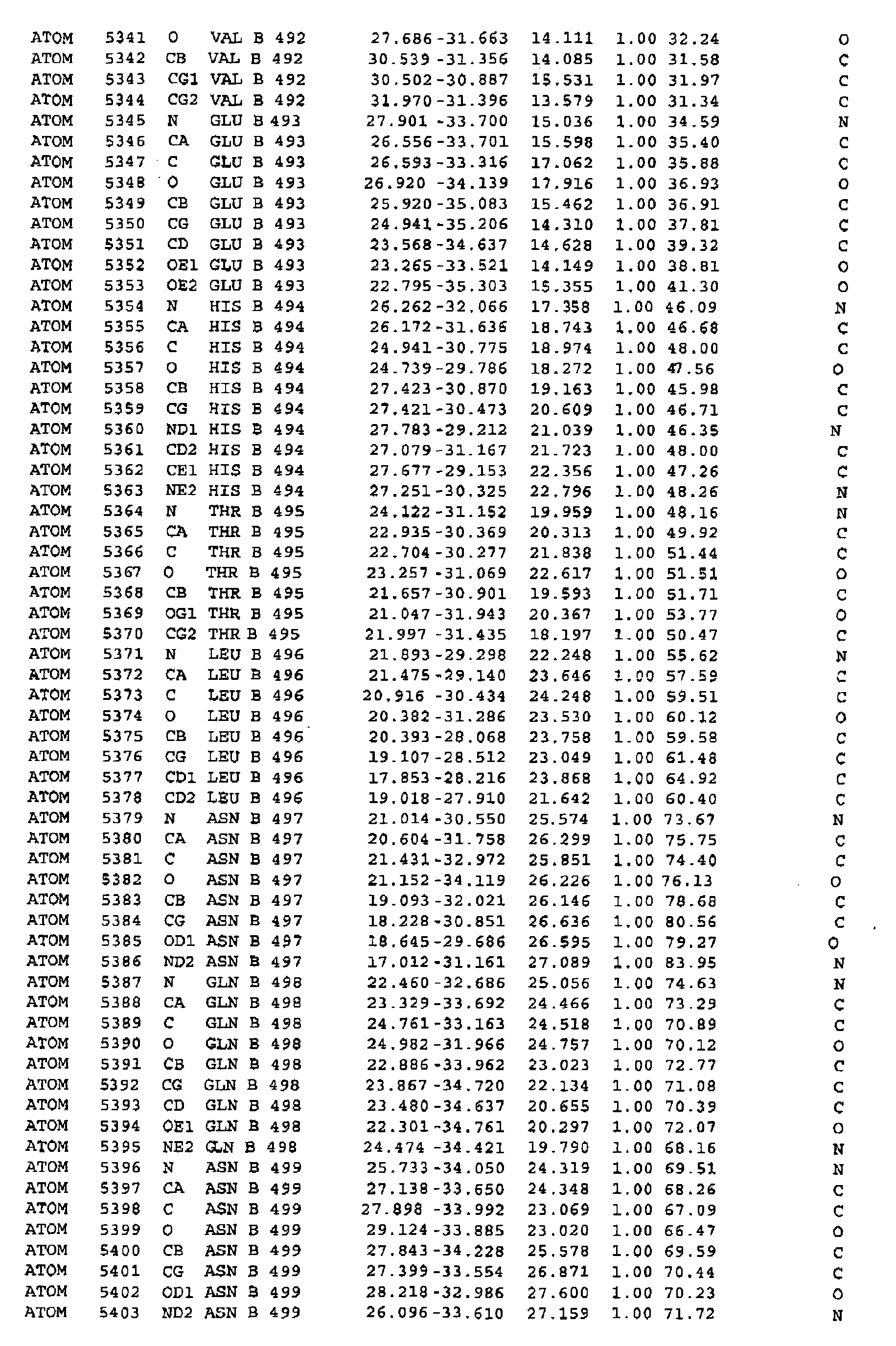

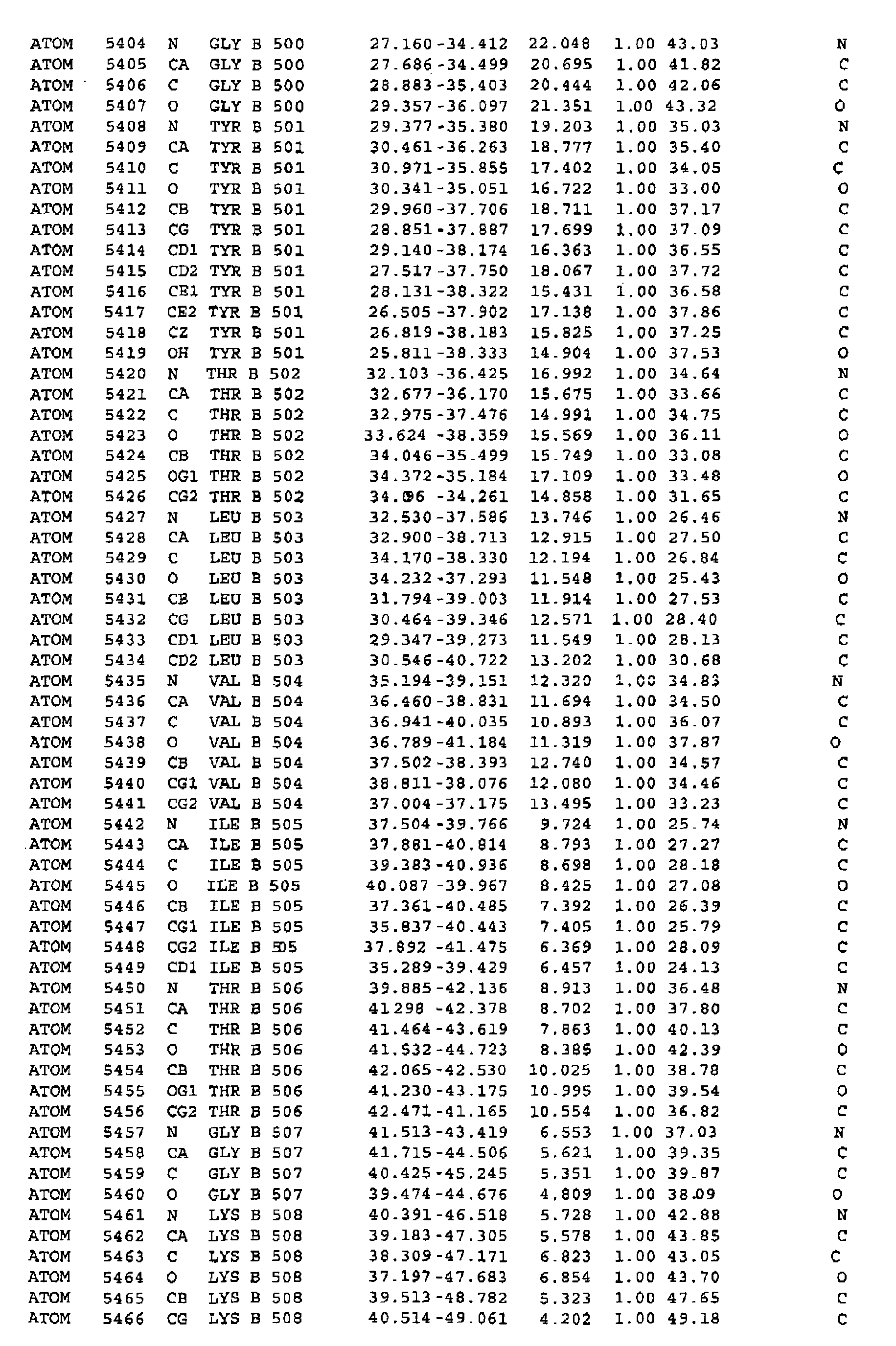

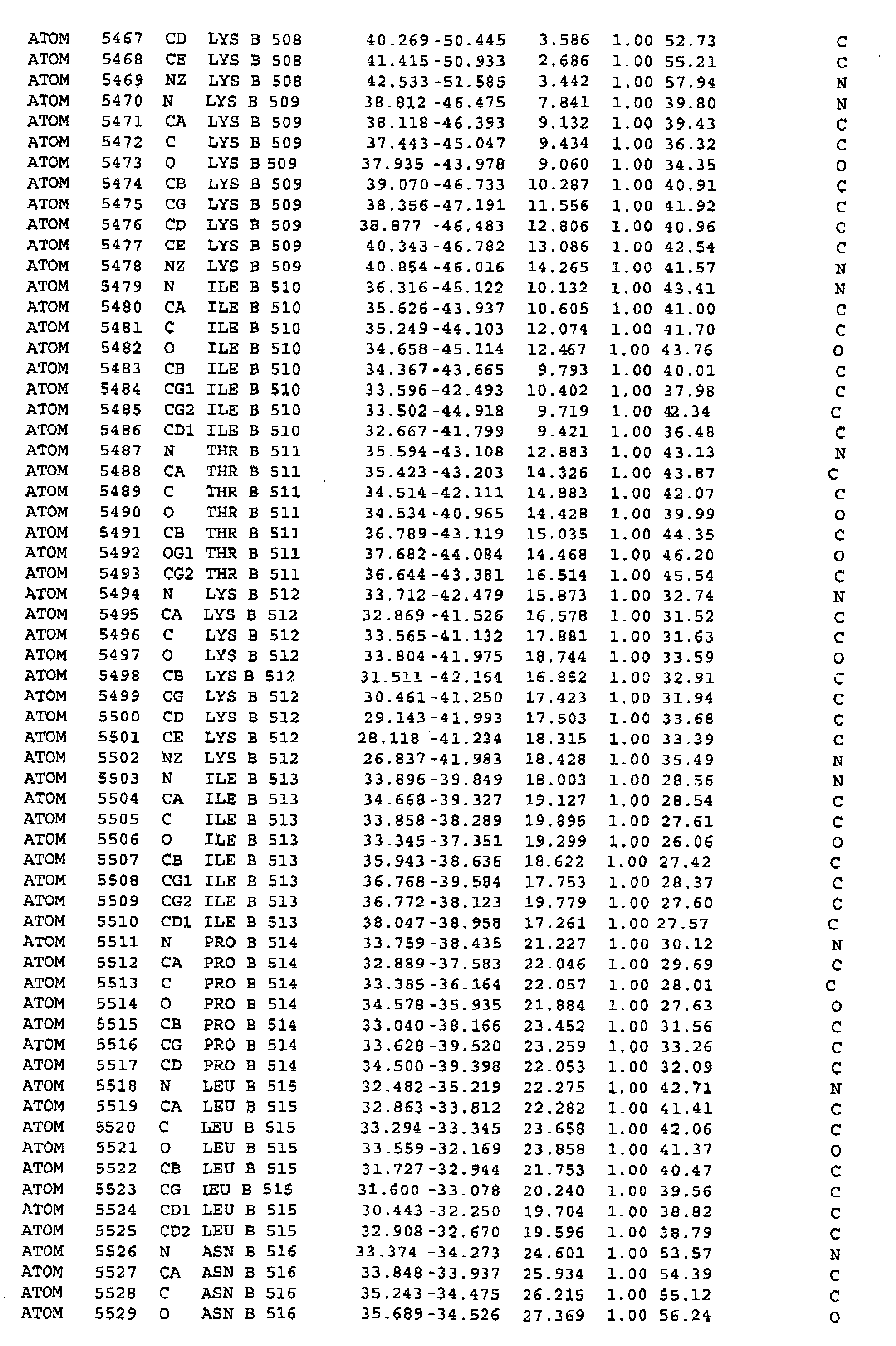

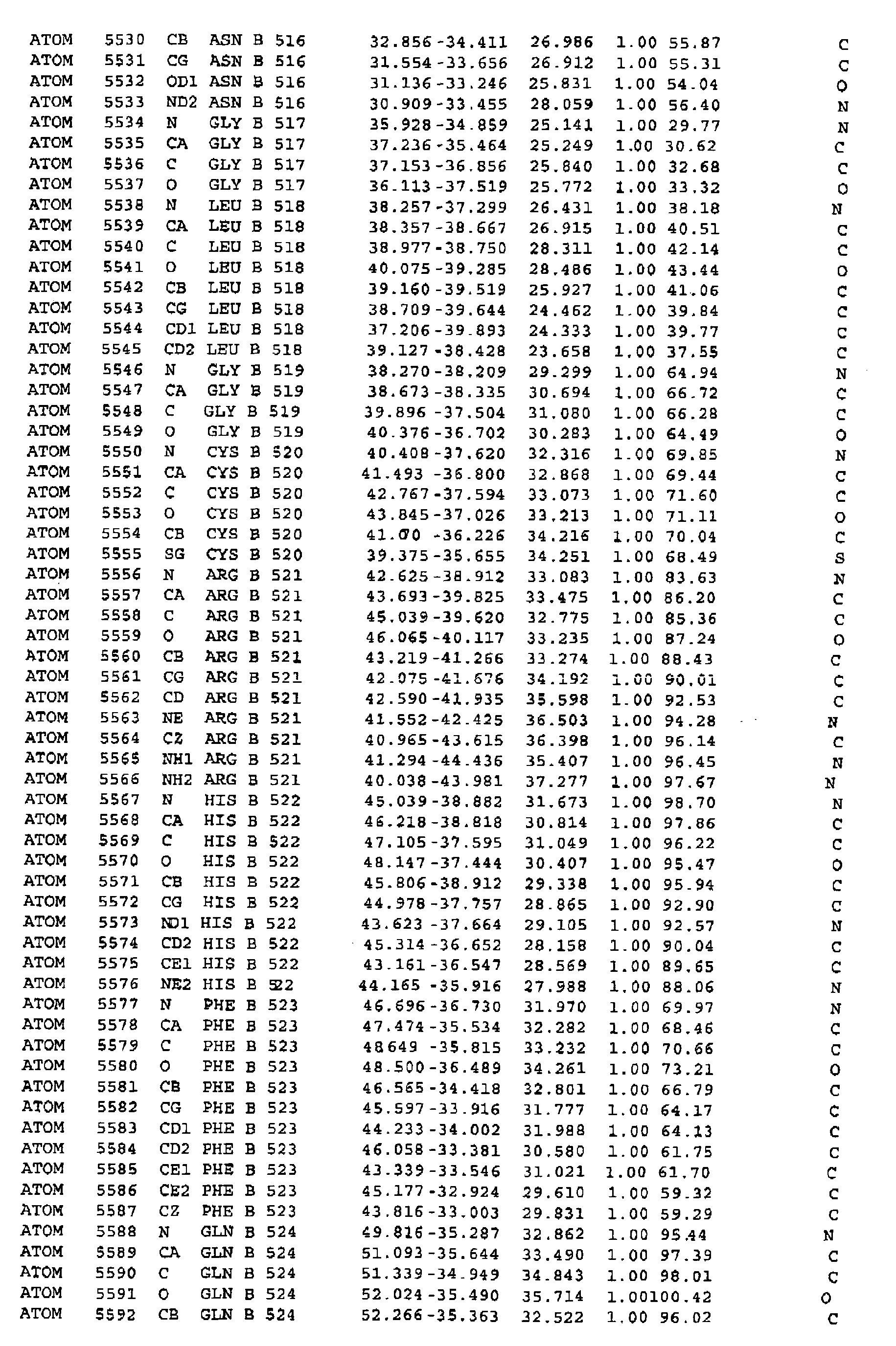

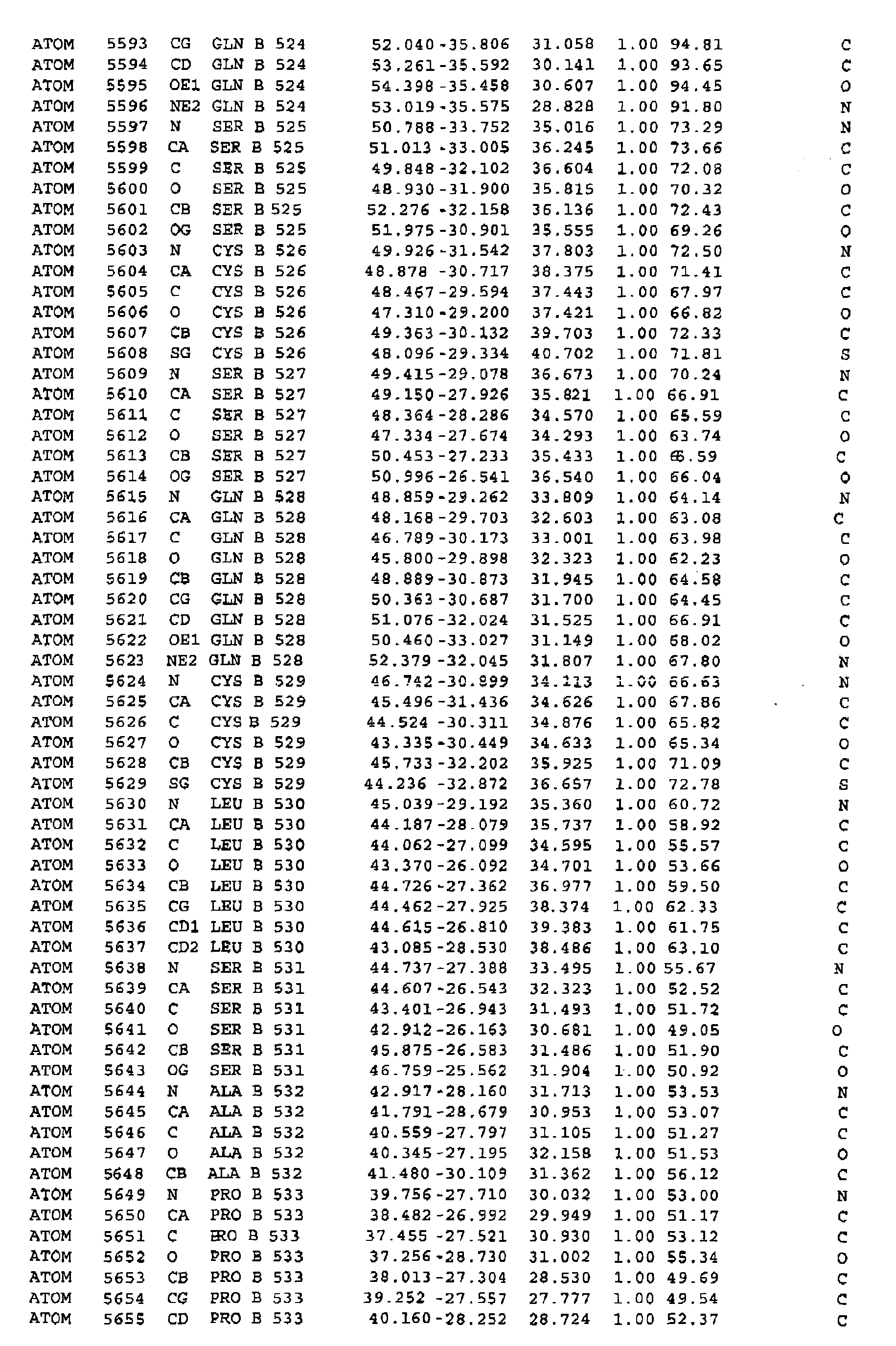

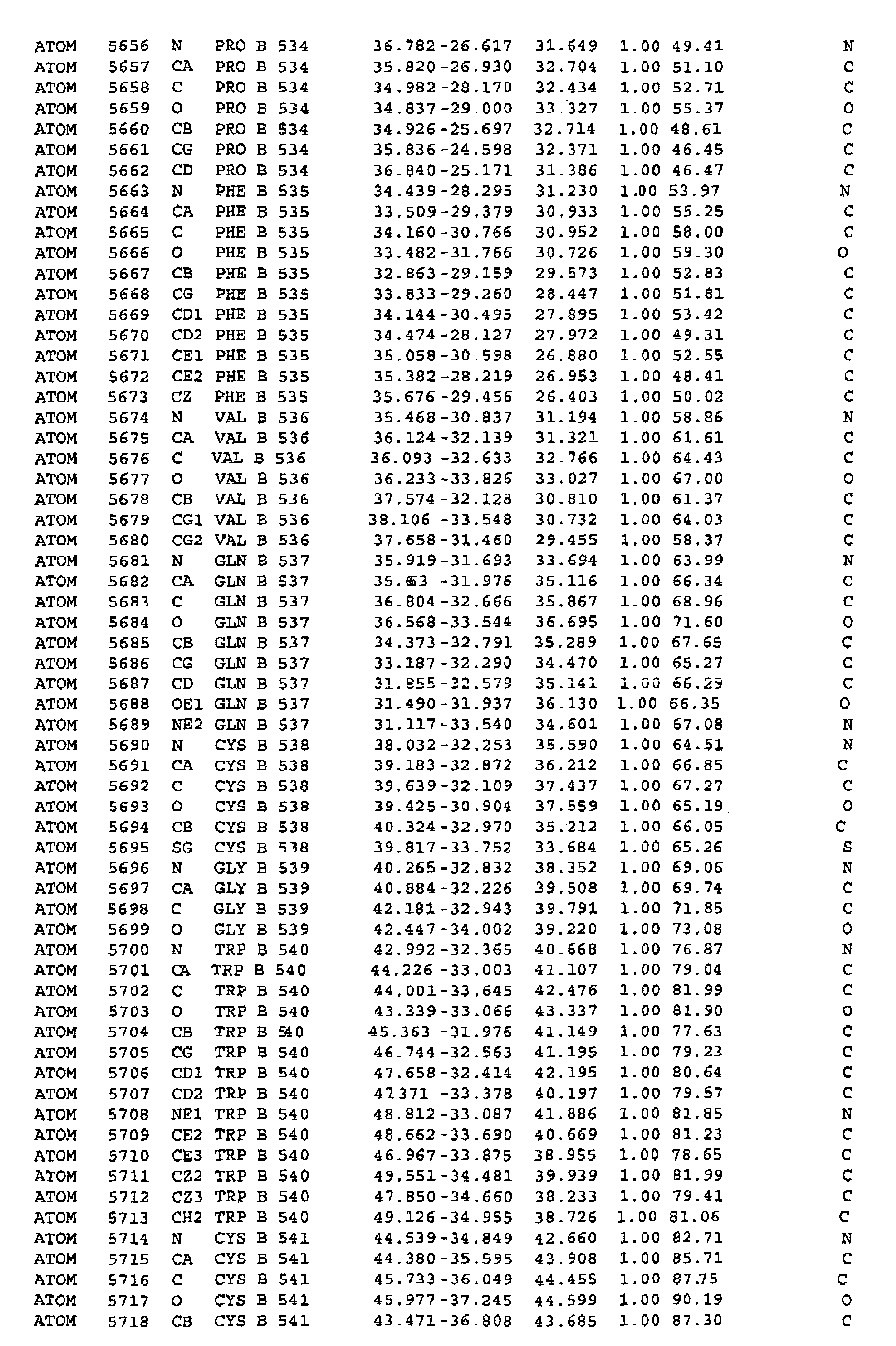

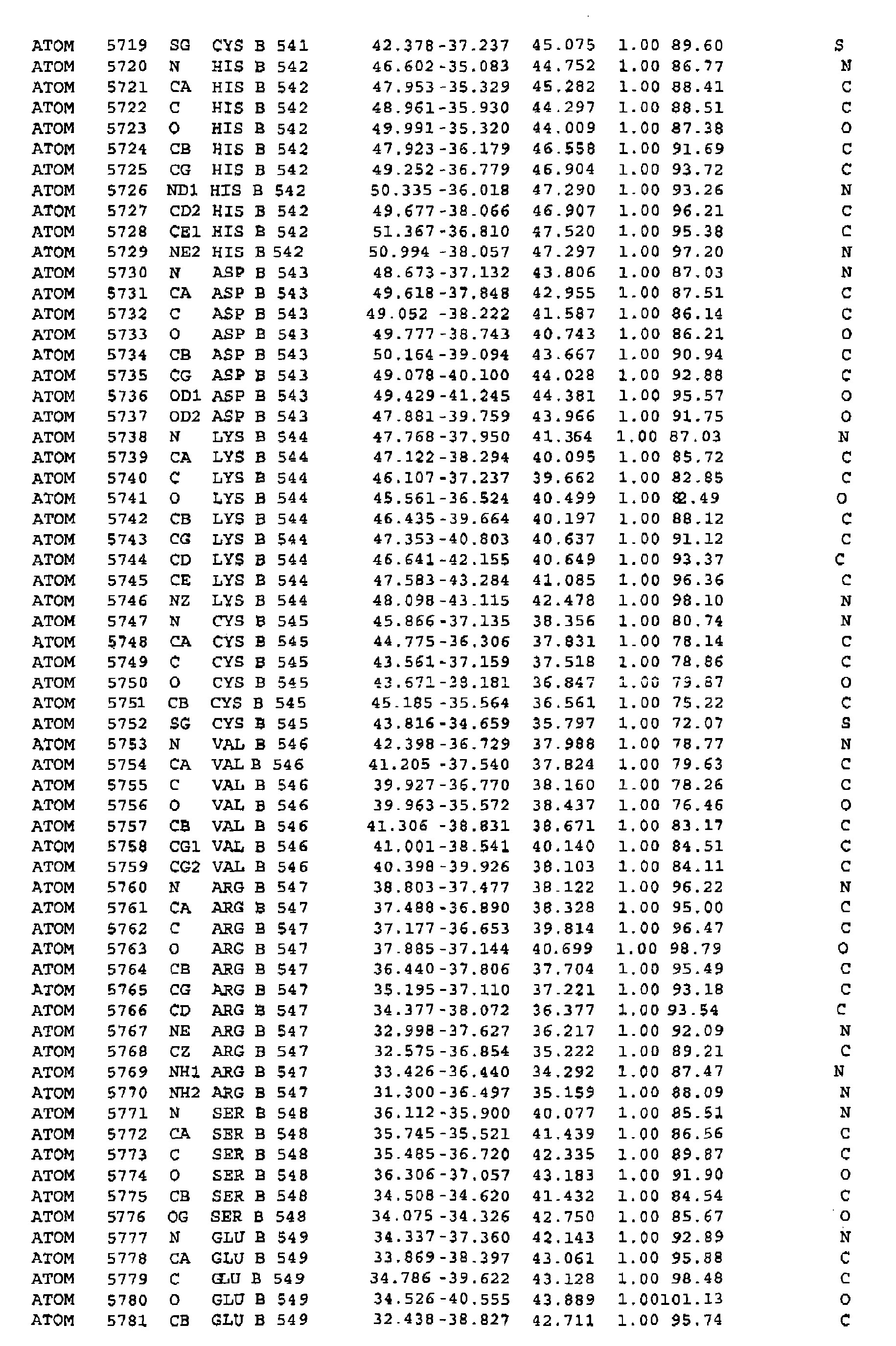

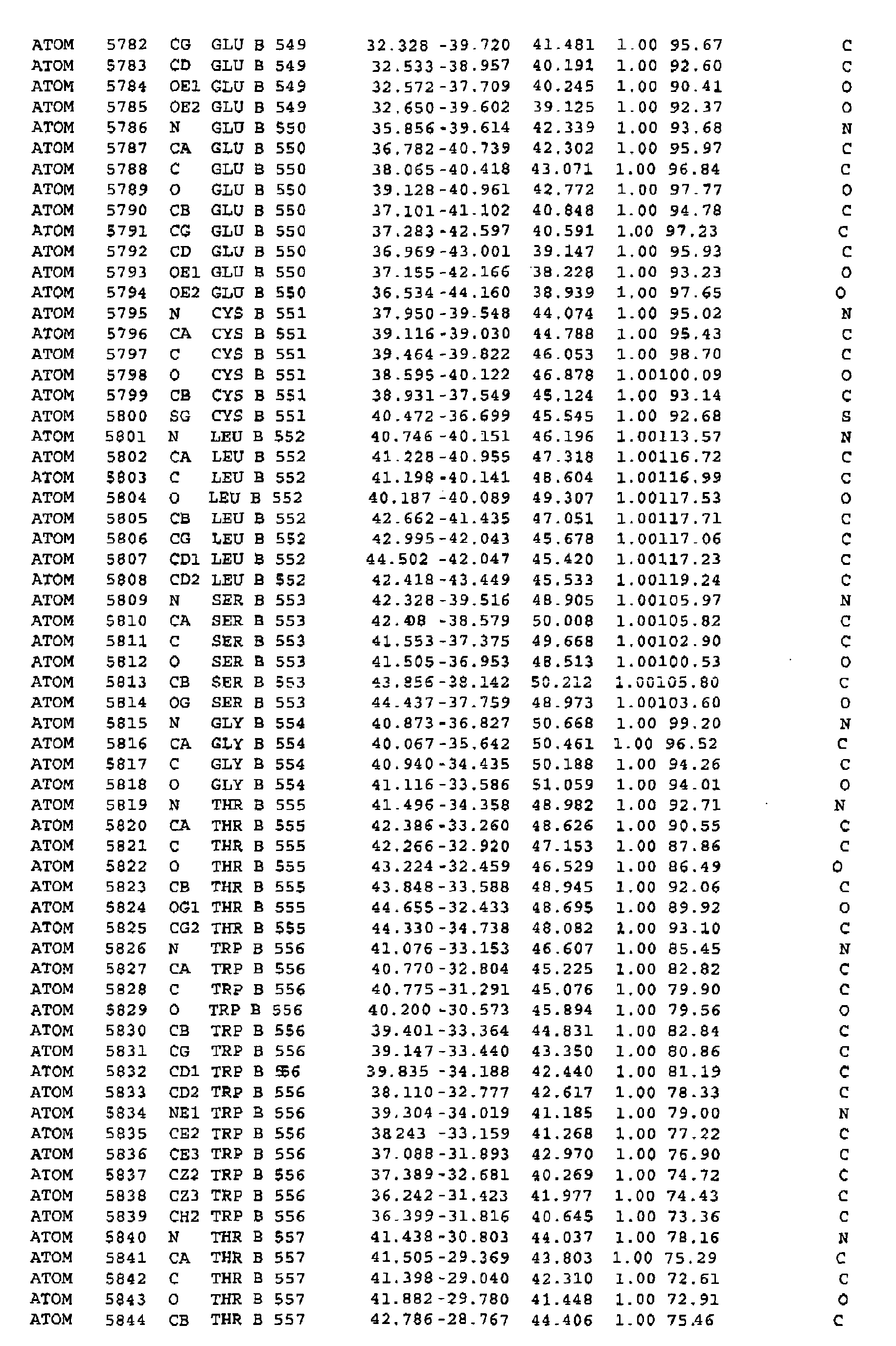

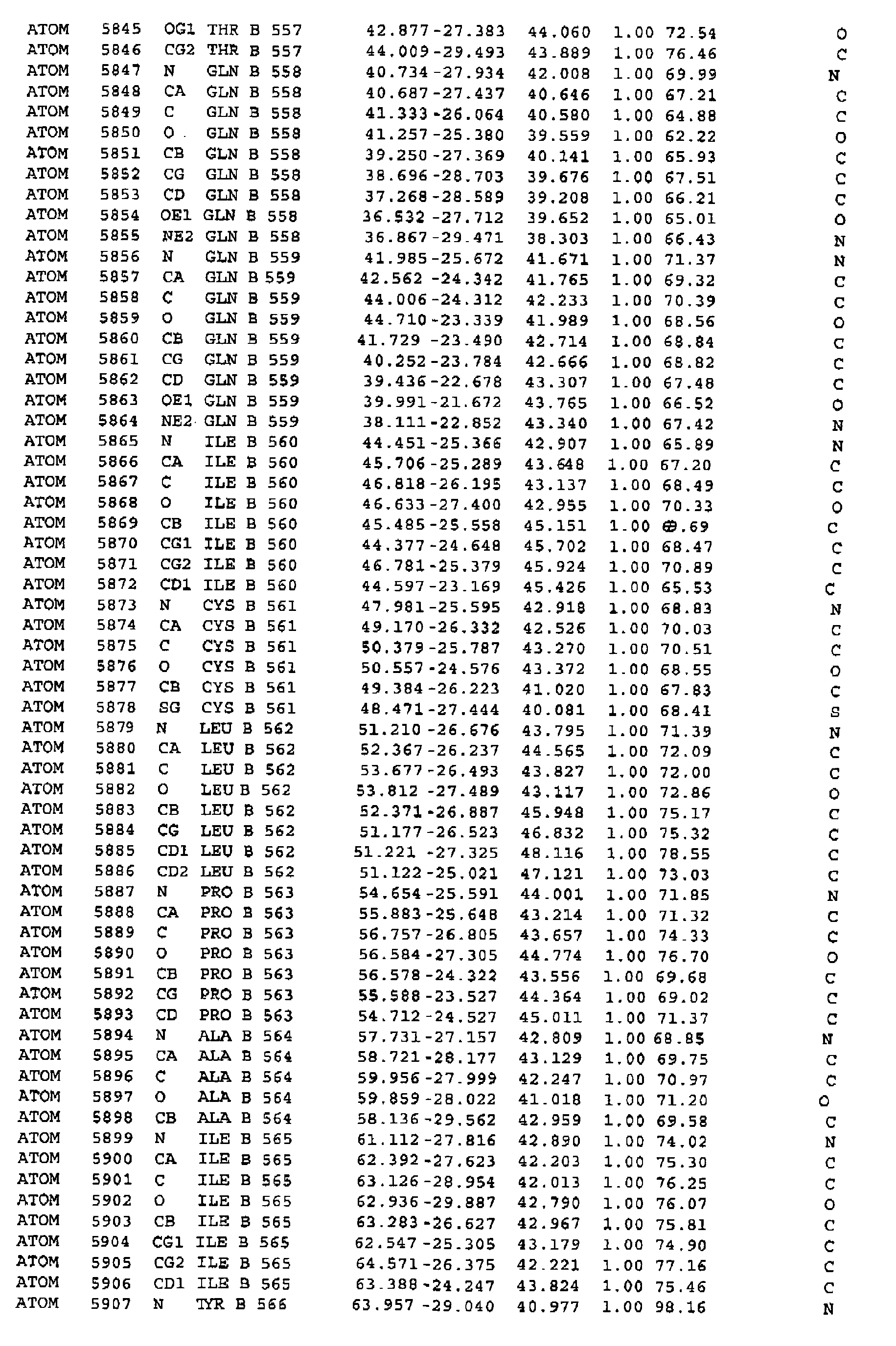

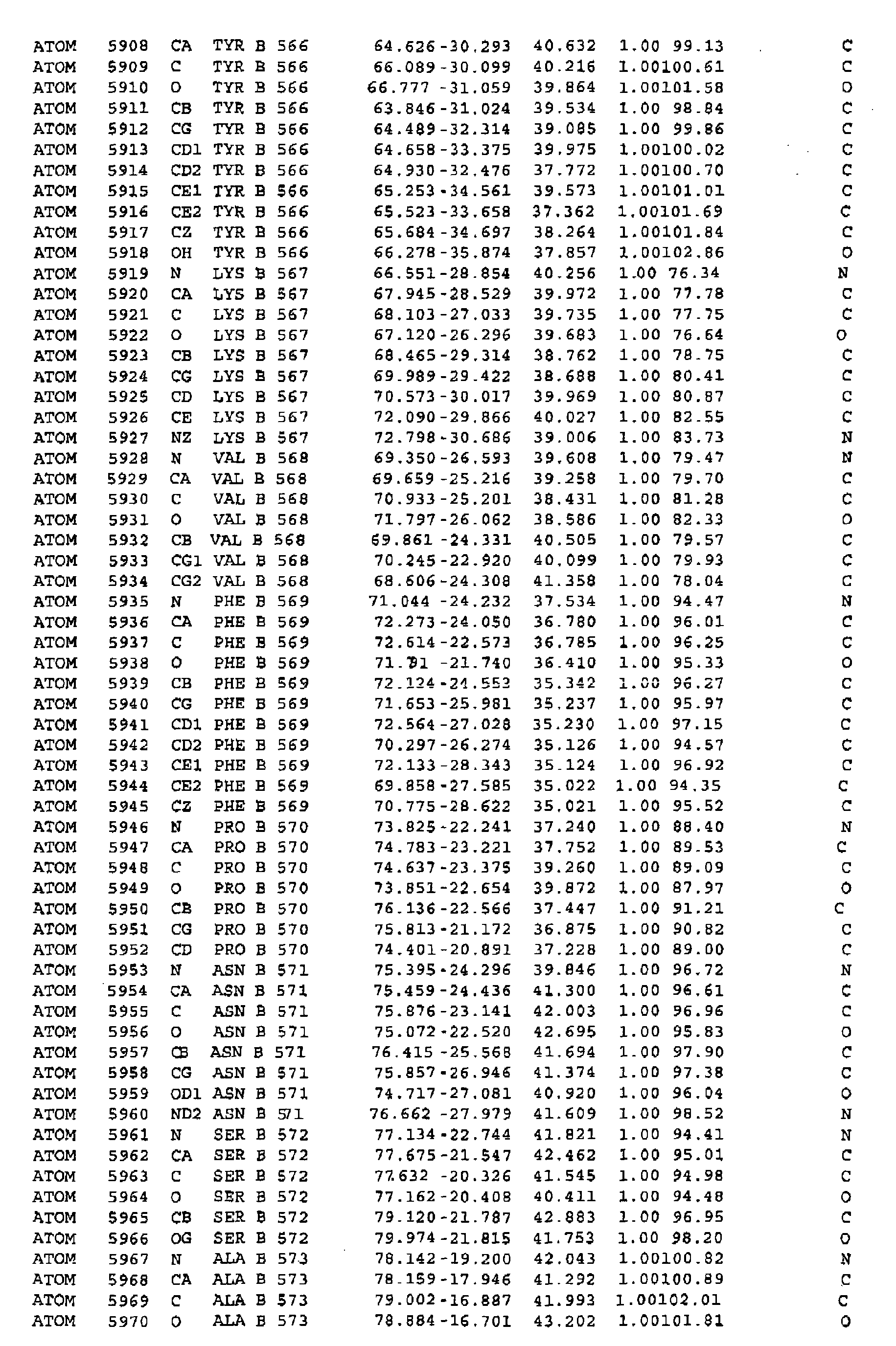

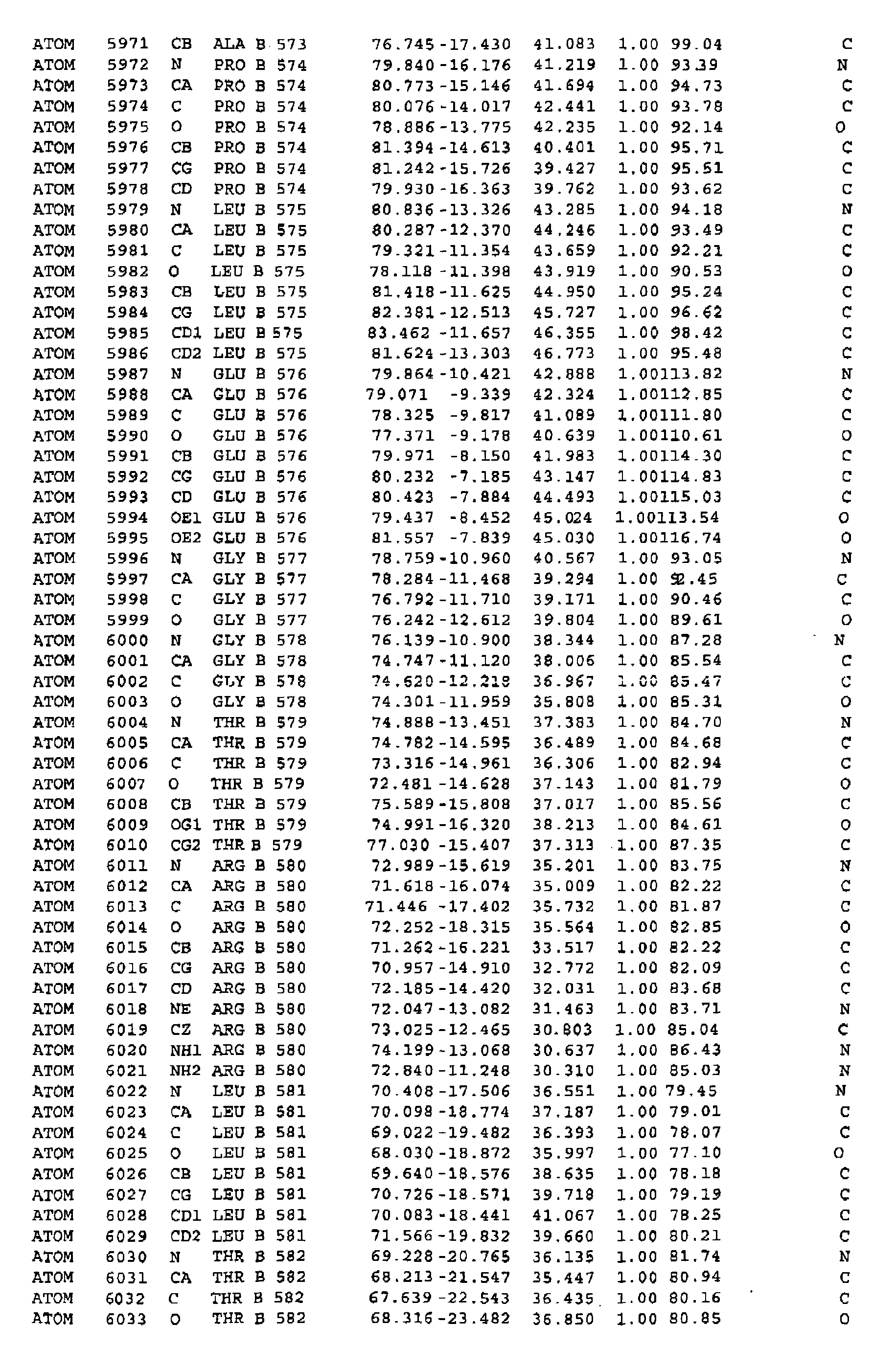

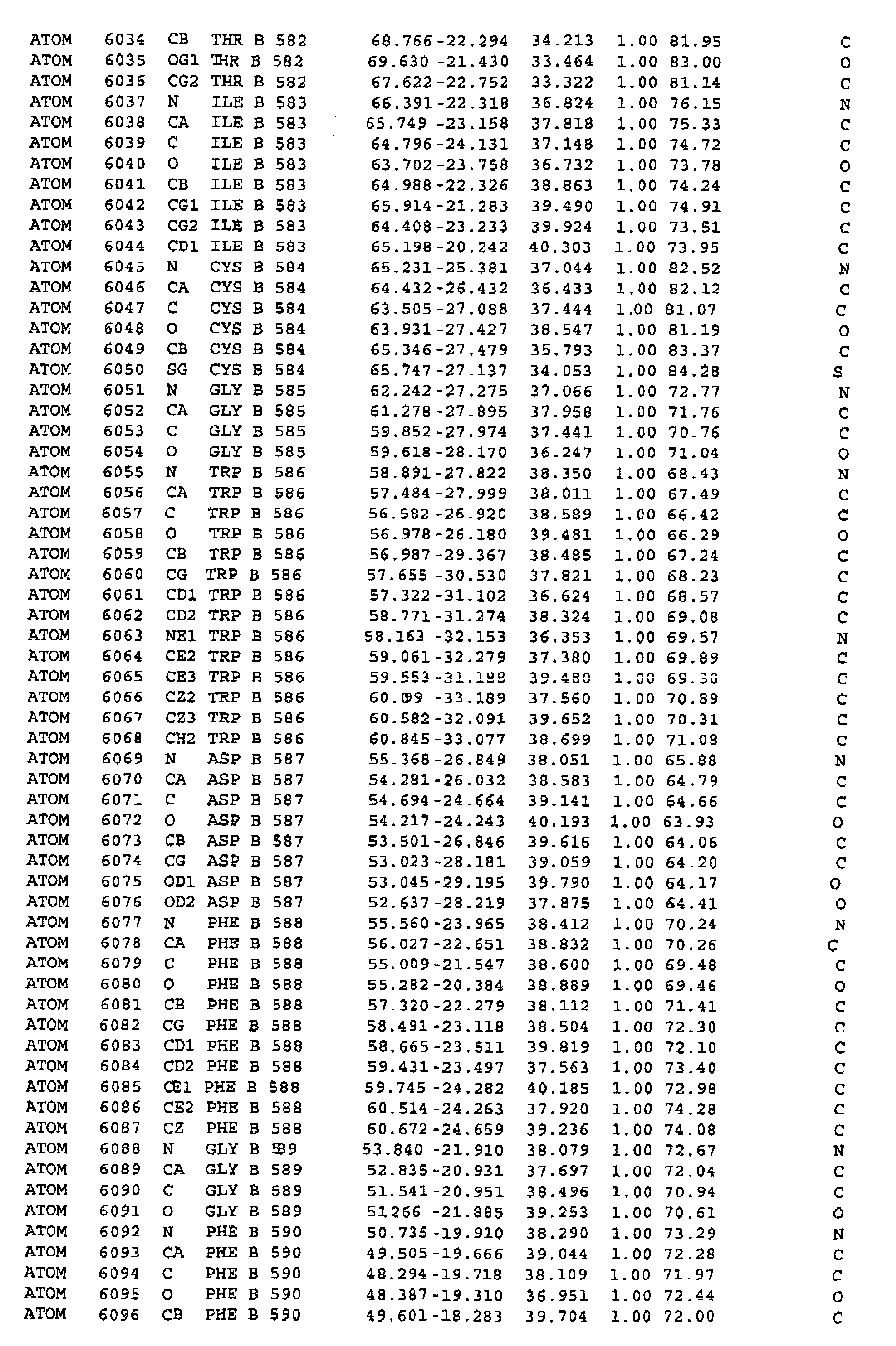

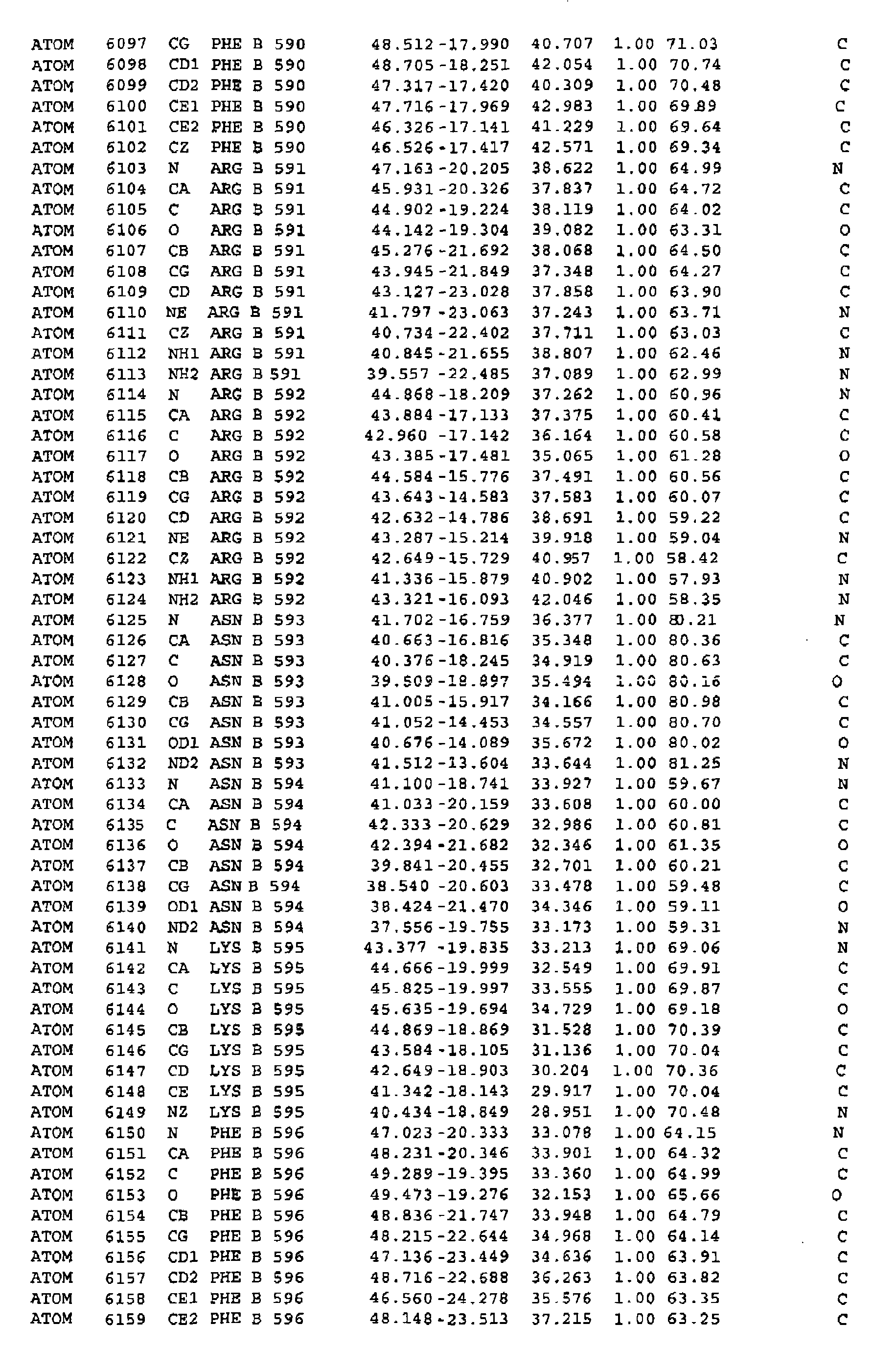

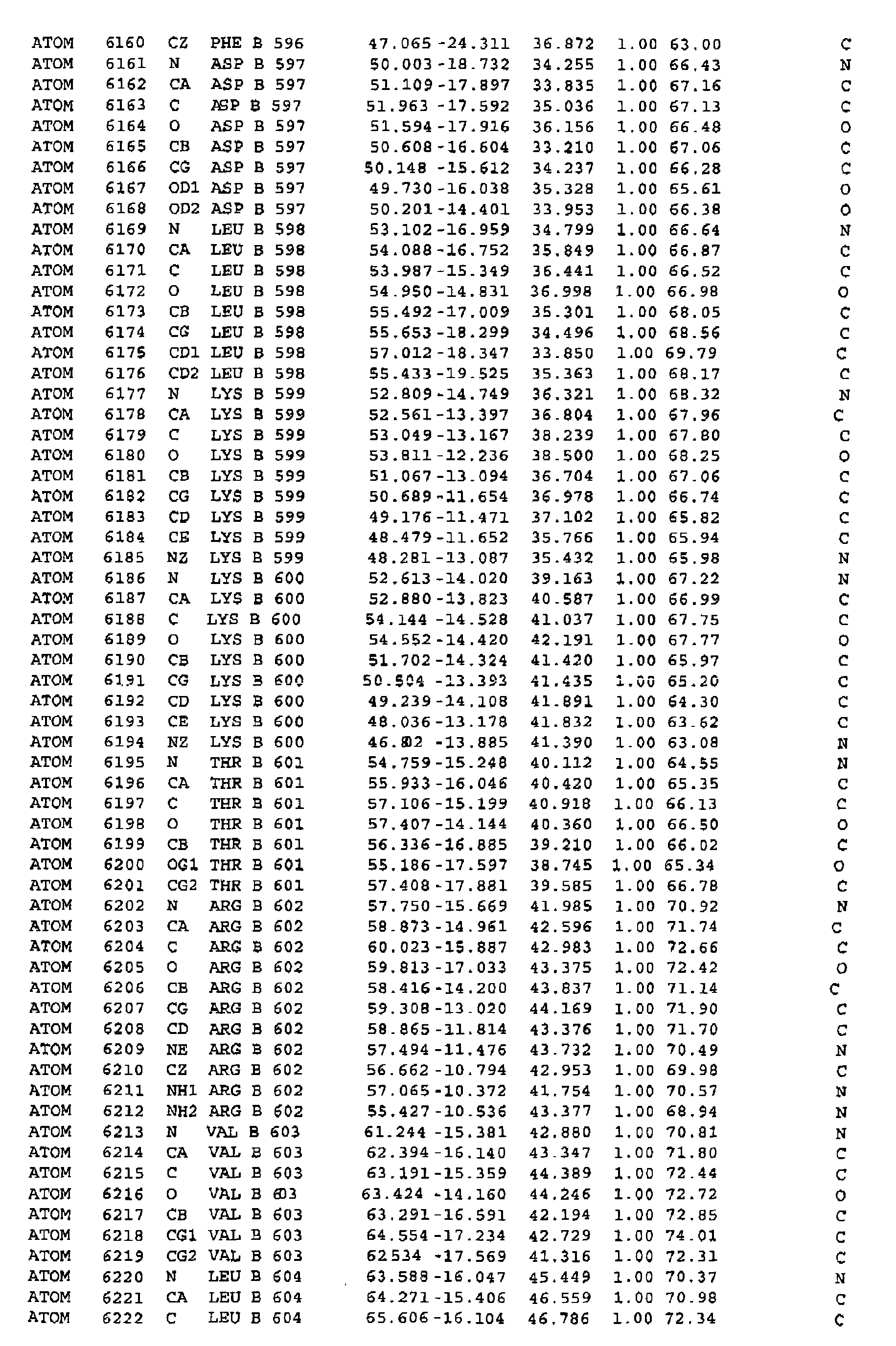

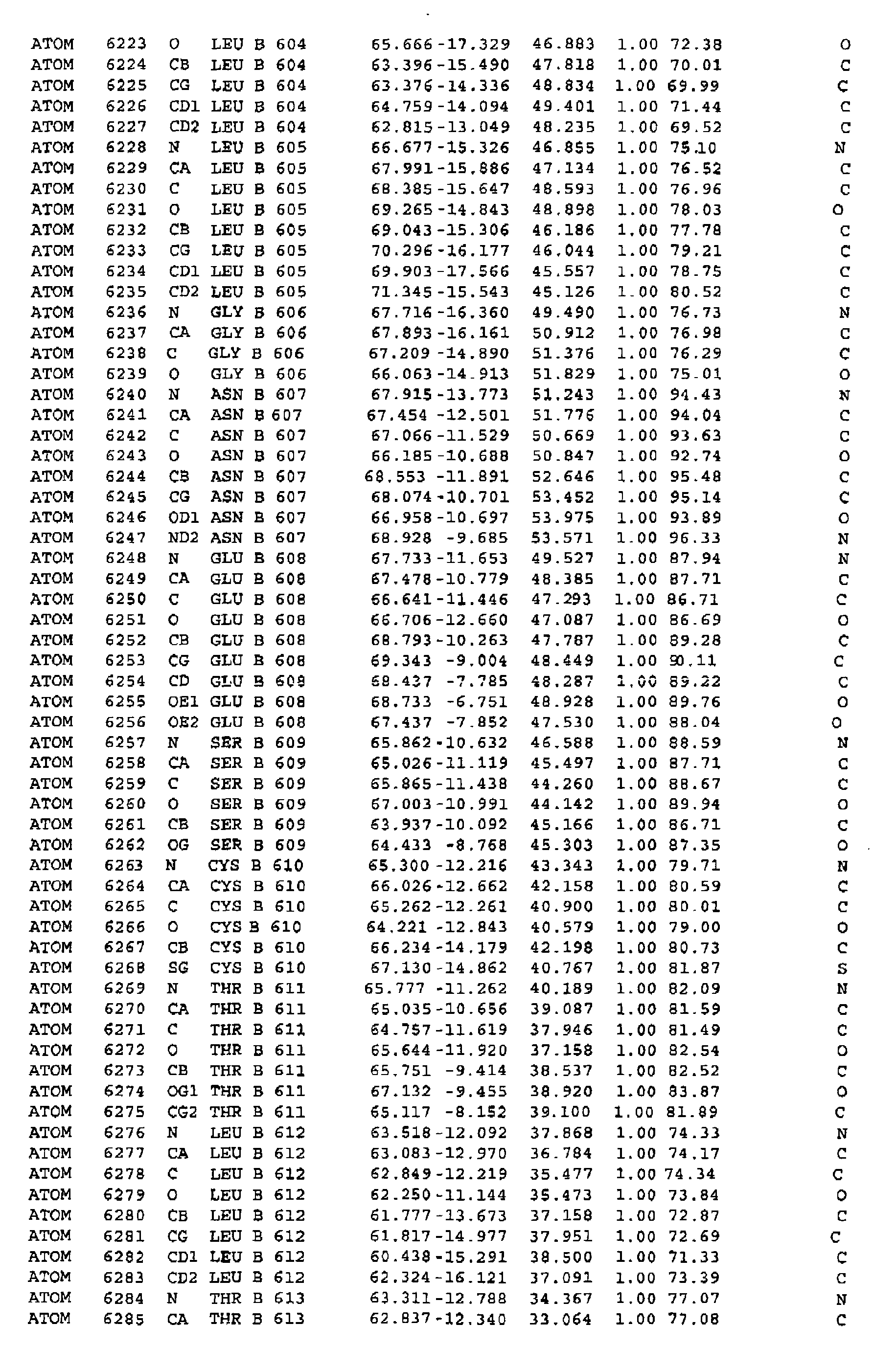

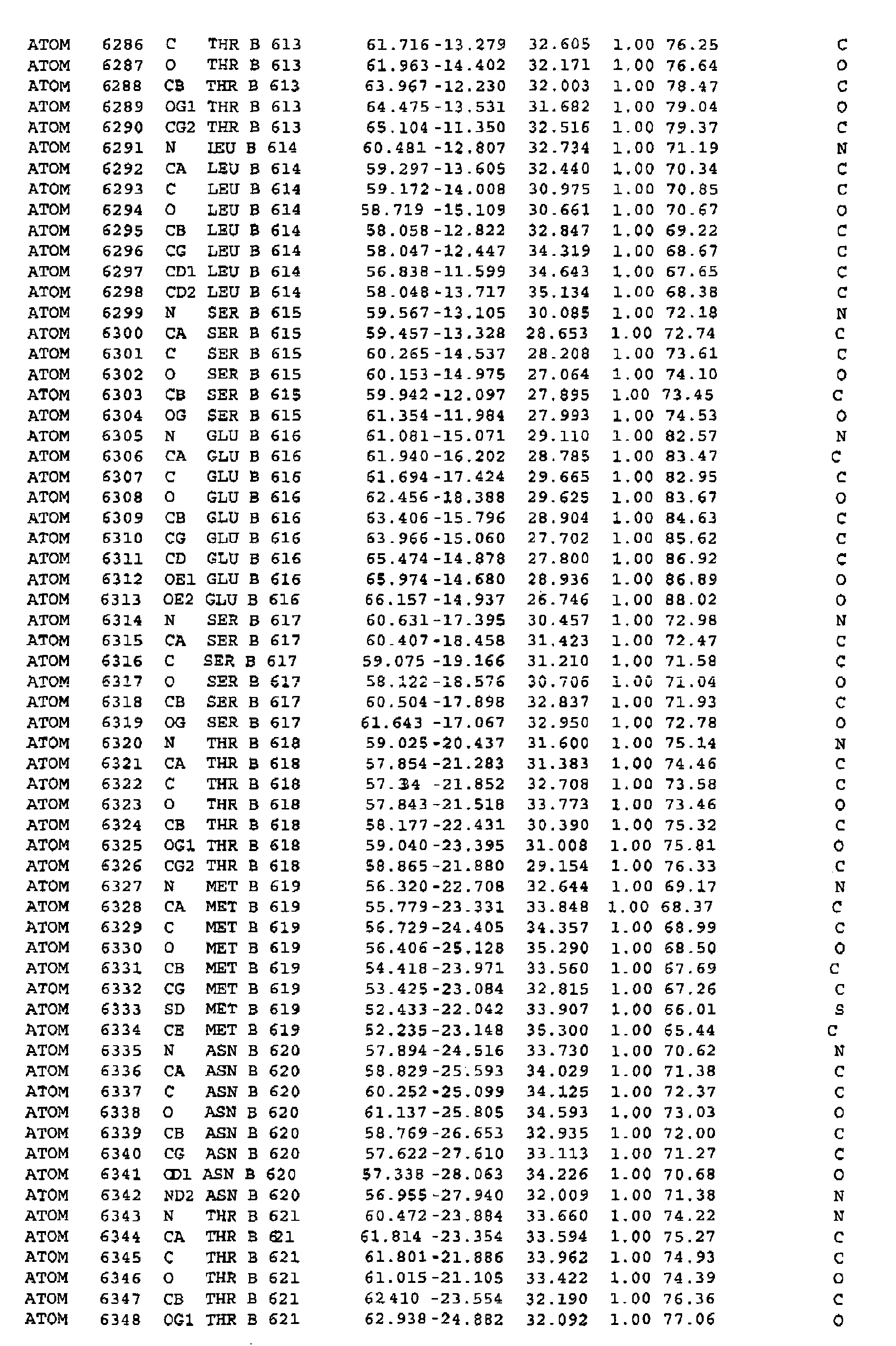

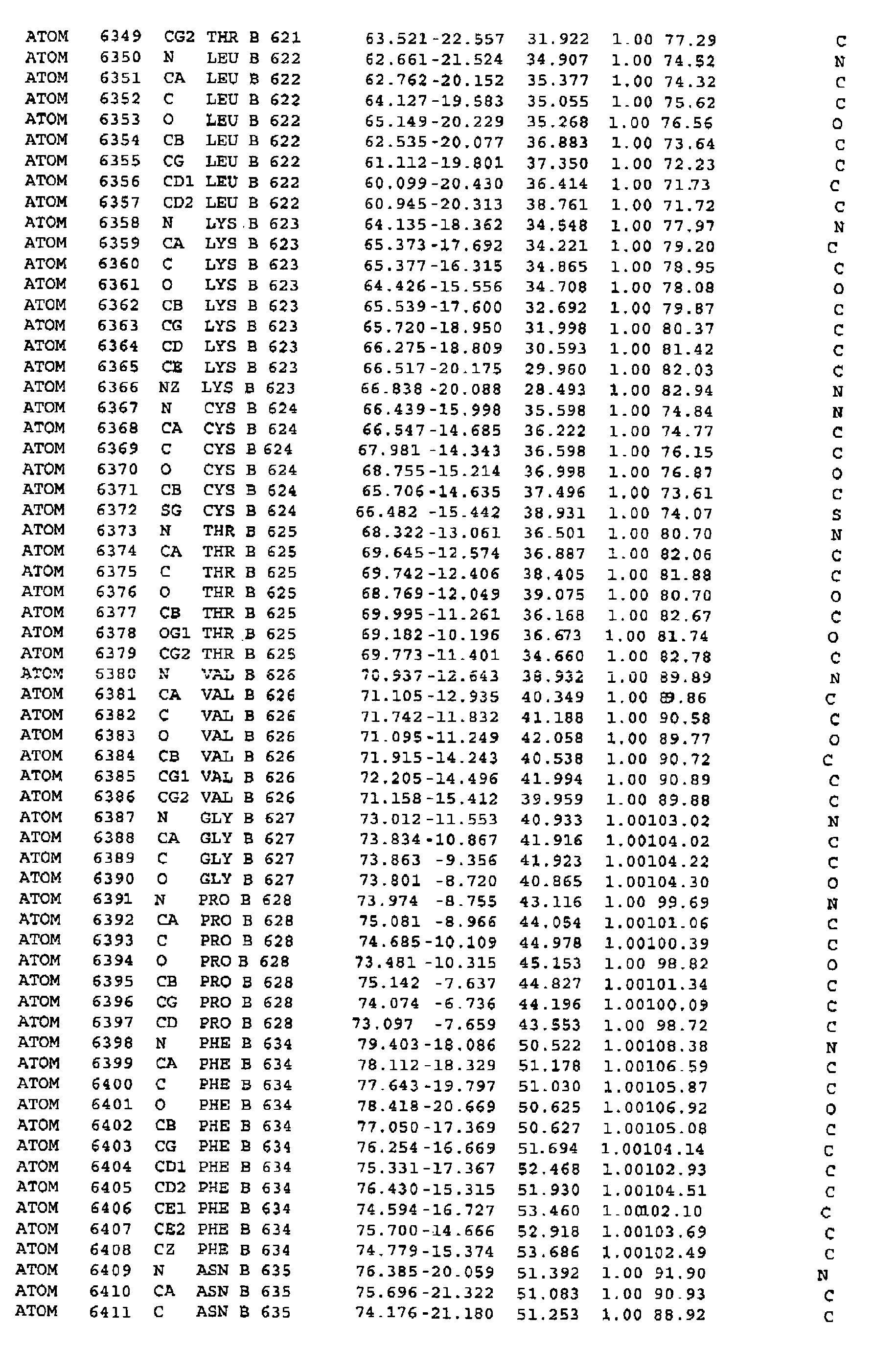

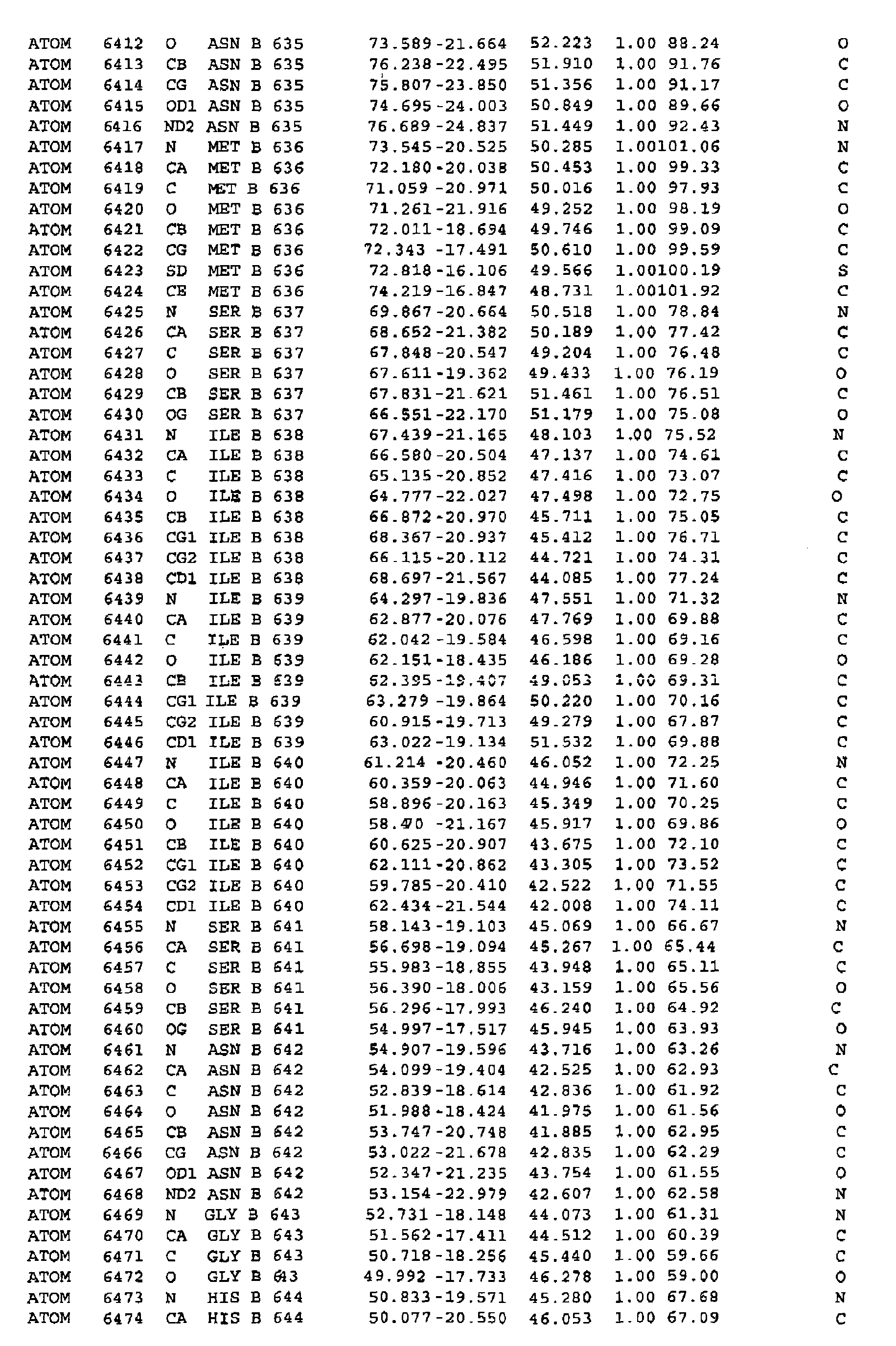

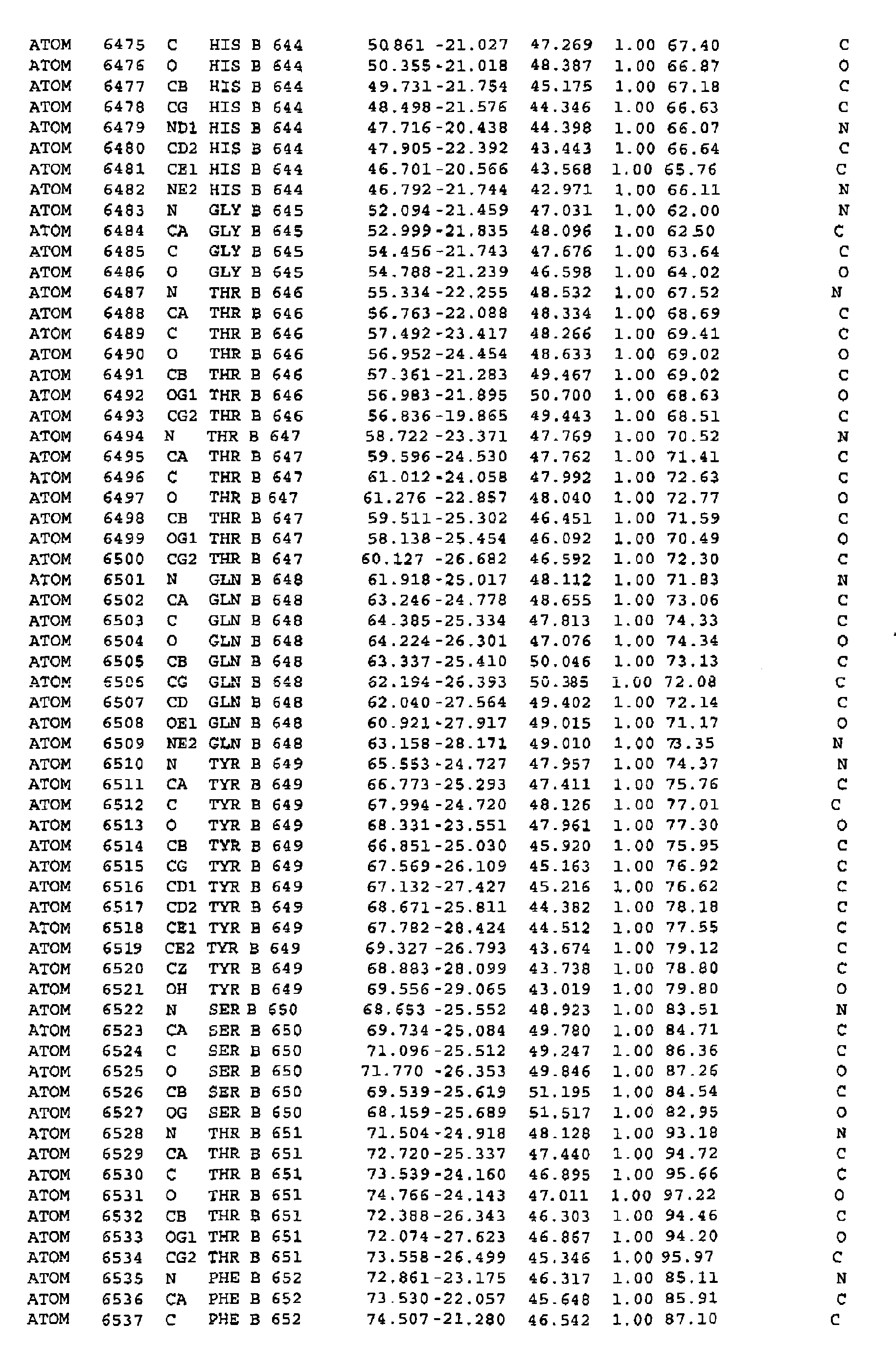

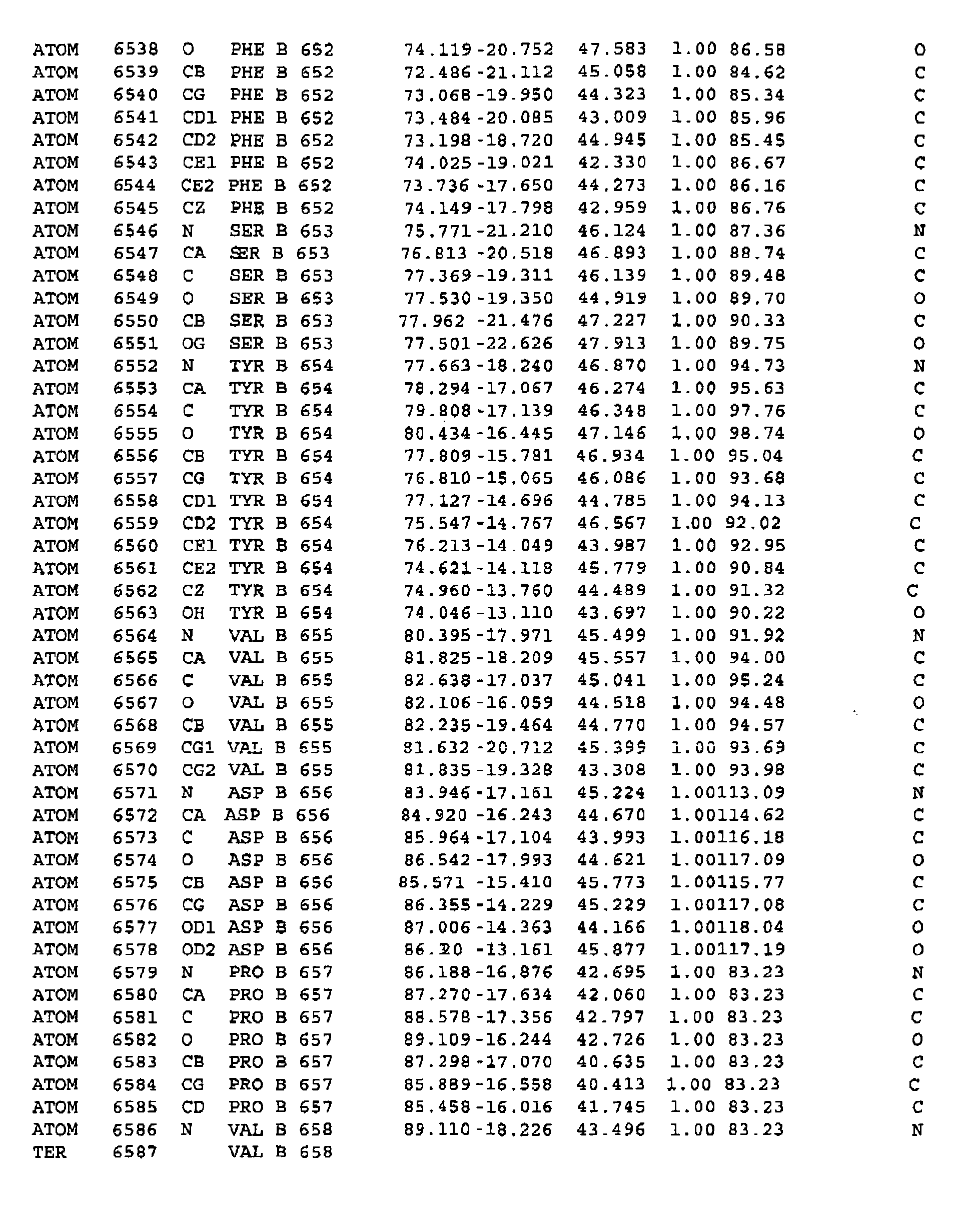

## Claims

1. Inhibitor of the Met-receptor wherein the binding site for said inhibitor comprises at least one or all amino acid (aa) residues corresponding to residues F590, R592, N593, D597, K599, K600, R602, L604, I639, S641, G643, H644, G645, T646 of Seq-ID No. 1.

2. Inhibitor according to claim 1 wherein said inhibitor does not bind to a binding site of the Met receptor comprising at least one or all of the amino acid residues corresponding to aa residues 124128, 148, 167, 190-192, 218, 220-224, 229, 230, 286, 414 of the Met-receptor of Seq-ID No. 1.

3. Inhibitor according to claim 1 or 2 wherein the inhibitor binding site comprises the Ig1 domain of the Met-receptor.

4. Inhibitor of the Met-receptor according to any one of the preceding claims wherein the binding site for said inhibitor comprises at least two or more of the amino acid residues 590-604, 639-646 of Seq-ID No. 1 or preferably all of the residues F590, R592, N593, D597, K599, K600, R602, L604, I639, S641, G643, H644, G645, T646 of Seq-ID No. 1,.

5. Inhibitor according to anyone of claims 1 to 4 wherein said inhibitor does not bind to the binding site of HGF / SF to the Sema domain of the Met receptor.

6. Inhibitor according to any one of the preceding claims wherein said inhibitor is a peptide able to bind covalently or non-covalently to at least one or all amino acid residues of F590, R592, N593, D597, K599, K600, R602, L604, I639, S641, G643, H644, G645, T646 and, optionally, R331, Q332, I333, G334, Y369, D372, R426, V427, M431, R469, S470 of the Met-receptor according to Seg-ID No. 1.

7. Inhibitor of a Met-receptor being a polypeptide having a LRR domain.

8. Inhibitor of a Met receptor being a compound comprising structural elements corresponding to at least two of the residues F104, W124, Y170 and Y214 of Seq. ID No. 2 and having a spatial configurartion of said aa residues as present in InIB as defined in table 1.

9. Inhibitor according to claim 8 having a spatial configuration of at least five or preferably all aa residues Q80, N84, F104, N106, W124, F126, D128, E129, E150, S168, Y170, N173, T190, E194, Q211, N212, Y214, D233 of Seq ID No. 2 as defined in table 1.

10. Inhibitor according to any one of claims 7 to 9 being an InIB fragment comprising amino acids 36 to 241 of Seq-ID No. 2 or a functional derivative thereof exhibiting binding to Met-receptor.

11. Inhibitor according to any one of claims 7 to 10 wherein the polypeptide
a) is a peptide of Seq-ID No. 3 or 4;
b) a polypeptide comprising an amino acid sequence of Seq-ID No. 3 or 4 and exhibiting i) bindhg to the Ig domain of the Met-receptor and ii) not activating said receptor thus inducing DNA synthesis in and/or scattering of cells;
c) a functional fragment or derivative of b) exhibiting i) binding to the Ig domain of the Met-receptor and ii) not activating said receptor thus inducing DNA synthesis in and/or scattering of cells;
d) a polypeptide having a 70% or more homology with an amino acid sequence of Seq-ID No. 3 or 4 or a functional fragment or derivative thereof as defined in c);
e) an altered polypeptide in which at least parts of the amino acids have been replaced by analogous parts of a different specificity, in particular human, while maintaining the ability to bind the Ig1 domain of the Met receptor, but not activating said receptor for including DNA synthesis in and/or scattering of cells.

12. Inhibitor according to anyone of claims 7 to 11, wherein said inhibitor has at least 6 β-strands having aromatic residues on its surface.

13. Use of a fragment of the Met-receptor comprising amino acids 590-604 and 639-646as a screening tool for an agent for treating or preventing cancer.

14. The use according to claim 13 wherein the fragment of the Met-receptor does not contain amino acids 124-128, 148, 167, 190-192, 218, 220-224, 229, 230, 286, 414 of Seq-ID No. 1.

15. The use according to claim 13 or 14 wherein said agent for treating or preventing cancer exhibits an activity of binding to said Me-receptor fragment but exhibits no activity of activating the Met-receptor for inducing DNA synthesis in and/or scattering of cells.

16. Use according to any one of claims 13 to 15, wherein said fragment of the Met-receptor is a peptide according to Seq-ID No. 5 or a functional derivative thereof able to bind the InIB241 fragment according to See ID No. 4.

17. Use of a cell which is transfected with an expressing vector comprising a polynucleotide encoding a polypeptide as defined in claim 13 to 16 as a screening tool for an agent for treating or preventing cancer.

18. A method for screening an agent for preventing or treating cancer comprising the step of contacting a Met-receptor molecule as defined in anyone of claims 1 to 6 with a compound to be tested and determining binding of said compound to be tested to said Met-receptor and contacting another peptide not containing the residues F590, R592, N593, D597, K599, K600, R602, L604, I639, S641, G643, H644, G645, T646 with the compound to be tested and determining binding of said compound to be tested.

19. The method according to claim 19 wherein the Met receptor molecule does not contain at least one or all of aa residues 124128, 148, 167, 190-192, 218, 220-224, 229, 230, 286, 414 according to Seq-ID No. comprising the binding site of the HGF / SF molecule to the Metreceptor.

20. A method for identifying a potential Met-receptor inhibitor compound for the Met-receptor which does not activate said Met-receptor, said method comprising the steps of a) using a three dimensional structure of the Met-receptor as defined by atomic coordinates according to table 1, b) employing said three dimensional structure to design or select said potential inhibitor such that said potential inhibitor is capable of binding to at least one amino acid to the InIB 321 binding site of Ig1 domain of the Met-receptor c) synthesising said potential inhibitor d) in an essay, contacting said potential inhibitor with said Met-receptor and e) determining the inhibitory activity of said potential inhibitor.

21. The method according to claim 20 wherein the potential Metreceptor inhibitor is designed or selected using computer modelling.

22. The method according to claim 20 or 21, wherein the potential Met-receptor inhibitor is designed de novo.

23. The method according to claim 20 to 22 wherein the potential Metreceptor inhibitor is designed based on a known inhibitor.

24. A method of using a crystal of the Met-receptor for screening for a novel drug wherein said crystal effectively diffracts Xrays for the determination of the atomic coordinates of said Met-receptor of greater than 4 Å and wherein the structure of said Met-receptor comprises aa residues 590-604 and 639 to 646 of Seq-ID No. 1 and wherein said structure of said Met receptor comprises a root mean square deviation of less than or equal to 1.0 Å in the positions of Calpha atoms as defined by the atomic coordinates of Met-receptor according to table 1 and wherein said method comprises a) selecting a potential ligand by performing rational drug design with a three dimensional structure determined for the crystal; b) in an essay, contacting the potential ligand with the ligand binding domain of the enzyme; and c) detecting the binding potential of the potential ligand for the ligand binding domain, wherein the potential ligand is selected as a novel drug based on the potential binding activity.

25. A crystalline form of the Met-receptor molecule, which has a crystal structure for which the structural coordinates of the back bone nitrogen, α-carbon and carbonyl carbon atoms of said Metreceptor molecule having a root mean square deviation from the structural coordinates of the equivalent back bone atoms of the Met-receptor as defined in table 1 of less than 1.0 Å Ǻ following structural alignment of equivalent back bone atoms, and wherein said Met-receptor protein consists of the Ig1, PSI and Sema domains, said domains folds up as a seven-bladed β-propeller for the Sema domain and an immunoglobulin-like domain for the Ig1 domain connected via the PSI domain, said Metreceptor molecule has an overall amino acid sequence that is at least 70% identical to the amino acid sequence of human Met-receptor set forth in Seq-ID No. 1.

26. A method for selecting, testing and/or regionally or semi-regionally designing a chemical compound which bind covalently or non-covalently to Met-receptors, **characterised by** applying in a computational analysis structure coordinates of a crystalline form according to claim 25.

27. A method for identifying a potential inhibitor of Metreceptor molecules comprising the following steps: a) using the atomic coordinates of a crystalline form according to claim 25 to define the inhibitor binding sites of said receptor; b) identifying a compound that fits the inhibitor binding site c) obtaining the compound, and d) contacting the compound with the Met receptor molecule determining the binding properties and/or effects of said compounds on and/or the inhibition of the Metreceptor molecule by said compound.

28. A method for selecting, testing and/or regionally or semi-regionally designing a modified Met-receptor molecule, **characterised by** applying in a computational analysis structure coordinates of a crystalline form according to claim 25.
